# EUROPEAN PATENT APPLICATION

(11) **EP 4 799 647 A1**
(43) Date of publication of application: **02.09.2026**
(21) Application number: 24881598.7
(22) Date of filing: 22.10.2024
(51) Int. Cl.: A61K 47/68, A61K 31/4745, C07D 491/22, A61P 35/00

(54) **CAMPTOTHECIN DRUG CONJUGATE, AND PREPARATION METHOD THEREFOR AND USE THEREOF**

(30) Priority: 23.10.2023 CN 202311382346; 26.01.2024 CN 202410118218; 30.05.2024 CN 202410693604; 23.08.2024 CN 202411176314; 12.10.2024 CN 202411427303
(71) Applicant: Shanghai Qilu Pharmaceutical Research and Development Centre Ltd., Shanghai 201203 (CN)
(72) Inventor: WEI, Wei, Shanghai 201203 (CN); BAI, Yuanchao, Shanghai 201203 (CN); WANG, Wei, Shanghai 201203 (CN); HUANG, Guoyin, Shanghai 201203 (CN); AI, Xianwei, Shanghai 201203 (CN); WANG, Xinmei, Shanghai 201203 (CN); YANG, Yang, Shanghai 201203 (CN); TAO, Weikang, Shanghai 201203 (CN)
(74) Representative: dompatent
(86) International application number: PCT/CN2024/126446
(87) International publication number: WO 2025/087233

(57) **Abstract**

A camptothecin conjugate compound and a preparation method therefor. The camptothecin compound has an excellent pharmacological activity and kinetic characteristics, and is suitable for preparing a drug for treating tumor-related diseases.

## Description

The present application claims priority to the Chinese patent application with the application number 202311382346.8, titled "camptothecin drug conjugate, and preparation method therefor and use thereof" and filed to the China National Intellectual Property Administration on October 23, 2023, the Chinese patent application with the application number 202410118218.0 titled "camptothecin drug conjugate, and preparation method therefor and use thereof" and filed to the China National Intellectual Property Administration on January 26, 2024, the Chinese patent application with the application number 202410693604.2 titled "camptothecin drug conjugate, and preparation method therefor and use thereof" and filed to the China National Intellectual Property Administration on May 30, 2024, the Chinese patent application with the application number 202411176314.7 titled "camptothecin drug conjugate, and preparation method therefor and use thereof" and filed to the China National Intellectual Property Administration on August 23, 2024, and the Chinese patent application with the application number 202411427303.1 titled "camptothecin drug conjugate, and preparation method therefor and use thereof" and filed to the China National Intellectual Property Administration on October 12, 2024, the entire contents of which are incorporated by reference in the present application.

### TECHNICAL FIELD

The present invention belongs to the technical field of medicinal chemistry, and particularly relates to a camptothecin conjugate compound and a preparation method therefor. The camptothecin compound has excellent pharmacological activity and kinetic properties, and is suitable for preparing a drug for treating tumor-related diseases.

### BACKGROUND

Camptothecin (CPT), a pyrroloquinoline cytotoxic alkaloid, is one of the most extensively studied natural anti-tumor drugs besides paclitaxel, and mainly found in the fruits or root barks of Camptotheca acuminata, a Nyssaceae plant native to China. In 1985, Hsiang et al. found a mechanism that the camptothecin and derivatives thereof exert anti-cancer effects by targeting Topoisomerase I (Topo I) and inhibiting DNA synthesis, and then the camptothecin raised people's extensive attention again, leading to the development of many derivatives, and subsequently became a new research focus in the anti-cancer field. Since then, a new generation of camptothecin drugs such as 10-hydroxycamptothecin (HCPT), irinotecan, topotecan, SN-38 and belotecan have been successively approved for marketing for treating a colorectal cancer, a small cell lung cancer, an ovarian cancer and other tumor diseases, and the research on indications and dosage forms of the drugs has also achieved gratifying results.

With the development of drug delivery system and antibody drug conjugate (ADC) technology, a series of camptothecin drugs which were previously undruggable or had significant side effects have been applied again. An ADC is formed by linking a monoclonal antibody targeting a specific antigen to a small molecular cytotoxic drug by a linker, which has both the powerful killing effect of traditional small molecular chemotherapy drugs and the tumor-targeting property of antibody drugs. The ADC technology can effectively overcome the side effects caused by poor water solubility and insufficient tissue distribution of the camptothecin drug, and a strong targeting ability of an antibody part of the ADC enables the carried camptothecin drug to be effectively delivered to a target tissue, thereby improving therapeutic effects of such compounds.

### SUMMARY

In a first aspect, the present invention provides an antibody drug conjugate as shown in formula I or a pharmaceutically acceptable salt thereof:

**Ab-[-L₁-L₂-L₃-L₄-D]_{q}** **formula I**

wherein, Ab is an antibody part or an antigen-binding fragment; and D is a drug molecule, -L₁-L₂-L₃-L₄- is a fragment linking the antibody Ab to the drug molecule D, and q is selected from 2-9;
L₁ is a linker linked to the antibody, which has a structure of -La-Lb-; wherein La is selected from the following structures: 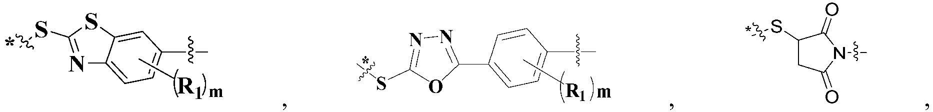 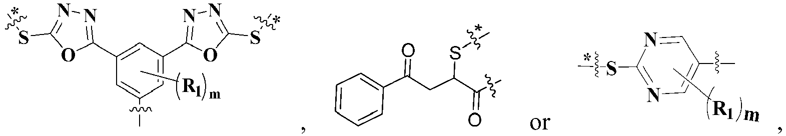 wherein R₁ is respectively independently selected from H, halogen atom, -CN, -OH, -NH₂, C₁₋₄ alkyl, C₁₋₄ haloalkyl, C₁₋₄ alkoxy, C₁₋₄ haloalkoxy or hydroxy-C₁₋₄ alkyl, and m is selected from 0, 1, 2 or 3; and the terminus of La marked with an asterisk * represents that the position is linked to the antibody Ab; and Lb is selected from 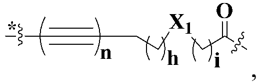 or Lb is a chemical bond, wherein n is selected from 0 or 1; h and i are respectively independently selected from 0, 1, 2, 3, 4 or 5, X₁ is selected from CH₂, N-Ra or O, and Ra is selected from H, C₁₋₄ alkyl, C₁₋₄ haloalkyl or C₁₋₄ alkyl-carbonyl; and the terminus of Lb marked with an asterisk * represents that the position is linked to La;
L₂ is a chemical bond, or L₂ is selected from 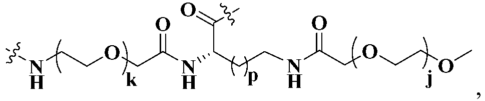 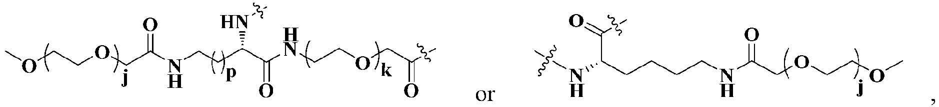 wherein k is selected from 0-8, j is selected from 0-20 or j is selected from 0-10, and p is selected from 0, 1, 2, 3 or 4; and the -NH- terminus of L₂ is linked to L₁;
L₃ is selected from a peptide group consisting of 2-10 amino acid residues; and the amino acids are selected from natural amino acids or unnatural amino acids; and
L₄ is a chemical bond, or L₄ is selected from the following groups: -NH-CH₂- or 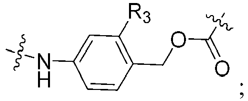 wherein, R₃ is selected from 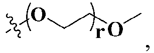 or R₃ is H, and r is selected from 0-10; and the -NH- terminus of L₄ is linked to L₃.

In one technical solution of formula I in the present invention, Ab is the antibody part or the antigen-binding fragment; and D is the drug molecule, -L₁-L₂-L₃-L₄- is the fragment linking the antibody Ab to the drug molecule D, and q is selected from 2-9; and L₁ is the linker linked to the antibody, which has the structure of -La-Lb-; wherein La is selected from the following structures: 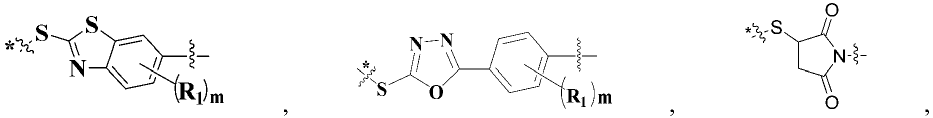 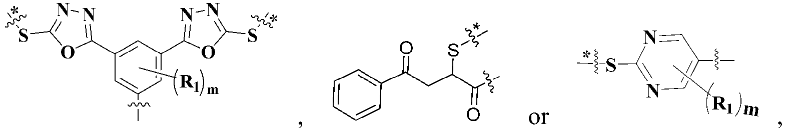 wherein R₁ is respectively independently selected from H, halogen atom, -CN, -OH, -NH₂, C₁₋₄ alkyl, C₁₋₄ haloalkyl, C₁₋₄ alkoxy, C₁₋₄ haloalkoxy or hydroxy-C₁₋₄ alkyl, and m is selected from 0, 1, 2 or 3; and the terminus of La marked with the asterisk * represents that the position is linked to the antibody Ab; and Lb is selected from 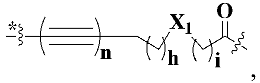 or Lb is the chemical bond, wherein n is selected from 0 or 1; h and i are respectively independently selected from 0, 1, 2, 3, 4 or 5, X₁ is selected from CH₂, N-Ra or O, and Ra is selected from H, C₁₋₄ alkyl, C₁₋₄ haloalkyl or C₁₋₄ alkyl-carbonyl; and the terminus of Lb marked with the asterisk * represents that the position is linked to La; L₂ is the chemical bond, or L₂ is selected from 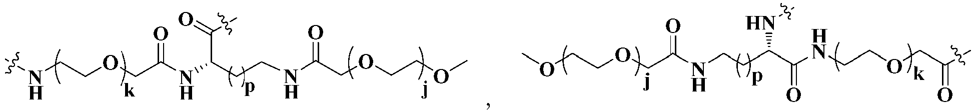 or 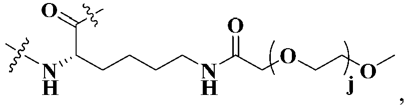 wherein k is selected from 0-8, j is selected from 0-10, and p is selected from 0, 1, 2, 3 or 4; and the -NH- terminus of L₂ is linked to L₁; L₃ is selected from the peptide group consisting of 2-10 amino acid residues; and the amino acids are selected from the natural amino acids or the unnatural amino acids; and L₄ is the chemical bond, or L₄ is 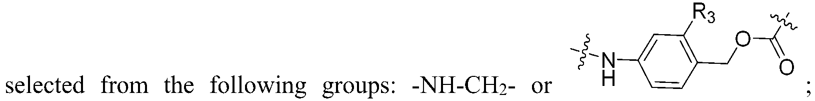 wherein, R₃ is selected from 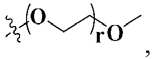 or R₃ is H, and r is selected from 0-10; and the -NH- terminus of L₄ is linked to L₃.

In one technical solution of formula I in the present invention, Ab is the antibody part; and D is the drug molecule, -L₁-L₂-L₃-L₄- is the fragment linking the antibody Ab to the drug molecule D, and q is selected from 2-6; and L₁ is the linker linked to the antibody, which has the structure of -La-Lb-; wherein La is selected from the following structures: 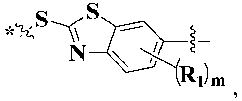 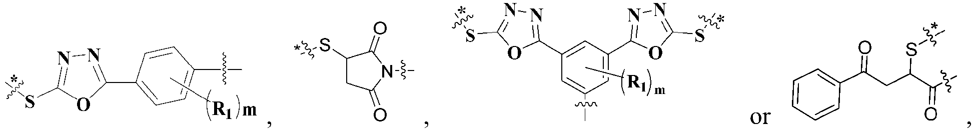 wherein R₁ is respectively independently selected from H, halogen atom, -CN, -OH, -NH₂, C₁₋₄ alkyl, C₁₋₄ haloalkyl, C₁₋₄ alkoxy, C₁₋₄ haloalkoxy or hydroxy-C₁₋₄ alkyl, and m is selected from 0, 1, 2 or 3; and the terminus of La marked with the asterisk * represents that the position is linked to the antibody Ab; and Lb is selected from 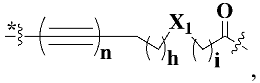 or Lb is the chemical bond, wherein n is selected from 0 or 1; h and i are respectively independently selected from 0, 1, 2, 3, 4 or 5, X₁ is selected from CH₂, N-Ra or O, and Ra is selected from H, C₁₋₄ alkyl, C₁₋₄ haloalkyl or C₁₋₄ alkyl-carbonyl; and the terminus of Lb marked with the asterisk * represents that the position is linked to La; L₂ is the chemical bond, or L₂ is selected from 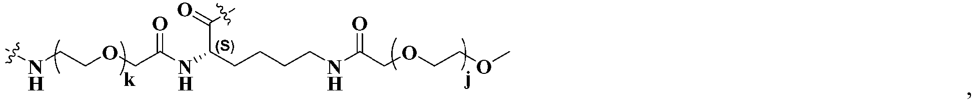 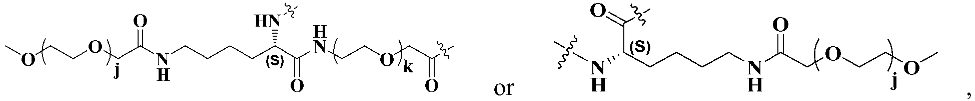 wherein k is selected from 0-8, and j is selected from 0-10; and the -NH- terminus of L₂ is linked to L₁; L₃ is selected from the peptide group consisting of 2-10 amino acid residues; and the amino acid is selected from the natural amino acid or the unnatural amino acid; and L₄ is the chemical bond, or L₄ is selected from the following groups: -NH-CH₂- or 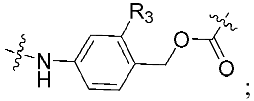 wherein, R₃ is selected from 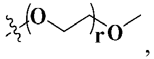 or R₃ is H, and r is selected from 0-10; and the -NH-terminus of L₄ is linked to L₃.

In any technical solution of formula I in the present invention, q is selected from 2-6, preferably 3-5, and more preferably 3.5-4.5.

In any technical solution of formula I in the present invention, q is selected from 7-9, preferably 7.5-8.5, and more preferably 7.5-8.0.

In any technical solution of formula I in the present invention, R₁ is selected from H.

In any technical solution of formula I in the present invention, n is selected from 1.

In any technical solution of formula I in the present invention, h and i are respectively independently selected from 0, 1 or 2.

In any technical solution of formula I in the present invention, h is 0, and i is 1.

In any technical solution of formula I in the present invention, X₁ is selected from CH₂.

In any technical solution of formula I in the present invention, X ₁ is selected from O.

In any technical solution of formula I in the present invention, X ₁ is selected from N-Ra.

In any technical solution of formula I in the present invention, Ra is selected from methyl, ethyl or isopropyl.

In any technical solution of formula I in the present invention, Ra is selected from methyl.

In any technical solution of formula I in the present invention, k is selected from 2-6, and preferably, k is selected from 4.

In any technical solution of formula I in the present invention, j is selected from 4-18, or j is selected from 6-16, or j is selected from 7-15.

In any technical solution of formula I in the present invention, j is selected from 7, 11 or 15.

In any technical solution of formula I in the present invention, j is selected from 15, or j is selected from 11.

In any technical solution of formula I in the present invention, j is selected from 7, or j is selected from 8.

In any technical solution of formula I in the present invention, L₂ is the chemical bond or L ₂ is selected from 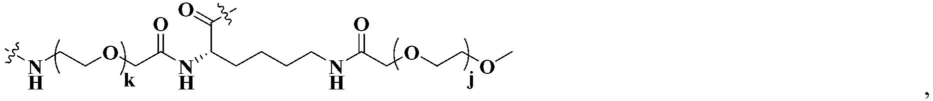 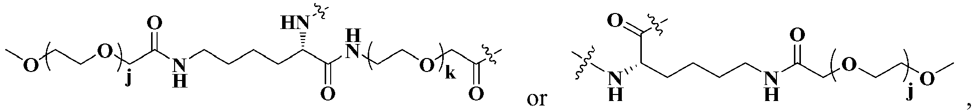 wherein k is selected from 0-8, and j is selected from 0-10; and the -NH- terminus of L₂ is linked to L₁.

In any technical solution of formula I in the present invention, L₂ is

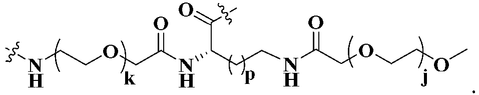

In any technical solution of formula I in the present invention, L₂ is

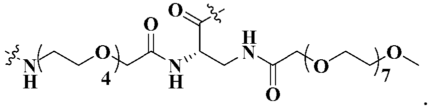

In any technical solution of formula I in the present invention, L₂ is

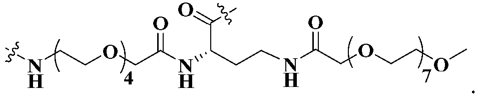

In any technical solution of formula I in the present invention, L₂ is

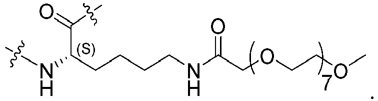

In any technical solution of formula I in the present invention, L₂ is

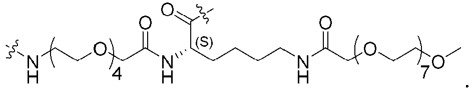

In any technical solution of formula I in the present invention, L₂ is

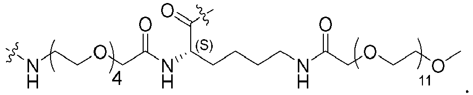

In any technical solution of formula I in the present invention, L₂ is

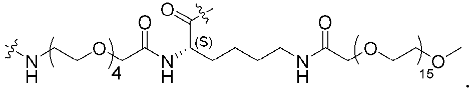

In any technical solution of formula I in the present invention, L₂ is

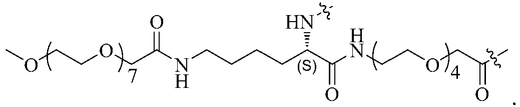

In any technical solution of formula I in the present invention, L₂ is the chemical bond.

In any technical solution of formula I in the present invention, the amino acid residues in L₃ are α-amino acid residues, selected from residues of the following amino acids: glycine, D-phenylalanine, D-valine, D-alanine, D-asparagine, citrulline, lysine, serine, glutamic acid and aspartic acid.

In any technical solution of formula I in the present invention, the amino acid residues in L₃ are α-amino acid residues, selected from the residues of the following amino acids: glycine, D-phenylalanine, D-valine, D-alanine, D-asparagine and citrulline.

In any technical solution of formula I in the present invention, L₃ is selected from the following peptide fragments: -Gly-Gly-Phe-Gly-, -Val-Ala-, -Ala-Ala-, -Ala-Ala-Asn- or -Val-Cit-; and the -NH- terminus of L₃ is linked to L₂.

In any technical solution of formula I in the present invention, r is selected from 6-10, or r is selected from 9.

In any technical solution of formula I in the present invention, L₄ is the chemical bond.

In any technical solution of formula I in the present invention, L₄ is selected from 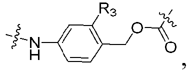 and R₃ has the same meaning as defined in formula I.

In any technical solution of formula I in the present invention, L₄ is selected from

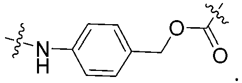

In any technical solution of formula I in the present invention, L₄ is selected from

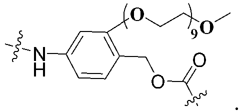

In any technical solution of formula I in the present invention, L₄ is selected from -NH-CH₂-.

In any technical solution of formula I in the present invention, La is selected from the following structures:

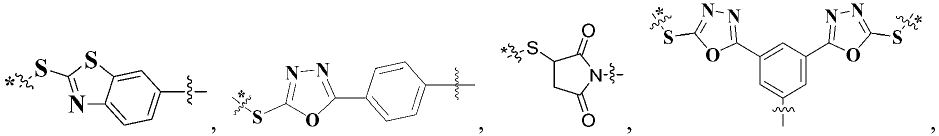

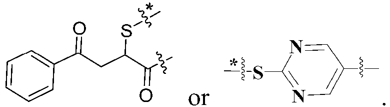

In any technical solution of formula I in the present invention, Lb is selected from the following structure:

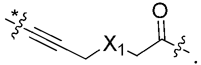

In any technical solution of formula I in the present invention, Lb is selected from the following structures:

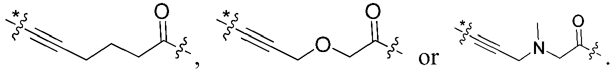

In any technical solution of formula I in the present invention, L₁ is selected from the following fragments: 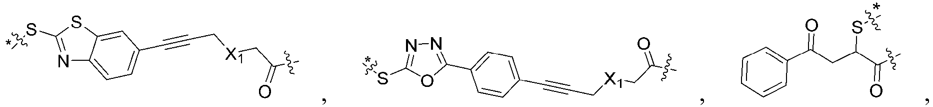 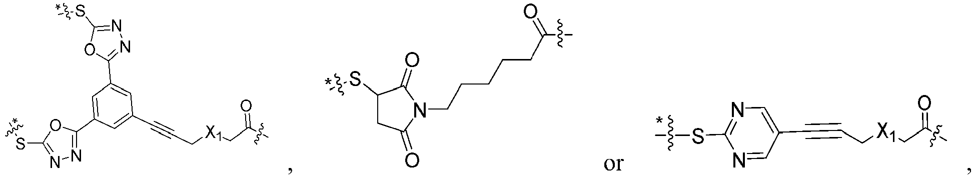 wherein, X₁ has the same meaning as defined in any technical solution of formula I.

In any technical solution of formula I in the present invention, L₁ is selected from the following fragments:

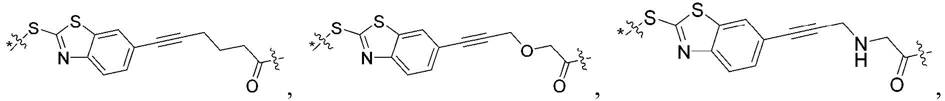

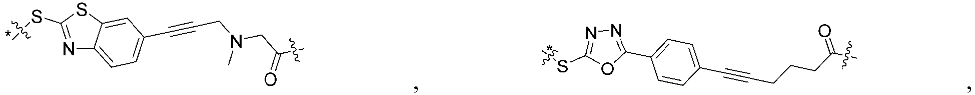

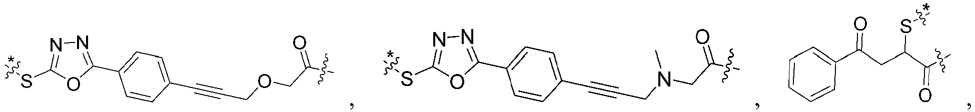

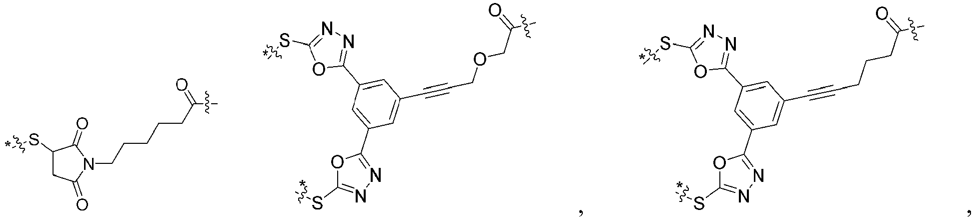

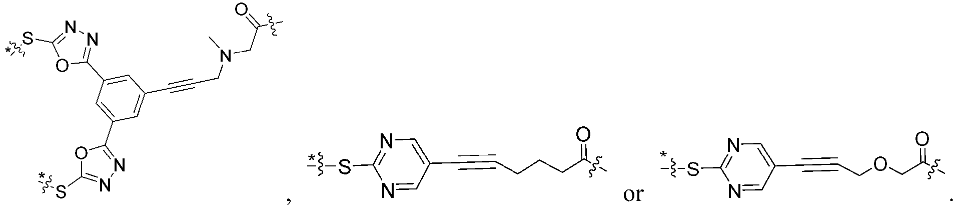

In any technical solution of formula I in the present invention, -L₁-L₂-L₃-L₄- is selected from the following structural fragments:

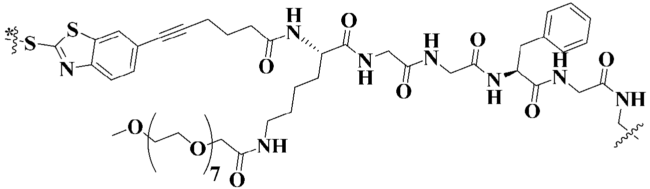

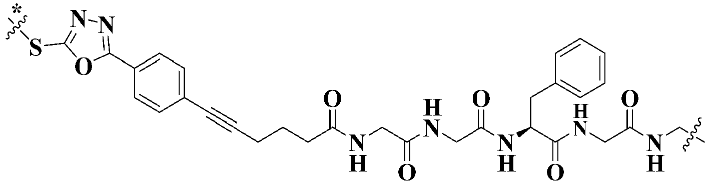

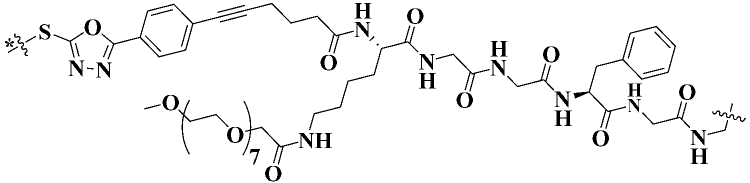

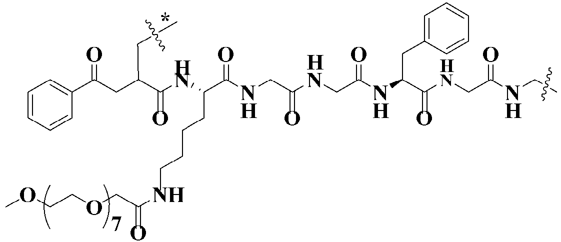

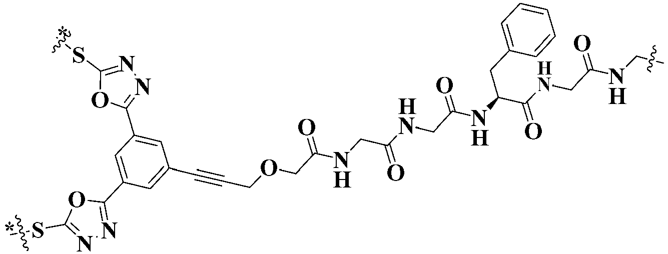

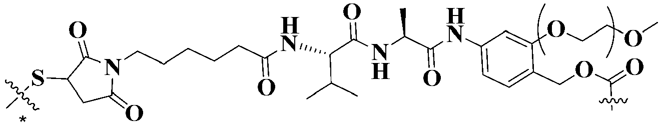

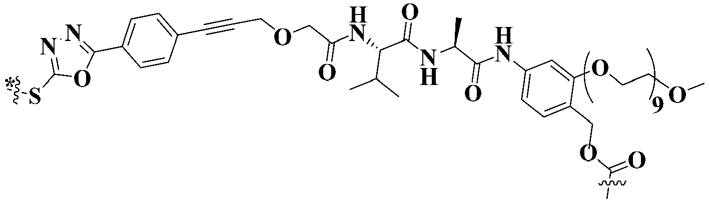

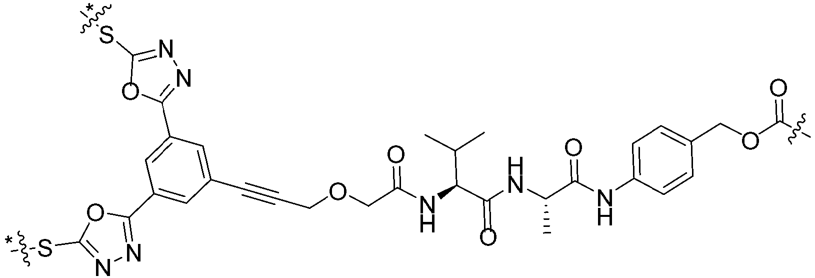

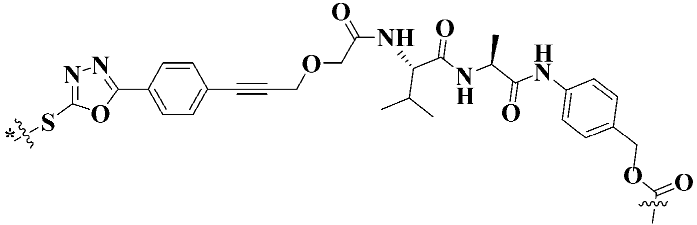

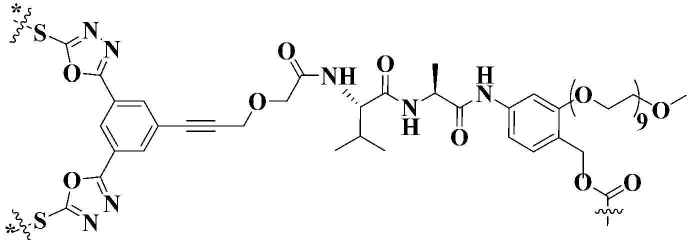

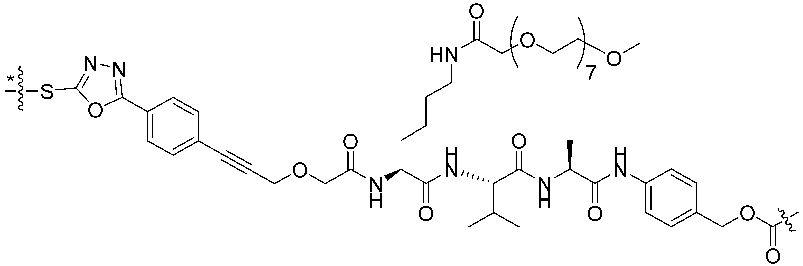

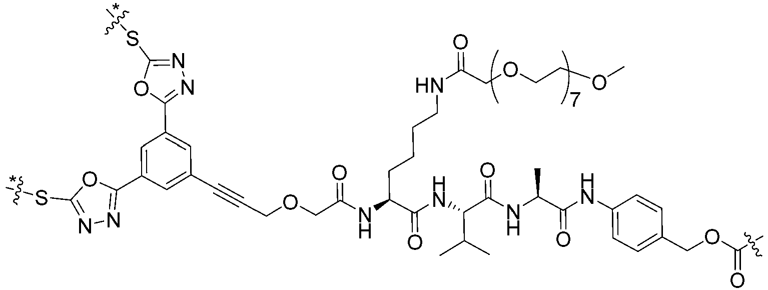

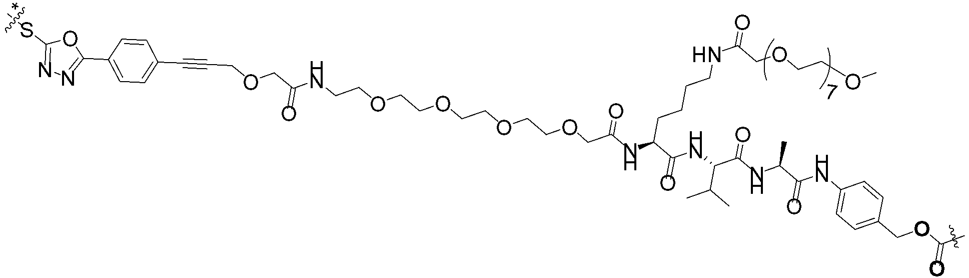

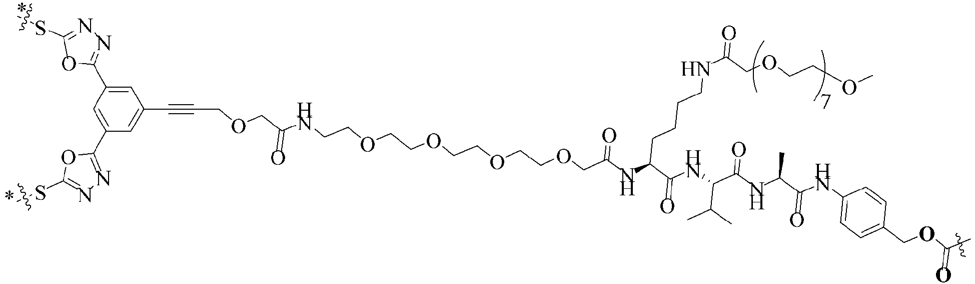

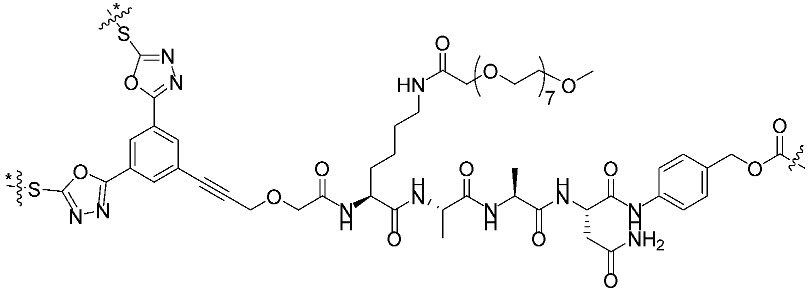

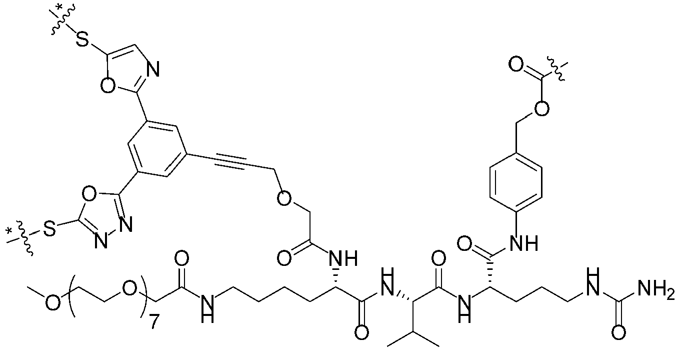

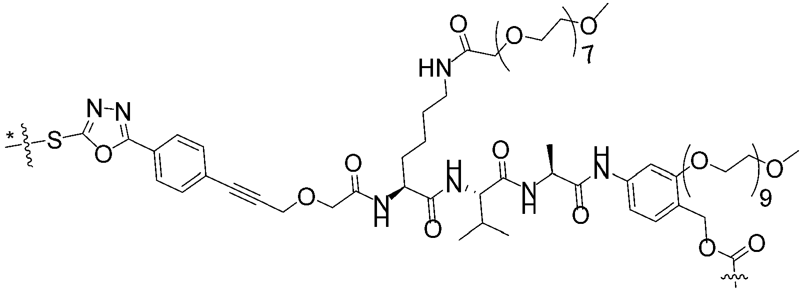

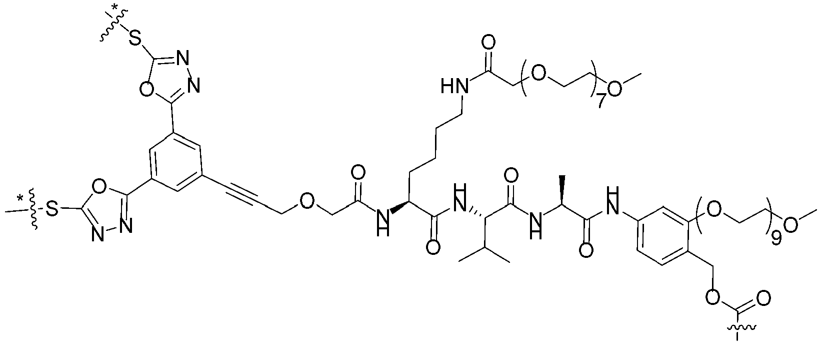

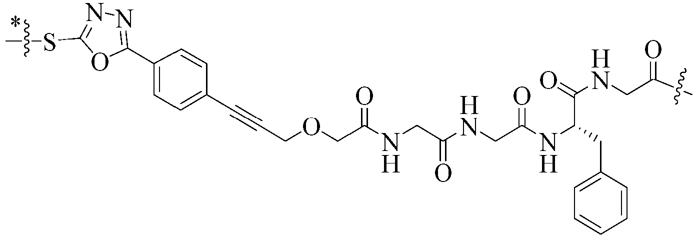

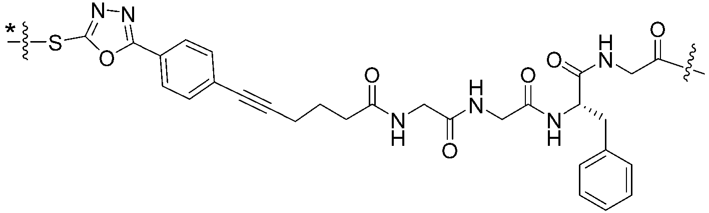

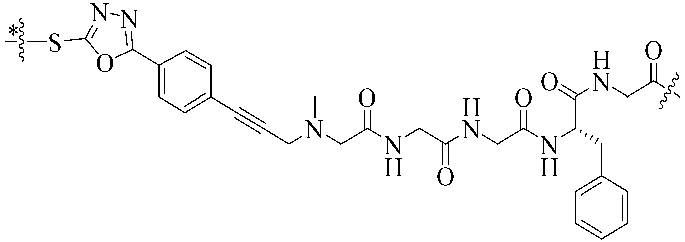

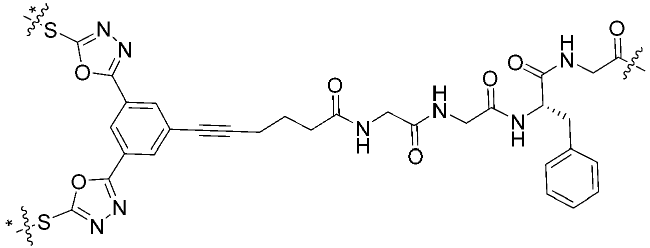

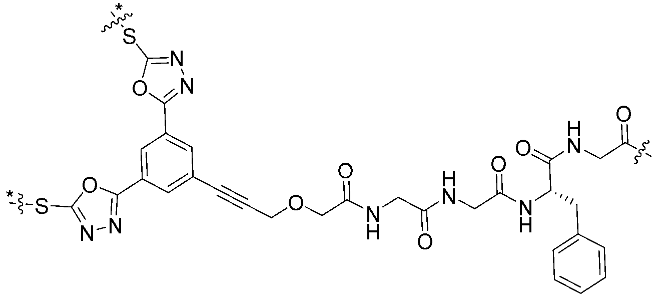

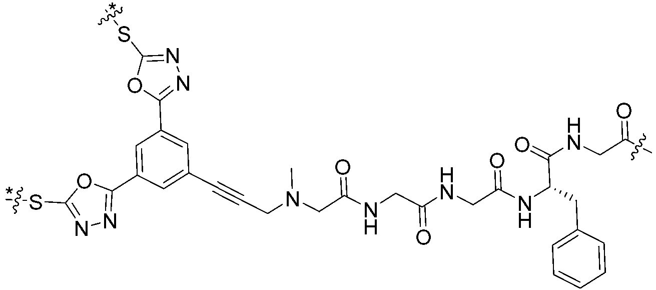

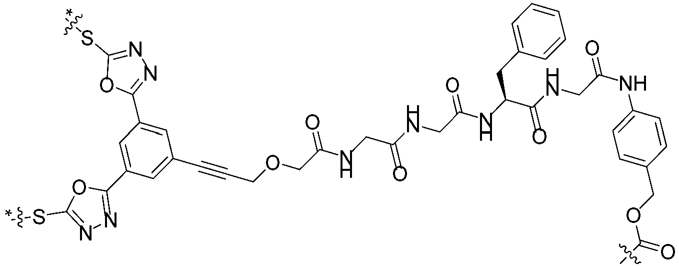

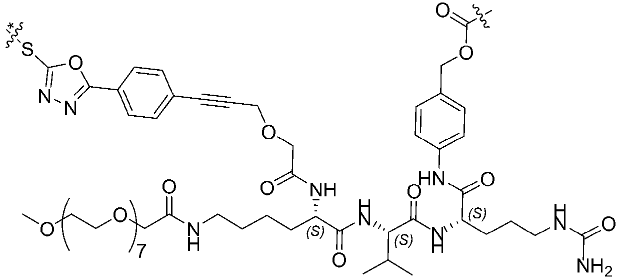

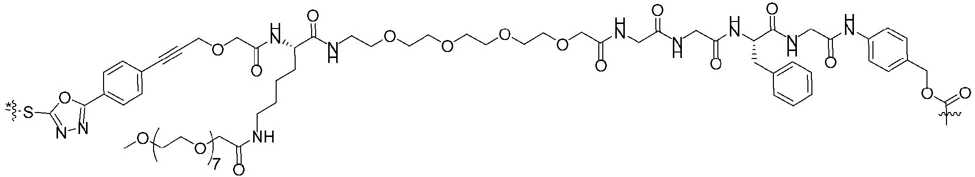

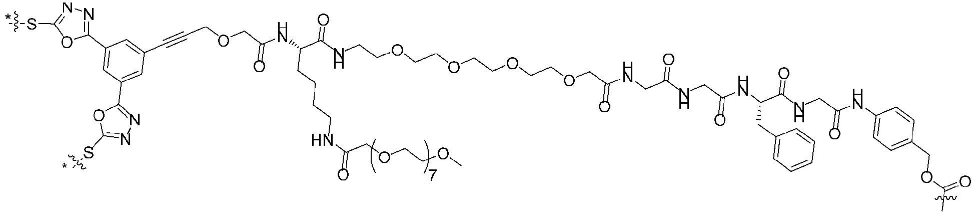

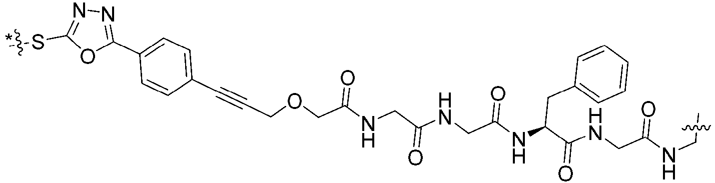

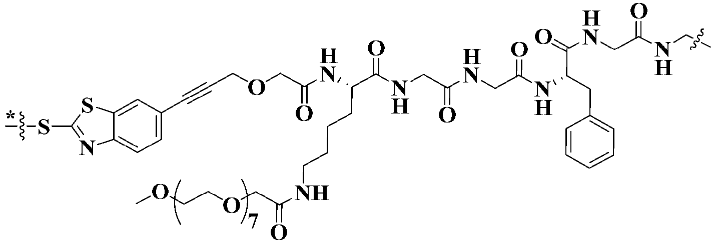

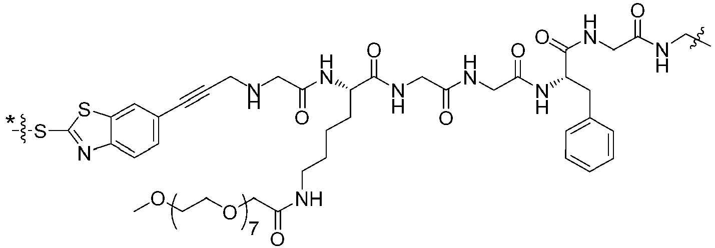

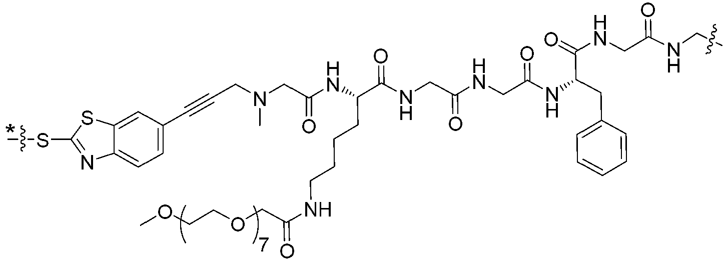

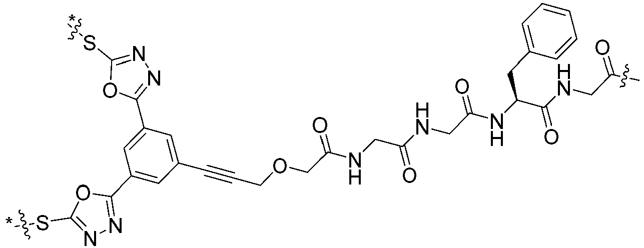

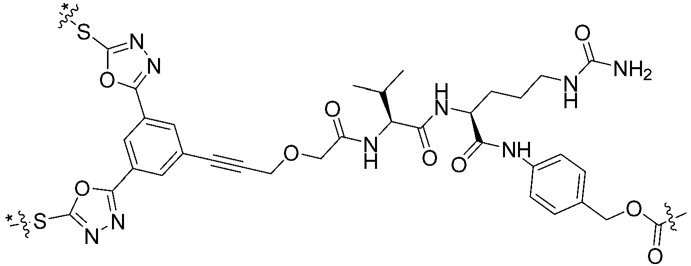

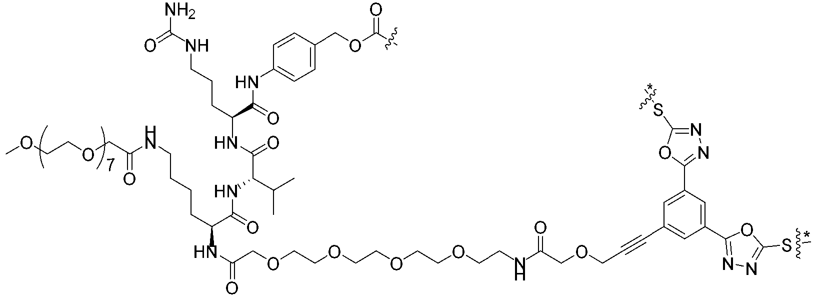

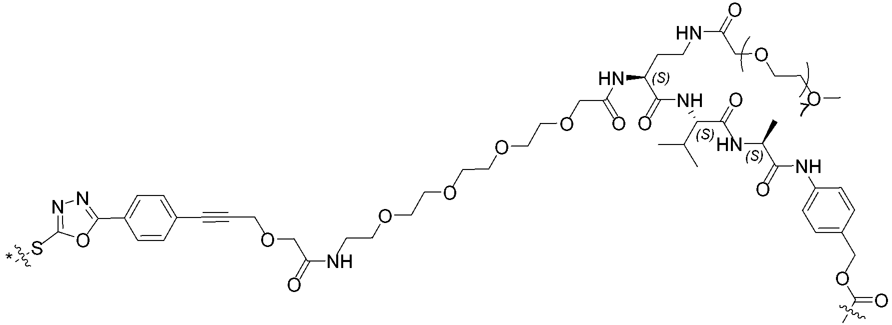

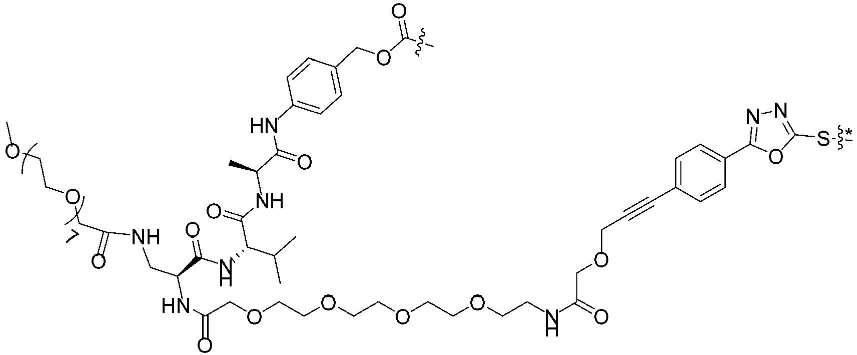

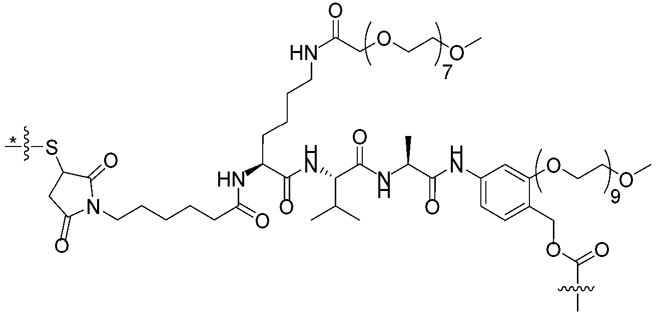

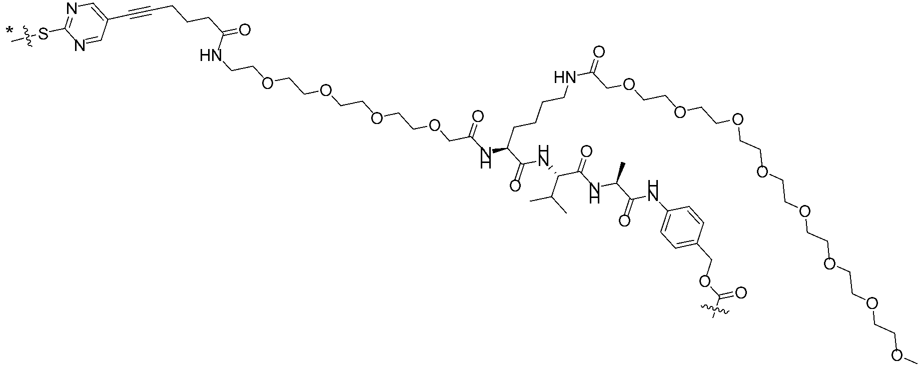

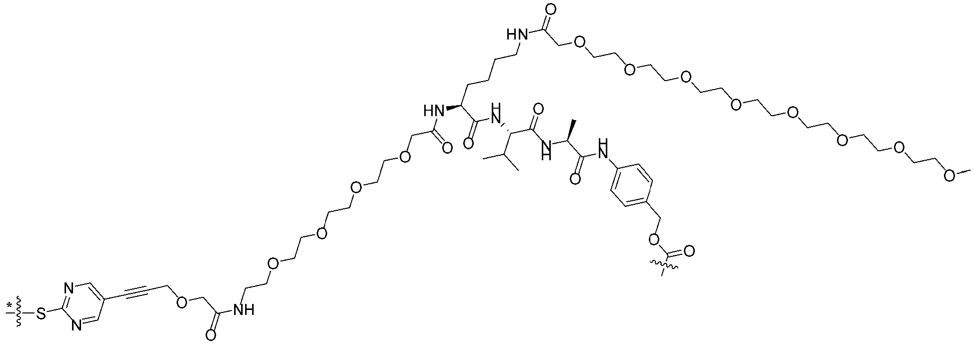

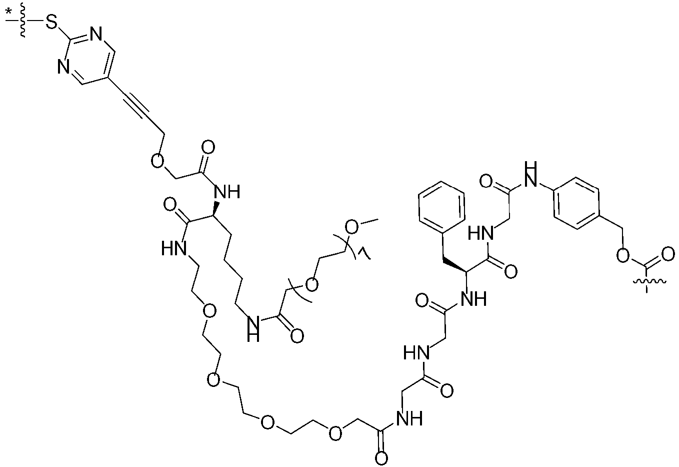

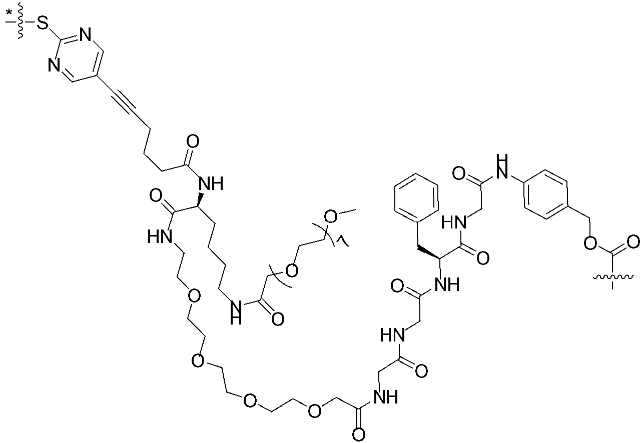

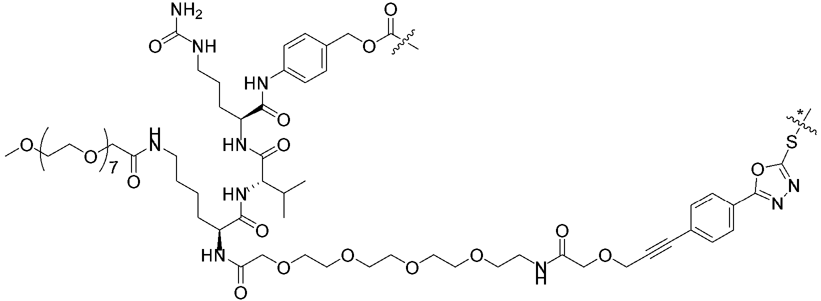

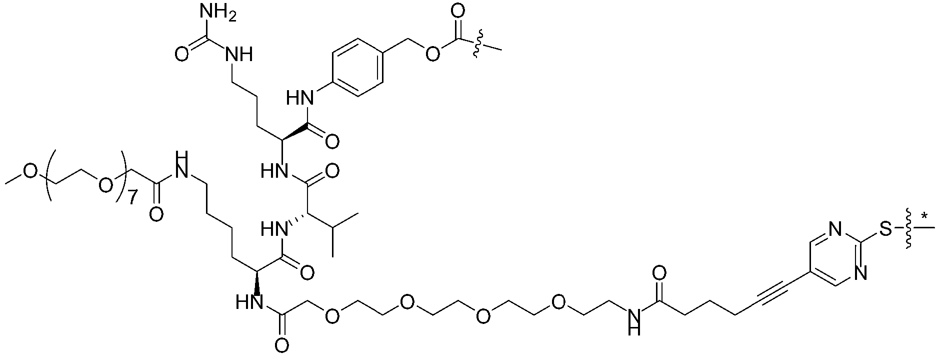

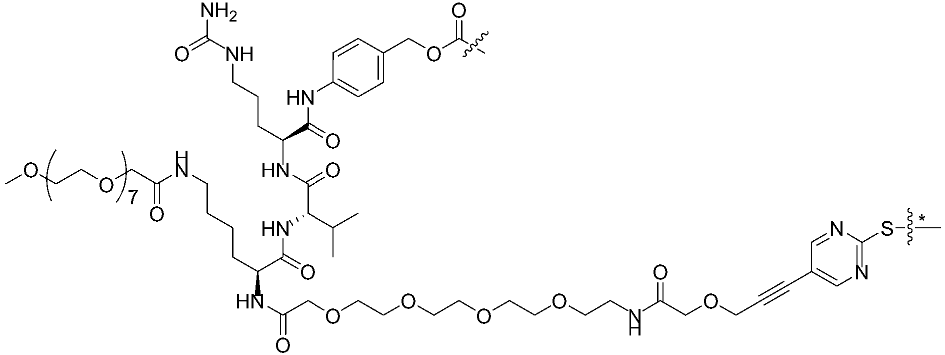

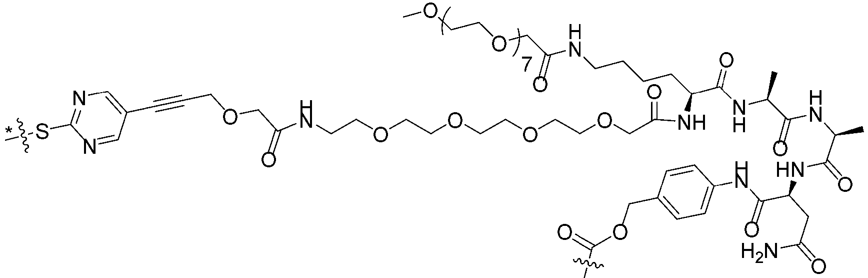

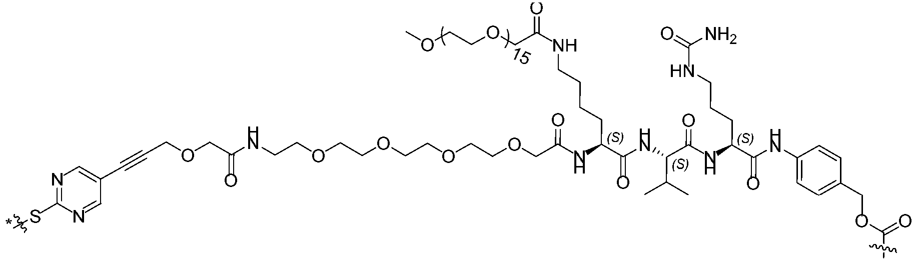

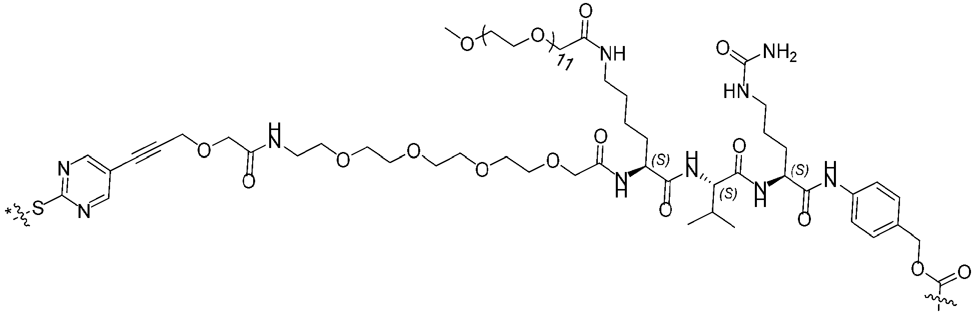

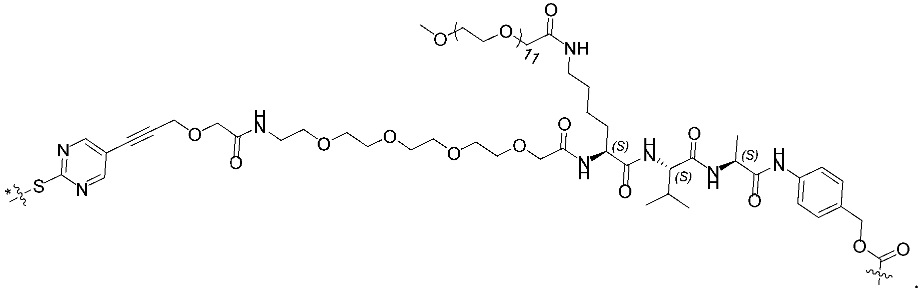

In any technical solution of the present invention, Ab may be selected from an anti-Her2 antibody, an anti-Trop2 antibody, an anti-Claudin18.2 antibody, an anti-LIV-1 antibody, an anti-5T4 antibody, an anti-CLDN6 antibody, an anti-CDH6 antibody or an anti-folate receptor antibody.

In any technical solution of formula I in the present invention, the anti-Her2 antibody is selected from Trastuzumab or Pertuzumab.

In any technical solution of formula I in the present invention, the anti-LIV-1 antibody comprises HCDR1, HCDR2 and HCDR3 as shown in SEQ ID NO: 1, SEQ ID NO: 2 and SEQ ID NO: 3.

In any technical solution of formula I in the present invention, the anti-LIV-1 antibody comprises a heavy chain variable region as shown in SEQ ID NO: 4.

In any technical solution of formula I in the present invention, the anti-LIV-1 antibody comprises a heavy chain as shown in SEQ ID NO: 5.

In any technical solution of formula I in the present invention, the anti-LIV-1 antibody comprises LCDR1, LCDR2 and LCDR3 as shown in SEQ ID NO: 6, SEQ ID NO: 7 and SEQ ID NO: 8.

In any technical solution of formula I in the present invention, the anti-LIV-1 antibody comprises a light chain variable region as shown in SEQ ID NO: 9.

In any technical solution of formula I in the present invention, the anti-LIV-1 antibody comprises a light chain as shown in SEQ ID NO: 10.

In any technical solution of formula I in the present invention, the anti-LIV-1 antibody comprises HCDR1, HCDR2 and HCDR3 as shown in SEQ ID NO: 1, SEQ ID NO: 2 and SEQ ID NO: 3, and LCDR1, LCDR2 and LCDR3 as shown in SEQ ID NO: 6, SEQ ID NO: 7 and SEQ ID NO: 8.

In any technical solution of formula I in the present invention, the anti-LIV-1 antibody comprises the heavy chain variable region as shown in SEQ ID NO: 4 and the light chain variable region as shown in SEQ ID NO: 9.

In any technical solution of formula I in the present invention, the anti-LIV-1 antibody comprises the heavy chain as shown in SEQ ID NO: 5 and the light chain as shown in SEQ ID NO: 10.

In any technical solution of formula I in the present invention, the anti-LIV-1 antibody is Ladiratuzumab.

In any technical solution of formula I in the present invention, the anti-Her2 antibody comprises HCDR1, HCDR2 and HCDR3 as shown in SEQ ID NO: 11, SEQ ID NO: 12 and SEQ ID NO: 13.

In any technical solution of formula I in the present invention, the anti-Her2 antibody comprises a heavy chain variable region as shown in SEQ ID NO: 14.

In any technical solution of formula I in the present invention, the anti-Her2 antibody comprises a heavy chain as shown in SEQ ID NO: 15.

In any technical solution of formula I in the present invention, the anti-Her2 antibody comprises LCDR1, LCDR2 and LCDR3 as shown in SEQ ID NO: 16, SEQ ID NO: 17 and SEQ ID NO: 18.

In any technical solution of formula I in the present invention, the anti-Her2 antibody comprises a light chain variable region as shown in SEQ ID NO: 19.

In any technical solution of formula I in the present invention, the anti-Her2 antibody comprises a light chain as shown in SEQ ID NO: 20.

In any technical solution of formula I in the present invention, the anti-Her2 antibody comprises HCDR1, HCDR2 and HCDR3 as shown in SEQ ID NO: 11, SEQ ID NO: 12 and SEQ ID NO: 13, and LCDR1, LCDR2 and LCDR3 as shown in SEQ ID NO: 16, SEQ ID NO: 17 and SEQ ID NO: 18.

In any technical solution of formula I in the present invention, the anti-Her2 antibody comprises the heavy chain variable region as shown in SEQ ID NO: 14 and the light chain variable region as shown in SEQ ID NO: 19.

In any technical solution of formula I in the present invention, the anti-Her2 antibody comprises the heavy chain as shown in SEQ ID NO: 15 and the light chain as shown in SEQ ID NO: 20.

In any technical solution of formula I in the present invention, the anti-Her2 antibody is Trastuzumab.

In any technical solution of formula I in the present invention, the drug molecule D is selected from a camptothecin compound, an auristatin compound, an eribulin compound, a maytansine compound, a calicheamicin compound, or an anthramycin compound.

In any technical solution of formula I in the present invention, the drug molecule D is selected from a fragment with a structure of formula IIa or a pharmaceutically acceptable salt thereof: 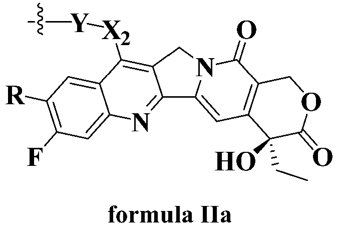 wherein, R is selected from F, C₁₋₄ alkoxy, C₁₋₄ haloalkyl or C₁₋₄ haloalkoxy; X₂ is selected from -(CH₂)s-, and s is selected from 0, 1, 2, 3 or 4; Y is selected from -O- or -N(Rc)-, and Rc is selected from H, C₁₋₄ alkyl, 3-5 membered cycloalkyl or 3-5 membered heterocycloalkyl; and when s=1, Rc is not H.

In any technical solution of formula IIa in the present invention, R is F, or R is methoxy.

In any technical solution of formula IIa in the present invention, s is 1, or s is 2, or s is 3.

In any technical solution of formula IIa in the present invention, Rc is selected from H, methyl or cyclopropyl.

In any technical solution of formula IIa in the present invention, Y is selected from -O-.

In any technical solution of formula IIa in the present invention, Y is -N(Rc)-, and Rc is methyl or cyclopropyl.

In any technical solution of formula I in the present invention, the drug molecule D is selected from the following structural fragments or pharmaceutically acceptable salts thereof:

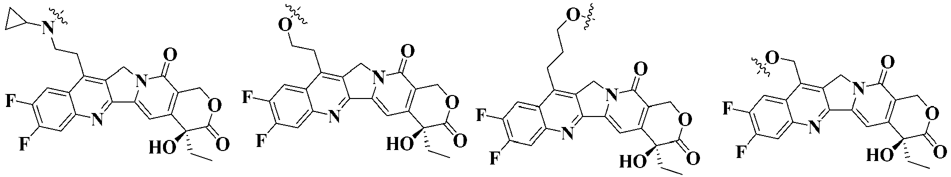

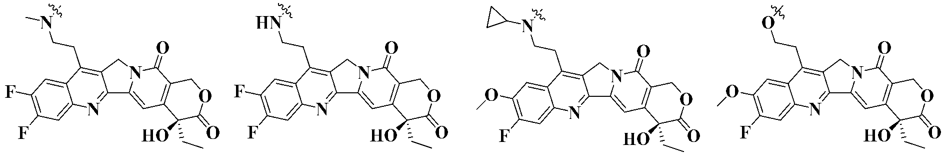

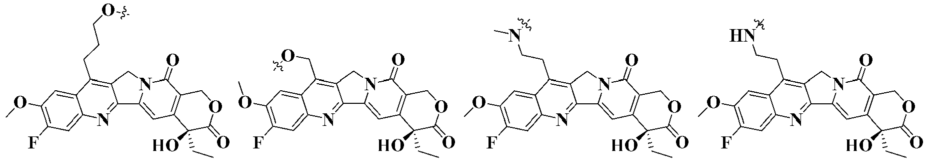

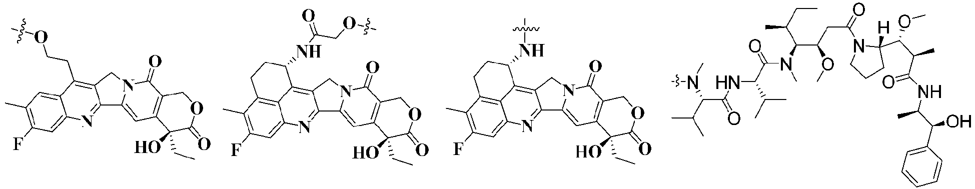

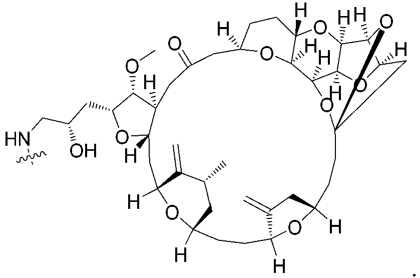

In any technical solution of formula I in the present invention, -L₁-L₂-L₃-L₄-D is selected from the following structural fragments or pharmaceutically acceptable salts thereof:

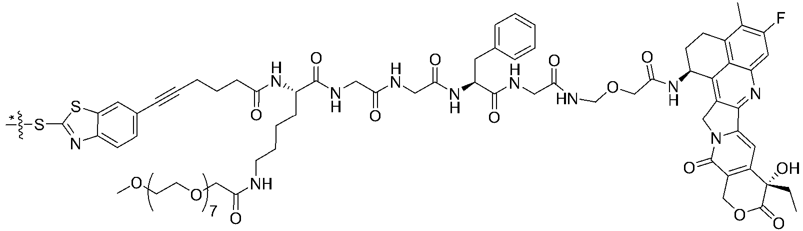

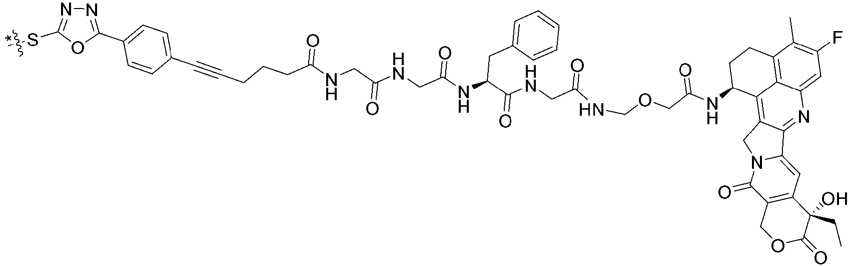

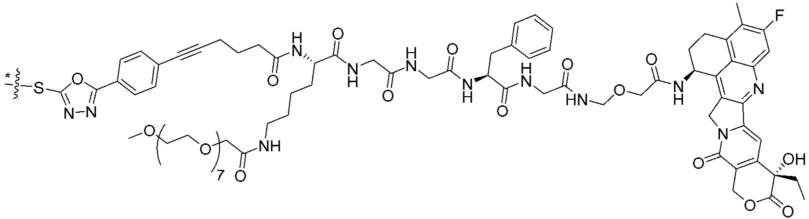

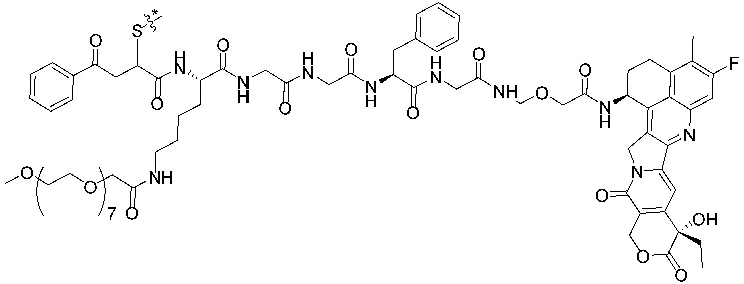

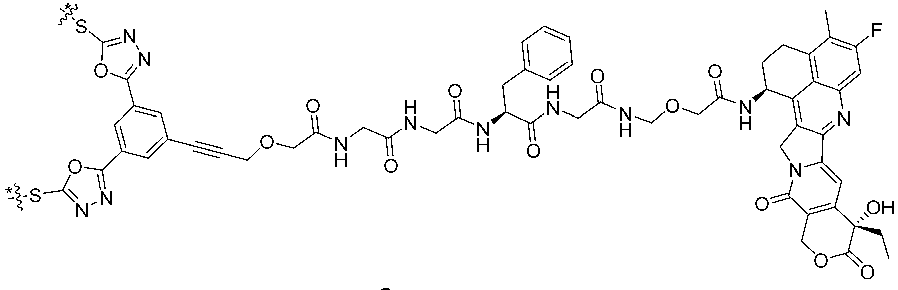

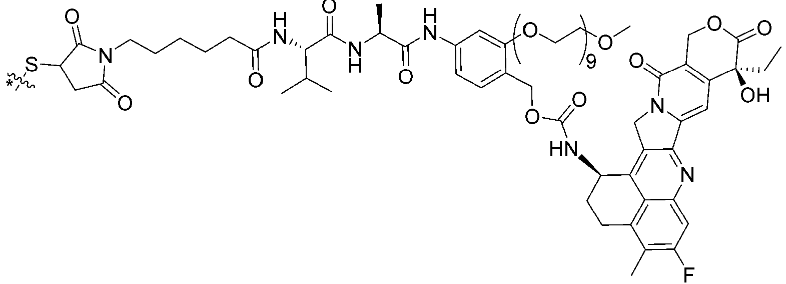

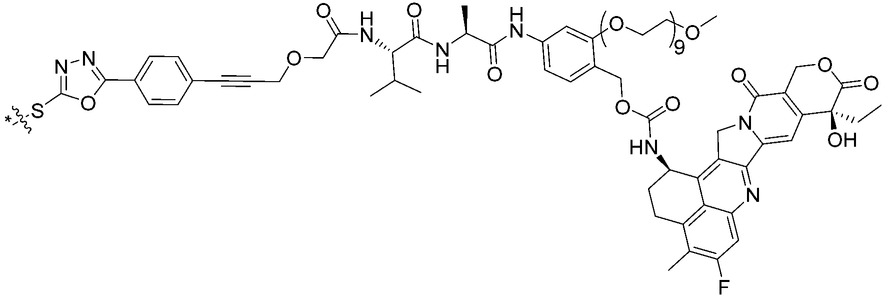

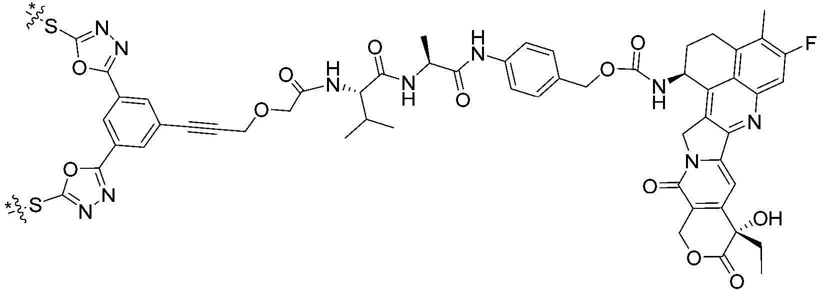

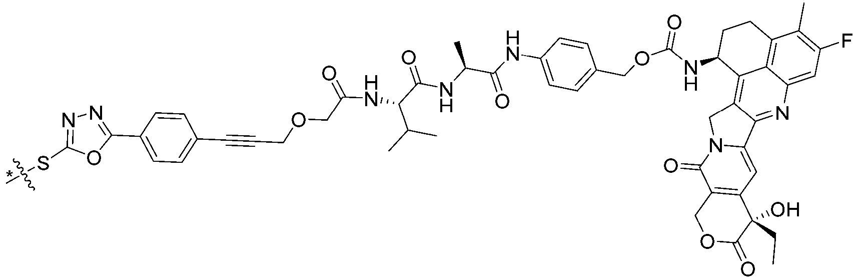

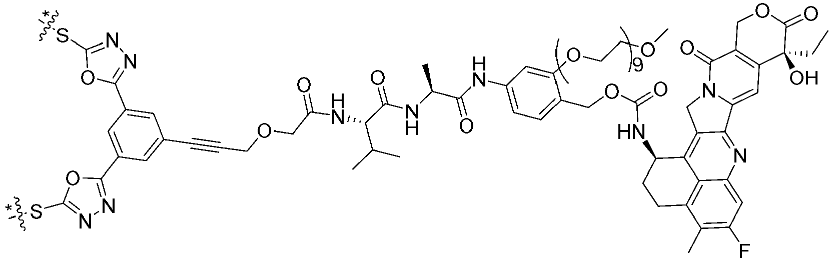

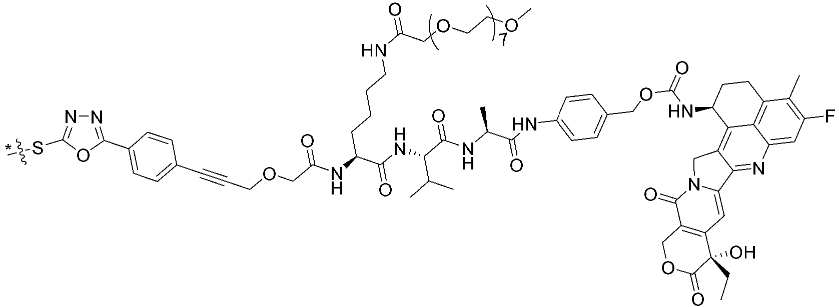

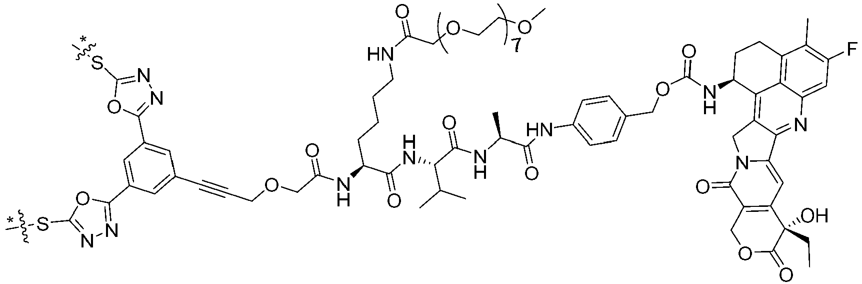

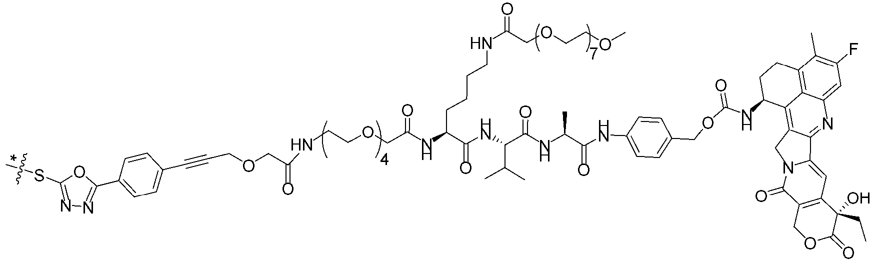

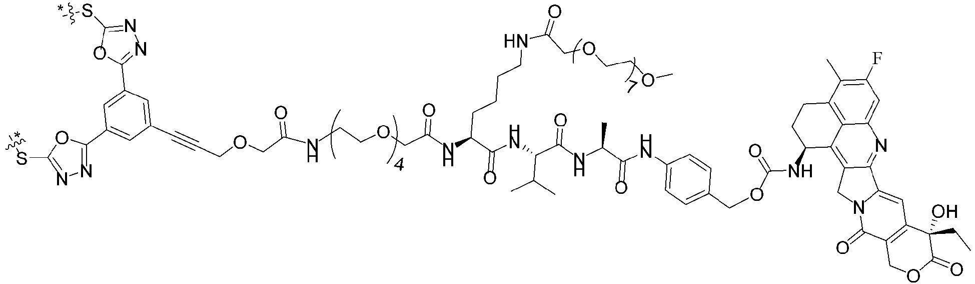

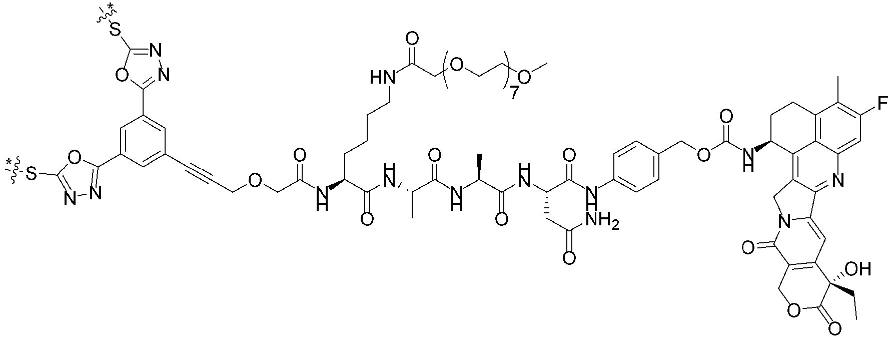

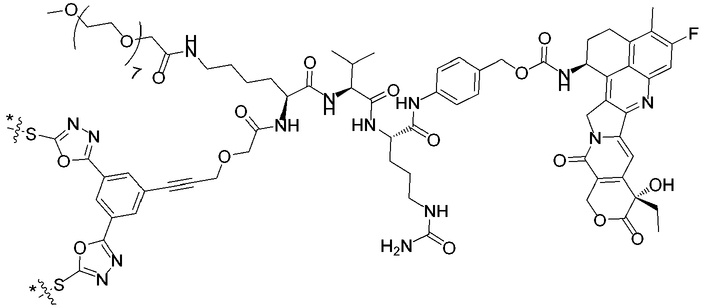

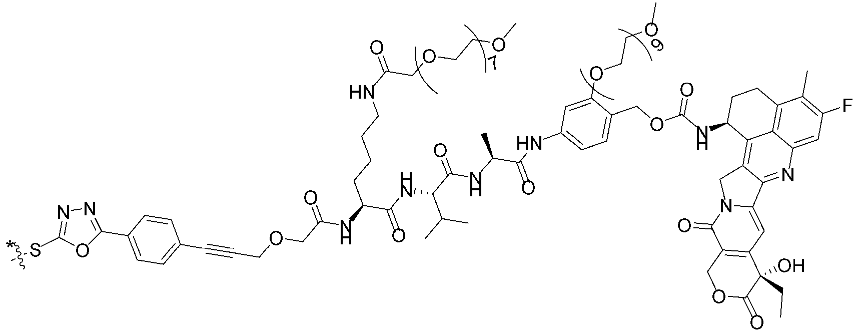

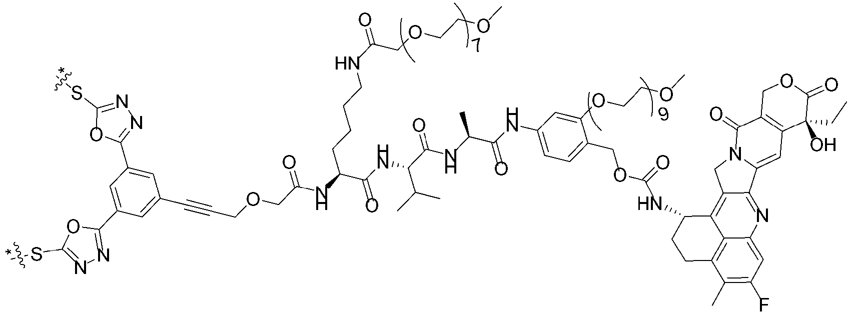

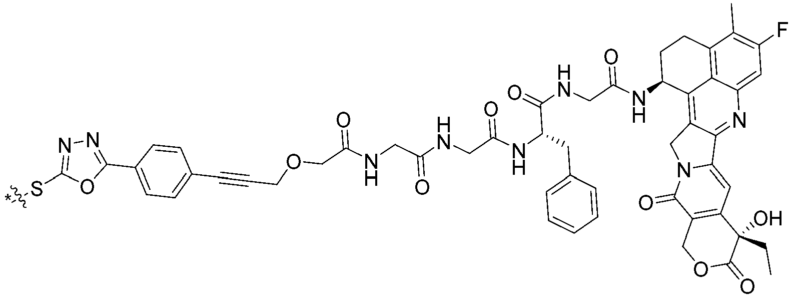

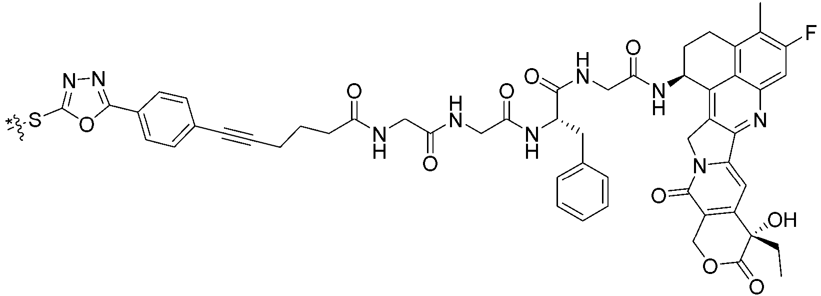

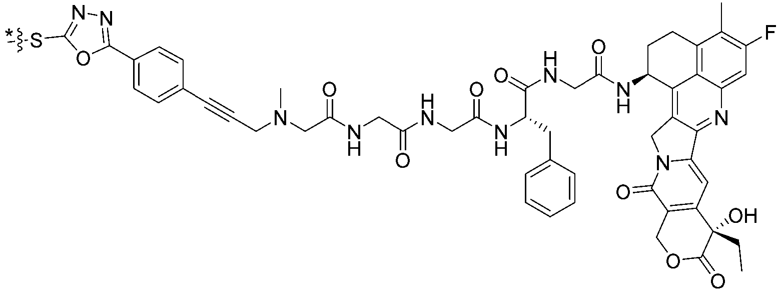

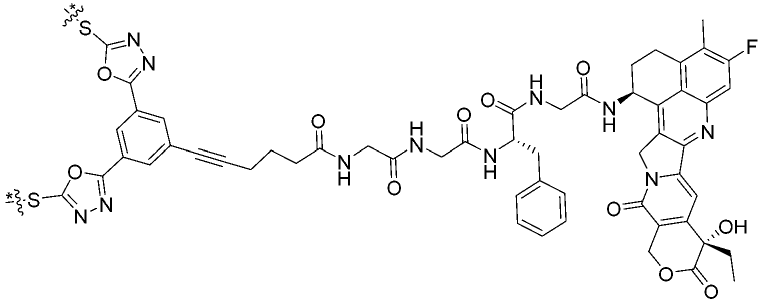

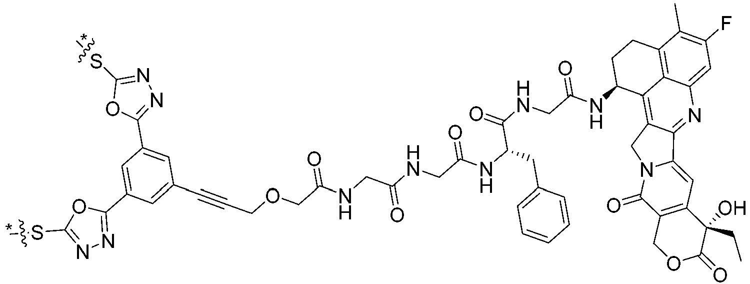

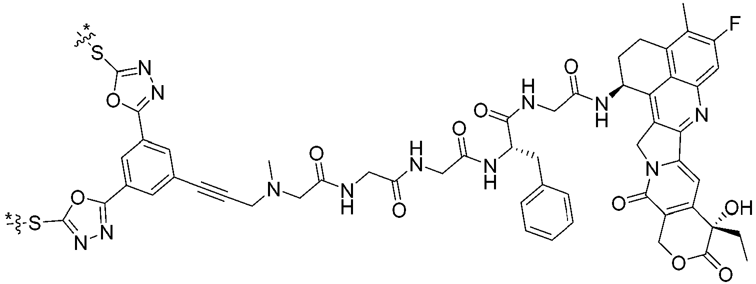

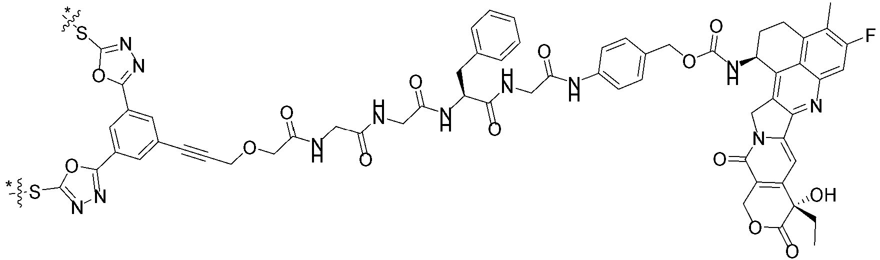

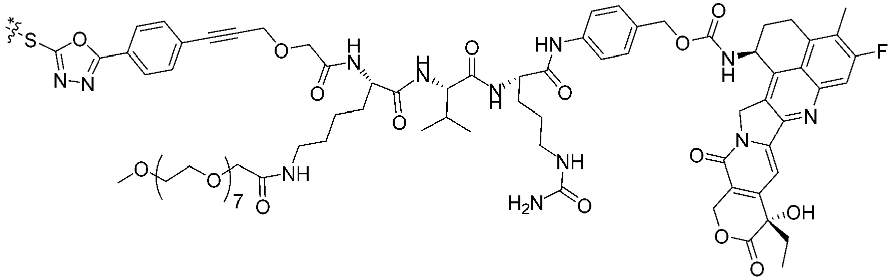

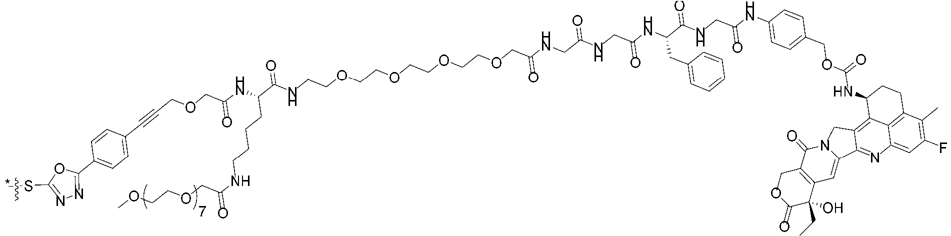

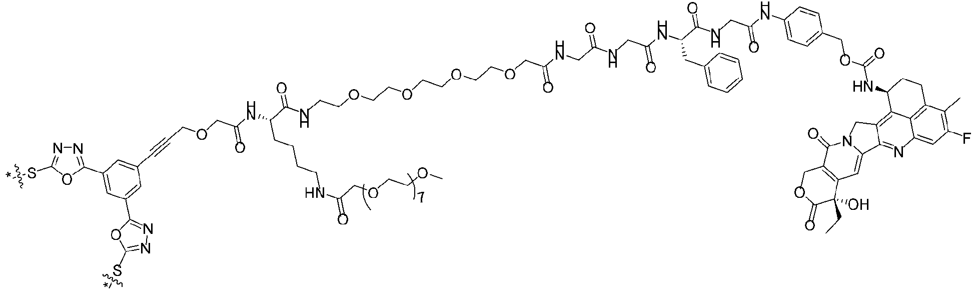

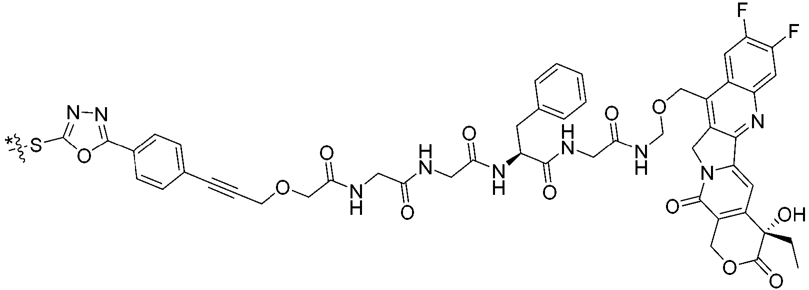

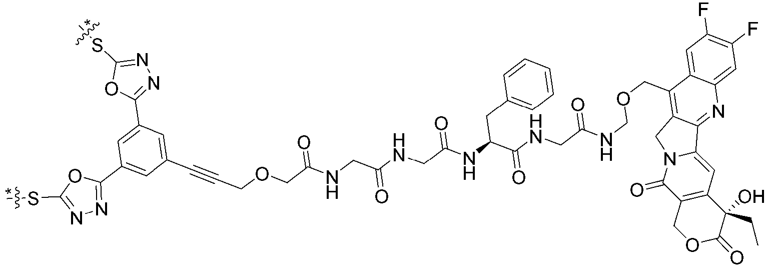

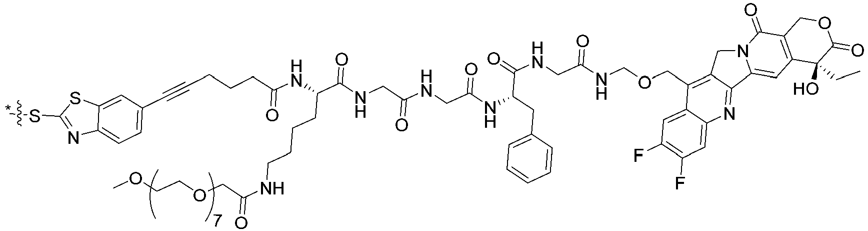

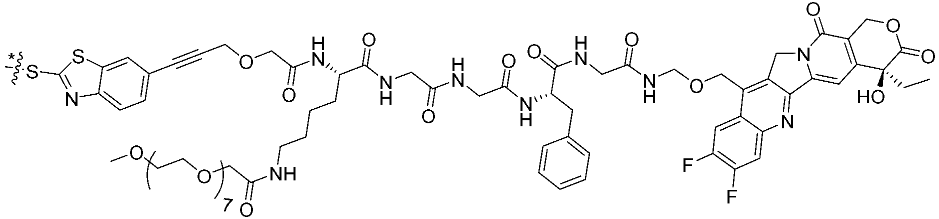

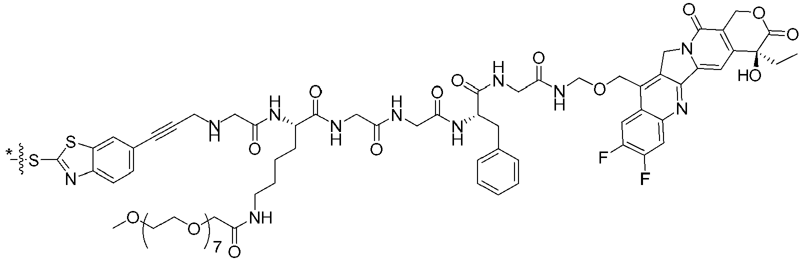

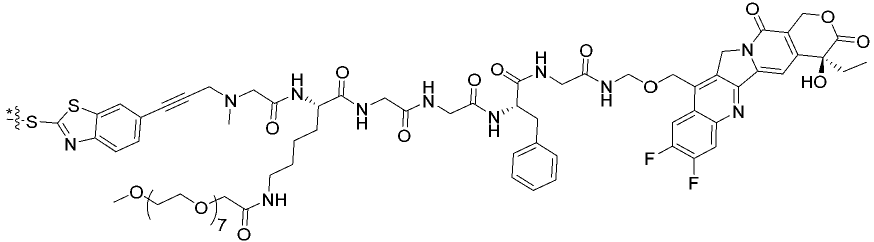

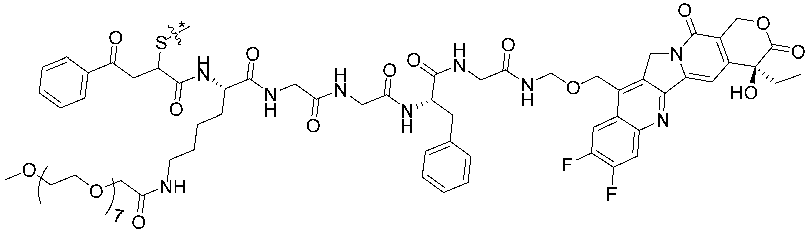

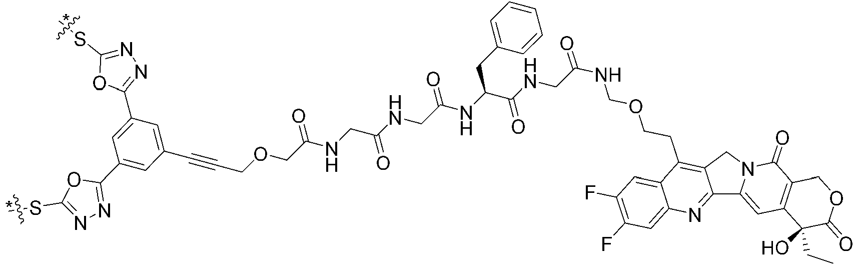

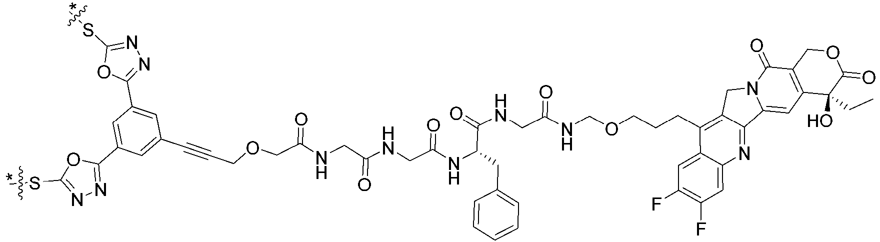

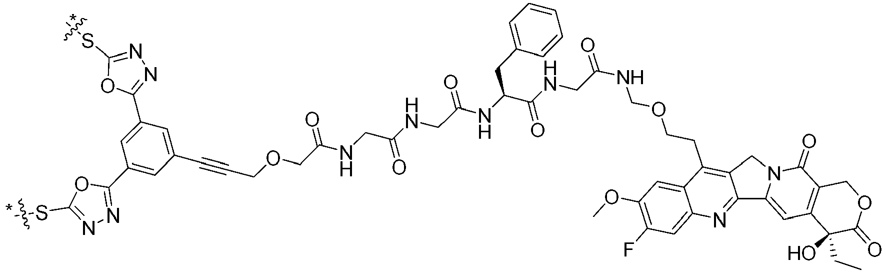

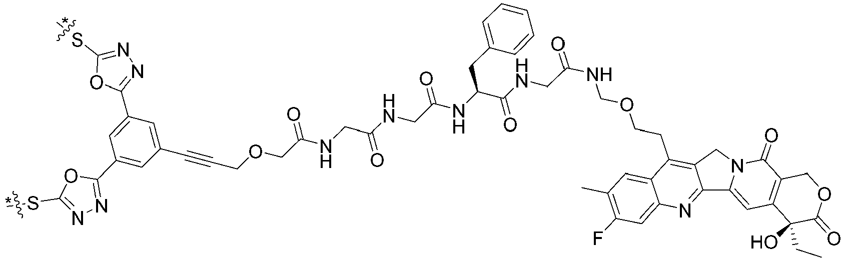

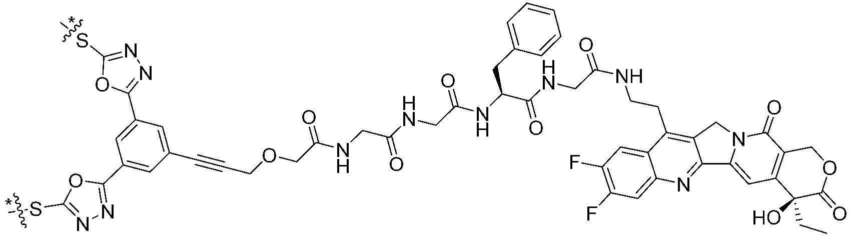

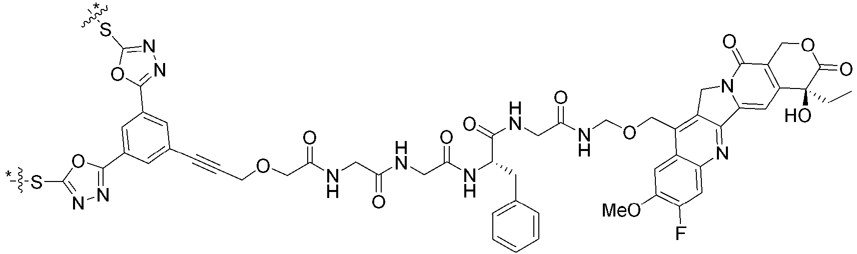

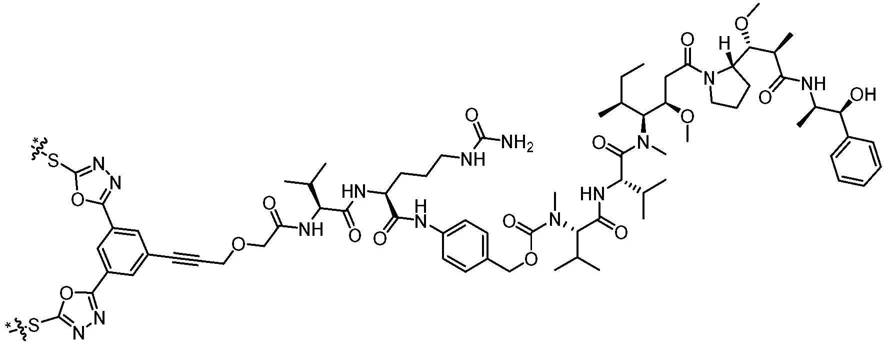

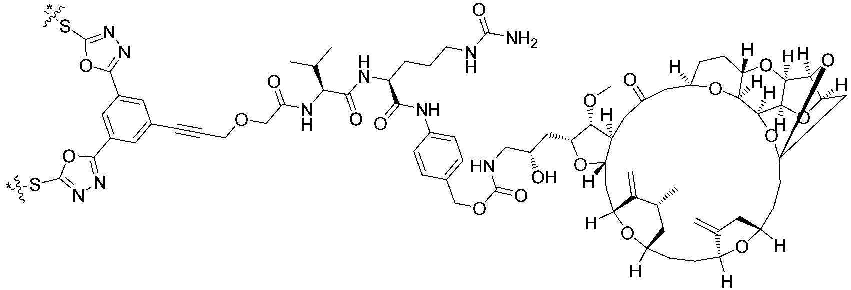

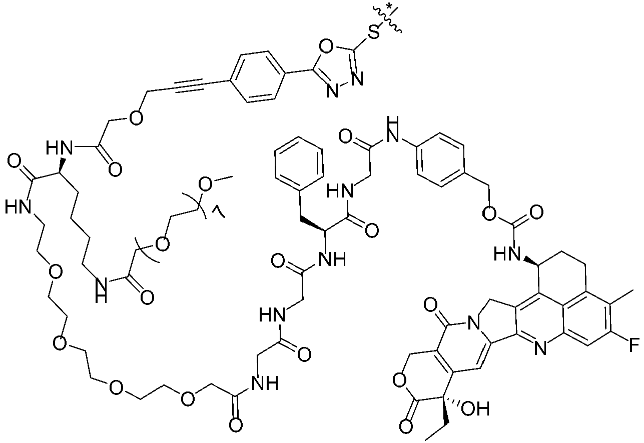

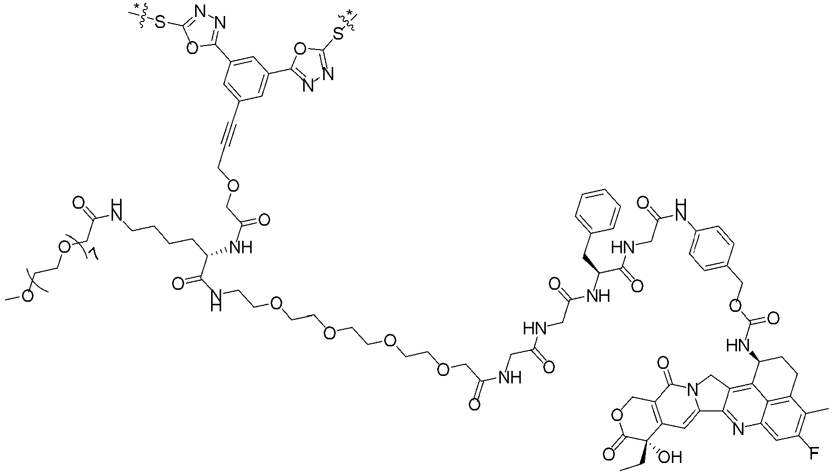

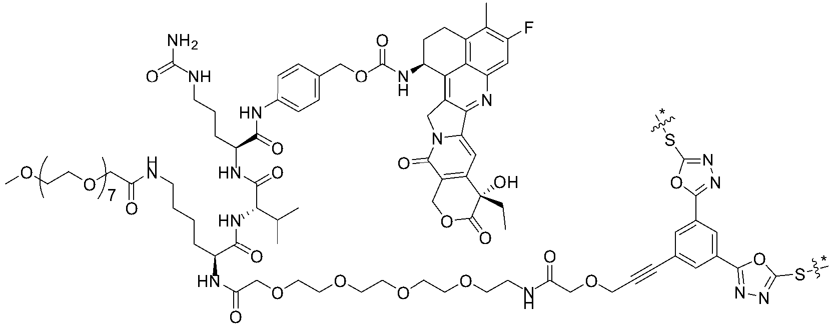

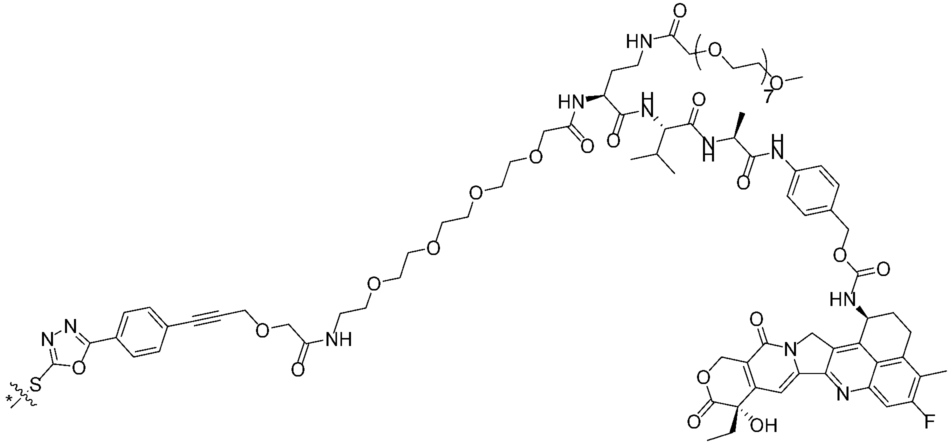

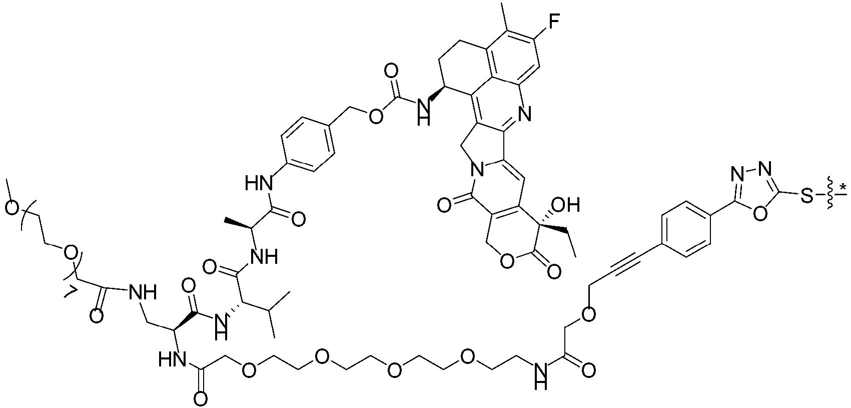

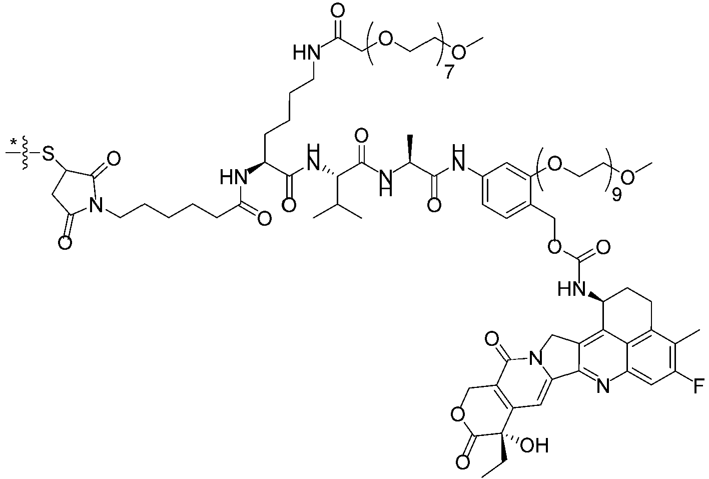

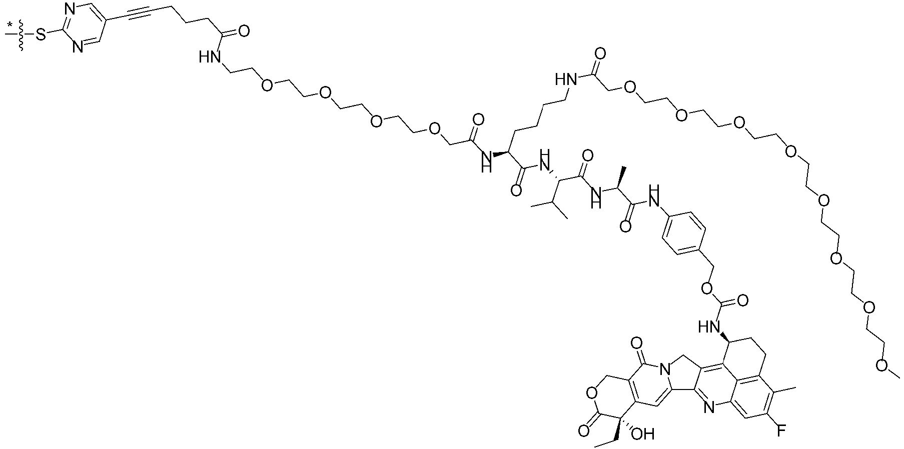

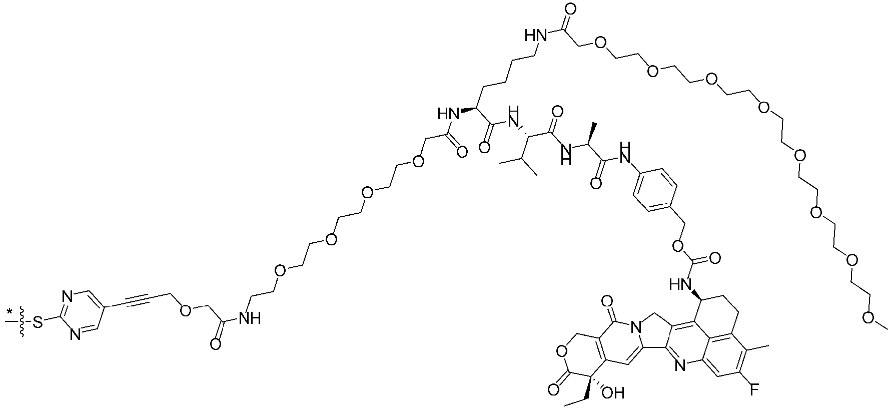

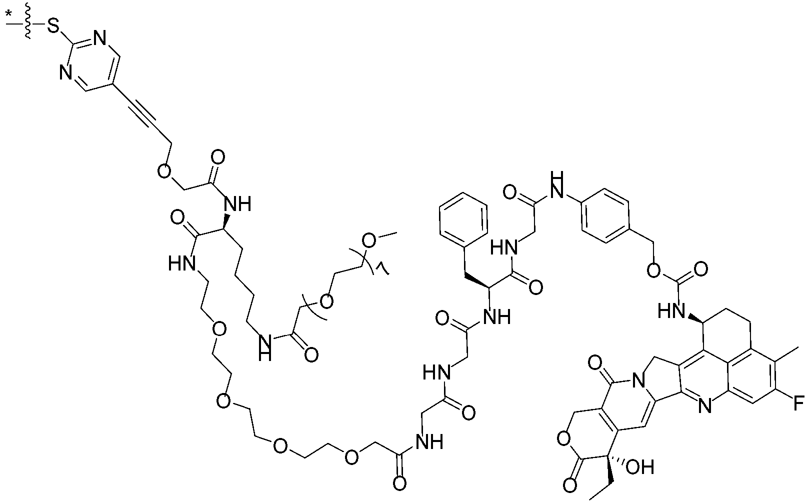

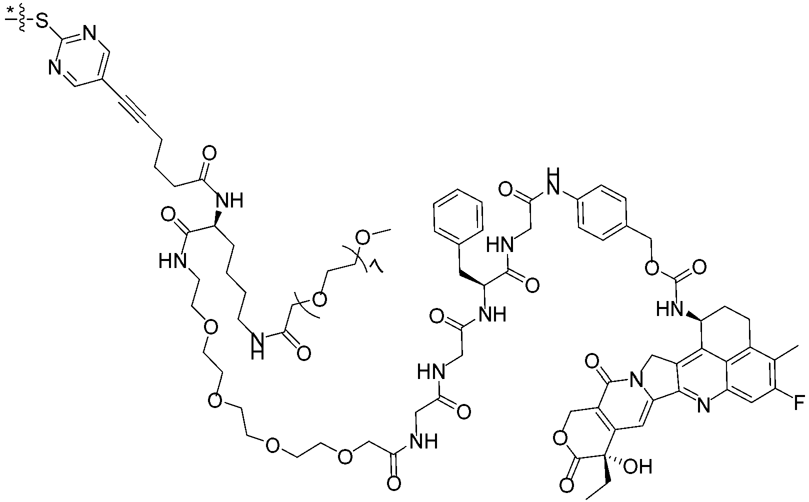

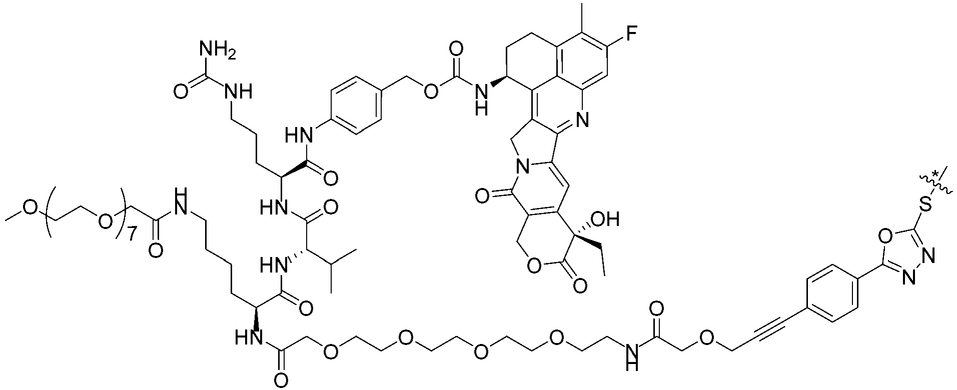

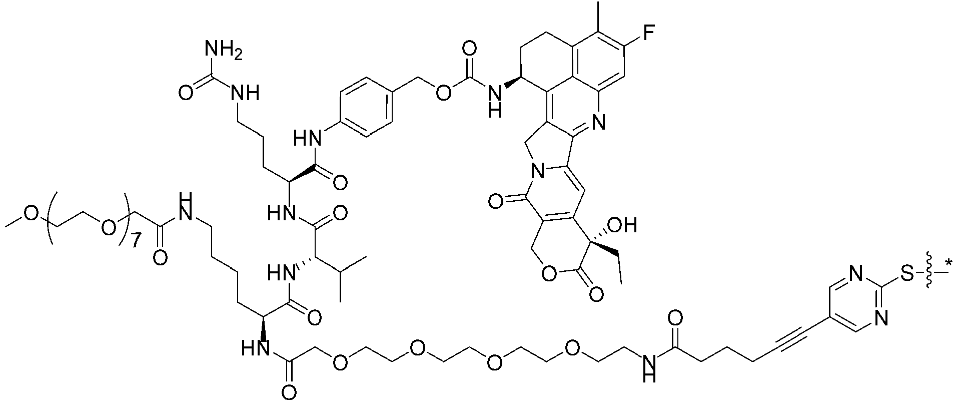

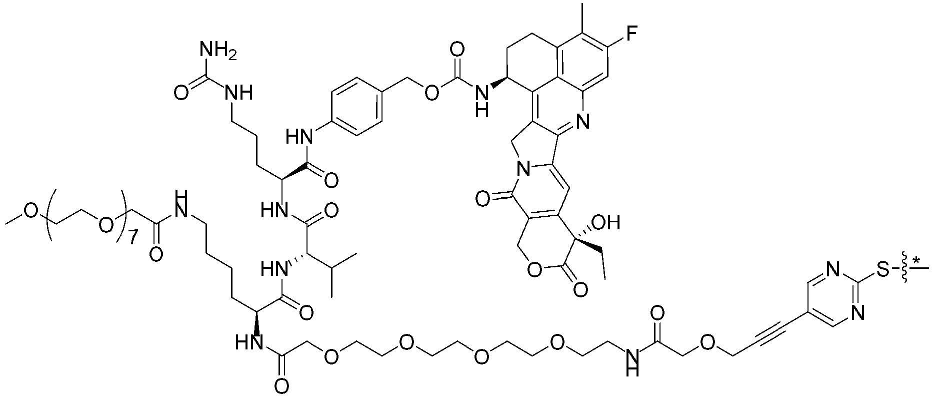

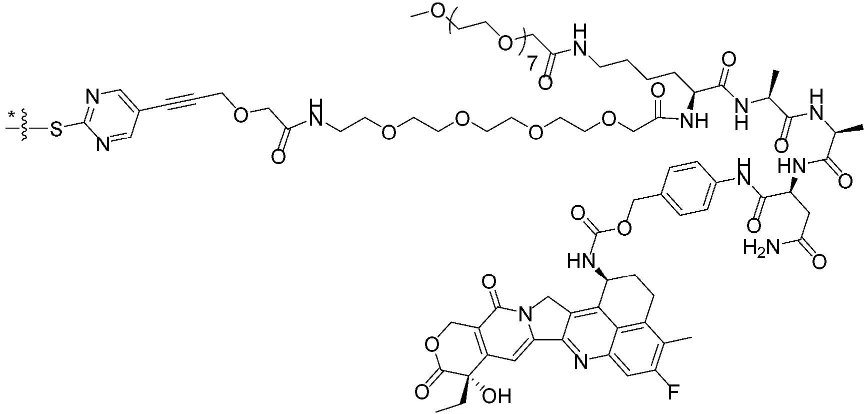

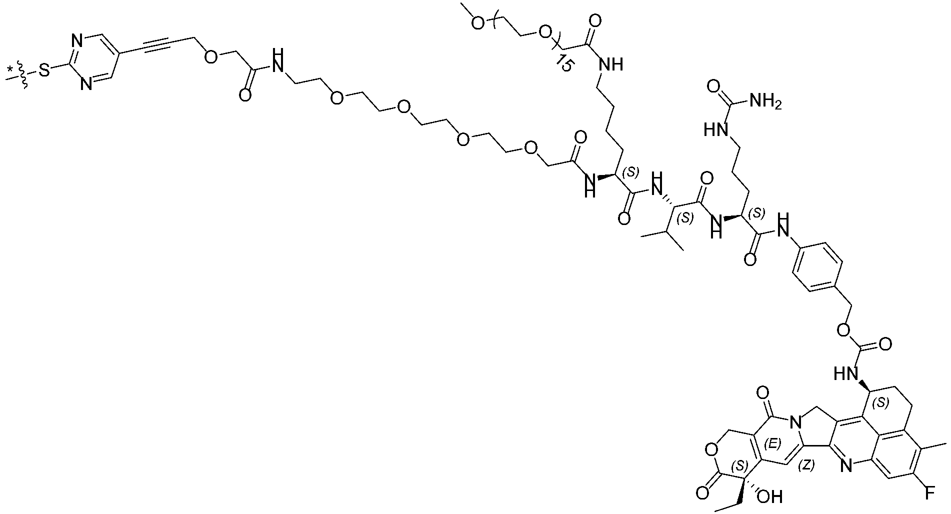

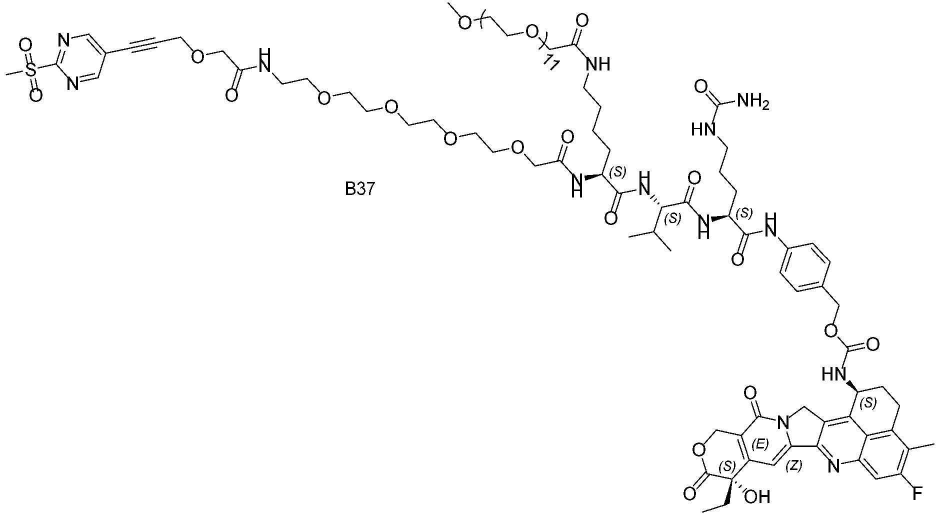

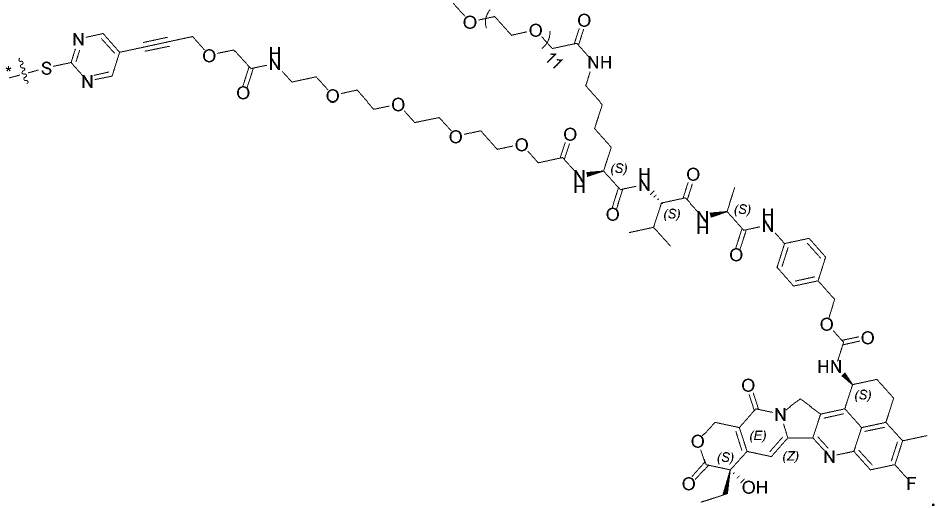

In one technical solution of the present invention, the antibody drug conjugate as shown in formula I or the pharmaceutically acceptable salt thereof is an antibody drug conjugate as shown in the following formula III or a pharmaceutically acceptable salt thereof: 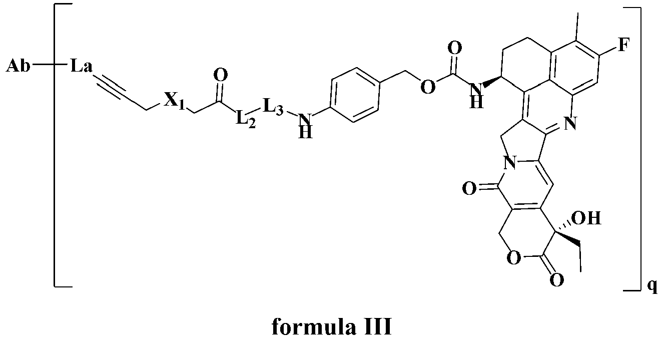 wherein, Ab, La, X₁, L₂, L₃ and q have the same meanings as defined in formula I or any other technical solution.

In any technical solution of formula III in the present invention, La may be selected from 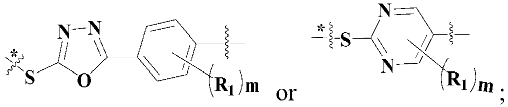 and R₁ and m have the same meanings as defined in formula I or any other technical solution.

In any technical solution of formula III in the present invention, La may be selected from

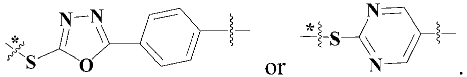

In any technical solution of formula III in the present invention, X₁ is selected from O or CH₂.

In any technical solution of formula III in the present invention, L₂ is selected from a chemical bond,

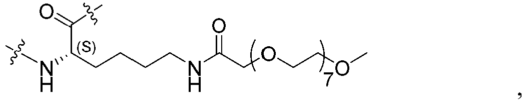

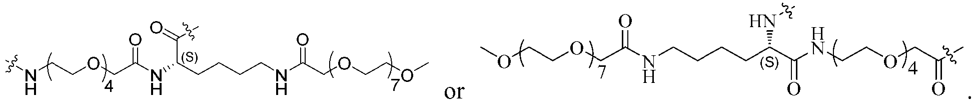

In any technical solution of formula III in the present invention, L₃ is a peptide fragment consisting of 2-4 amino acids selected from glycine, D-phenylalanine, D-valine, D-alanine, D-asparagine and citrulline, and the -NH- terminus of L₃ is linked to L₂.

In any technical solution of formula III in the present invention, L₃ is selected from -Val-Ala-, -Val-Cit- or -Gly-Gly-Phe-Gly-, wherein the -NH- terminus of L₃ is linked to L₂.

In any technical solution of formula III in the present invention, q is selected from 7.5-8.5, preferably 7.5-8.0.

In any technical solution of formula III in the present invention, q is selected from 4.0-5.0, preferably 4.0-4.5.

In any technical solution of formula III in the present invention, La is selected from 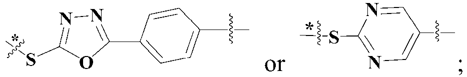 X₁ is selected from O or CH₂; L₂ is selected from a chemical bond, 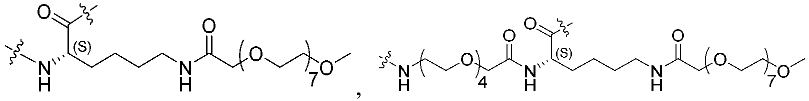 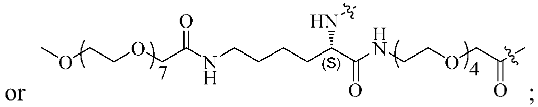 L₃ is selected from -Val-Ala-, -Val-Cit- or -Gly-Gly-Phe-Gly-, wherein the -NH- terminus of L₃ is linked to L₂; and q is selected from 7.5-8.5.

In one technical solution of the present invention, the antibody drug conjugate as shown in formula I or III or the pharmaceutically acceptable salt thereof is an antibody drug conjugate as shown in the following formula IV or a pharmaceutically acceptable salt thereof: 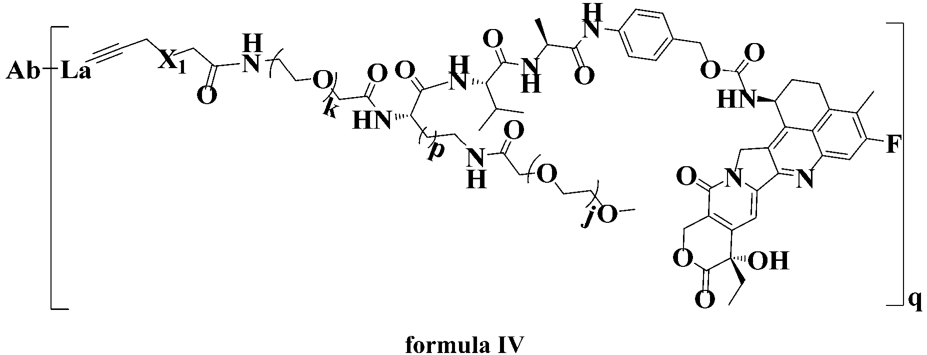 wherein, Ab, La, X₁, k, p, j and q have the same meanings as defined in formula I, formula III or any other technical solution.

In any technical solution of formula IV in the present invention, La is selected from 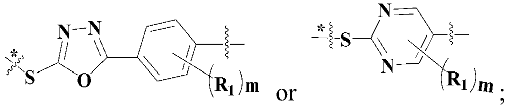 and R₁ and m have the same meanings as defined in formula I or any other technical solution.

In any technical solution of formula IV in the present invention, La is selected from

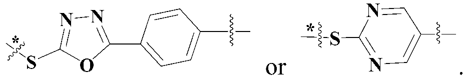

In any technical solution of formula IV in the present invention, X₁ is CH₂ or O.

In any technical solution of formula IV in the present invention, k is selected from 3, 4 or 5, preferably 4; p is selected from 2, 3 or 4, preferably 3; and j is selected from 6, 7 or 8, preferably 7.

In any technical solution of formula IV in the present invention, q is selected from 7-9, preferably 7.5-8.5, and more preferably 7.5-8.0.

In any technical solution of formula IV in the present invention, q is selected from 4.5-5.5, preferably 4.0-4.5.

In any technical solution of formula IV in the present invention, La is selected from 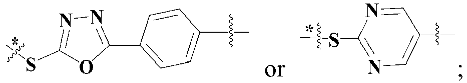 X₁ is CH₂ or O; and k is 4, p is 3, j is 7, and q is 7.5-8.0.

In any technical solution of formula III or IV in the present invention, Ab is selected from an anti-Her2 antibody, an anti-Trop2 antibody, an anti-Claudin18.2 antibody, an anti-LIV-1 antibody, an anti-5T4 antibody, an anti-CLDN6 antibody, an anti-CDH6 antibody or an anti-folate receptor antibody.

In any technical solution of formula III or IV in the present invention, the anti-Her2 antibody is selected from Trastuzumab or Pertuzumab.

In any technical solution of formula III or IV in the present invention, the anti-LIV-1 antibody comprises HCDR1, HCDR2 and HCDR3 as shown in SEQ ID NO: 1, SEQ ID NO: 2 and SEQ ID NO: 3, and LCDR1, LCDR2 and LCDR3 as shown in SEQ ID NO: 6, SEQ ID NO: 7 and SEQ ID NO: 8.

In any technical solution of formula III or IV in the present invention, the anti-LIV-1 antibody comprises the heavy chain variable region as shown in SEQ ID NO: 4 and the light chain variable region as shown in SEQ ID NO: 9.

In any technical solution of formula III or IV in the present invention, the anti-LIV-1 antibody comprises the heavy chain as shown in SEQ ID NO: 5 and the light chain as shown in SEQ ID NO: 10.

In one technical solution of the present invention, the antibody drug conjugate as shown in formula I or the pharmaceutically acceptable salt thereof is selected from the following antibody drug conjugates or pharmaceutically acceptable salts thereof: 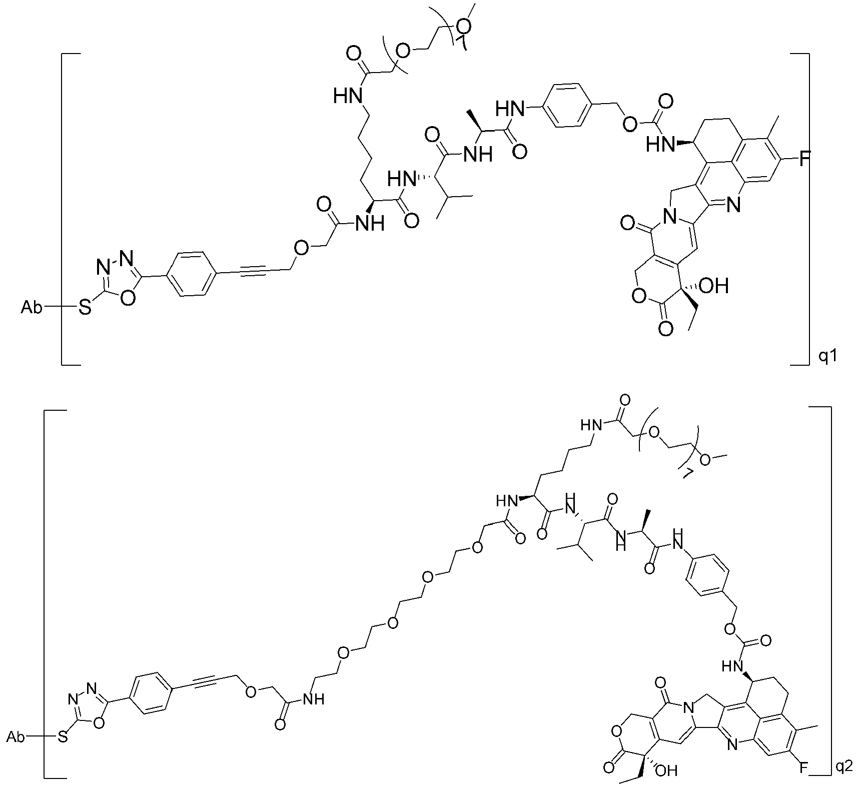 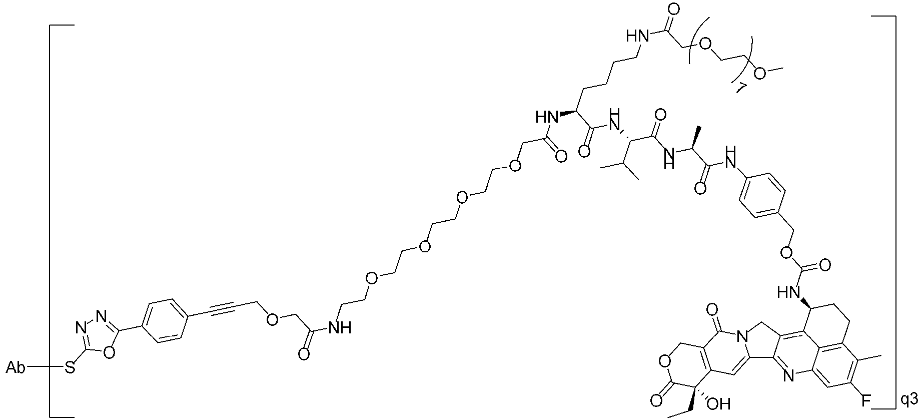 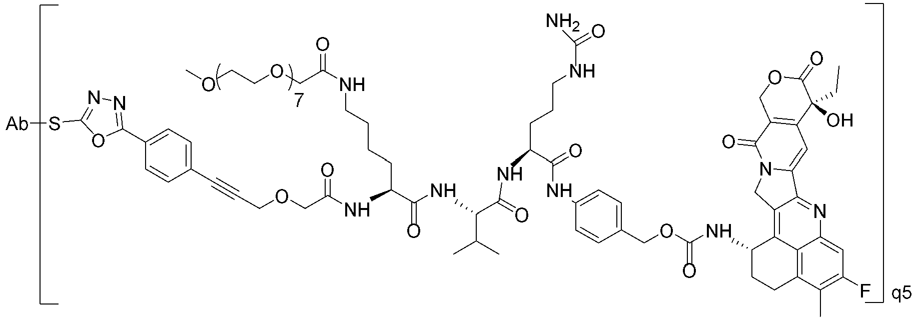 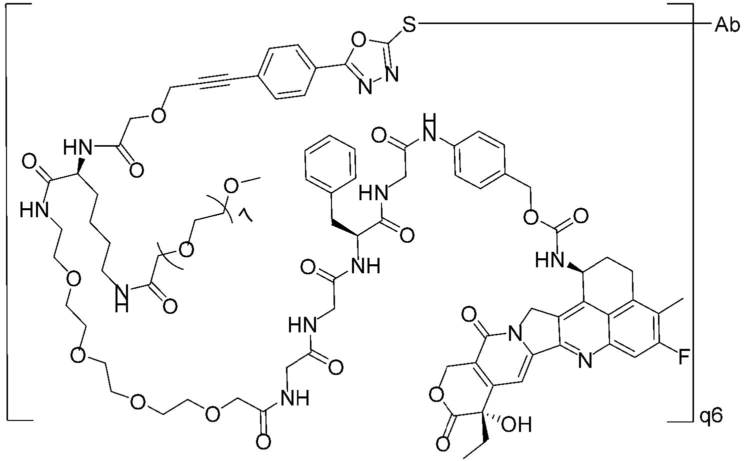 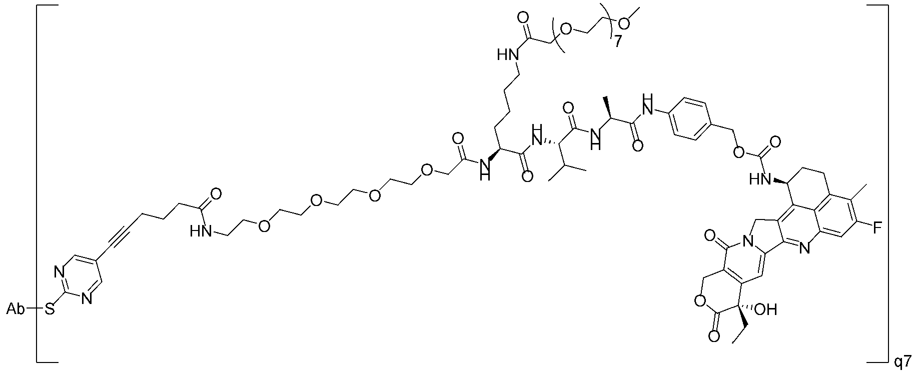 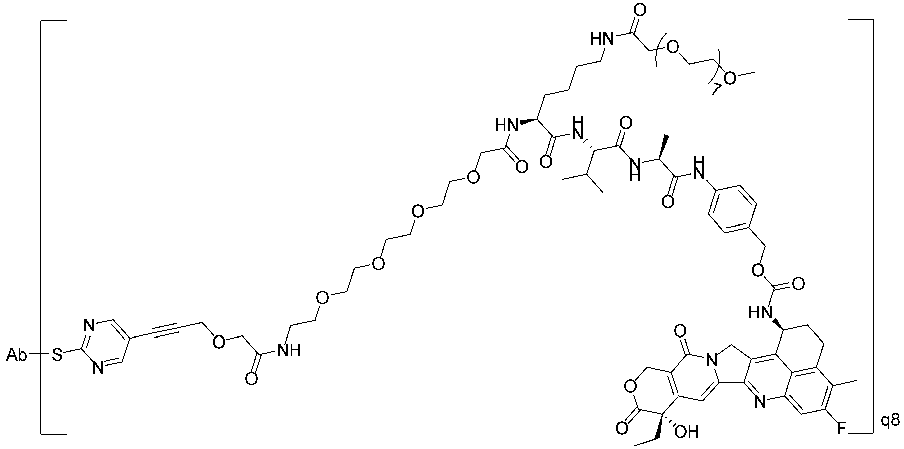 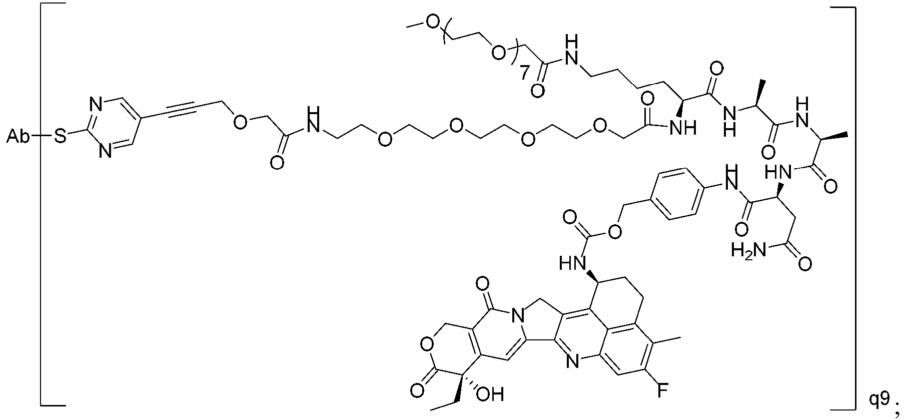 wherein, q1, q2, q4, q5, q6, q7, q8 and q9 are respectively independently selected from 7.5-8.0, preferably 7.6-7.7; q3 is selected from 4.0-5.0, preferably 4.2-4.6, and Ab is respectively independently selected from Ladiratuzumab or Trastuzumab.

In a second aspect, the present invention provides a compound as shown in formula II or a pharmaceutically acceptable salt thereof: 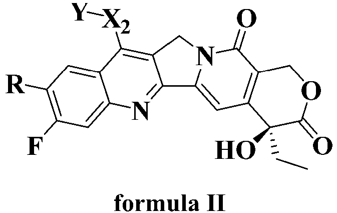 wherein, R is selected from F, C₁₋₄ alkoxy, C₁₋₄ haloalkyl or C₁₋₄ haloalkoxy; X₂ is selected from -(CH₂)s-, and s is selected from 0, 1, 2, 3 or 4; Y is selected from -OH or -NH-Rc, and Rc is selected from H, C₁₋₄ alkyl, 3-5 membered cycloalkyl or 3-5 membered heterocycloalkyl; and when s=1, Rc is not H.

In any technical solution of formula II in the present invention, R is F, or R is methoxy.

In any technical solution of formula II in the present invention, s is 1, or s is 2, or s is 3.

In any technical solution of formula II in the present invention, Rc is selected from H, methyl or cyclopropyl.

In any technical solution of formula II in the present invention, Y is selected from -OH.

In any technical solution of formula II in the present invention, Y is -NH-Rc, and Rc is methyl or cyclopropyl.

In any technical solution of formula II in the present invention, the compound as shown in formula II is selected from the following compounds:

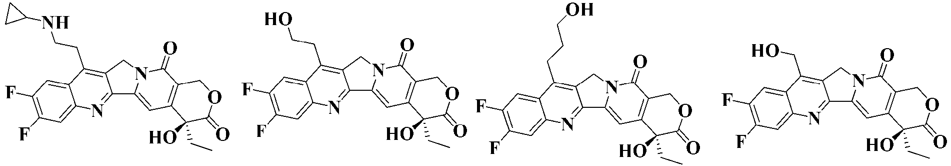

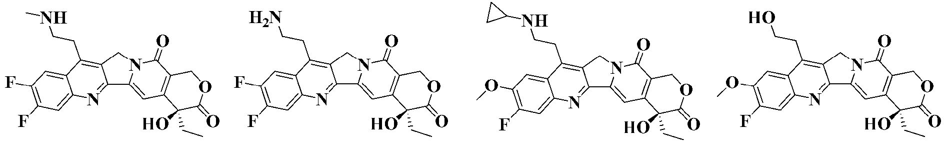

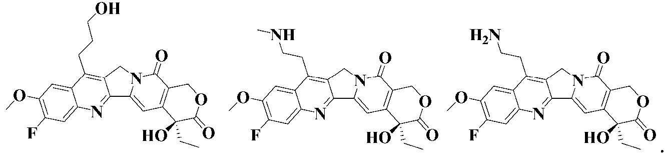

In a third aspect, the present invention provides compounds as follows or pharmaceutically acceptable salts thereof:

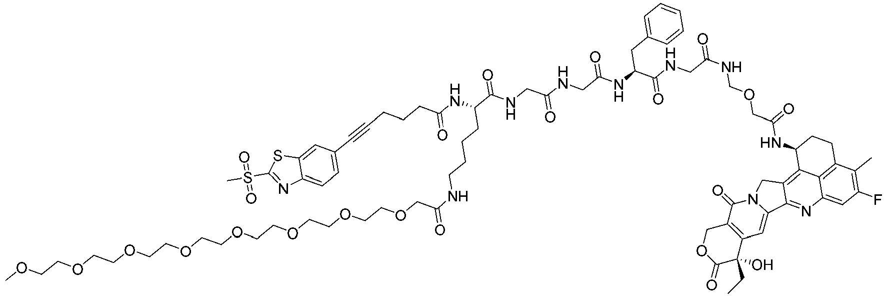

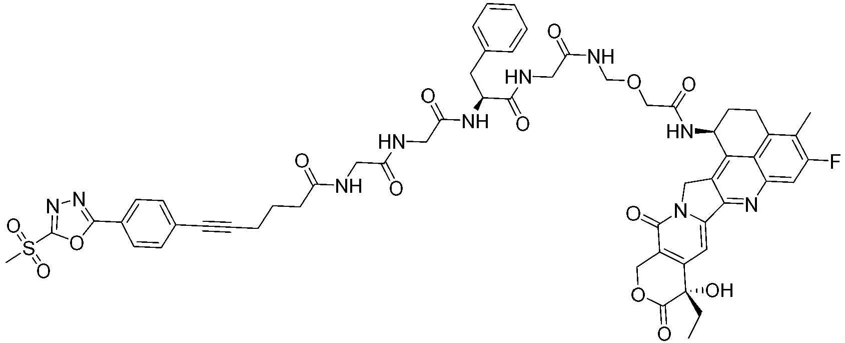

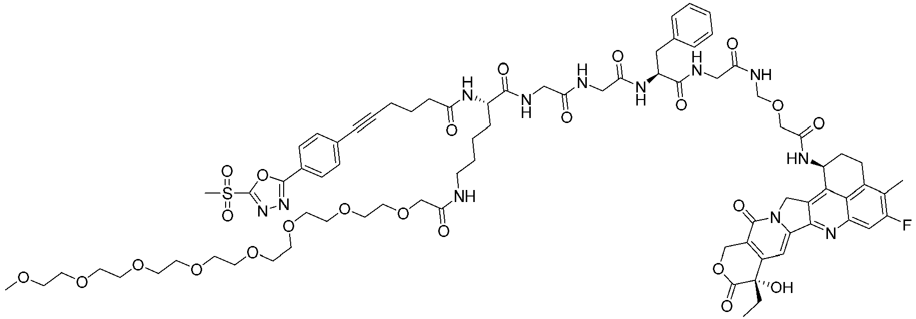

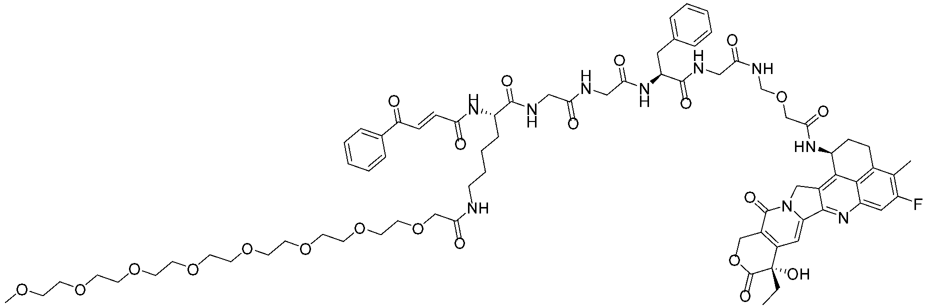

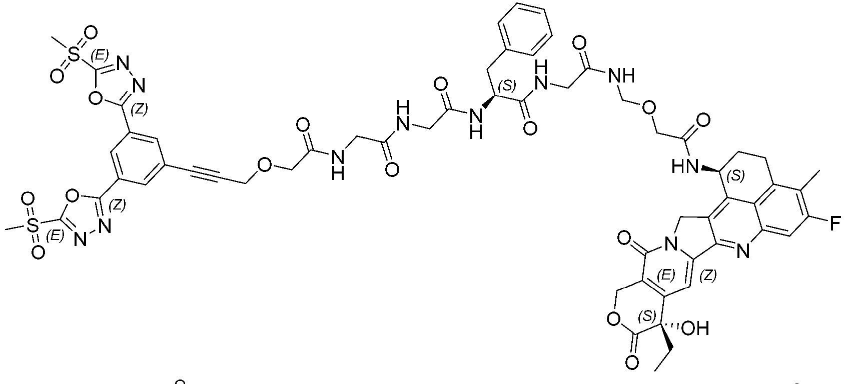

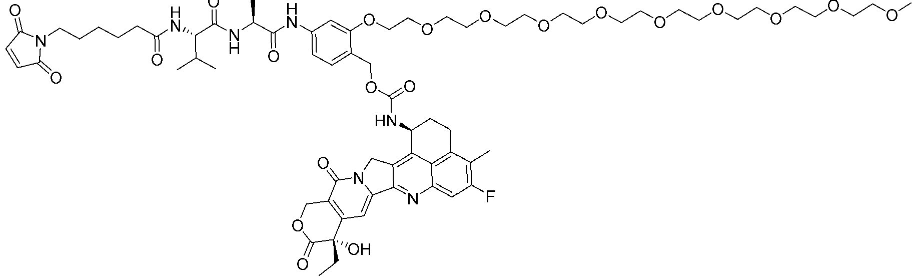

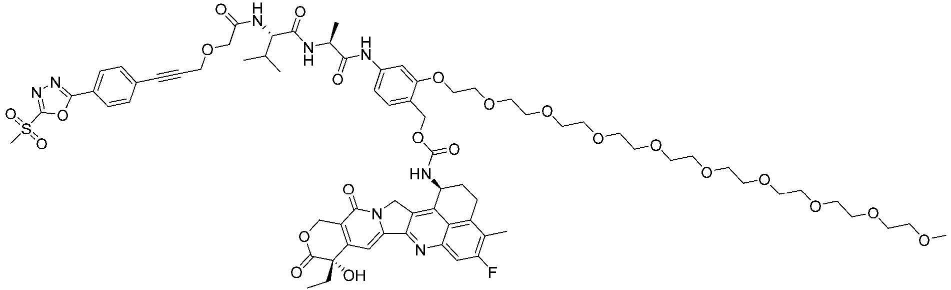

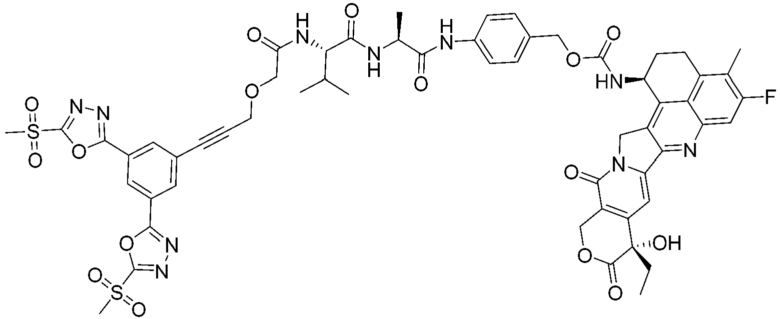

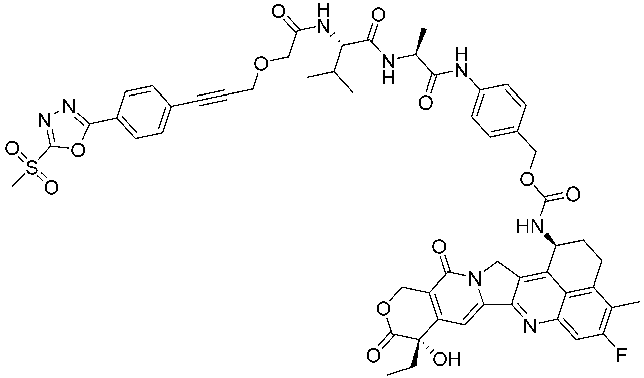

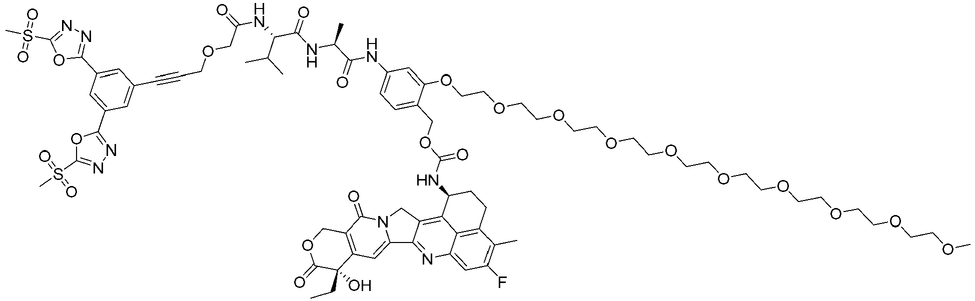

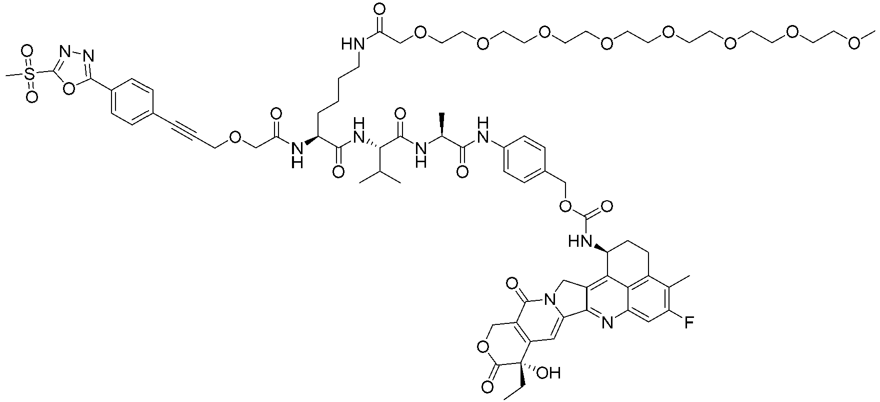

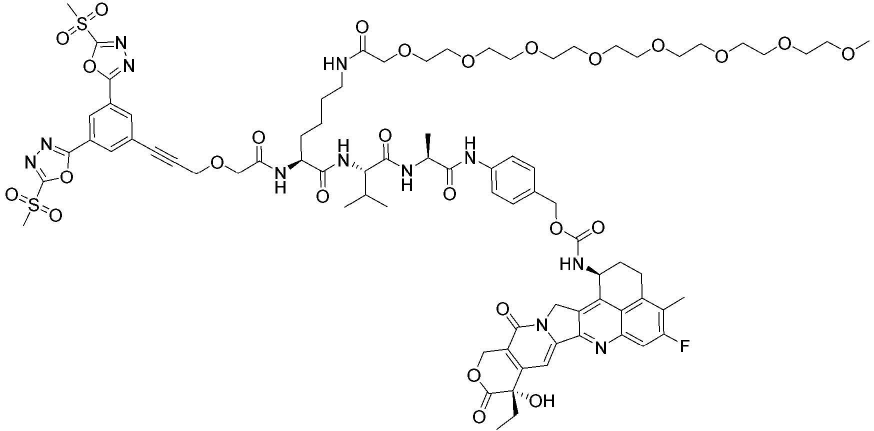

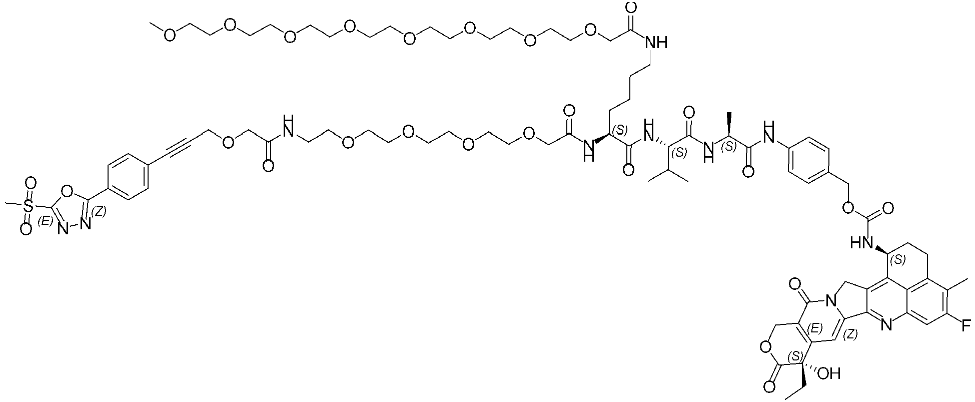

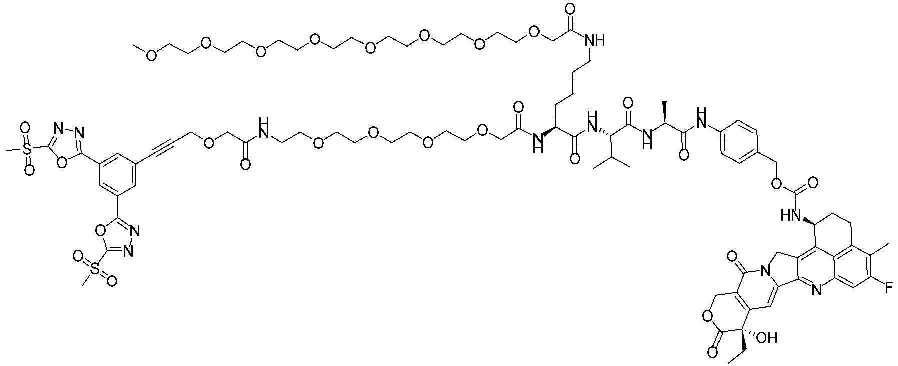

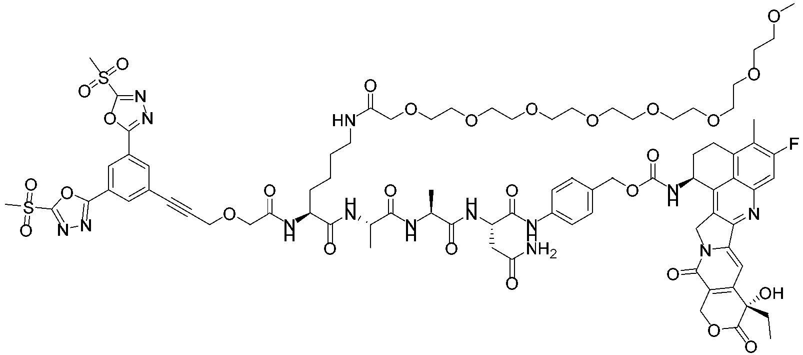

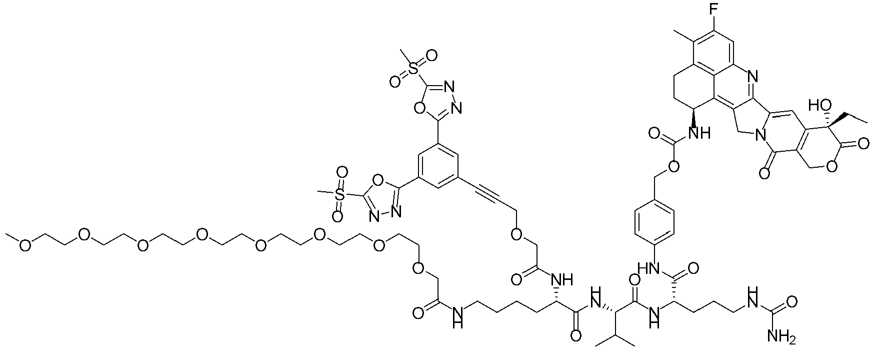

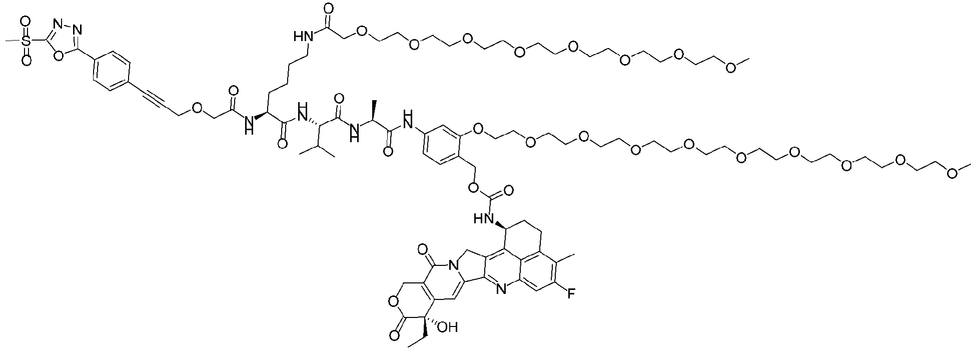

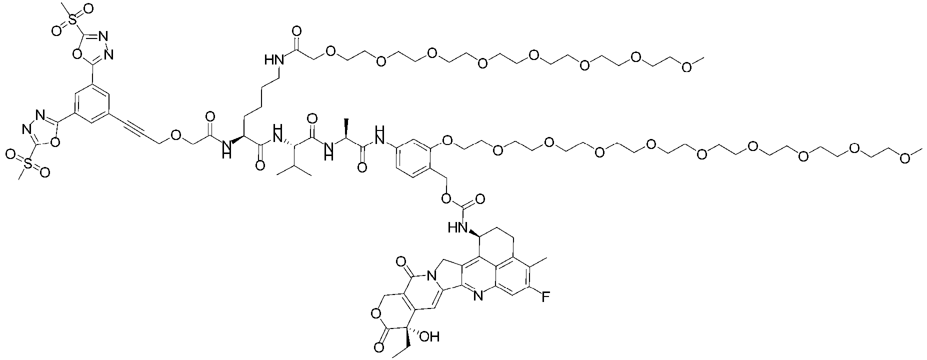

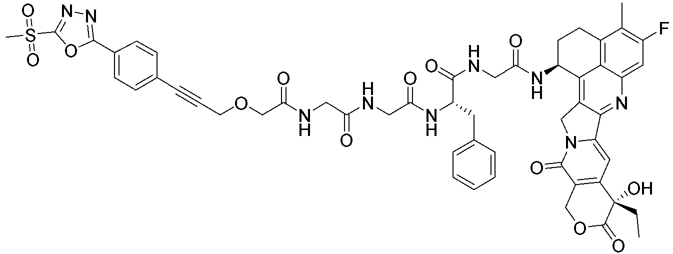

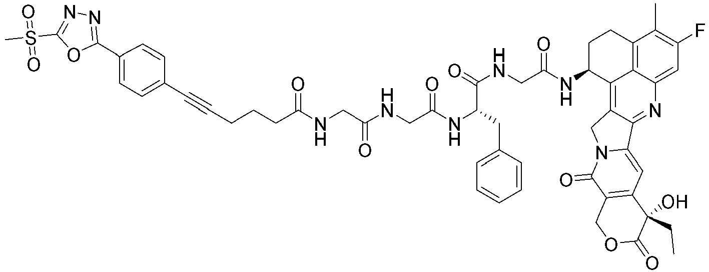

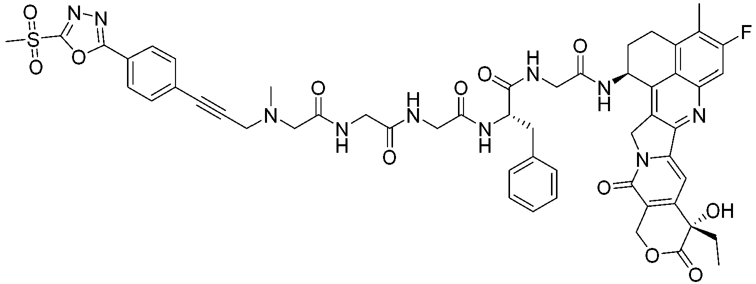

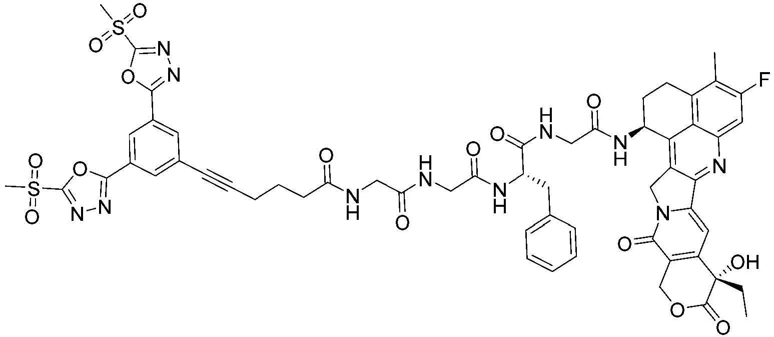

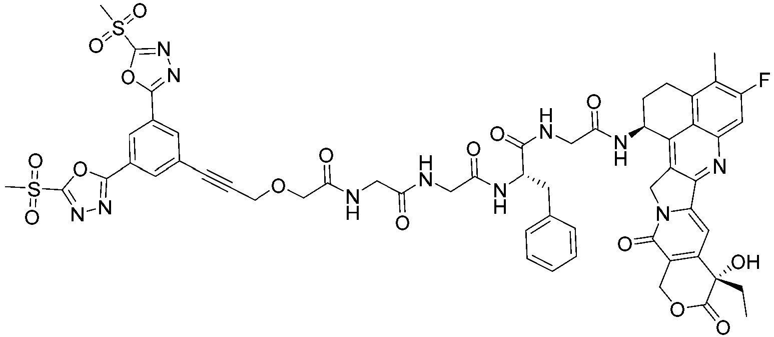

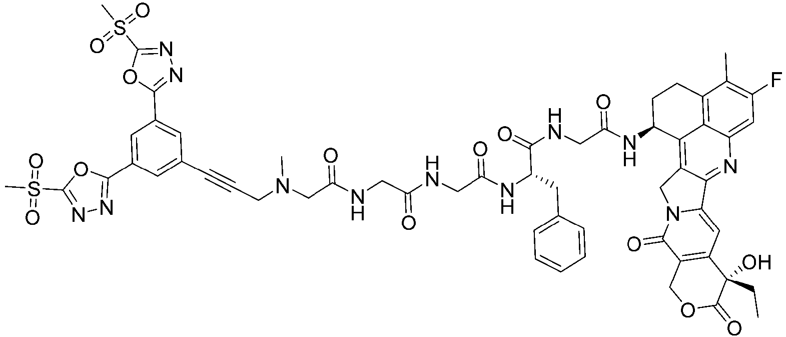

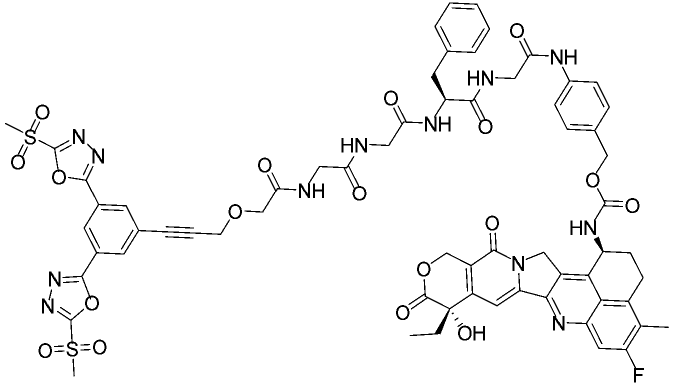

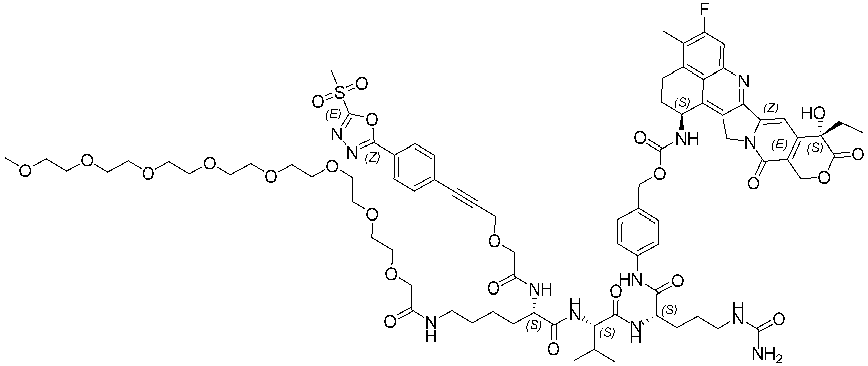

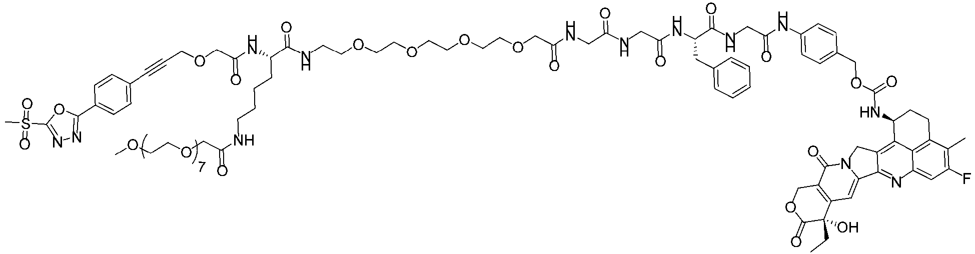

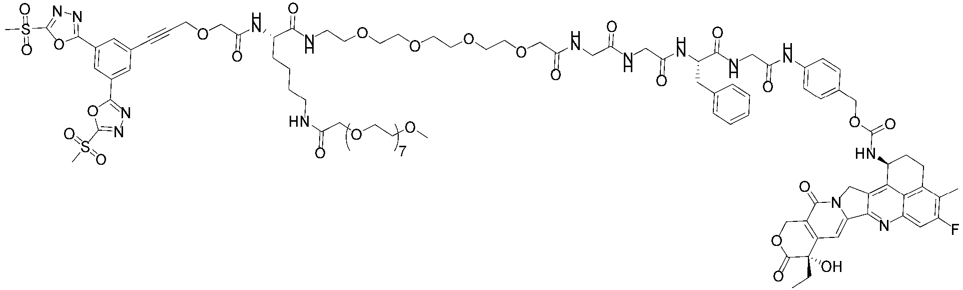

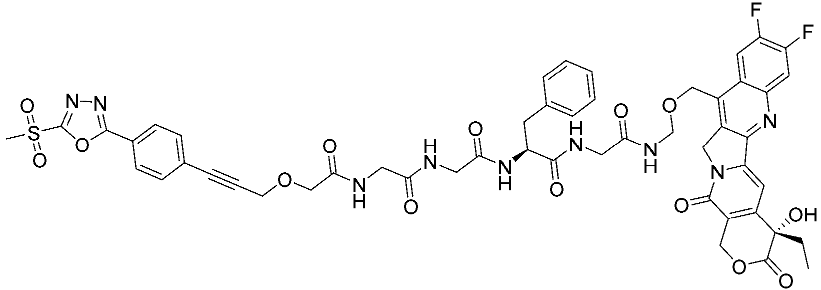

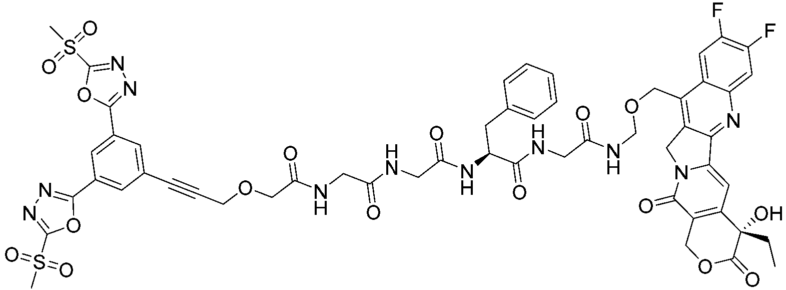

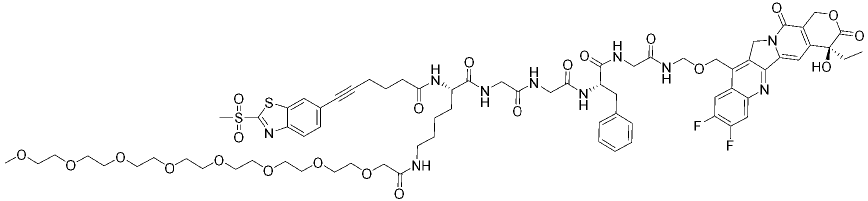

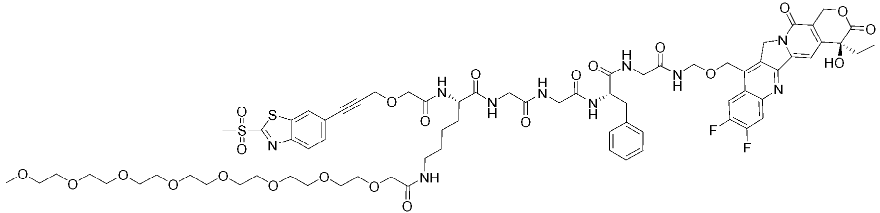

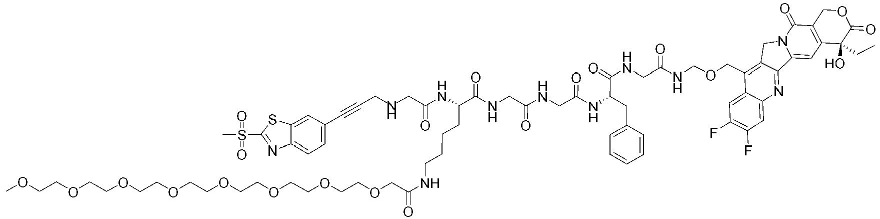

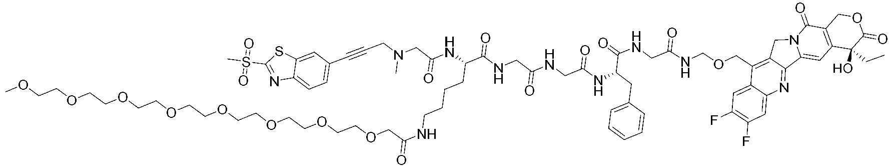

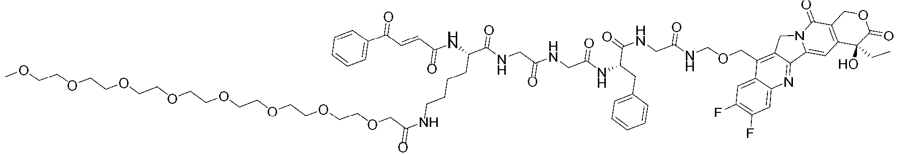

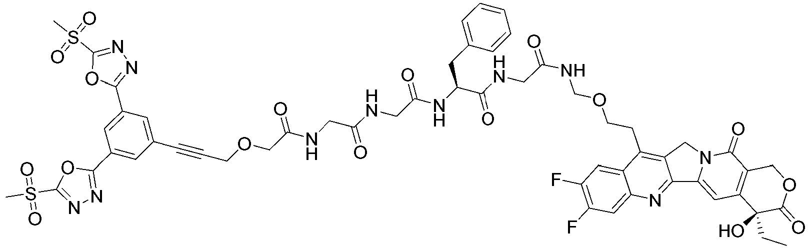

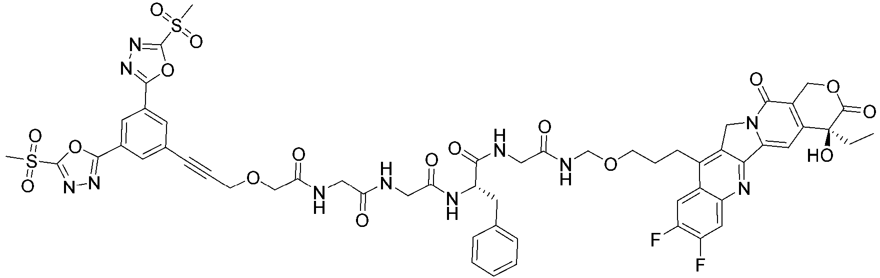

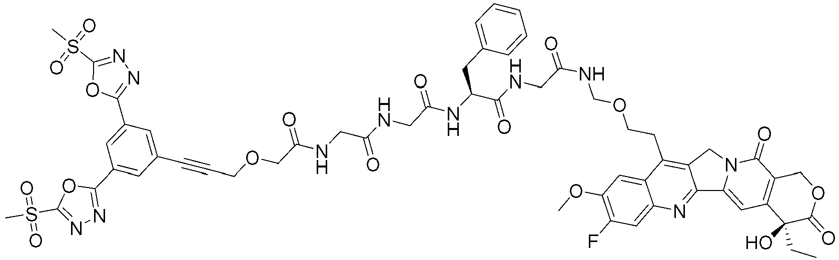

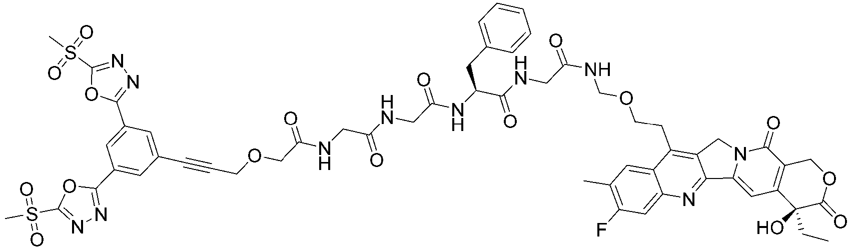

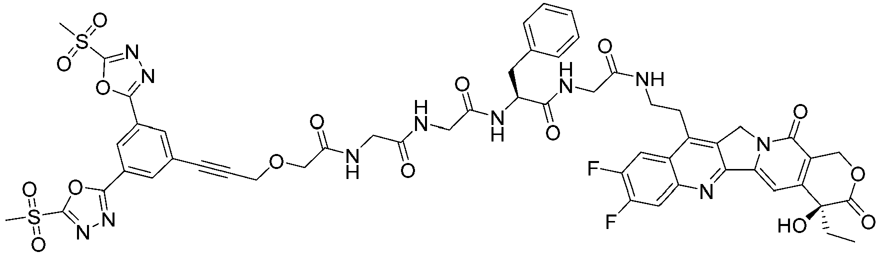

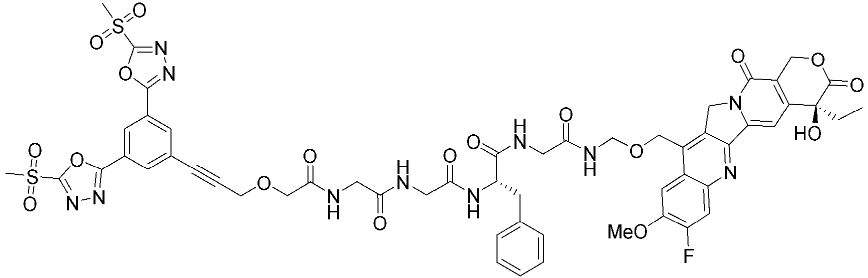

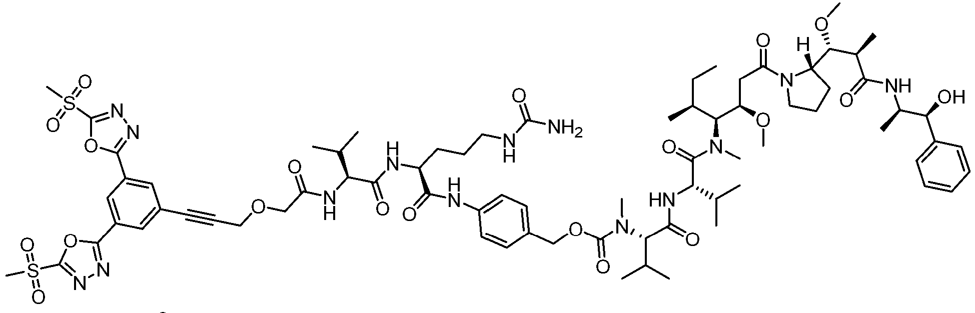

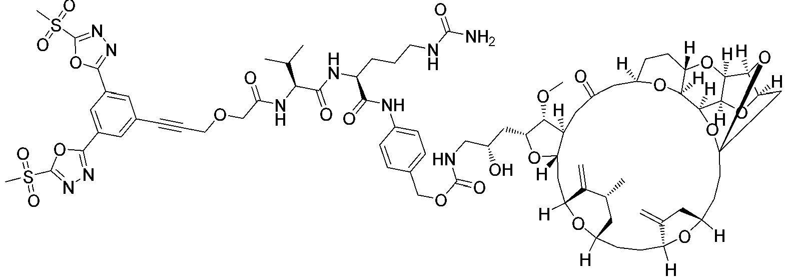

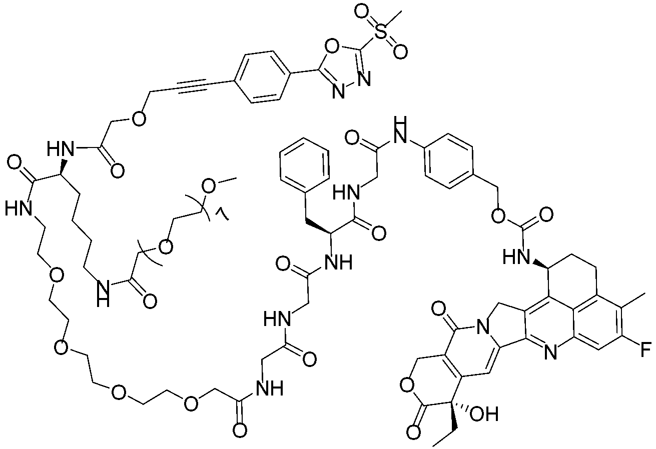

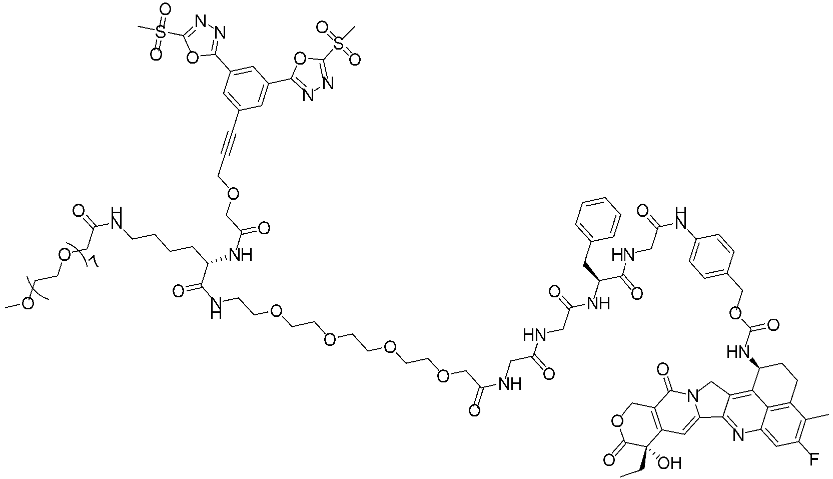

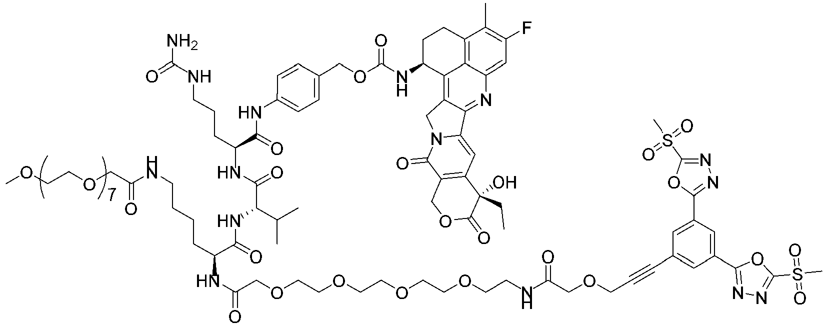

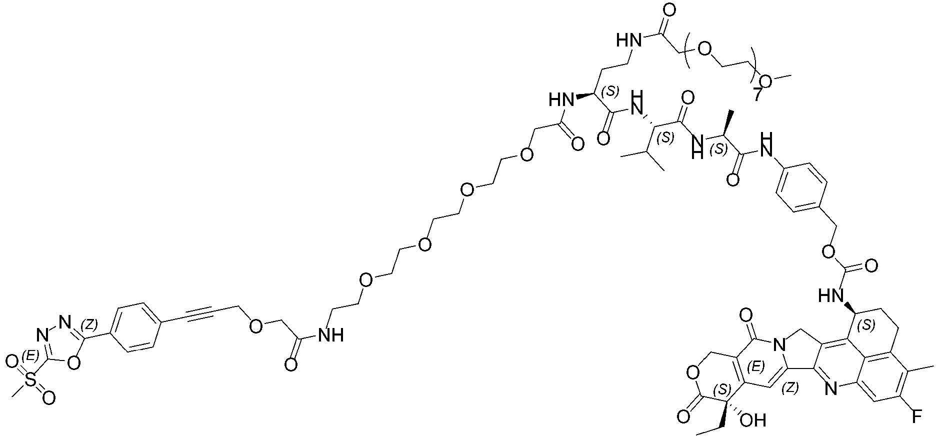

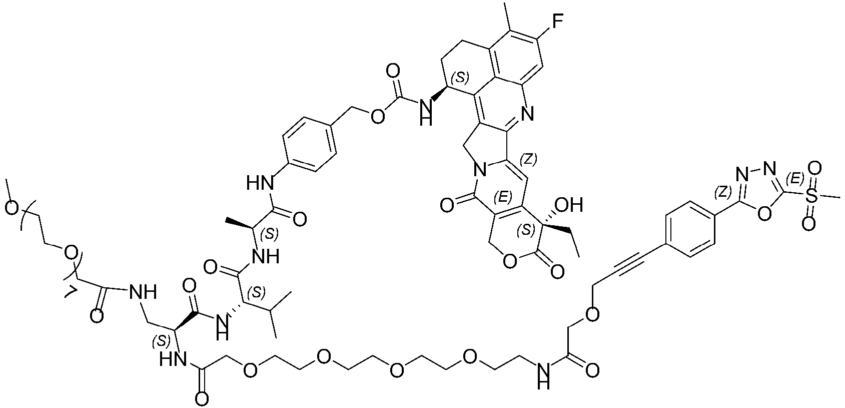

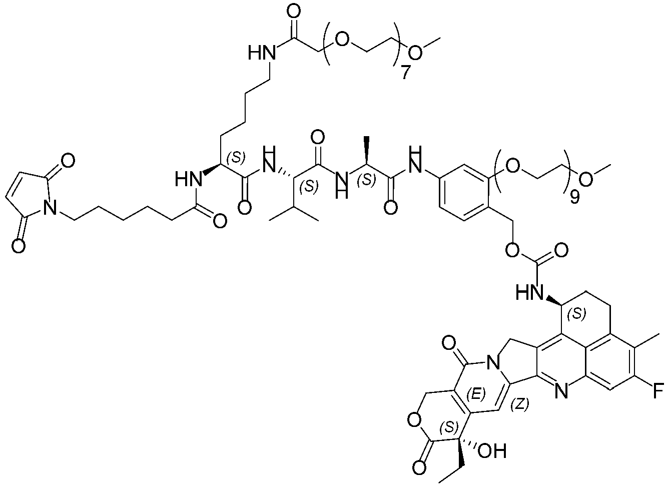

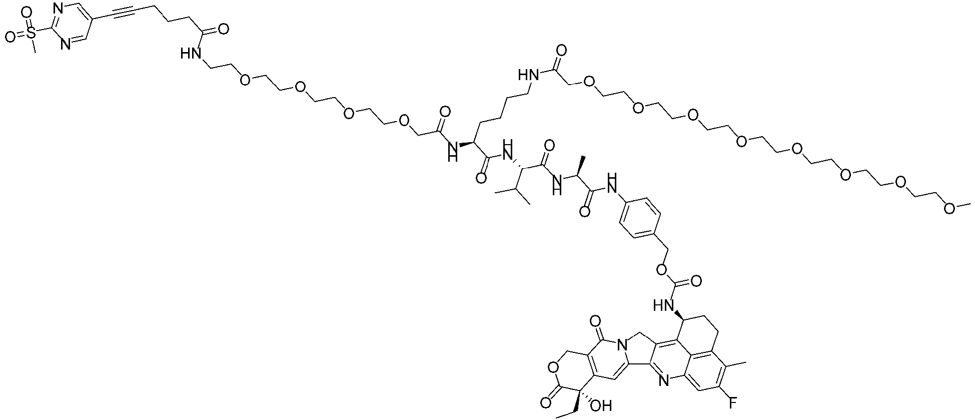

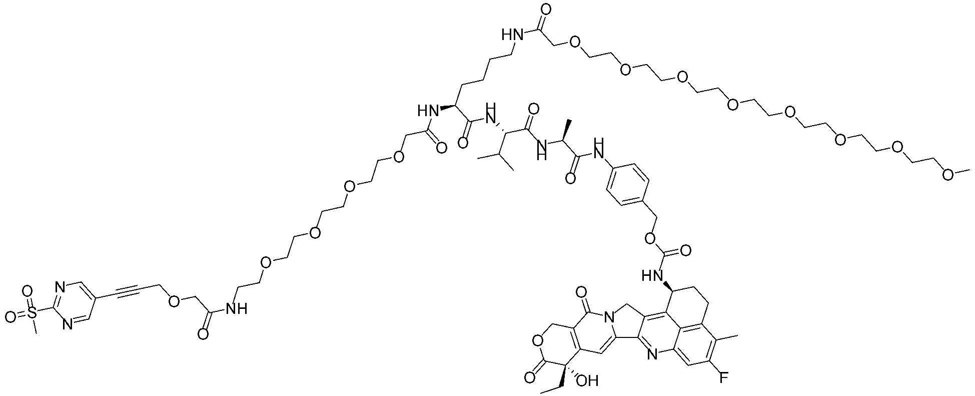

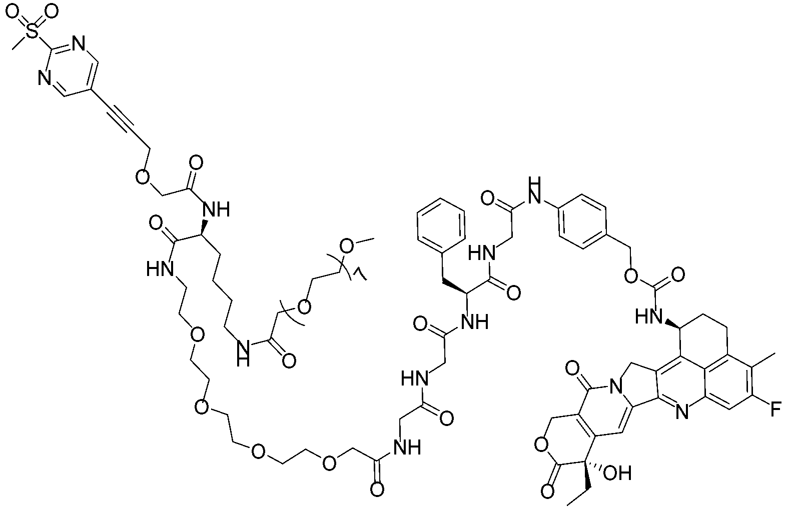

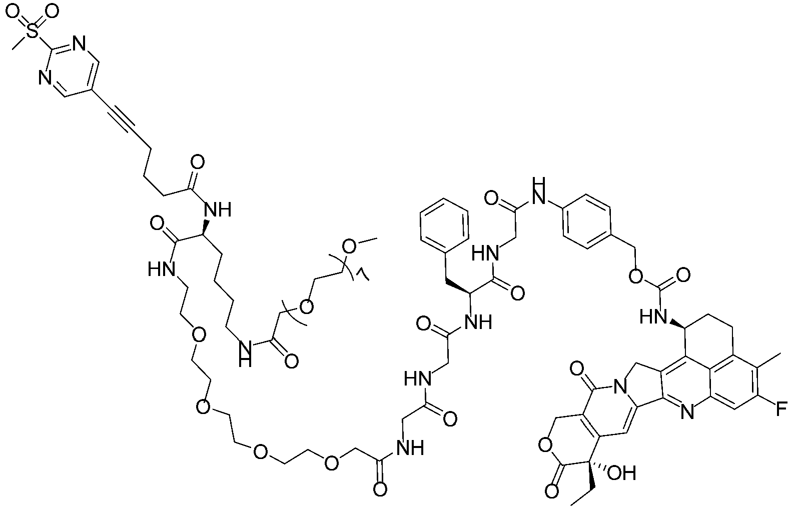

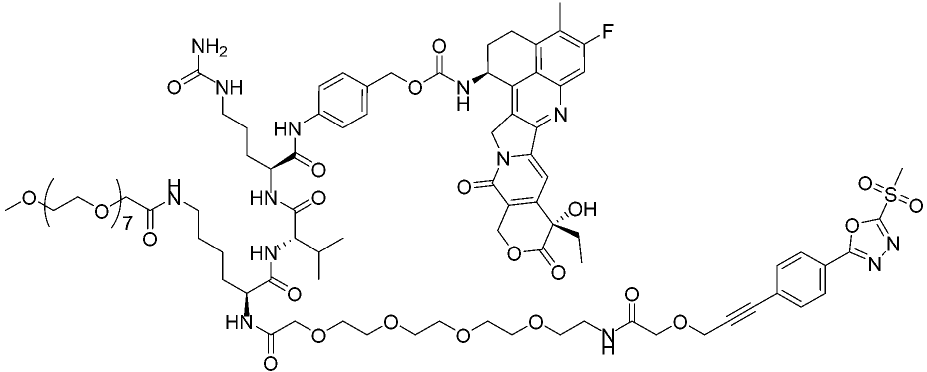

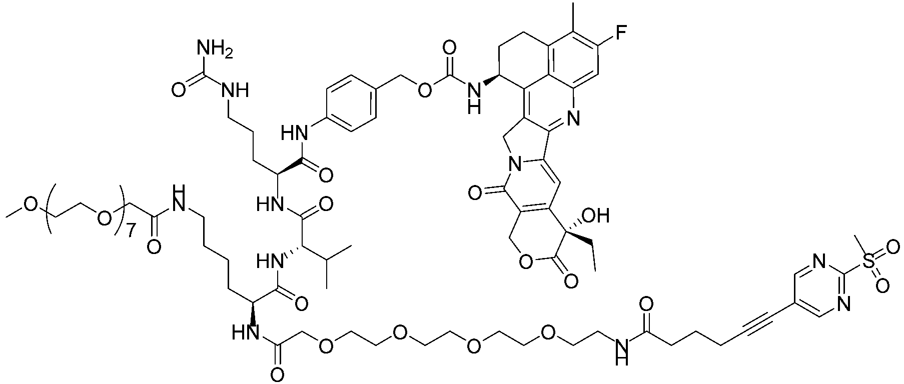

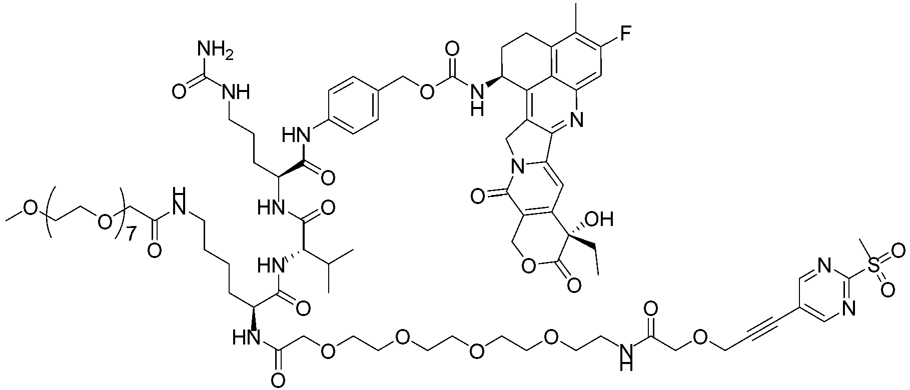

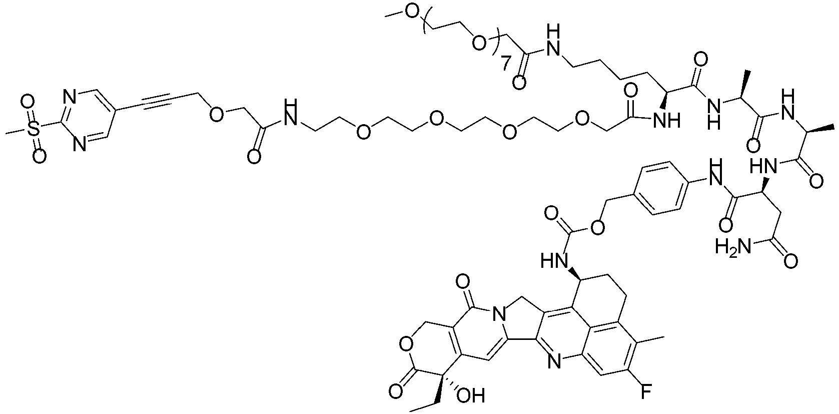

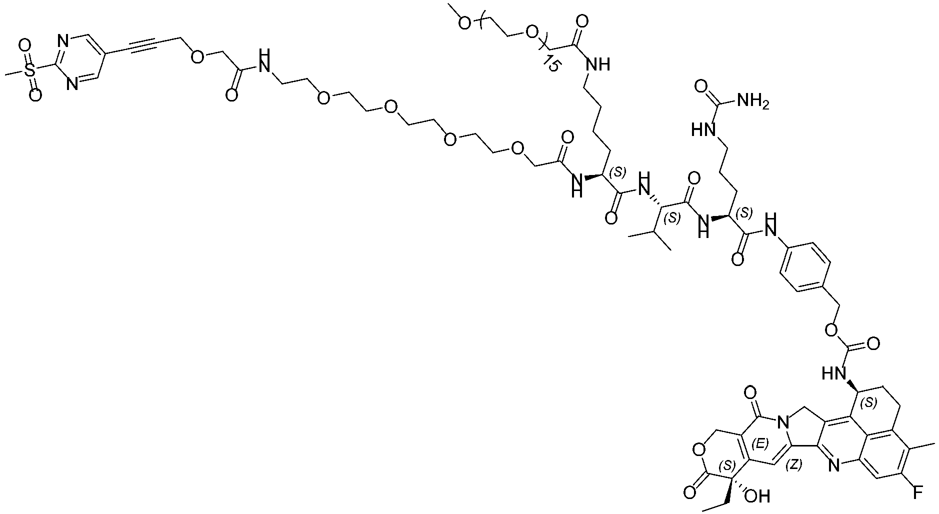

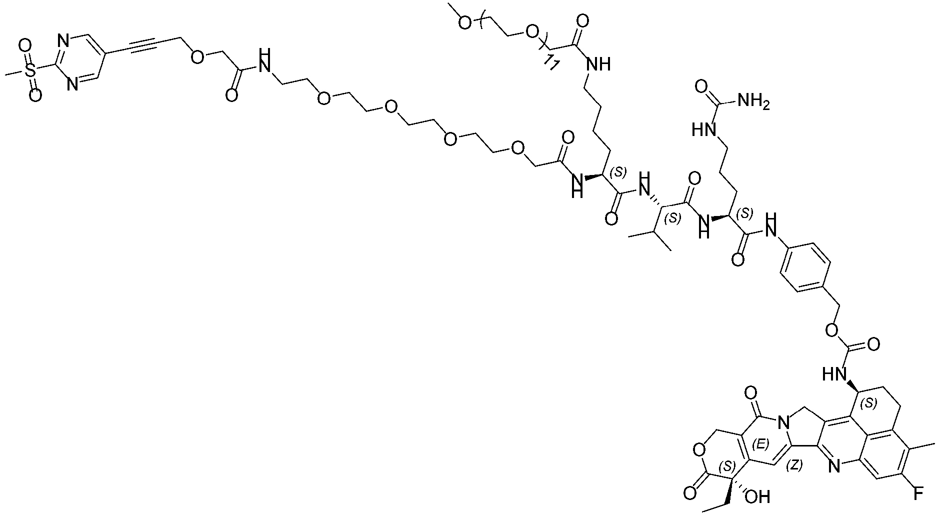

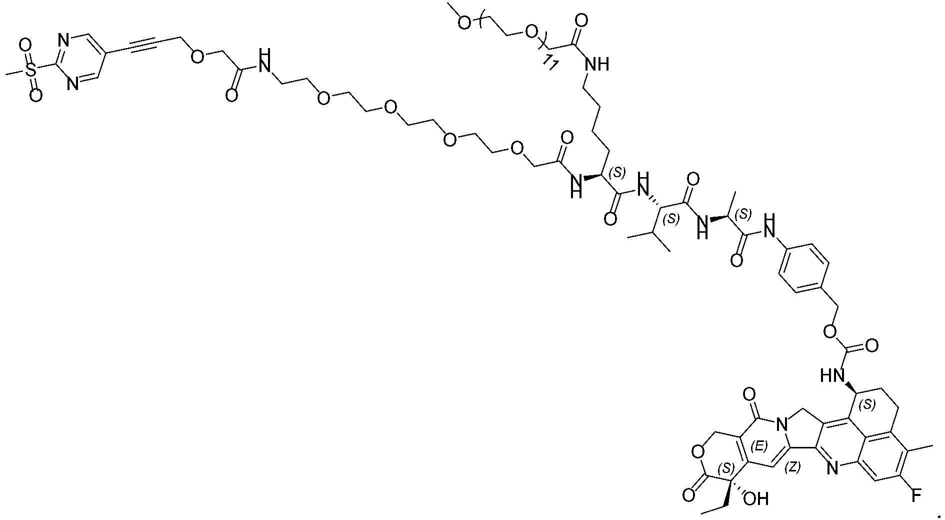

In one technical solution of the present invention, the present invention provides a compound as shown in formula IIIa or a pharmaceutically acceptable salt thereof: 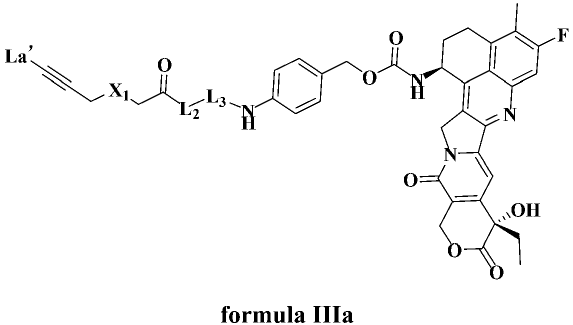 wherein, X₁, L₂ and L₃ have the same meanings as defined in formula I, formula III or any other technical solution, and La' is selected from the following groups: 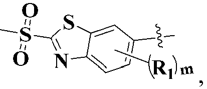 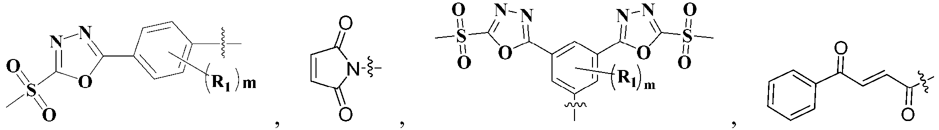 or 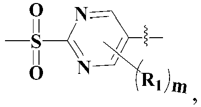 wherein R₁ is respectively independently selected from H, halogen atom, -CN, -OH, -NH₂, C₁₋₄ alkyl, C₁₋₄ haloalkyl, C₁₋₄ alkoxy, C₁₋₄ haloalkoxy or hydroxy-C₁₋₄ alkyl, and m is selected from 0, 1, 2 or 3.

In any technical solution of formula IIIa in the present invention, La' may be selected from 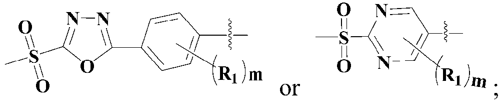 and R₁ and m have the same meanings as defined in formula I or any other technical solution.

In any technical solution of formula IIIa in the present invention, La' may be selected from

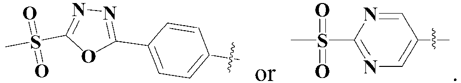

In any technical solution of formula IIIa in the present invention, X₁ is selected from O or CH₂.

In any technical solution of formula IIIa in the present invention, L₂ is selected from a chemical bond,

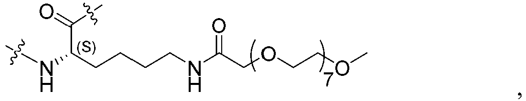

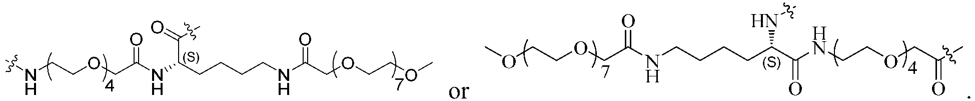

In any technical solution of formula IIIa in the present invention, L₃ is a peptide fragment consisting of 2-4 amino acids selected from glycine, D-phenylalanine, D-valine, D-alanine, D-asparagine and citrulline, and the -NH- terminus of L₃ is linked to L₂.

In any technical solution of formula IIIa in the present invention, L₃ is selected from -Val-Ala-, -Val-Cit- or -Gly-Gly-Phe-Gly-, wherein the -NH- terminus of L₃ is linked to L₂.

In any technical solution of formula IIIa in the present invention, La' may be selected from 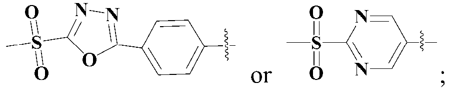 X₁ is selected from O or CH₂; L₂ is selected from a chemical bond, 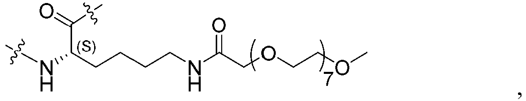 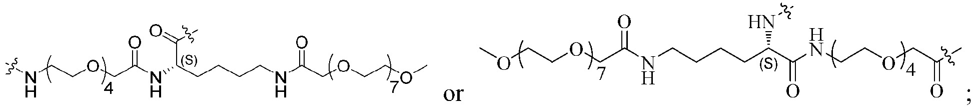 and L₃ is selected from -Val-Ala-, -Val-Cit- or -Gly-Gly-Phe-Gly-, wherein the -NH- terminus of L₃ is linked to L₂.

In one technical solution of the present invention, the present invention provides a compound as shown in formula IVa or a pharmaceutically acceptable salt thereof: 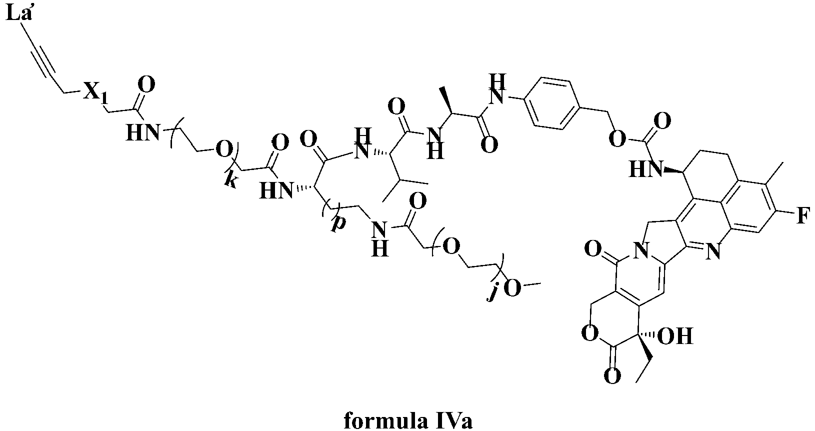 wherein, X₁, k, p and j have the same meanings as defined in formula I, formula III, formula IIIa, formula IV or any other technical solution, and La' has the same meaning as defined in formula IIIa.

In any technical solution of formula IVa in the present invention, La' is selected from 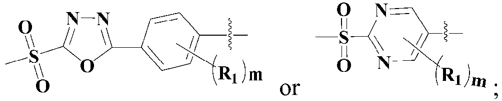 and R₁ and m have the same meanings as defined in formula I or any other technical solution.

In any technical solution of formula IVa in the present invention, La' is selected from

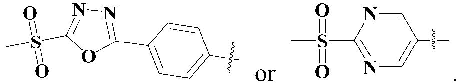

In any technical solution of formula IVa in the present invention, X₁ is CH₂ or O.

In any technical solution of formula IVa in the present invention, k is selected from 3, 4 or 5, preferably 4; p is selected from 2, 3 or 4, preferably 3; and j is selected from 6, 7 or 8, preferably 7.

In any technical solution of formula IVa in the present invention, q is selected from 7-9, preferably 7.5-8.5, and more preferably 7.5-8.0.

In any technical solution of formula IVa in the present invention, La' is selected from 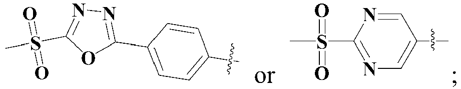 X₁ is CH₂ or O; and k is 4, p is 3, j is 7, and q is 7.5-8.0.

In any technical solution of formula IVa in the present invention, La' is selected from 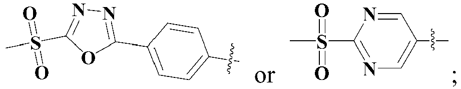 X₁ is CH₂ or O; and k is 4, p is 3, j is 7, and q is 4.5-5.0.

In a fourth aspect, the present invention provides the following structural fragments:

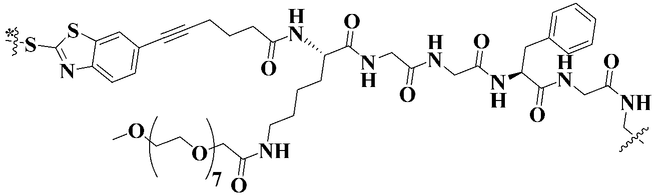

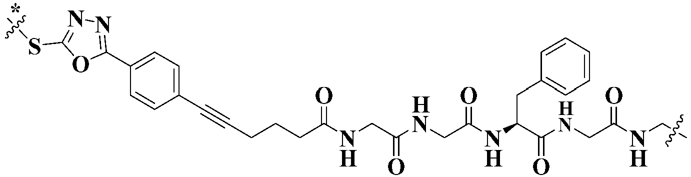

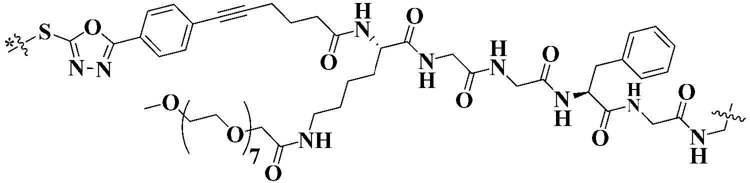

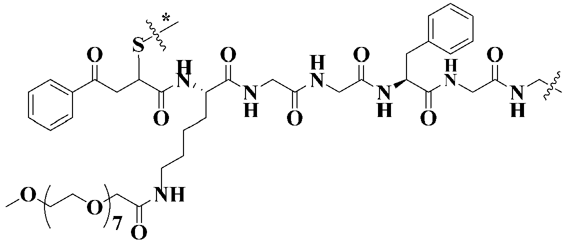

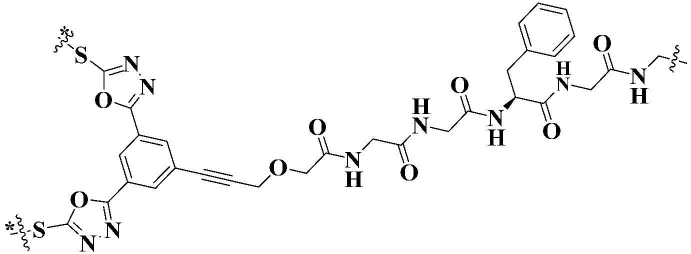

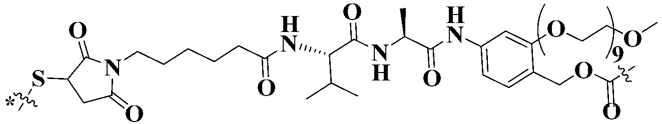

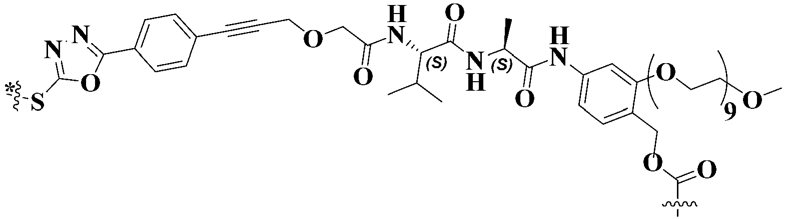

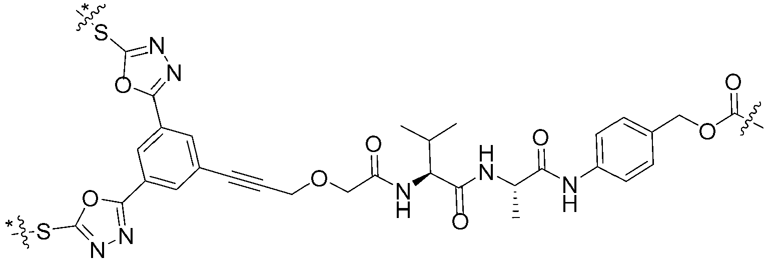

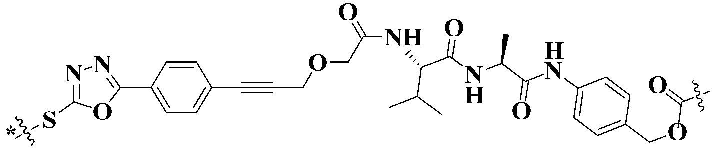

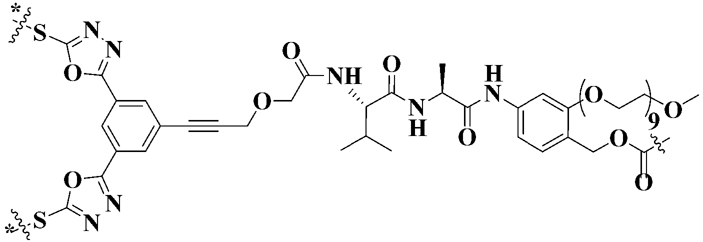

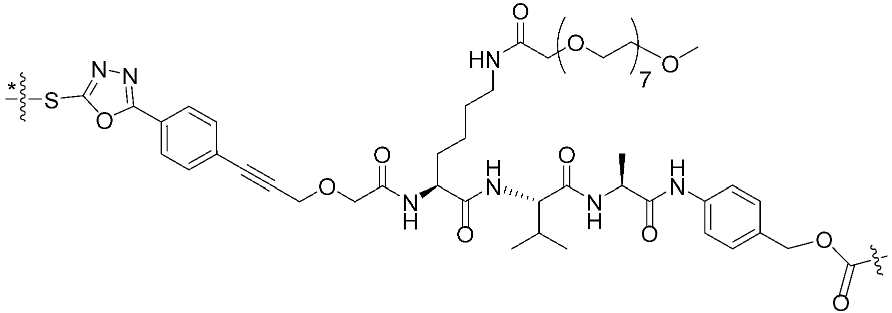

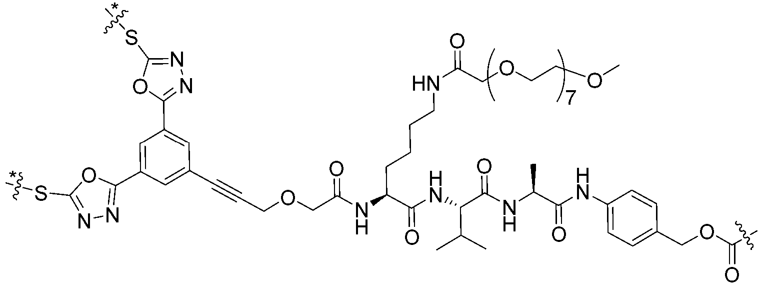

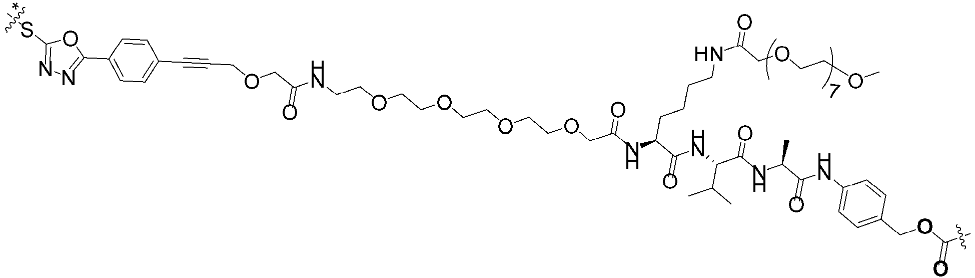

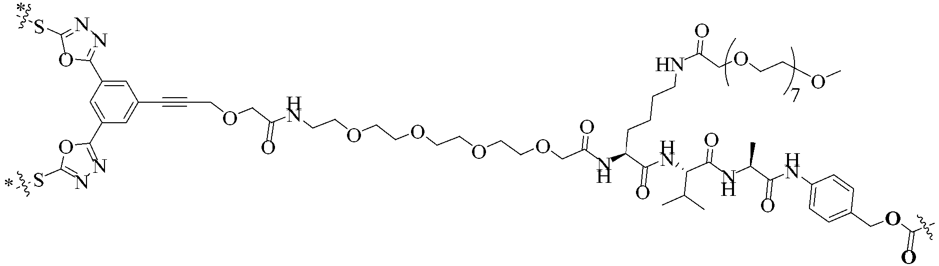

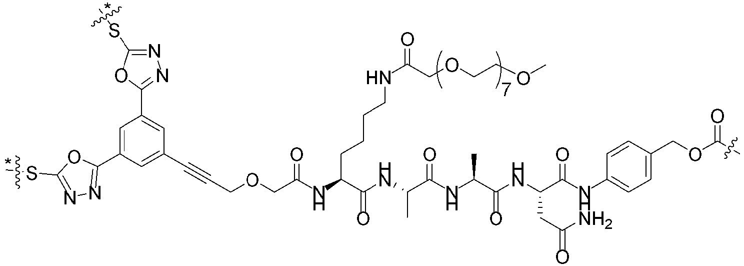

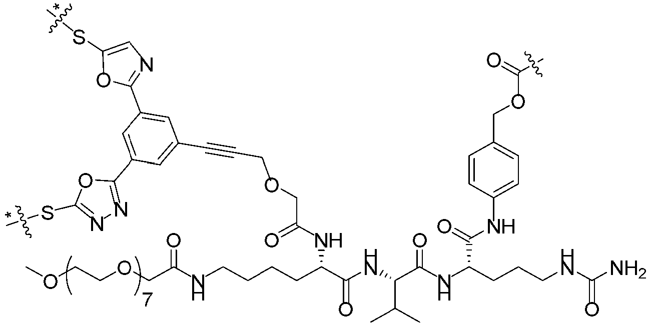

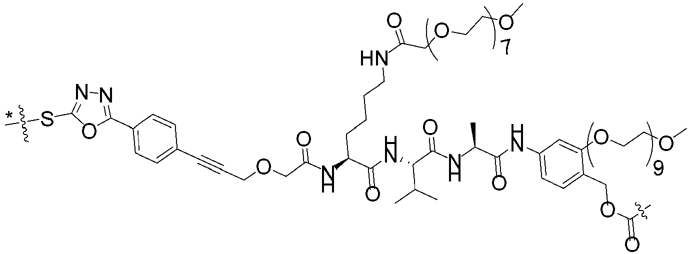

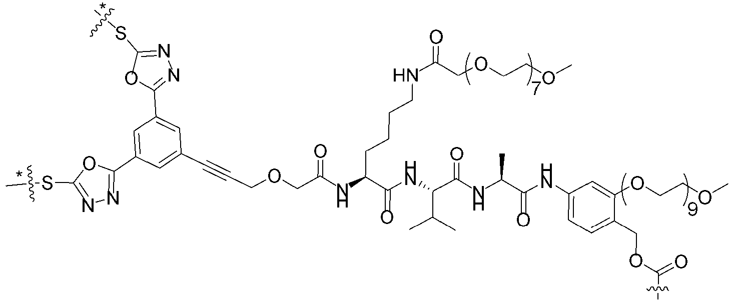

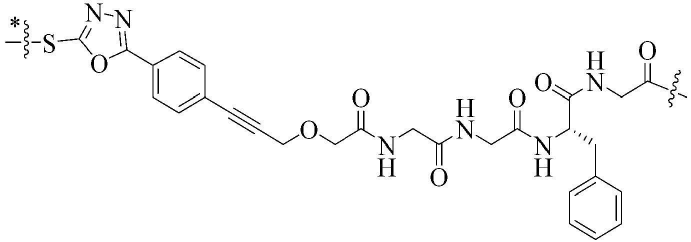

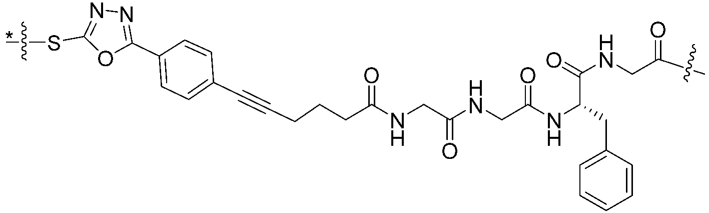

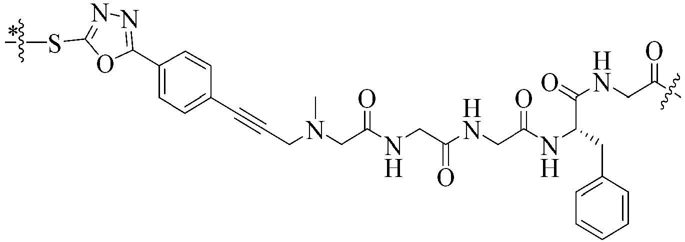

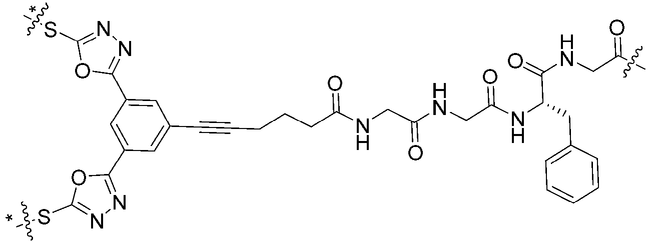

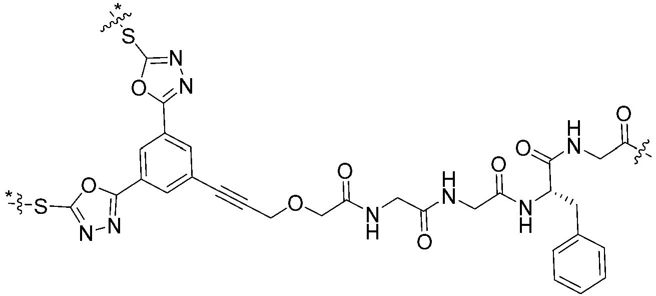

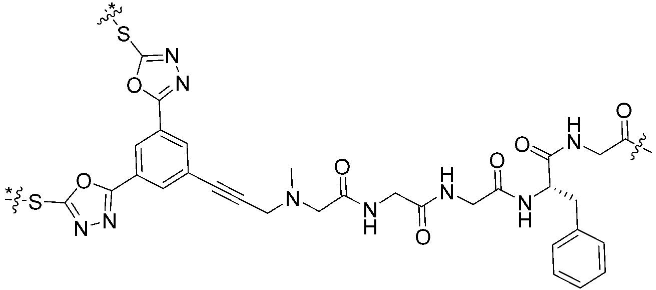

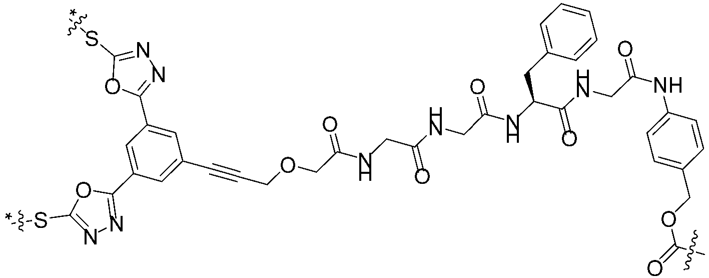

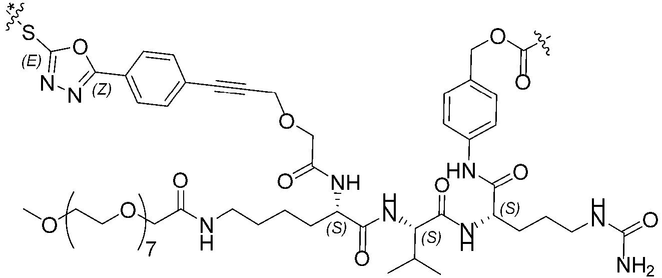

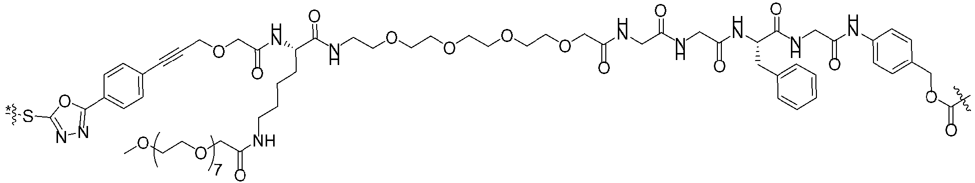

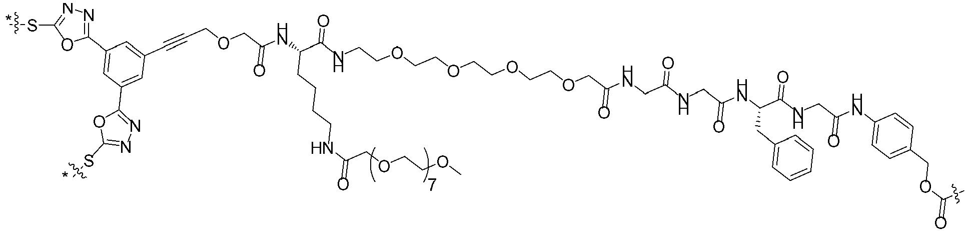

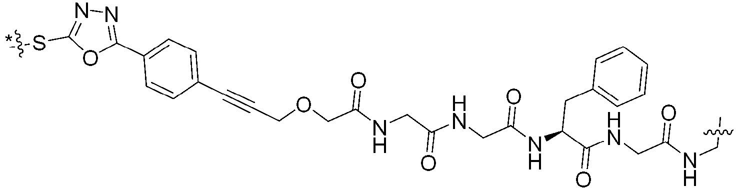

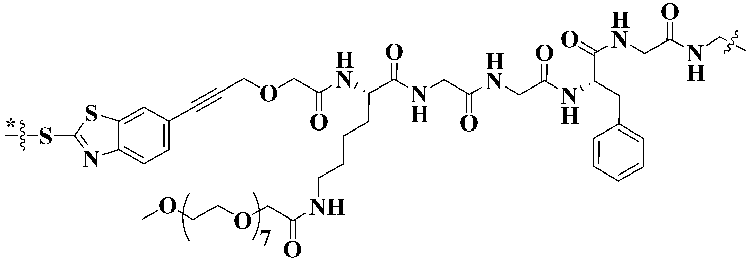

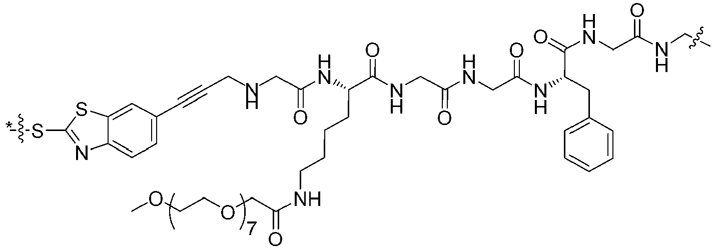

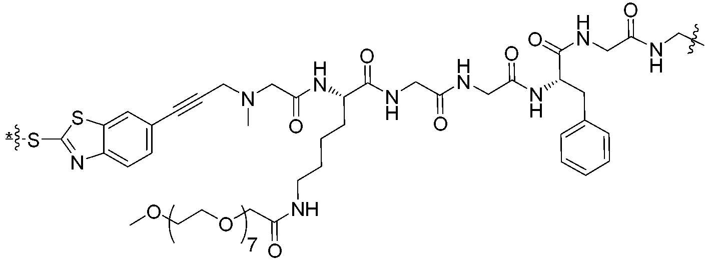

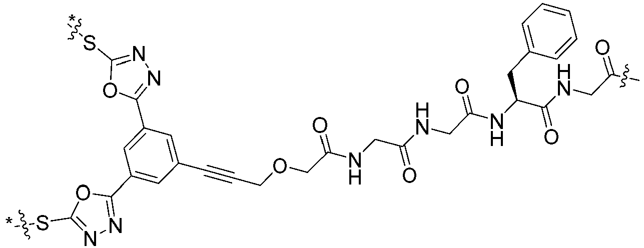

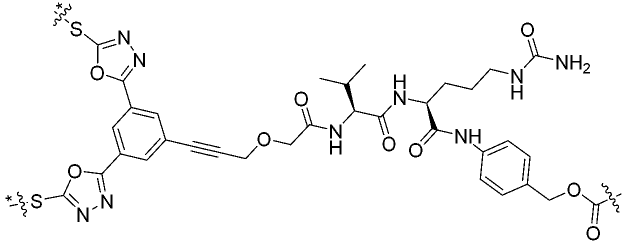

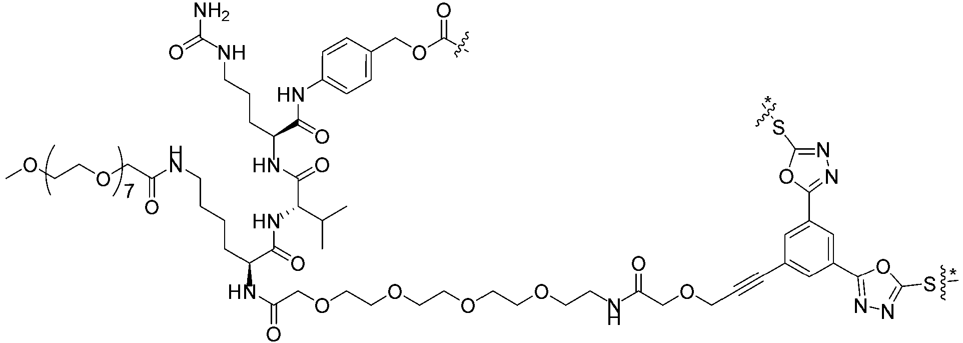

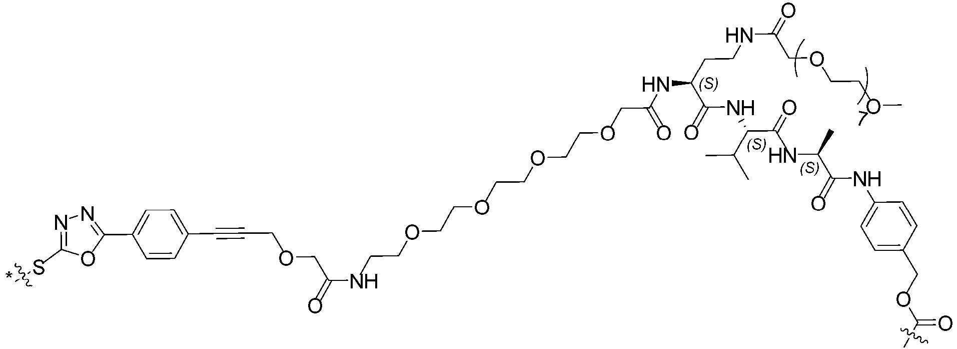

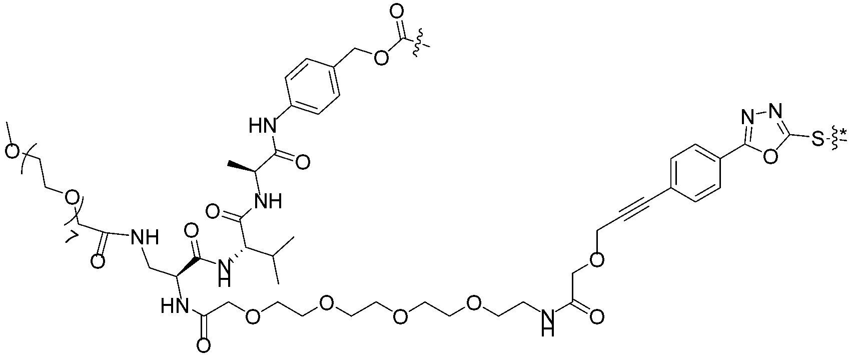

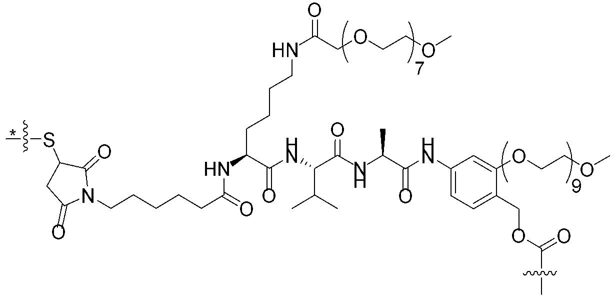

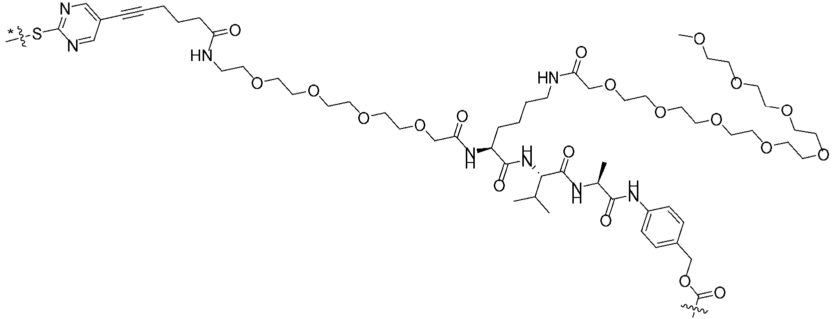

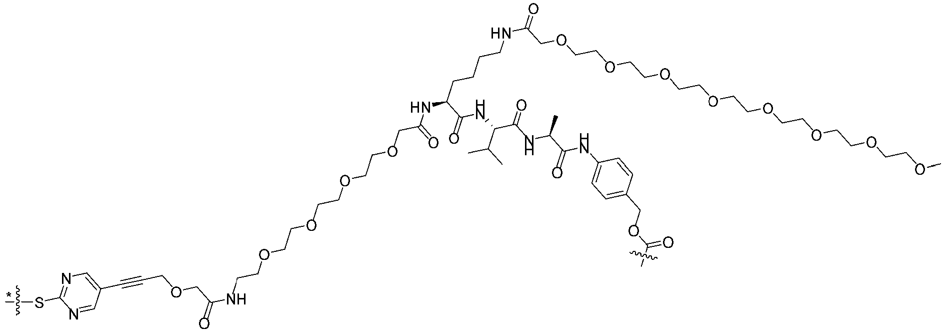

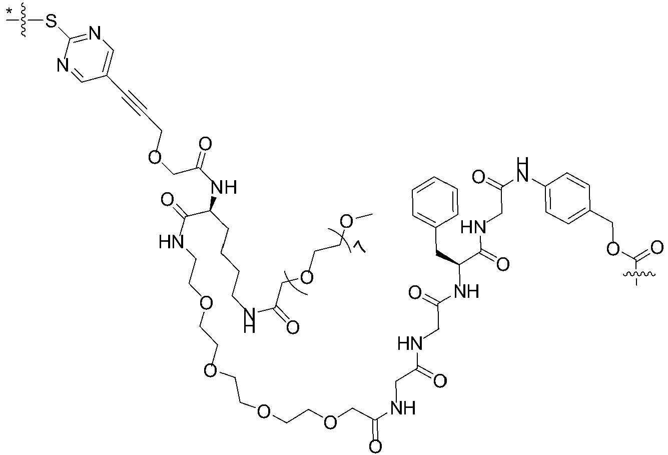

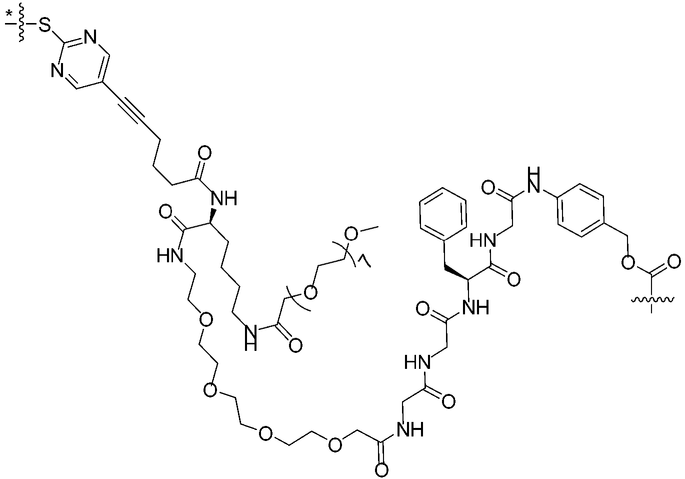

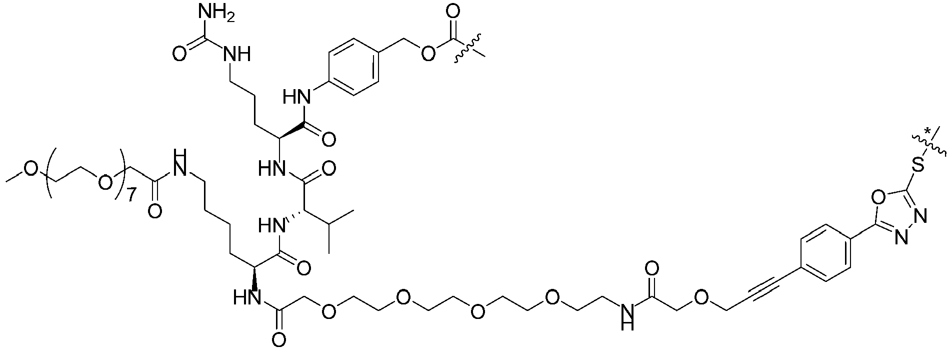

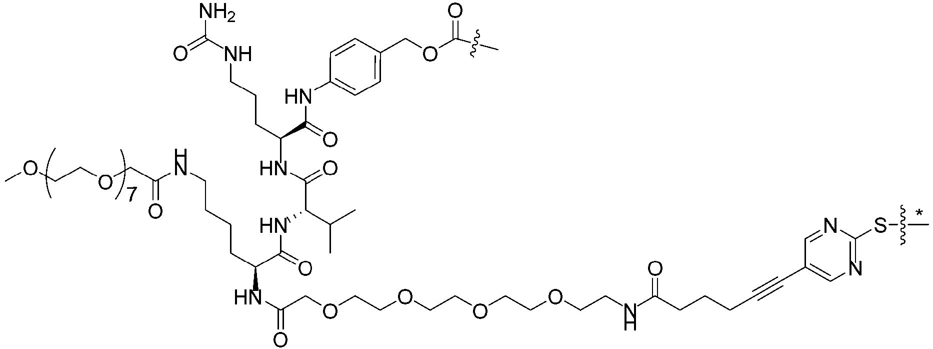

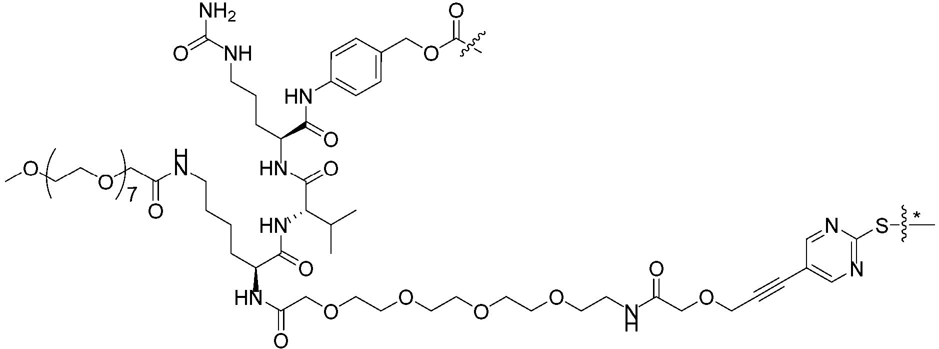

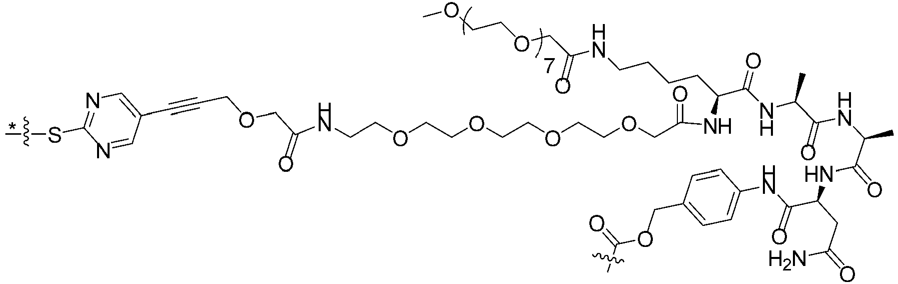

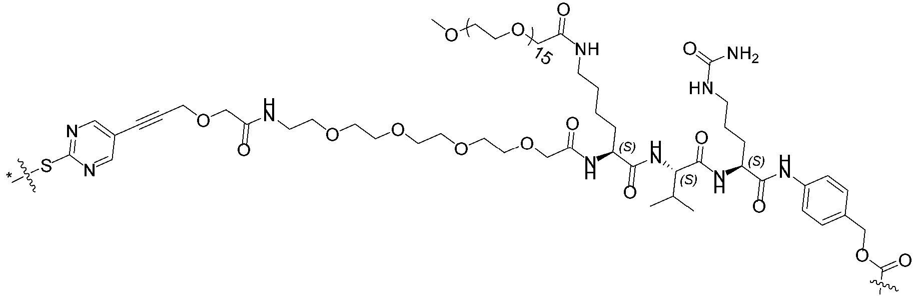

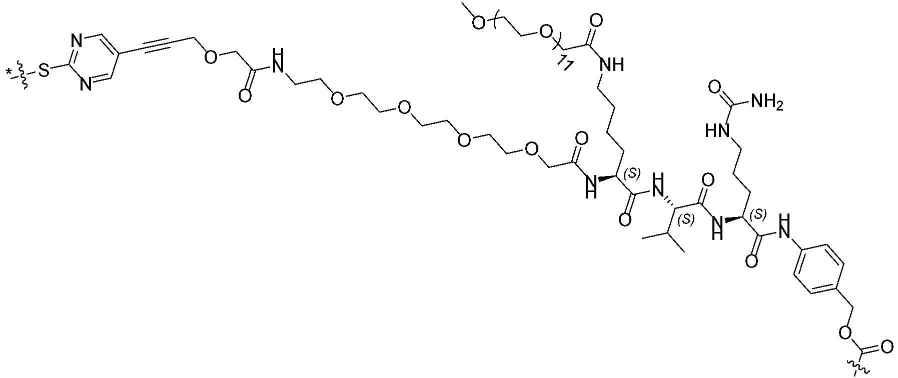

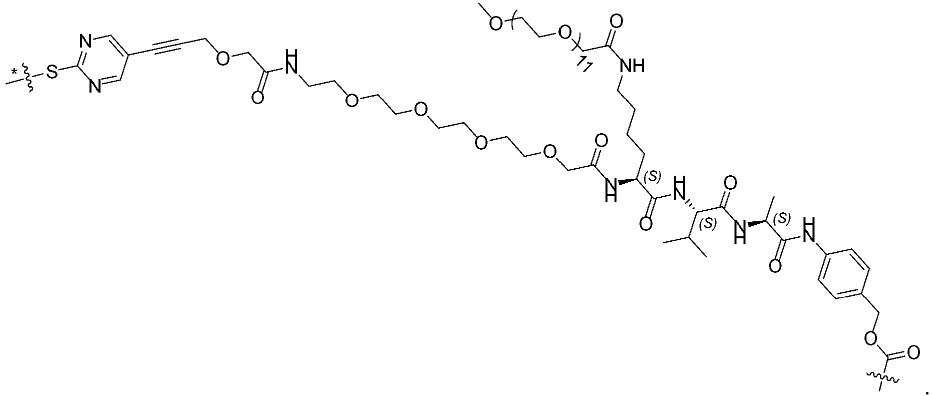

In a fifth aspect, the present invention provides a use of the antibody drug conjugate or the pharmaceutically acceptable salt thereof (comprising the antibody drug conjugate as shown in formula I, formula III or formula IV or the pharmaceutically acceptable salt thereof) in the first aspect of the present invention in preparation of a drug for treating cancers.

In a sixth aspect, the present invention provides a use of the compound or the pharmaceutically acceptable salt thereof (comprising the compound as shown in formula II or the pharmaceutically acceptable salt thereof) in the second aspect of the present invention in preparation of a drug for treating cancers.

In a seventh aspect, the present invention provides a use of the compound or the pharmaceutically acceptable salt thereof in the second aspect of the present invention in preparation of the antibody drug conjugate or the pharmaceutically acceptable salt thereof in the first aspect of the present invention.

In a eighth aspect, the present invention provides a use of the compound or the pharmaceutically acceptable salt thereof in the second aspect of the present invention in preparation of the compound or the pharmaceutically acceptable salt thereof in the third aspect of the present invention.

In a ninth aspect, the present invention provides a use of the compound or the pharmaceutically acceptable salt thereof in the third aspect of the present invention in preparation of the antibody drug conjugate or the pharmaceutically acceptable salt thereof in the first aspect of the present invention.

In a tenth aspect, the present invention provides a use of the structral fragments in the fourth aspect of the present invention in preparation of the antibody drug conjugate or the pharmaceutically acceptable salt thereof in the first aspect of the present invention.

In an eleventh aspect, the present invention provides a method for treating cancers, which comprises administering an effective amount of the antibody drug conjugate or the pharmaceutically acceptable salt thereof in the first aspect of the present invention to a patient in need thereof, or administering an effective amount of the compound or the pharmaceutically acceptable salt thereof in the second aspect of the present invention to the patient in need thereof.

In a twelfth aspect, the present invention provides a pharmaceutical composition, which comprises the antibody drug conjugate or the pharmaceutically acceptable salt thereof in the first aspect of the present invention.

In a thirteenth aspect, the present invention provides a use of the pharmaceutical composition in the twelfth aspect of the present invention in preparation of a drug for treating cancers.

In a fourteenth aspect, the present invention provides a use of the antibody drug conjugate or the pharmaceutically acceptable salt thereof (comprising the antibody drug conjugate as shown in formula I, formula III or formula IV or the pharmaceutically acceptable salt thereof) in the first aspect of the present invention, the compound or the pharmaceutically acceptable salt thereof (comprising the compound as shown in formula II or the pharmaceutically acceptable salt thereof) in the second aspect of the present invention, and the pharmaceutical composition in the twelfth aspect of the present invention as a drug (i.e., use for treatment).

In any technical solution of the present invention, the cancers comprise breast cancer.

It should be noted that, in Ab-[-L₁-L₂-L₃-L₄-D]_{q}, the S atom at the terminus of L₁ or La marked with an asterisk * should be understood by those skilled in the art as an S atom generated by linking L₁ to a thiol group contained in Ab itself, which means that the S atom between L₁ and Ab is not an additionally externally linked sulfur atom. Even if the S atom at the marked asterisk in L₁ or La in the present application is not shown and written, the S atom is generated when Ab is coupled with L₁-L₂-L₃-L₄-D, and an obtained ADC molecule also contains the S atom.

In the present invention, the antibody hLIV22 is Ladiratuzumab, which has a sequence shown in the following table S1 encoded by a Kabat system.

### S1 Sequence of antibody hLIV22

| | |
|---|---|
| Heavy chain variable region | QVQLVQSGAE VKKPGASVKV SCKASGLTIE DYYMHWVRQA PGQGLEWMGW IDPENGDTEY GPKFQGRVTM TRDTSINTAY MELSRLRSDD TAVYYCAVHN AHYGTWFAYW GQGTLVTVSS (SEQ ID NO: 4) |
| HCDR1 | DYYMH (SEQ ID NO: 1) |
| HCDR2 | WIDPENGDTEYGPKFQG (SEQ ID NO: 2) |
| HCDR3 | HNAHYGTWFAY (SEQ ID NO: 3) |
| Light chain variable region | DVVMTQSPLS LPVTLGQPAS ISCRSSQSLL HSSGNTYLEW YQQRPGQSPR PLIYKISTRFSGVPDRFSGS GSGTDFTLKI SRVEAEDVGV YYCFQGSHVP YTFGGGTKVE IK (SEQ ID NO: 9) |
| LCDR1 | RSSQSLLHSSGNTYLE (SEQ ID NO: 6) |
| LCDR2 | KISTRFS (SEQ ID NO: 7) |
| LCDR3 | FQGSHVPYT (SEQ ID NO: 8) |

More specifically, the antibody hLIV22 has the heavy chain as shown in SEQ ID NO: 5 and the light chain as shown in SEQ ID NO: 10:

QVQLVQSGAEVKKPGASVKVSCKASGLTIEDYYMHWVRQAPGQGLEWMGWIDPE
NGDTEYGPKFQGRVTMTRDTSINTAYMELSRLRSDDTAVYYCAVHNAHYGTWFAYWGQ
GTLVTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPPCPA
PELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFFLYSKL
TVDKSRWQQGNVFSCSVMHEALHNHYTQKSLSLSPG (SEQ ID NO: 5);

DVVMTQSPLSLPVTLGQPASISCRSSQSLLHSSGNTYLEWYQQRPGQSPRPLIYKIST
RFSGVPDRFSGSGSGTDFTLKISRVEAEDVGVYYCFQGSHVPYTFGGGTKVEIKRTVAAP
SVFIFPPSDEQLKSGTASVVCLLNNFYPREAKVQWKVDNALQSGNSQESVTEQDSKDST

YSLSSTLTLSKADYEKHKVYACEVTHQGLSSPVTKSFNRGEC (SEQ ID NO: 10).

In the present invention, the anti-Her2 antibody may be Trastuzumabwhich, which has a sequence shown in the following table S2 encoded by the Kabat system.

### S2 Sequence of antibody Trastuzumab

| | |
|---|---|
| Heavy chain variable region |  (SEQ ID NO: 14) |
| HCDR1 | DTYIH (SEQ ID NO: 11) |
| HCDR2 | RIYPTNGYTRYADSVKG (SEQ ID NO: 12) |
| HCDR3 | WGGDGFYAMDY (SEQ ID NO: 13) |
| Light chain variable region |  (SEQ ID NO: 19) |
| LCDR1 | RASQDVNTAVA (SEQ ID NO: 16) |
| LCDR2 | SASFLYS (SEQ ID NO: 17) |
| LCDR3 | QQHYTTPPT (SEQ ID NO: 18) |

More specifically, the antibody Trastuzumab has the heavy chain as shown in SEQ ID NO: 15 and the light chain as shown in SEQ ID NO: 20:

EVQLVESGGGLVQPGGSLRLSCAASGFNIKDTYIHWVRQAPGKGLEWVARIYPTNG
YTRYADSVKGRFTISADTSKNTAYLQMNSLRAEDTAVYYCSRWGGDGFYAMDYWGQGT
LVTVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFLYSKLTV
DKSRWQQGNVFSCSVMHEALHNHYTQKSLSLSPGK (SEQ ID NO: 15);

DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKRTVAAPSVFIFPP
SDEQLKSGTASVVCLLNNFYPREAKVQWKVDNALQSGNSQESVTEQDSKDSTYSLSSTL
TLSKADYEKHKVYACEVTHQGLSSPVTKSFNRGEC (SEQ ID NO: 20).

Excellent technical effects of the present invention: according to the present invention, the antibody drug conjugate or the pharmaceutically acceptable salt thereof in the first aspect of, and the compound or the pharmaceutically acceptable salt thereof in the second aspect have excellent cancer inhibiting effects and acceptable toxicities. The preparation method of the present invention also has better technical advantages. When preparing the ADC, proportions of DAR4 ingredients of the ADC obtained in the present invention are obviously superior to those others in the prior art.

### Definitions and descriptions of terms

Unless otherwise specified, the terms used herein have the general meanings in the technical field. In the case where a specific term or phrase does not have a special definition, the specific term or phrase should not be regarded as being uncertain or unclear, but should be understood according to the general meaning in the technical field. When a trade name appears herein, it is intended to refer to a corresponding commodity or active ingredients thereof.

The term "drug molecule" is also called a "load", a "loaded drug" or a "payload", which refers to a substance with disease prevention and treatment effects. A drug in an antibody drug conjugate generally refers to a cytotoxic drug, which refers to a chemical molecule with a strong destructive effect on normal growth of tumor cells.

The term "linker" refers to a chemical structure fragment or bond with one end linked to a ligand and the other end linked to the drug, which may also be linked to other linkers and then linked to the drug.

The term "drug-linker conjugate" is also called a load-linker conjugate and a linker-load conjugate, which have the same meaning in the present invention.

In the present invention, the sulfur atom (S) at the end of La marked with * for linking to Ab in formula I or any technical solution thereof actually comes from Ab, and the sulfur atom S may be embodied in the structural formula of La or omitted. For example, La of 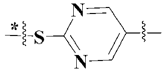 has the same meaning as La of 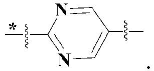 The S atom in corresponding position in structures of other ADC compounds recorded in the present invention is explained in the same way.

In the present invention, the H refers to hydrogen, which is a class of atoms in which nuclei contain one proton, and comprises three isotopes: protium (P), deuterium (D) and tritium (T), and meanwhile, the H also represents one atom of the aforementioned hydrogen element.

In the present invention, the "independently selected from" means that, when there are multiple variables exist simultaneously among different varible groups and among the same substituents,, each variable group may be selected from the same or different scopes or options.

In the present invention, the "halogen" or "halogen atom" refers to fluorine, chlorine, bromine and iodine; and the "halo" refers to a group formed by substituting one or more hydrogen atoms in a substituent with halogen atoms.

The term "alkyl" refers to straight-chain or branched-chain hydrocarbyl in which carbon atoms, as well as the carbon atoms and hydrogen atoms are linked by single bonds. The alkyl is preferably C₁₋₄ or C₁₋₆ alkyl, wherein "C₁₋₄ alkyl" represents straight-chain or branched-chain alkyl having 1-4 carbon atoms; and specific examples of C₁₋₄ alkyl comprise, but are not limited to, methyl, ethyl, n-propyl, isopropyl, n-butyl, sec-butyl, isobutyl and tert-butyl, preferably methyl, ethyl, n-propyl and isopropyl; and "C₁₋₆ alkyl" represents straight-chain or branched-chain alkyl having 1-6 carbon atoms; and specific examples of C₁₋₆ alkyl comprise, but are not limited to, methyl, ethyl, n-propyl, isopropyl, n-butyl, sec-butyl, isobutyl, tert-butyl, n-pentyl and n-hexyl, preferably methyl, ethyl, n-propyl, isopropyl, n-butyl, sec-butyl, isobutyl, tert-butyl, n-pentyl and n-hexyl.

The term "haloalkyl" refers to alkyl in which one or more hydrogen atoms are substituted with halogen atoms. Haloalkyl is preferably C₁₋₄ haloalkyl and C₁₋₆ haloalkyl. Specific examples of C₁₋₄ haloalkyl comprise, but are not limited to, monofluoromethyl, monochloromethyl, difluoromethyl, dichloromethyl, trifluoromethyl, trichloromethyl and tribromomethyl.

The term "alkoxy" refers to alkyl-O-, which is a substituent formed by substituting a hydrogen atom in -OH with alkyl. Alkyl is as mentioned above, and alkoxy comprises C₁₋₄ alkoxy and C₁₋₆ alkoxy. Specific examples of C₁₋₄ alkoxy comprise, but are not limited to, methoxy, ethoxy, n-propoxy, isopropoxy, n-butoxy, sec-butoxy and tert-butoxy, preferably methoxy, ethoxy and isopropoxy; and specific examples of C₁₋₆ alkoxy comprise n-pentyloxy, neopentyloxy and n-hexyloxy in addition to the specific examples of C₁₋₄ alkoxy.

The term "haloalkoxy" refers to a substituent obtained by substituting one or more hydrogen atoms of alkoxy with halogen atoms. Examples of C₁₋₄ haloalkoxy comprise, but are not limited to, trifluoromethoxy, trichloromethoxy, 2,2,2-trifluoroethoxy and 2,2,2-trichloroethoxy.

The term "hydroxy-C₁₋₄ alkyl" refers to a substituent obtained by substituting one or more hydrogen atoms of C₁₋₄ alkyl with hydroxyl. Specific examples of hydroxy-C₁₋₄ alkyl comprise hydroxymethyl and hydroxyethyl.

The term "cycloalkyl" refers to a cyclic saturated monocyclic structure formed by linking carbon atoms by single bonds. Specific examples of 3-6 membered cycloalkyl comprise, but are not limited to, cyclopropyl, cyclobutyl, cyclopentyl and cyclohexyl; and specific examples of 5-6 membered cycloalkyl comprise, but are not limited to, cyclopentyl and cyclohexyl.

The term "heterocycloalkyl" refers to a substituent group obtained by substituting one or more carbon atoms in a cyclic skeleton of cycloalkyl with one or more heteroatoms or heteroatom groups; and the heteroatoms or the heteroatom groups are generally selected from N, N(O), O, S, S(O) and S(O)₂. Specific examples of 5-6 membered heterocycloalkyl comprise, but are not limited to, tetrahydropyrrolyl, tetrahydrofuranyl, piperidinyl and piperazinyl.

In the present invention, the "natural amino acid" refers to the following amino acids having an α-configuration: Glycine (Gly), Alanine (Ala), Valine (Val), Leucine (Leu), Isoleucine (Ile), Proline (Pro), Phenylalanine (Phe), Tyrosine (Tyr), Tryptophan (Trp), Serine (Ser), Threonine (Thr), Cysteine (Cys), Methionine (Met), Asparagine (Asn), Glutamine (Gln), Aspartic acid (Asp), Glutamic acid (Glu), Lysine (Lys), Arginine (Arg), and Histidine (His).

The "unnatural amino acid" refers to amino acids other than the natural amino acids, such as citrulline.

The "optionally" means that the substituent may or may not be substituted with another substituent.

The term "composition" as used in the present invention refers to a product comprising specified amounts of specified ingredients, and any product directly or indirectly generated from a combination of the specified amounts of specified ingredients. Those skilled in the art can adjust actual dosage levels of active ingredients in the pharmaceutical composition of the present invention, so that the amount of the active compound obtained can effectively provide a desired therapeutic response for specific patient, composition, and administration mode.

The term "pharmaceutically acceptable carrier" refers to a medium generally accepted in the art for delivering a biologically active agent to an animal, particularly a mammal.

The term "excipient" generally refers to a carrier, diluent, and/or medium required to formulate an effective pharmaceutical composition.

The term "effective amount" refers to a sufficient amount of the compound or the pharmaceutically acceptable salt thereof in the present invention for treating a disorder with a reasonable benefit/risk ratio applicable to any medical treatment and/or prevention.

The term "pharmaceutically acceptable salt" refers to a salt which is suitable for contact with tissues of humans and lower animals without undue toxicity, irritation, allergic response, and the like, within a scope of sound medical judgment, and is commensurate with a reasonable benefit/risk ratio.

In the present invention, when a certain chemical bond in a substituent is marked with " 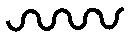 ", this position of the substituent is a linking position to an adjacent group or a structural fragment. The dash "-" appeared in the structure of the substituent indicates a linking point of the substituent, for example, -CH₃ is linked via a carbon atom. " 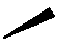 " and " 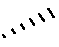 " indicate an absolute configuration of a stereogenic center, which is an R or S configuration.

When a bond of a substituent may be cross-linked to two atoms on a ring, the substituent may be bonded to any atom on the ring. For example, a structural unit 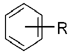 means that a substituent R may be substituted at any position on a benzene ring.

The "isomer" in the present invention comprises a geometric isomer and a stereoisomer, such as an atropisomer, a cis-trans isomer, an enantiomer, a diastereomer, a tautomer, and a racemic mixture thereof and other mixtures, and all these mixtures are within the scope of the present invention. The term "enantiomer" refers to stereisomers in a mirror-image relation. The term "tautomer" refers to one of the functional group isomers, which has different hydrogen linkage points by shifting of one or more double bonds, for example, ketone and its enol form are keto-enol tautomers. The term "diastereomer" refers to a stereoisomer in which molecules have two or more chiral centers and the molecules are in a non-mirror-image relation. The term "cis-trans isomer" refers to different spatial configurations in which a double bond in a molecule or a single bond of a ring-forming carbon atom cannot rotate freely. The term "atropisomer" refers to a stereoisomer capable of being isolated due to hindered rotation or very slow rotation of a single bond.

The stereoisomer of the compound of the present invention may be prepared by chiral synthesis or a chiral reagent or other conventional techniques. For example, an enantiomer of some compound of the present invention may be prepared by an asymmetric catalysis technology or a chiral auxiliary derivation technology. Alternatively, a compound with a single stereo configuration is obtained from a mixture by a chiral resolution technology. Alternatively, the compound is directly prepared from a chiral starting material. The separation of optically pure compound in the present invention is generally completed by preparative chromatography, wherein a chiral chromatographic column is adopted to achieve the purpose of separating a chiral compound.

In the present invention, a solution concentration unit M represents mol/L, and a solution concentration unit mM represents mmol/L.

In the present invention, (S)-2-(2-(2-aminoacetamido)acetamido)-N-(2-((((1S,9S)-9-ethyl-5-fluoro-9-hydroxy-4-methyl-1 0,13-dioxy-2,3,9,10,13,15-hexahydro-1H,12H-benzo[de]pyran[3',4':6,7]indolizino[1,2-b]quinolin -1-yl)amino)-2-oxoethoxy)methyl)amino)-2-oxoethyl)-3-phenylpropanamide refers to a compound with the following structure:

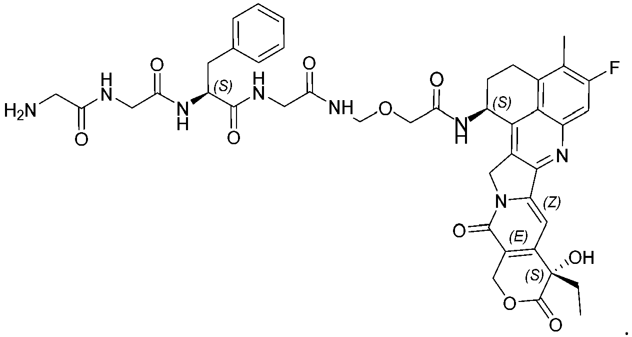

Compound A refers to a compound with the following structure, which may be prepared according to WO2022228493:

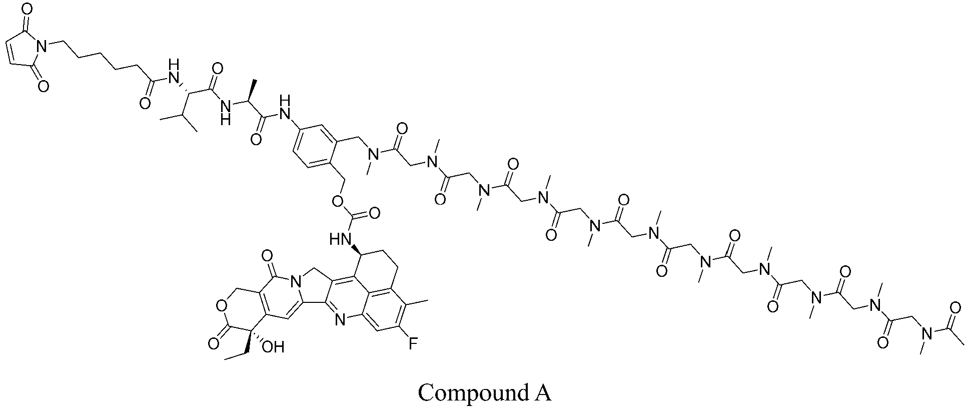

Compound Deruxtecan refers to a compound with the following structure:

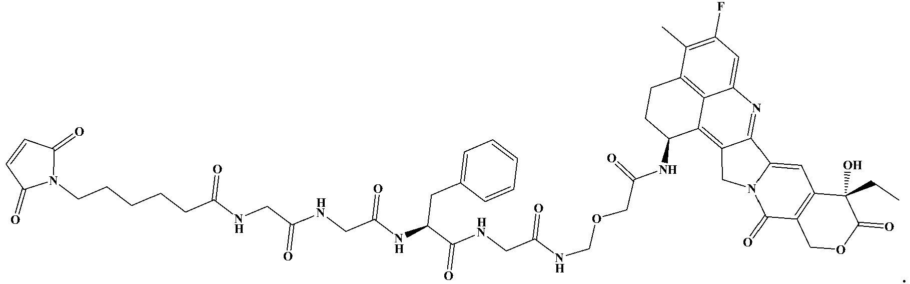

SN-38 refers to a compound with the following structure:

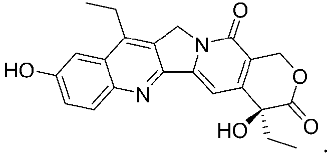

Dxd refers to a compound with the following structure:

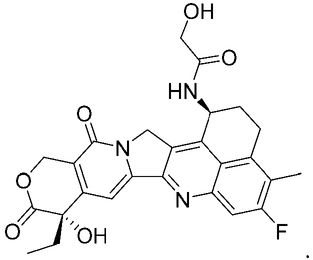

The chemical abbreviations used in the present invention and the chemical names they refer to are as follows:

| **Chemical abbreviation** | **Chemical name** |
|---|---|
| DBU | 1,8-diazabicyclo[5.4.0]undec-7-ene |
| HOBT | 1-hydroxybenzotriazole |
| EDCI | Carbodiimide |
| DIPEA | Diisopropylethylamine |
| DME | Dimethoxyethane |
| DMSO | Dimethyl sulfoxide |
| DMF | N,N-dimethylformamide |
| DMAC | N,N-dimethylacetamide |
| NAC | N-acetylcysteine |
| TFA | Trifluoroacetic acid |

### BRIEF DESCRIPTION OF THE DRAWINGS

FIG. 1 shows pharmacodynamic evaluation of ADC1-ADC6 of the present invention in the mouse xenograft HCC1806 breast cancer model;
FIG. 2 shows a body weight change caused by ADC1-ADC6 of the present invention in the mouse xenograft HCC1806 breast cancer model;
FIG. 3 shows pharmacodynamic evaluation of ADC7-ADC9 of the present invention in the mouse xenograft HCC1806 breast cancer model;
FIG. 4 shows a body weight change caused by ADC7-ADC9 of the present invention in the mouse xenograft HCC1806 breast cancer model; and
FIG. 5 shows a drug concentration-time curve of ADC 10-ADC11 of the present invention.

### DETAILED DESCRIPTION

The present invention is further described in detail hereinafter with reference to specific preparation examples and biological experiments. However, it should be understood that these examples and experiments are provided solely for illustrative purposes and should not be construed as limiting the present invention in any form. Those skilled in the art know that, unless otherwise specified, the materials used hereinafter are well-known in the art, and are commercially available or may be obtained by those skilled in the art based on published literatures or conventional methods. Unless otherwise specified, all reactions in the present invention are carried out by continuous magnetic stirring under a dry nitrogen or argon atmosphere, in which a solvent is a dry solvent, wherein: (i) temperatures are given in degrees Celsius (°C), operations are carried out at room temperature, and the room temperature generally refers to 15-35°C, preferably 20-30°C, and more preferably 20-25°C; (ii) solvents are removed by rotary evaporation under a reduced pressure with a rotary evaporator, and a bath temperature is not higher than 60°C; (iii) reaction processes is monitored by thin-layer chromatography (TLC); and (iv) final products exhibit satisfactory ¹H nuclear magnetic resonance spectroscopy (¹H-NMR) and/or mass spectrometry (MS) data.

### Test instruments:

The compound structures of the present invention are determined by nuclear magnetic resonance (NMR) or/and liquid chromatography-mass spectrometry (LC-MS). NMR chemical shifts (δ) are given in parts per million (ppm). In the determination carried out by the NMR, a Bruker Neo 400M or Bruker Ascend 400 nuclear magnetic instrument is used, solvents comprise deuterated dimethyl sulfoxide (DMSO-d₆), deuterated methanol (CD₃OD) and/or deuterated chloroform (CDCl₃), and the internal standard is tetramethylsilane (TMS).

In the determination carried out by the liquid chromatography-mass spectrometry (LC-MS), Shimadzu 2030Plus-LCMS2020 mass spectrometer, Agilent 1260-6125B single quadrupole mass spectrometer or Shimadzu LCMS-2020 mass spectrometer is used. In determination carried out by HPLC, Shimadzu LCMS-2020 or Agilent 1260 high performance liquid chromatographic instrument is used.

Preparative high performance liquid chromatography was performed using Shimadzu FRC-40 equipped with LC-20AP and PDA-20A (chromatographic column: Synergi Max-RP, 150 × 30 mm, 4 m) or GILSON GX-281 LC (chromatographic column: Boston Prime C18 150*30 mm*5 µm; YMC-Actus Triart C18 150*30 mm*5 µm; YMC-Triart PFP 150*30 mm*5 µm; YMC-Triart Phenyl 150*30 mm*5 µm).

In the examples of the present application, preparation and separation conditions for a payload-linker conjugate are as follows: (chromatographic column: Boston Prime C18 150*30 mm*5 µm; A: 0.225% FA in Water; B: MeCN). Those skilled in the art may appropriately adjust the elution gradient according to different compounds in the examples.

### Example A1: preparation of compound A1

### Step 1: synthesis of 6-iodo-2-(methylthio)benzothiazole (A1-2)

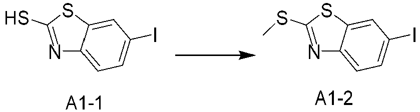

6-iodo-2-mercaptobenzothiazole A1-1 (2.8 g, 9.55 mmol) was added into a 100 mL single-neck flask and dissolved with tetrahydrofuran (20 mL). The obtained solution was added with methyl iodide (1.8 g, 12.42 mmol) and potassium carbonate (2.6 g, 19.10 mmol) at 0°C. The reaction solution was stirred at room temperature for 4 hours. The reaction solution was added with water (30 mL) and extracted with ethyl acetate (30 mL*3). Organic phases were combined, washed with saturated brine (50 mL*3), dried with anhydrous sodium sulfate, filtered, and concentrated under a reduced pressure. Residues were purified by silica gel column chromatography (PE: EA = 90: 10) to obtain A1-2 (2.9 g, 99%).

LC-MS:308.0[M+H]⁺.

### Step 2: synthesis of 6-(2-(methylthio)benzo[d]thiazol-6-yl)hex-5-ynoic acid (A1-3)

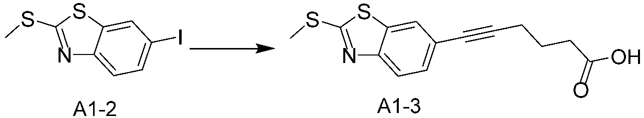

A1-2 (500.0 mg, 1.63 mmol) was added into a 10 mL single-neck flask and dissolved with diisopropylamine (3 mL). The obtained solution was added with bis(acetonitrile)palladium(II) chloride (12.7 mg, 0.05 mmol), triphenylphosphine (42.7 mg, 0.16 mmol) and cuprous iodide (10.3 mg, 0.03 mmol). The reaction solution was stirred at room temperature for 5 minutes, and then dropwise added with 5-hexynoic acid (273.8 mg, 2.44 mmol). The reaction solution was stirred at 50°C overnight. The reaction solution was concentrated under a reduced pressure. Residues were purified by silica gel column chromatography (dichloromethane: methanol = 93: 7) to obtain A1-3 (500.0 mg, 95%).

LC-MS: 292.0[M+H]⁺.

### Step 3: synthesis of 6-(2-(methylsulfonyl)benzo[d]thiazol-6-yl)hex-5-ynoic acid (A1-4)

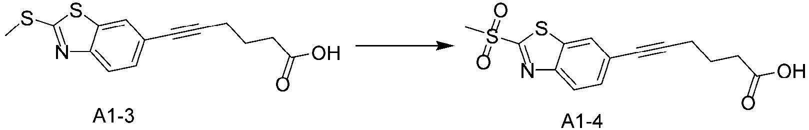

A1-3 (500.0 mg, 1.72 mmol) was added into a 10 mL single-neck flask and dissolved with dichloromethane (20 mL). The obtained solution was added with meta-chloroperoxybenzoic acid (888.3 mg, 5.15 mmol). The reaction solution was stirred at room temperature overnight. The reaction solution was concentrated under a reduced pressure to half of its original volume, and filtered. The organic phase was concentrated under a reduced pressure. Residues were purified by preparative high performance liquid chromatography (chromatographic column: Boston Prime C18 150*30 mm*5 µm; A: 0.225% FA in Water; B: MeCN) to obtain A1-4 (80.0 mg, 14%).

LC-MS: 324.0[M+H]⁺.

### Step 4: synthesis of 1,1-dimethylethyl (2S)-4-(2-benzothiazolyl sulfonyl)-2-[[(1,1-dimethoxy)carbonyl)amino)butyrate (A1-5)

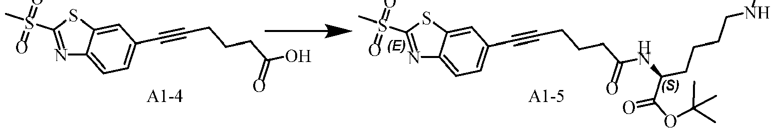

A1-4 (500.0 mg, 1.55 mmol) was added into a 10 mL single-neck flask and dissolved with dichloromethane (10 mL). The obtained solution was added with N(e)-Boc-L-lysine tert-butyl ester hydrochloride (524.0 mg, 1.55 mmol), 1-(3-dimethylaminopropyl)-3-ethylcarbodiimide hydrochloride (444.6 mg, 2.32 mmol), and 4-dimethylaminopyridine (566.7 mg, 4.64 mmol). The reaction solution was stirred at room temperature for 2 hours. The reaction solution was concentrated under a reduced pressure. Residues were purified by silica gel column chromatography (dichloromethane: methanol = 90: 10) to obtain A1-5 (180.0 mg, 19%).

LC-MS:552.2[M+H]⁺.

### Step 5: synthesis of 6-[[[2-methylsulfonyl)-6-benzothiazolyl]carbonyl]amino]hexanoic acid (A1-6)

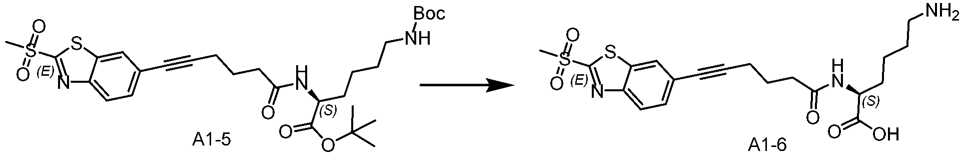

A1-5 (180.0 mg, 0.30 mmol) was added into a 50 mL single-neck flask and dissolved with trifluoroacetic acid (2.5 mL) and dichloromethane (5 mL). The reaction solution was stirred at room temperature for 1 hour, and then heated to 30°C and stirred for 2 hours. The reaction solution was concentrated under a reduced pressure, dissolved with 10 mL of toluene, and concentrated under a reduced pressure again to obtain A1-6 (167.4 mg, 100%).

LC-MS 452.2[M+H]⁺.

### Step 6: synthesis of (S)-31-(6-(2-(methylsulfonyl)benzo[d]thiazol-6-yl)hex-5-ynamino)-25-oxo-2,5,8,11,14,17,20,2 3-octaoxa-26-azatricontan-32-oic acid (A1-7)

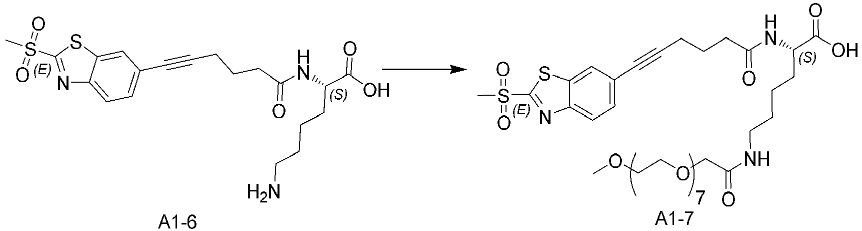

A1-6 (133.7 mg, 0.30 mmol) was added into a 100 mL single-neck flask and dissolved with dichloromethane (10 mL). The obtained solution was added with a dichloromethane solution (1 mL) of methyl-heptaethylene glycol-acetyl chloride (123.4 mg, 0.30 mmol) and diisopropylethylamine (0.10 mL, 0.59 mmol). The reaction solution was stirred at room temperature overnight. The reaction solution was concentrated under a reduced pressure. Residues were purified by silica gel column chromatography (dichloromethane: methanol = 82: 18) to obtain A1-7 (110.0 mg, 45%).

LC-MS: 832.4[M+H]⁺.

### Step 7: synthesis of N-((10S,19S)-10-benzyl-1-(((1S,9S)-9-ethyl-5-fluoro-9-hydroxy-4-methyl-10,13-dioxy-2,3,9,1 0,13,15-hexahydro-1H,12H-benzo[d]pyrano[3',4':6,7]indolizino[1,2-b]quinolin-1-yl)amino)-19-(6-(2-(methylsulfonyl)benzo[d]thiazol-6-yl)hex-5-ynamino)-1,6,9,12,15,18-hexaoxo-3-oxa-5,8,11,14,17-pentaazatriazole-23-yl)-2,5,8,11,11,14,17,20,23-octaoxapentacosan-25-amide (A1)

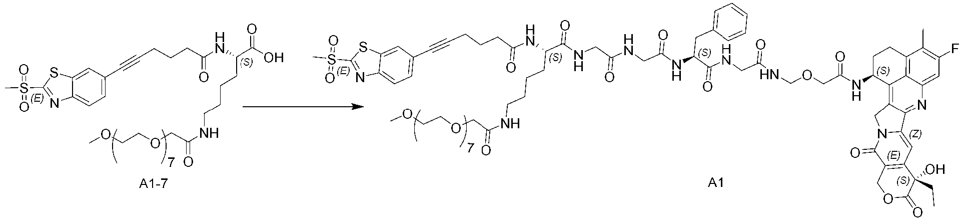

A1-7 (80.0 mg, 0.10 mmol), diisopropylethylamine (0.05 mL, 0.3 mmol) and (S)-2-(2-(2-aminoacetamido)acetamido)-N-(2-((((1S,9S)-9-ethyl-5-fluoro-9-hydroxy-4-methyl-1 0,13-dioxy-2,3,9,10,13,15-hexahydro-1H,12H-benzo[de]pyran[3',4':6,7]indolizino[1,2-b]quinolin -1-yl)amino)-2-oxoethoxy)methyl)amino)-2-oxoethyl)-3-phenylpropanamide (80.9 mg, 0.10 mmol) were added into a 100 mL single-neck flask, and dissolved with N,N-dimethylformamide (2 mL). The obtained solution was added with 4-(4,6-dimethoxy-triazin-2-yl)-4-methylmorpholine hydrochloride (28.3 mg, 0.10 mmol) in an ice bath. The reaction solution was stirred in an ice bath for 1 hour. The reaction solution was filtered, and then purified by preparative high performance liquid chromatography (chromatographic column: Boston Prime C18 150*30 mm*5 µm; A: 0.225% FA in Water; B: MeCN) to obtain the compound A1.

LC-MS: 1654.6[M+H]⁺.

### Example A2: preparation of compound A2

### Step 1: synthesis of 5-(4-iodophenyl)-1,3,4-oxadiazole-2-thiol (A2-2)

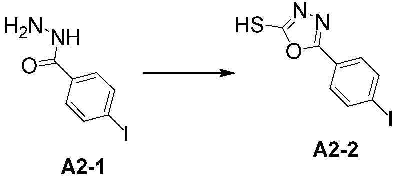

4-iodobenzohydrazide (A2-1) (5.0 g, 19.08 mmol) and ethanol (200 mL) were added into a 500 mL single-neck flask, added with potassium hydroxide (1.1 g, 19.08 mmol) and carbon disulfide (4.4 g, 57.24 mmol) at room temperature, and refluxed overnight. The reaction solution was cooled to room temperature, dried by rotary evaporation first and then diluted with water (30 mL), added with 2 M HCl to adjust the pH to 6, and filtered to obtain A2-2 (5.0 g, 86%).

LC-MS: 305.0[M+H]⁺.

### Step 2: synthesis of 2-(4-iodophenyl)-5-(methylthio)-1,3,4-oxadiazole (A2-3)

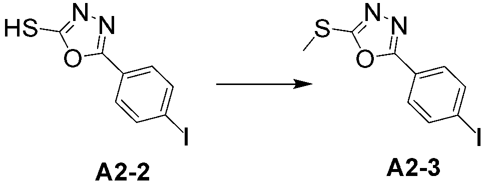

A2-2 (10.0 g, 32.88 mmol) and tetrahydrofuran (200 mL) were added into a 500 mL three-neck flask, added with triethylamine (10.0 g, 98.65 mmol), iodomethane (5.1 g, 36.17 mmol) and 4-dimethylaminopyridine (0.4 g, 3.29 mmol) at room temperature, subjected to nitrogen replacement, and then stirred at 25°C overnight. The reaction solution was poured into water (100 mL), and then extracted with dichloromethane (50 mL*3). Organic phases were combined, dried with anhydrous sodium sulfate, concentrated and then purified by column chromatography (petroleum ether: ethyl acetate =1: 1) to obtain A2-3 (10.0 g, 96%).

LC-MS:319.0[M+H]⁺.

### Step 3: synthesis of 6-(4-(5-(methylthio)-1,3,4-oxadiazol-2-yl)phenyl)hex-5-ynoic acid (A2-4)

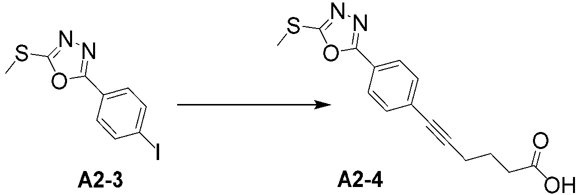

A2-3 (10.0 g, 31.43 mmol) and toluene (100 mL) were added into a 250 mL three-neck flask, added with hex-5-ynoic acid (7.1 g, 62.87 mmol), cuprous iodide (0.9 g, 4.72 mmol), triphenylphosphine (0.8 g, 3.14 mmol), bis(acetonitrile)palladium(II) chloride (0.4 g, 1.57 mmol) and diisopropylamine (19.1 g, 188.60 mmol) at room temperature, subjected to nitrogen replacement for 3 times, and stirred at room temperature overnight. The reaction solution was filtered, concentrated and then purified by column chromatography (dichloromethane: methanol = 10: 1) to obtain A2-4 (5.0 g, 32%).

LC-MS:303.0[M+H]⁺.

### Step 4: synthesis of 6-(4-(5-(methylsulfonyl)-1,3,4-oxadiazol-2-yl)phenyl)hex-5-ynoic acid (A2-5)

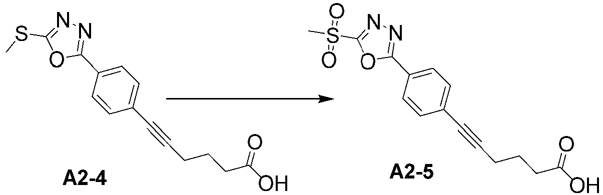

A2-4 (1.4 g, 4.63 mmol) and dichloromethane (30 mL) were added into a 100 mL three-neck flask, added with meta-chloroperbenzoic acid (4.0 g, 23.18 mmol) at room temperature, subjected to nitrogen replacement for 3 times, and stirred at room temperature overnight. The reaction solution was filtered, and the filtrate was concentrated and then seperated by preparative high performance liquid chromatography to obtain A2-5 (250.0 mg, 16%).

LC-MS: 335.0[M+H]⁺.

### Step 5: synthesis of N-(((S)-10-benzyl-1-(((1S,9S)-9-ethyl-5-fluoro-9-hydroxy-4-methyl-10,13-dioxo-1,2,3,9,10,12, 13,15-octahydrobenzo[d]pyrano[3',4':6,7]indolizino[1,2-b]quinolin-1-yl)amino)-1,6,9,12,15-pentaoxo-3-oxa-5,8,11,14-tetraazahexadecan-16-yl)-6-(4-(5-(methylsulfonyl)-1,3,4-oxadiazol -2-yl)phenyl)hex-5-ynamide (A2)

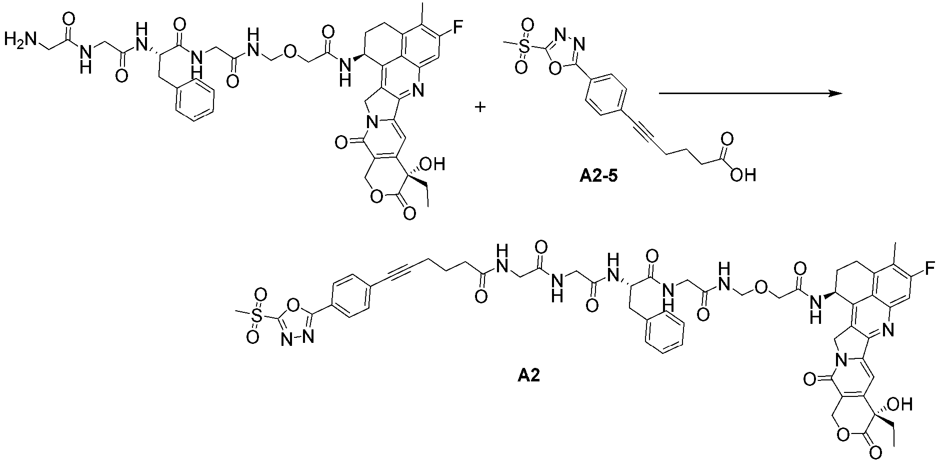

A2-5 (40.0 mg, 0.12 mmol) and N,N-dimethylformamide (3 mL) were added into a 50 mL single-neck flask, added with (7-azabenzotriazol-1-yl)-N,N,N',N'-tetramethyluronium hexafluorophosphate (91.0 mg, 0.24 mmol) and N,N-diisopropylethylamine (46.4 mg, 0.36 mmol) at 0°C and stirred for 5 minutes, and then dropwise added with an N,N-dimethylformamide (2 mL) solution of (S)-2-(2-(2-aminoacetamido)acetamido)-N-(2-(((((1S,9S)-9-ethyl-5-fluoro-9-hydroxy-4-methyl-1 0,13-dioxo-1,2,3,9,10,12,13,15-octahydrobenzo[de]pyrano[3',4':6,7]indolizino[1,2-b]quinolin-1-y l)amino)-2-oxoethoxy)methyl)amino)-2-oxoethyl)-3-phenylpropenamide 7 (100.6 mg, 0.12 mmol) and stirred at room temperature for 1 hour. The reaction solution was purified by preparative high performance liquid chromatography to obtain the compound A2 (20.2 mg, 15%).

LC-MS: 1157.2[M+H]⁺.

### Example A3: preparation of compound A3

### Step 1: synthesis of (S)-tert-butyl 31-(6-(4-(5-(methylsulfonyl)-1,3,4-oxadiazol-2-yl)phenyl)hex-5-ynamido)-25-oxo-2,5,8,11,14, 17,20,23-octaoxa-26-azatriane-32-oate (A3-1)

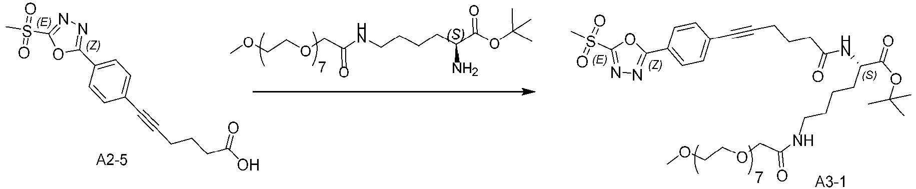

6-(4-(5-(methylsulfonyl)-1,3,4-oxadiazol-2-yl)phenyl)hex-5-ynoic acid (compound A2-5) (70.0 mg, 0.21 mmol) and acetonitrile (3 mL) were added into a 100 mL three-neck flask, added with N,N,N',N'-tetramethylchloroformamidinium hexafluorophosphate (88.1 mg, 0.31 mmol) and N-methylimidazole (34.4 mg, 0.42 mmol) at room temperature and stirred for 10 minutes, and then added with an acetonitrile (2 mL) solution of 2-methylpropan-2-yl(2S)-2-amino-6-[(25-oxo-2,5,8,11,14,17,20,23-octaoxypentacosyloxy)amino ]hexanoate (122.0 mg, 0.21 mmol) and stirred for 50 minutes. The reaction solution was concentrated and purified by silica gel column chromatography (DCM: MeOH=10: 1) to obtain the compound A3-1 (188.0 mg, 100%).

LC-MS:899.4[M+H]⁺.

### Step 2: synthesis of (S)-31-(6-(4-(5-(methylsulfonyl)-1,3,4-oxadiazol-2-yl)phenyl)hex-5-ynamido)-25-oxo-2,5,8,11 ,14,17,20,23-octyloxy-26-azatriane-32-oic acid (A3-2)

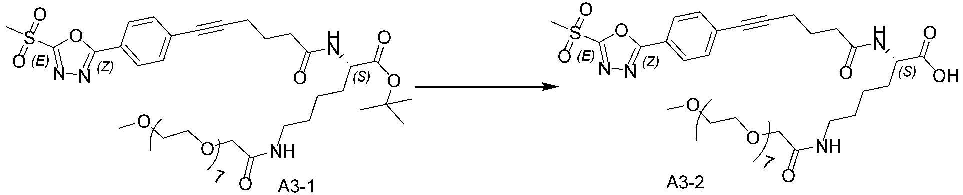

In a 50 mL three-neck flask, A3-1 (100.0 mg, 0.11 mmol) was added into a solvent of dichloromethane (1 mL) and trifluoroacetic acid (0.5 mL), and stirred at room temperature for 1 hour. The reaction solution was concentrated and then separated by preparative high performance liquid chromatography to obtain A3-2 (25.0 mg, 27%).

LC-MS:843.2[M+H]⁺.

### Step 3: synthesis of N-((10S,19S)-10-benzyl-1-(((1S,9S)-9-ethyl-5-fluoro-9-hydroxy-4-methyl-10,13-dioxo-1,2,3,9, 10,12,13,15-octahydrobenzo[d]pyrano[3',4':6,7]indolizino[1,2-b]quinolin-1-yl)amino)-19-(6-(4-(5-(methylsulfonyl)-1,3,4-oxadiazol-2-yl)phenyl)hex-5-ynamido)-1,6,9,12,15,18-hexaoxo-3 -oxa-5,8,11,14,17-pentaazatriazin-23-yl)-2,5,8,14,17,20,20,3-octaoxapentacosan-25-amide A3

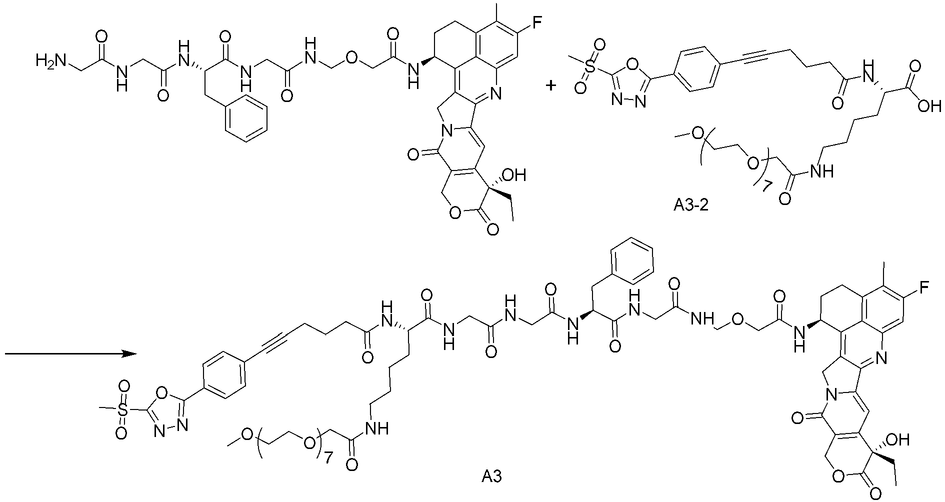

A3-2 (25.0 mg, 0.03 mmol) and acetonitrile (4 mL) were added into a 50 mL single-neck flask, added with (S)-2-(2-(2-(2-(2-aminoacetamido-acetamido)-acetamido)-N-(2-ethyl)-(1S,9S)-9-ethyl-5-fluoro-9 -hydroxy-4-methyl-10,13-dioxo-1,2,3,9,10,12,13,13-dioxo-1,2,3,10,10,12,13,5-octahydrobenzop yran[3',4':6,7':6,7]indolylpentaquin-1-yl)-amino-2-oxoethoxy-2-ethoxyethyl-2-methyl-2-oxoethyl )-3-phenylpropylisopropylamide (24.9 mg, 0.03 mmol) and 4-(4,6-dimethoxy-triazin-2-yl)-4-methylmorphine hydrochloride (8.7 mg, 0.03 mmol) at 0°C, and stirred at room temperature for 1 hour. The reaction solution was purified by preparative high performance liquid chromatography to obtain the compound A3 (11.4 mg, 23%).

LC-MS:1665.6[M+H]⁺.

### Example A4: preparation of compound A4

### Step 1: synthesis of tert-butyl (S)-31-(((9H-fluoren-9-yl)methoxy)carbonyl)amino)-25-oxo-2,5,8,11,14,17,20,23-octaoxa-26-azatriaza-32-oate (A4-2)

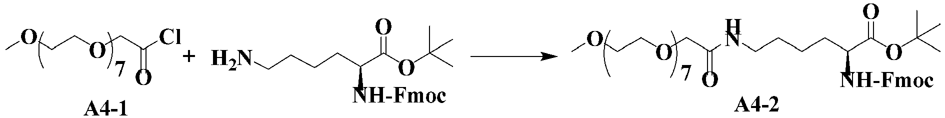

Tert-butyl ((9H-fluoren-9-yl)methoxy)carbonyl)-L-lysinate (176.9 mg, 0.38 mmol) was added into a 10 mL single-neck flask and dissolved with dichloromethane (5 mL). The obtained solution was added with a dichloromethane solution (1 mL) of methyl-heptaethylene glycol-acetyl chloride (160.0 mg, 0.38 mmol) and diisopropylethylamine (0.1 mL, 0.77 mmol). The reaction solution was stirred at room temperature for 1 hour. The reaction solution was concentrated under a reduced pressure. Residues were purified by silica gel column chromatography (dichloromethane: methanol = 95: 5) to obtain A4-2 (251.0 mg, 87%).

LC-MS:805.6[M+H]⁺.

### Step 2: synthesis of tert-butyl (S)-31-amino-25-oxo-2,5,8,11,14,17,20,23-octaoxa-26-azatricontan-32-oate (A4-3)

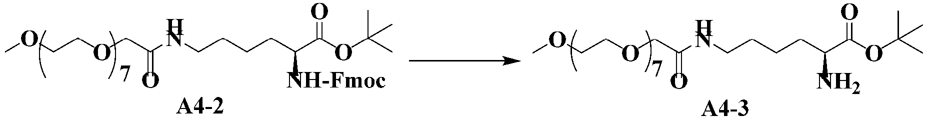

A4-2 (250.0 mg, 0.31 mmol) was added into a 10 mL single-neck flask and dissolved with dimethylformamide (5 mL). The obtained solution was added with diethylamine (0.3 mL, 3.11 mmol). The reaction solution was stirred at room temperature for 1 hour. The reaction solution was concentrated under a reduced pressure to obtain A4-3 (181.2 mg, 100%).

LC-MS: 583.4[M+H]⁺.

### Step 3: synthesis of tert-butyl (S)-31-(((9H-fluoren-9-yl)methoxy)carbonyl)amino)-25-oxo-2,5,8,11,14,17,20,23-octaoxa-26-azatriaza-32-oate (A4-4)

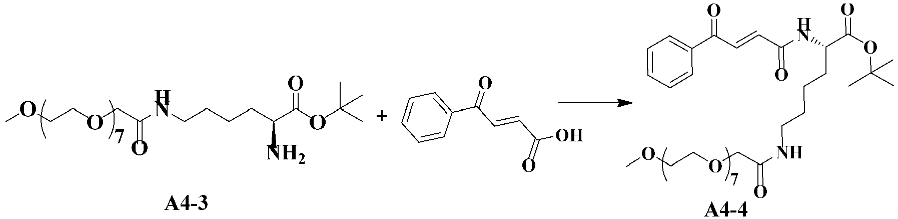

Benzoylacrylic acid (61.3 mg, 0.35 mmol), N,N,N',N'-tetramethylchloroformamidinium hexafluorophosphate (146.5 mg, 0.52 mmol) and N-methylimidazole (57.2 mg, 0.70 mmol) were added into a 10 mL single-neck flask, and dissolved with acetonitrile (2 mL). The obtained solution was stirred at room temperature for 10 minutes. Then, A4-3 (202.8 mg, 0.35 mmol) was added into the mixed solution. The reaction solution was stirred at room temperature for 1 hour. The reaction solution was concentrated under a reduced pressure. Residues were purified by silica gel column chromatography (dichloromethane: methanol = 89: 11) to obtain A4-4 (150.0 mg, 58%).

LC-MS: 741.6[M+H]⁺.

### Step 4: synthesis of (S,E)-25-oxo-31-(4-oxo-4-phenylbut-2-enamido)-2,5,8,11,14,17,20,23-octaoxa-26-azatriaza-3 2-enoic acid (A4-5)

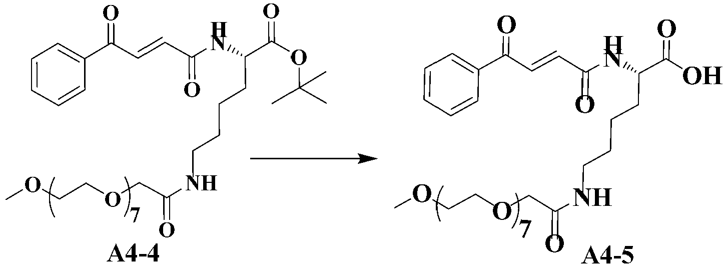

A4-4 (150.0 mg, 0.20 mmol) was added into a 10 mL single-neck flask and dissolved with dichloromethane (2 mL). The obtained reaction solution was added with trifluoroacetic acid (1 mL). The reaction solution was stirred at 30°C for 1 hour. The reaction solution was concentrated under a reduced pressure to obtain **A4-5** (138.3 mg, 100%).

LC-MS: 684.8[M]⁺.

### Step 5: synthesis of N-((10S,19S)-10-benzyl-1-(((1S,9S)-9-ethyl-5-fluoro-9-hydroxy-4-methyl-10,13-dioxo-2,3,9,1 0,13,15-hexahydro-1H,12H-benzo[d]pyrano[3',4':6,7]indolizino[1,2-b]quinolin-1-yl)amino)-1,6,9,12,15,18-hexaoxo-19-((E)-4-oxo-4-phenylbut-2-enamido)-3-oxa-5,8,11,14,17-pentaazatr iazin-23-yl)-2,5,8,11,1,14,17,20,23-octaoxapentan-25-amide A4

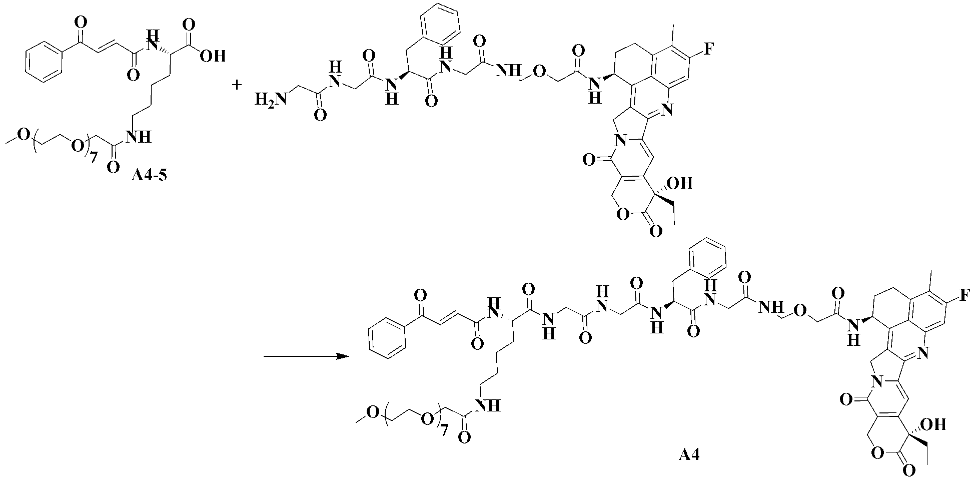

A4-5 (138.3 mg, 0.20 mmol) and (S)-2-(2-(2-aminoacetamido)acetamido)-N-(2-((((1S,9S)-9-ethyl-5-fluoro-9-hydroxy-4-methyl-1 0,13-dioxy-2,3,9,10,13,15-hexahydro-1H,12H-benzo[de]pyran[3',4':6,7]indolizino[1,2-b]quinolin -1-yl)amino)-2-oxoethoxy)methyl)amino)-2-oxoethyl)-3-phenylpropanamide (169.9 mg, 0.20 mmol) were added into a 100 mL single-neck flask, and dissolved with N,N-dimethylformamide (1 mL). The obtained solution was added with 4-(4,6-dimethoxy-triazin-2-yl)-4-methylmorpholine hydrochloride (59.54 mg, 0.202 mmol) in an ice bath. The reaction solution was stirred in an ice bath for 1 hour. The reaction solution was filtered and purified by preparative high performance liquid chromatography to obtain A4 (23.0 mg, 8%).

LC-MS: 1507.6[M+H]⁺.

### Example A5: preparation of compound A5

### Step 1: synthesis of N-[(1S,9S)-9-ethyl-5-fluoro-9-hydroxy-4-methyl-10,13-dioxo-2,3,9,10,12,15-hexahydro-1H-c yclohexo[1,2,3-de]pyrano[3',4':6,7]indolizino[1,2-b]quinolin-1-yl]-2-{[(7S)-13-amino-7-benz yl-3,6,9,12-tetraoxo-2,5,8,11-tetraazatridecan-1-yl]oxy}acetamide A5-2

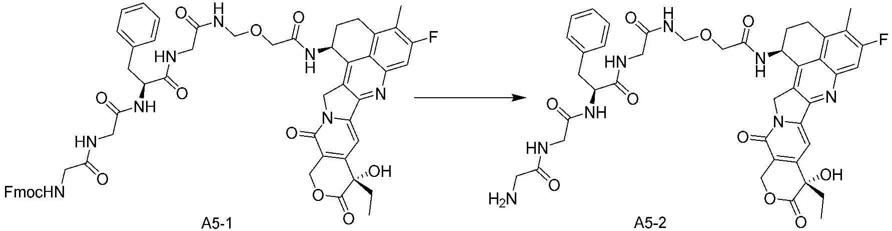

A5-1 (200.0 mg, 0.19 mmol) was added into a 50 mL single-neck flask and dissolved with N,N-dimethylformamide (2 mL). The obtained solution was added with diethylamine (0.19 mL, 1.9 mmol). The reaction solution was stirred at room temperature for 1 hour. The reaction solution was concentrated under a reduced pressure. Residues were slurried with petroleum ether to obtain A5-2 (120 mg, 75.1%).

LC-MS: 841.3[M+H]⁺.

### Step 2: synthesis of N-[(1S,9S)-9-ethyl-5-fluoro-9-hydroxy-4-methyl-10,13-dioxo-2,3,9,10,12,15-hexahydro-1H-c yclohexo[1,2,3-de]pyrano[3',4':6,7]indolizino[1,2-b]quinolin-1-yl]-2-{[(14S)-14-benzyl-1-{3,5 -bis[5-(methyldioxo-16-sulfanyl)-1,3,4-oxadiazol-2-yl]phenyl}-6,9,12,15,18-pentaoxo-4-oxa-7 ,10,13,16,19-pentaazaicos-1-yn-20-yl]oxy}acetamide A5

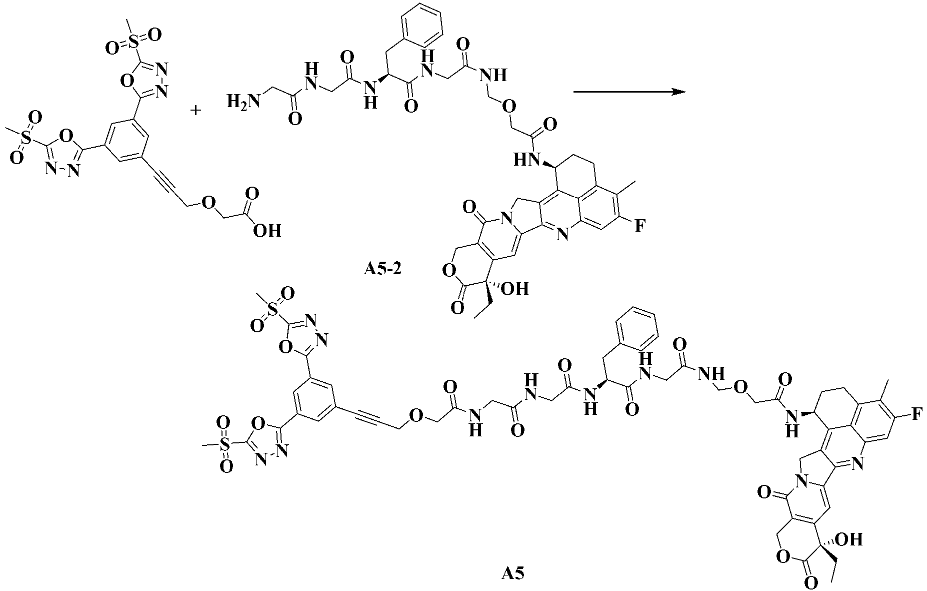

[(3-{3,5-bis[5-(methyldioxo-λ6-sulfanyl)-1,3,4-oxadiazol-2-yl]phenyl}prop-2-ynyl)oxy]acet ic acid (B3-6) (19.3 mg, 0.04 mmol) and 2-(7-azabenzotriazol-1-yl)-N,N,N',N'-tetramethyluronium hexafluorophosphate (24.5 mg, 0.06 mmol) were added into a 10 mL single-neck flask, and dissolved with N,N-dimethylformamide (1 mL). The obtained solution was added with diisopropylethylamine (0.01 mL, 0.09 mmol). The mixed solution was stirred at room temperature for 15 minutes. Then, A5-2 (33.7 mg, 0.04 mmol) was added into the mixed solution. The reaction solution was stirred at room temperature for 3 hours. The reaction solution was filtered and purified by preparative high performance liquid chromatography to obtain A5 (22.5 mg, 43.1%).

LC-MS: 1305.3[M+H]⁺.

### Example B1: preparation of compound B1

### Step 1: synthesis of 2,5,8,11,14,17,20,23,26-nonoxyoctadecan-28-ylmethanesulfonate (B1-2)

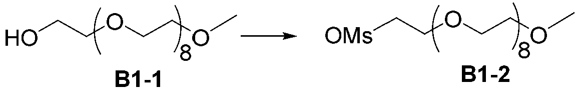

Nonaethylene glycol monomethyl ether (22.0 g, 51.34 mmol) was added into a 100 mL single-neck flask and dissolved with dichloromethane (50 mL). The obtained solution was added with triethylamine (14.3 mL, 102.68 mmol) and methylsufonyl chloride (8.8 g, 77.01 mmol) at 0°C. The reaction solution was stirred at room temperature for 4 hours. The reaction solution was concentrated under a reduced pressure. The obtained residues were purified by silica gel column chromatography (dichloromethane: methanol = 92: 8) to obtain B1-2 (23.5 g, 90%).

LC-MS: 507.4[M+H]⁺.

### Step 2: synthesis of methyl-nonaethylene glycol-bromide (B1-3)

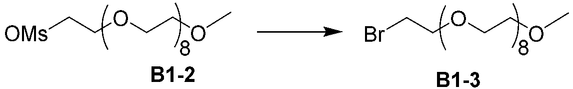

B1-2 (23.5 g, 46.39 mmol) was added into a 100 mL single-neck flask and dissolved with acetonitrile (200 mL). The obtained solution was added with tetrabutylammonium bromide (2.7 g, 8.29 mmol). The reaction solution was stirred at 50°C overnight. The reaction solution was concentrated under a reduced pressure. Residues were purified by silica gel column chromatography (dichloromethane: methanol = 96: 4) to obtain B1-3 (17.5 g, 77%).

LC-MS: 491.2[M+H]⁺.

### Step 3: synthesis of 2-((2,5,8,11,14,17,20,23,26-nonoxaoctan-28-yl)oxy)-4-nitrobenzaldehyde (B1-4)

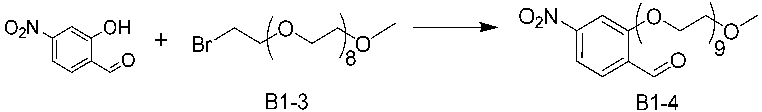

B1-3 (5.5 g, 11.19 mmol) and 2-hydroxy-4-nitrobenzaldehyde (1.9 g, 11.19 mmol) were added into a 100 mL single-neck flask and dissolved with N,N-dimethylformamide (10 mL). The obtained solution was added with potassium carbonate (7.7 g, 55.96 mmol) and potassium iodide (40.0 mg, 0.22 mmol). The reaction solution was stirred at 100 °C for 3 hours. The reaction solution was cooled to room temperature, concentrated under a reduced pressure, then diluted with water (50 mL), and extracted with ethyl acetate (30 mL*3). Organic phases were combined, dried with anhydrous sodium sulfate, filtered, and concentrated under a reduced pressure. Residues were purified by silica gel column chromatography (dichloromethane: methanol = 96: 4) to obtain B1-4 (14.3 g, 55%).

LC-MS: 578.4[M+H]⁺.

### Step 4: synthesis of (2-((2,5,8,11,14,17,20,23,26-nonoxyoctan-28-yl)oxy)-4-aminophenyl)methanol (B1-5)

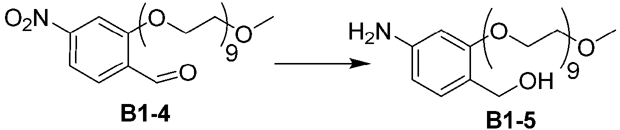

B1-4 (500.0 mg, 0.87 mmol) was added into a 100 mL single-neck flask and dissolved with ethyl acetate (50 mL). The obtained solution was added with triethylamine (0.1 mL) and subjected to nitrogen replacement. Then, the reaction solution was added with 10% palladium on carbon (100.0 mg, 10%) and subjected to hydrogen replacement. The reaction solution was stirred at room temperature under a hydrogen atmosphere (15 psi) overnight. After the palladium on carbon was filtered off, the filtrate was concentrated under a reduced pressure. Residues were purified by silica gel column chromatography (dichloromethane: methanol = 92: 8) to obtain B1-5 (500.0 mg, 53%).

LC-MS: 550.4[M+H]⁺.

### Step 5: synthesis of (9H-fluoren-9-yl)methyl((S)-1-(((S)-1-(3-((2,5,8,11,14,17,20,23,26-nonoxyoctan-28-yl)oxy)-4-( hydroxymethyl)phenyl)amino)-1-oxopropan-2-yl)amino)-3-methyl-1-oxobutan-2-yl)carbam ate (B1-6)

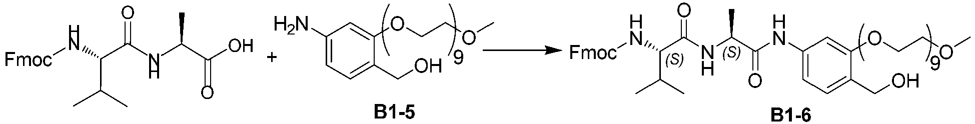

B1-5 (300.0 mg, 0.55 mmol), N-[fluorenylmethoxycarbonyl]-L-valyl-L-alanine (224.0 mg, 0.55 mmol) and 4-(4,6-dimethoxytriazin-2-yl)-4-methylmorpholine hydrochloride (160.9 mg, 0.55 mmol) were added into a 100 mL single-neck flask, and dissolved with dichloromethane (10 mL) and methanol (5 mL). The reaction solution was stirred at room temperature overnight. The reaction solution was concentrated under a reduced pressure. Residues were purified by silica gel column chromatography (dichloromethane: methanol = 96: 4) to obtain B1-6 (250.0 mg, 49%).

LC-MS: 964.6[M+Na]⁺.

### Step 6: synthesis of 9H-fluoren-9-ylmethyl (S)-1-((S)-1-((3-(2,5,8,11,14,17,20,23,26-nonaoxaoctadecan-28-yl)oxy)-4-((((4-nitrophenoxy)c arbonyl)oxy)methyl)phenyl)amino)-1-oxopropan-2-yl)amino)-3-methyl-1-oxobutan-2-ylcarb amate (B1-7)

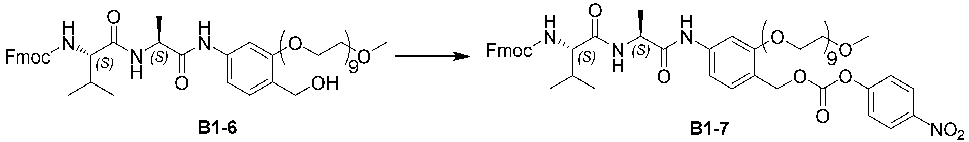

B1-6 (600.0 mg, 0.64 mmol) and bis(p-nitrophenyl)carbonate (581.2 mg, 1.91 mmol) were added into a 100 mL single-neck flask and dissolved with N,N-dimethylformamide (3 mL). The obtained solution was added with diisopropylethylamine (0.4 mL, 2.55 mmol) and 4-dimethylaminopyridine (0.8 mg, 0.01 mmol). The reaction solution was stirred at room temperature overnight. The reaction solution was concentrated under a reduced pressure. Residues were purified by silica gel column chromatography (dichloromethane: methanol = 96: 4) to obtain B1-7 (300.0 mg, 43%).

LC-MS: 1129.6[M+Na]⁺.

### Step 7: synthesis of 9H-fluoren-9-ylmethyl {[(2S)-1-{[(2S)-1-[(4-{[({[(1S,9S)-9-ethyl-5-fluoro-9-hydroxy-4-methyl-10,13-dioxo-2,3,9,10,1 2,15-hexahydro-1H-cyclohexo[1,2,3-de]pyrano[3',4':6,7]indolizino[1,2-b]quinolin-1-yl)amin o}carbonyl)oxy)methyl}-3-(2,5,8,11,14,17,20,23,26-nonaoxaoctacos-28-yloxy)phenyl)amino]-1-oxopropan-2-yl]amino}-3-methyl-1-oxobutan-2-yl]amino}formate (B1-8)

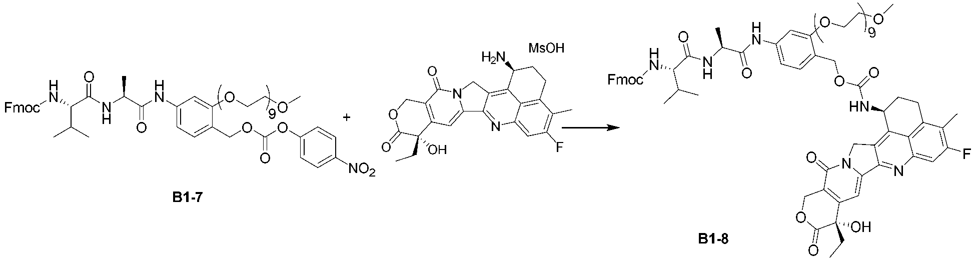

B1-7 (100.0 mg, 0.09 mmol), exatecan mesylate (48.0 mg, 0.09 mmol) and 1-hydroxybenzotriazole (12.2 mg, 0.09 mmol) were added into a 10 mL single-neck flask and dissolved with N,N-dimethylformamide (1 mL). The obtained solution was stirred at room temperature for 30 minutes, and then added with diisopropylethylamine (0.03 mL, 0.18 mmol). The reaction solution was stirred at room temperature for 3 hours, and the turbid solution became clear. The reaction solution was concentrated under a reduced pressure. Residues were purified by silica gel column chromatography (dichloromethane: methanol = 94: 6) to obtain B1-8 (110.0 mg, 61%).

LC-MS: 1403.6[M+H]⁺.

### Step 8: synthesis of 2-((2,5,8,11,14,17,20,23,26-nonoxyoctan-28-yl)oxy)-4-((S)-2-(S)2-amino-3-methylbutanamid o)benzyl((1S,9S)-9-ethyl-5-fluoro-9-hydroxy-4-methyl-10,13-dioxo-2,9,10,13,5-hexahydro-1 H,12H-benzo[d]pyran[3',4':6,7]indolizin[1,2-b]quinolin-1-yl)carbamate (B1-9)

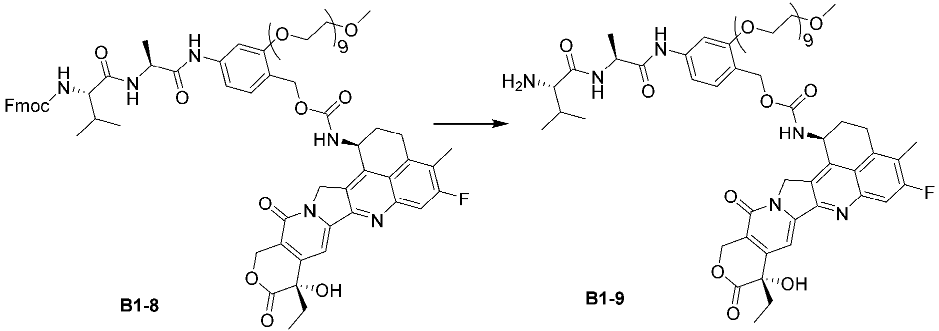

B1-8 (100.0 mg, 0.07 mmol) was added into a 10 mL single-neck flask and dissolved with N,N-dimethylformamide (2 mL). The obtained solution was added with diethylamine (0.07 mL, 0.71 mmol). The reaction solution was stirred at room temperature for 1 hour. The reaction solution was concentrated under a reduced pressure. Residues were slurried with petroleum ether to obtain B1-9 (83.9 mg, 100%).

LC-MS: 1181.6[M+H]⁺.

### Step 9: synthesis of 2-((2,5,8,11,14,17,20,23,26-nonoxyoctan-28-yl)oxy)-4-((S)-2-(S)2-amino-3-methylbutanamid o)benzyl((1S,9S)-9-ethyl-5-fluoro-9-hydroxy-4-methyl-10,13-dioxo-2,9,10,13,5-hexahydro-1 H,12H-benzo[d]pyran[3',4':6,7]indolizin[1,2-b]quinolin-1-yl)carbamate B1

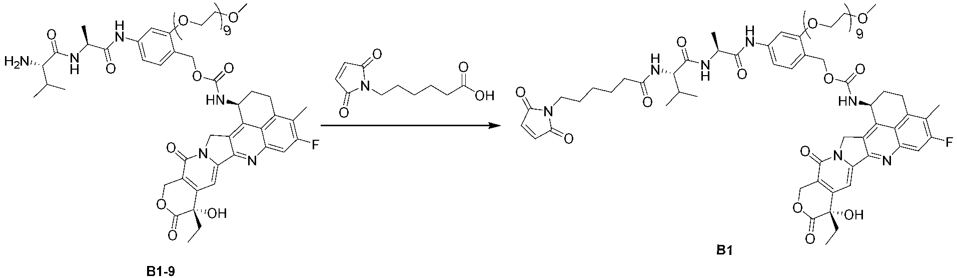

6-(2,5-dioxo-2,5-dihydro-1H-pyrrol-1-yl)hexanoic acid (1.1 mg, 0.01 mmol) and 2-(7-azabenzotriazol-1-yl)-N,N,N',N'-tetramethyluronium hexafluorophosphate (2.9 mg, 0.01 mmol) were added into a 10 mL single-neck flask, and dissolved with N,N-dimethylformamide (1 mL). The obtained solution was added with diisopropylethylamine (0.01 mL, 0.03 mmol). The mixed solution was stirred at room temperature for 10 minutes. Then, B1-9 (8.4 mg, 0.01 mmol) was added into the mixed solution. The reaction solution was stirred at room temperature for 18 hours. The reaction solution was filtered and purified by preparative high performance liquid chromatography to obtain B1 (13.2 mg, 13%).

LC-MS: 1374.6[M+H]⁺.

### Example B2: preparation of compound B2

### Step 1: synthesis of 5-(4-iodophenyl)-1,3,4-oxadiazole-2-thiol (B2-2)

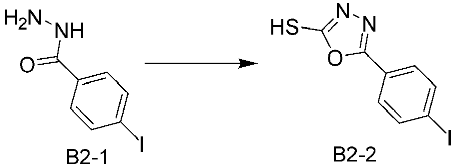

4-iodobenzohydrazide (B2-1) (5.0 g, 19.08 mmol) and ethanol (200 mL) were added into a 500 mL single-neck flask, added with potassium hydroxide (1.1 g, 19.08 mmol) and carbon disulfide (4.4 g, 57.24 mmol) at room temperature, and stirred under reflux overnight. The reaction solution was directly concentrated, diluted with water (30 mL), added with 2 M hydrochloric acid to adjust the pH to about 6, and filtered to obtain B2-2 (5.0 g, 86%).

LC-MS: 305.0[M+H]⁺.

### Step 2: synthesis of 2-(4-iodophenyl)-5-(methylthio)-1,3,4-oxadiazole (B2-3)

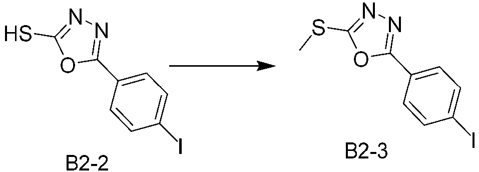

B2-2 (10.0 g, 32.88 mmol) and tetrahydrofuran (200 mL) were added into a 500 mL three-neck flask, added with triethylamine (10.0 g, 98.65 mmol), iodomethane (5.1 g, 36.17 mmol) and 4-dimethylaminopyridine (0.4 g, 3.29 mmol) at room temperature, and stirred at room temperature overnight. The reaction solution was directly dried by rotary evaporation, then slurried with n-hexane, and filtered to obtain B2-3 (10.0 g, 96%).

LC-MS: 319.0[M+H]⁺.

### Step 3: synthesis of 2-((3-(4-(5-(methylthio)-1,3,4-oxadiazol-2-yl)phenyl)prop-2-yn-1-yl)oxy)acetic acid (B2-4)

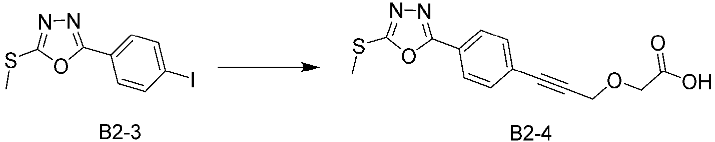

B2-3 (4.0 g, 12.57 mmol) and N,N-dimethylformamide (40 mL) were added into a 250 mL three-neck flask, added with 2-(prop-2-yn-1-yloxy)acetic acid (1.9 g, 16.35 mmol), cuprous iodide (0.4 g, 1.89 mmol), triphenylphosphine (0.3 g, 1.26 mmol), bis(acetonitrile)palladium(II) chloride (0.2 g, 0.63 mmol) and diisopropylamine (7.6 g, 75.44 mmol) at room temperature, and stirred at 50°C for 4 hours. The reaction solution was cooled to room temperature, poured into water (400 mL) to adjust the pH to 6, and then extracted with ethyl acetate (100 mL*3). Organic phases were combined, washed with saturated brine (150 mL*3), dried with anhydrous sodium sulfate, filtered, concentrated, and then purified by column chromatography (dichloromethane: methanol = 10: 1) to obtain B2-4 (3.2 g, 84%).

LC-MS: 305.2[M+H]⁺.

### Step 4: synthesis of 2-((3-(4-(5-(methylsulfonyl)-1,3,4-oxadiazol-2-yl)phenyl)prop-2-yn-1-yl)oxy)acetic acid (B2-5)

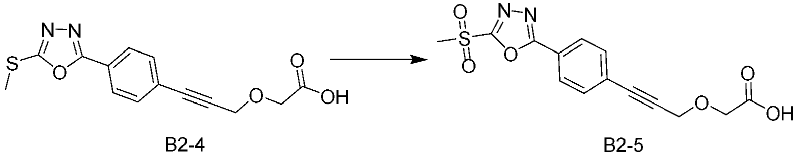

B2-4 (2.0 g, 6.57 mmol) and dichloromethane (30 mL) were added into a 100 mL three-neck flask, added with meta-chloroperbenzoic acid (5.7 g, 32.86 mmol) at room temperature, and stirred at room temperature overnight. The reaction solution was filtered, and directly purified by column chromatography (DCM: MeOH=10: 1) to obtain 0.6 g of crude product. Then, the crude product was separated by preparative high performance liquid chromatography to obtain B2-5 (0.1 g, 5%).

LC-MS: 337.0[M+H]⁺.

### Step 5: synthesis of 2-(2,5,8,11,14,17,20,23,26-nonaoxaoctadecan-28-yl)oxy)-4-(S)-2-(S)-3-methyl-2-(3-(4-(5-meth ylsulfonyl)-1,3,4-oxadiazol-2-yl)phenyl)propyl-2-yn-1-oxy)acetamido)butanamide)propana mido)benzyl((1S,9S)-9-ethyl-5-fluoro-9-hydroxy-4-methyl-10,13-dioxo-2,3,9,10,13,15-hexahy dro-1H,12H-benzopyran[3',4':6,7]indolizin[1,2-b]quinolin-1-yl)-carbamate B2

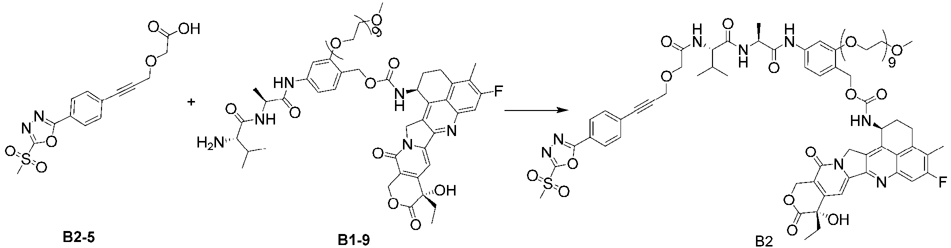

B2-5 (14.5 mg, 0.04 mmol) and 2-(7-azabenzotriazol-1-yl)-N,N,N',N'-tetramethyluronium hexafluorophosphate (24.5 mg, 0.06 mmol) were added into a 10 mL single-neck flask, and dissolved with N,N-dimethylformamide (1 mL). The obtained solution was added with diisopropylethylamine (0.01 mL, 0.09 mmol). The mixed solution was stirred at room temperature for 15 minutes. Then, B1-9 (50.8 mg, 0.04 mmol) was added into the mixed solution. The reaction solution was stirred at room temperature for 3 hours. The reaction solution was filtered and purified by preparative high performance liquid chromatography to obtain B2 (23.3 mg, 36%).

LC-MS: 1499.6[M+H]⁺.

### Example B3: preparation of compound B3

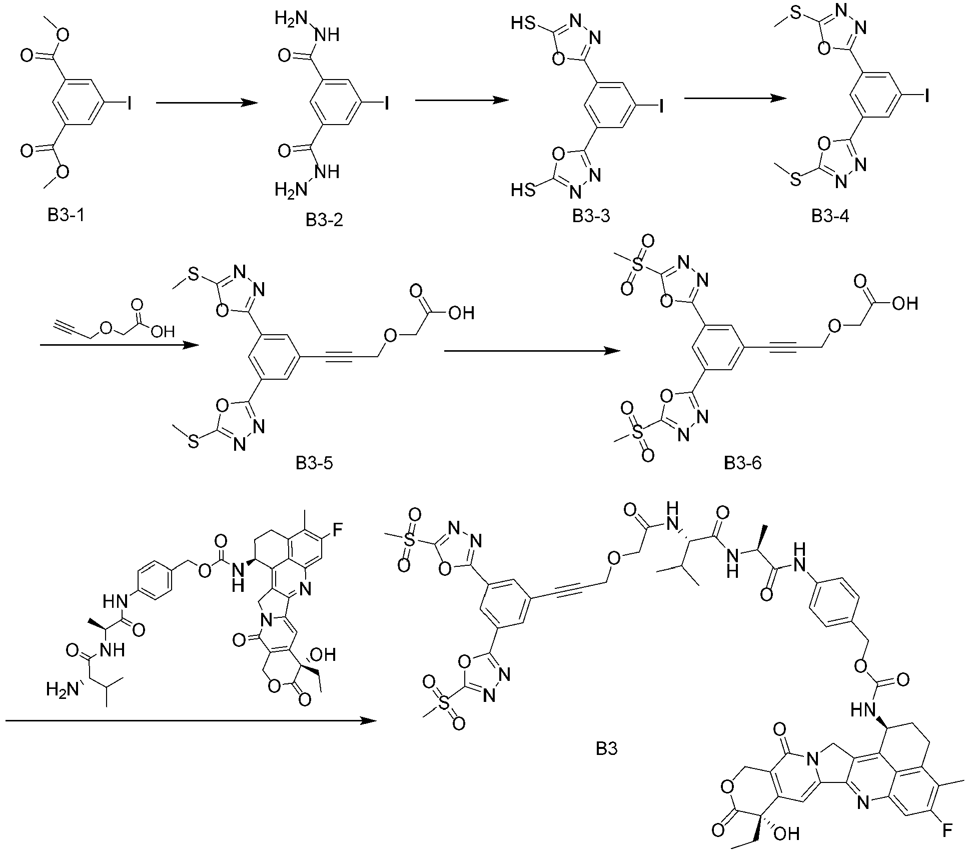

### Step 1: synthesis of 5-iodoisophthalohydrazide (B3-2)

Dimethyl 5-iodoisophthalate B3-1 (1.0 g, 3.12 mmol) and methanol (10 mL) were added into a 100 mL three-neck flask, added with hydrazine hydrate (0.8 g, 15.62 mmol) at room temperature, and stirred at 70°C overnight. The reaction solution was directly concentrated and slurried with methanol to obtain B3-2 (1.0 g, 100%).

LC-MS: 321.0[M+H]⁺.

### Step 2: synthesis of 5,5'-(5-iodo-1,3-phenylene)bis(1,3,4-oxadiazole-2-thiol) (B3-3)

B3-2 (1.0 g, 3.12 mmol) and ethanol (30 mL) were added into a 100 mL single-neck flask, added with potassium hydroxide (0.4 g, 6.25 mmol) and carbon disulfide (1.4 g, 18.75 mmol) at room temperature, and stirred under reflux overnight. The reaction solution was directly concentrated, diluted with water (10 mL), added with 2 M hydrochloric acid to adjust the pH to about 6, and filtered to obtain B3-3 (1.3 g, 100%).

LC-MS: 404.9[M+H]⁺.

### Step 3: synthesis of 5,5'-(5-iodo-1,3-phenylene)bis(2-(methylthio)-1,3,4-oxadiazole) (B3-4)

B3-3 (1.3 g, 3.22 mmol) and tetrahydrofuran (30 mL) were added into a 50 mL three-neck flask, added with triethylamine (2.0 g, 19.30 mmol), iodomethane (1.8 g, 12.86 mmol) and 4-dimethylaminopyridine (80.0 mg, 0.64 mmol) at room temperature, and stirred at room temperature overnight. The reaction solution was directly dried by rotary evaporation, then slurried with n-hexane, and filtered to obtain B3-4 (1.4 g, 100%).

LC-MS: 433.0[M+H]⁺.

### Step 4: synthesis of 2-((3-(3,5-bis(5-methylthio)-1,3,4-oxadiazol-2-yl)phenyl)prop-2-yn-1-yl)oxy)acetic acid (B3-5)

B3-4 (900.0 mg, 2.08 mmol) and N,N-dimethylformamide (10 mL) were added into a 100 mL three-neck flask, added with 2-(prop-2-yn-1-yloxy)acetic acid (356.4 mg, 3.12 mmol), cuprous iodide (99.1 mg, 0.31 mmol), triphenylphosphine (54.6 mg, 0.21 mmol), bis(acetonitrile)palladium(II) chloride (27.0 mg, 0.10 mmol) and diisopropylamine (1.3 g, 12.49 mmol) at room temperature, and stirred at 50°C for 3 hours. The reaction solution was cooled to room temperature, poured into water (50 mL) to adjust the pH to 6, and then extracted with ethyl acetate (30 mL*3). Organic phases were combined, washed with saturated brine (50 mL*3), dried with anhydrous sodium sulfate, filtered, concentrated, and then purified by column chromatography (dichloromethane: methanol = 10: 1) to obtain B3-5 (0.6 g, 69%).

LC-MS: 419.0[M+H]⁺.

### Step 5: synthesis of 2-((3-(3,5-bis(5-(methylsulfonyl)-1,3,4-oxadiazol-2-yl)phenyl)prop-2-yn-1-yl)oxy)acetic acid (B3-6)

B3-5 (0.6 g, 1.43 mmol) and dichloromethane (20 mL) were added into a 100 mL three-neck flask, added with meta-chloroperbenzoic acid (1.2 g, 7.17 mmol) at room temperature, and stirred at room temperature overnight. The reaction solution was filtered, and directly purified by column chromatography (DCM: MeOH=10: 1) to obtain 300 mg of crude product. Then, the crude product was separated by preparative high performance liquid chromatography to obtain B3-6 (70.0 mg, 10%).

LC-MS: 483.0[M+H]⁺.

### Step 6: synthesis of 4-((S)-2-(S)-3-methyl-2-(2-((3-(4-(5-(methylsulfonyl)-1,3,4-oxadiazol-2-yl)phenyl)prop-2-yn-1-yl)oxy)acetamido)propionamido)benzyl((1S,9S)-9-fluoro-9-hydroxy-4-methyl-10,13,10,13, 15-hexahydro-1H,12H-benzo[d]pyrano[3':6,7]indolizino[1,2-b]quinolin-1-yl)carbamate B3

B3-6 (63.9 mg, 0.13 mmol) and 2-(7-azabenzotriazol-1-yl)-N,N,N',N'-tetramethyluronium hexafluorophosphate (75.6 mg, 0.20 mmol) were added into a 10 mL single-neck flask, dissolved with N,N-dimethylformamide (2 mL), and then added with N,N-diisopropylethylamine (34.3 mg, 0.26 mmol). The obtained solution was stirred at room temperature for 10 minutes. Then, 4-((S)-2-(S)-2-amino-3-methylbutanamido)propionamido)benzyl((1S,9S)-9-ethyl-5-fluoro-9-hydr oxy-4-methyl-10,13-dioxo-2,3,9,10,13,15-hexahydro-1H,12H-benzo[d]pyrano[3',4':6,7]indolizin o[1,2-b]quinolin-1-yl )carbamate (100.0 mg, 0.13 mmol) was added into the mixed solution, and stirred at room temperature for 2 hours. The reaction solution was filtered, and separated and purified by preparative high performance liquid chromatography to obtain B3 (22.1 mg, 14%).

LC-MS: 1219.2[M+H]⁺.

### Example B4: preparation of compound B4

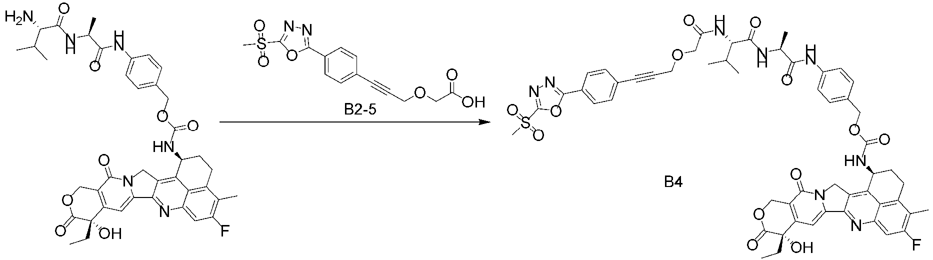

B2-5 (44.6 mg, 0.13 mmol) and 2-(7-azabenzotriazol-1-yl)-N,N,N',N'-tetramethyluronium hexafluorophosphate (75.6 mg, 0.20 mmol) were added into a 10 mL single-neck flask, dissolved with N,N-dimethylformamide (2 mL), and then added with N,N-diisopropylethylamine (34.3 mg, 0.26 mmol). The obtained solution was stirred at room temperature for 10 minutes. Then, 4-((S)-2-(S)-2-amino-3-methylbutanamido)propionamido)benzyl((1S,9S)-9-ethyl-5-fluoro-9-hydr oxy-4-methyl-10,13-dioxo-2,3,9,10,13,15-hexahydro-1H,12H-benzo[d]pyrano[3',4':6,7]indolizin o[1,2-b]quinolin-1-yl)carbamate (100.0 mg, 0.13 mmol) was added into the mixed solution, and stirred at room temperature for 2 hours. The reaction solution was filtered, and separated and purified by preparative high performance liquid chromatography to obtain B4 (21.3 mg, 15%).

LC-MS: 1073.4[M+H]⁺.

### Example B5: preparation of compound B5

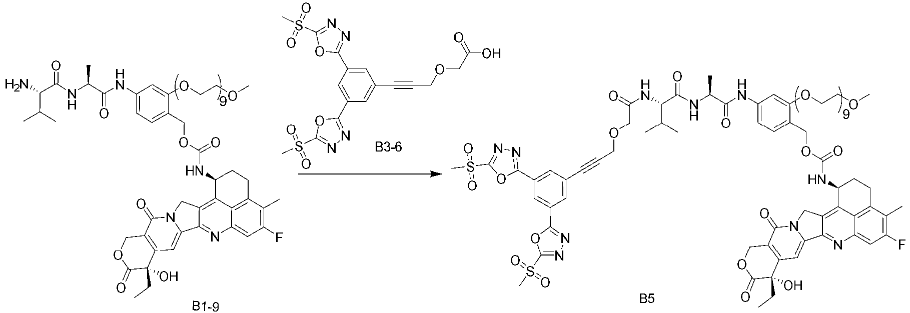

B3-6 (20.0 mg, 0.04 mmol) and 2-(7-azabenzotriazol-1-yl)-N,N,N',N'-tetramethyluronium hexafluorophosphate (23.6 mg, 0.06 mmol) were added into a 10 mL single-neck flask, and dissolved with N,N-dimethylformamide (1 mL). The obtained solution was added with diisopropylethylamine (0.01 mL, 0.08 mmol). The mixed solution was stirred at room temperature for 15 minutes. Then, B1-9 (48.4 mg, 0.04 mmol) was added into the mixed solution. The reaction solution was stirred at room temperature for 3 hours. The reaction solution was filtered and purified by preparative high performance liquid chromatography to obtain B5 (20.5 mg, 29%).

LC-MS: 1645.6[M+H]⁺.

### Example B6: preparation of compound B6

### Step 1: synthesis of 2-methylpropan-2-yl (2S)-2-({[(9H-fluoren-9-ylmethyl)oxy]carbonyl}amino)-6-[(25-oxo-2,5,8,11,14,17,20,23-octao xapentacosan-25-yl)amino]hexanoate (B6-3)

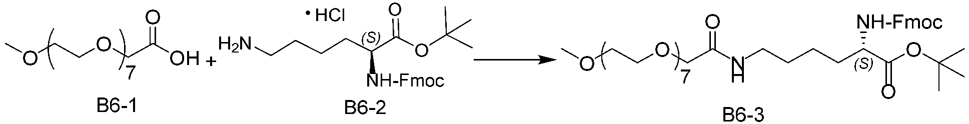

(2,5,8,11,14,17,20-heptaoxadocosan-22-yloxy)acetic acid (B6-1) (400.0 mg, 1.00 mmol) and 2-(7-azabenzotriazol-1-yl)-N,N,N',N'-tetramethyluronium hexafluorophosphate (380.0 mg, 1.00 mmol) were added into a 10 mL single-neck flask, and dissolved with N,N-dimethylformamide (5 mL). The obtained solution was added with diisopropylethylamine (0.25 mL, 2.00 mmol). The mixed solution was stirred at room temperature for 15 minutes. Then, B6-2 (460.2 mg, 1.00 mmol, prepared according to WO 2019208820) was added into the mixed solution. The reaction solution was stirred at room temperature for 3 hours. The reaction solution was filtered and purified by preparative high performance liquid chromatography to obtain B6-3 (620 mg, 77.1%).

LC-MS: 805.4[M+H]⁺.

### Step 2: synthesis of (2S)-2-({[(9H-fluoren-9-ylmethyl)oxy]carbonyl}amino)-6-[(25-oxo-2,5,8,11,14,17,20,23-octao xapentacosan-25-yl)amino]hexanoic acid (B6-4)

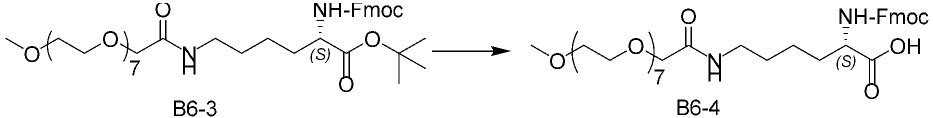

B6-3 (580.0 mg, 0.72 mmol) was added into a 50 mL single-neck flask and dissolved with trifluoroacetic acid (2 mL) and dichloromethane (4 mL). The reaction solution was stirred at room temperature for 1 hour, and then heated to 30°C and stirred for 2 hours. The reaction solution was concentrated under a reduced pressure, dissolved with 10 mL of toluene, and concentrated under a reduced pressure again to obtain B6-4 (540.0 mg, 100%).

LC-MS: 749.4[M+H]⁺.

### Step 3: synthesis of (4-{[(31S,34S,37S)-31-({[(9H-fluoren-9-ylmethyl)oxy]carbonyl}amino)-37-methyl-25,32,35,3 8-tetraoxo-34-(prop-2-yl)-2,5,8,11,14,17,20,23-octaoxa-26,33,36-triazaoctatriacontan-38-yl]a mino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate (B6-6)

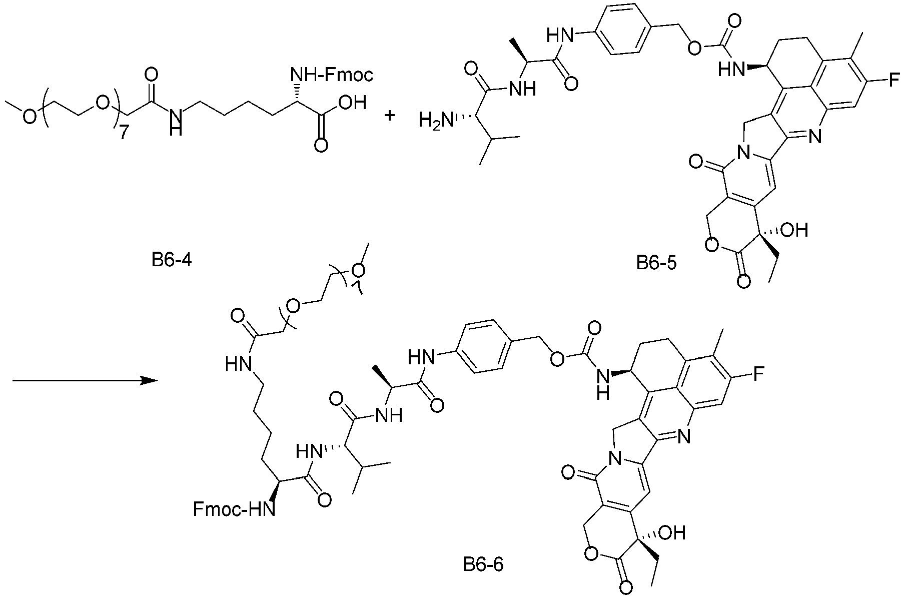

B6-5 (75.4 mg, 0.10 mmol), diisopropylethylamine (0.05 mL, 0.3 mmol) and B6-4 (74.9 mg, 0.10 mmol) were added into a 100 mL single-neck flask, and dissolved with N,N-dimethylformamide (2 mL). The obtained solution was added with 4-(4,6-dimethoxy-1,3,5-triazin-2-yl)-4-methylmorpholine hydrochloride (28.3 mg, 0.10 mmol) in an ice bath. The reaction solution was stirred in an ice bath for 1 hour. The reaction solution was filtered and then purified by preparative high performance liquid chromatography to obtain B6-6 (100 mg, 67.3%).

LC-MS: 1485.7[M+H]⁺.

### Step 4: synthesis of (4-{[(31S,34S,37S)-31-amino-37-methyl-25,32,35,38-tetraoxo-34-(prop-2-yl)-2,5,8,11,14,17,2 0,23-octaoxa-26,33,36-triazaoctatriacontan-38-yl]amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate (B6-7)

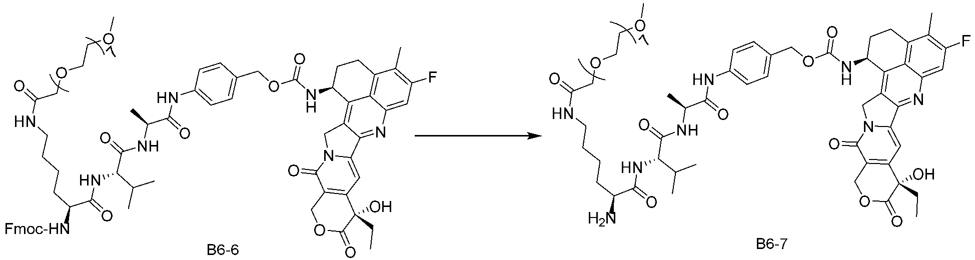

B6-6 (100.0 mg, 0.07 mmol) was added into a 10 mL single-neck flask and dissolved with N,N-dimethylformamide (2 mL). The obtained solution was added with diethylamine (0.07 mL, 0.71 mmol). The reaction solution was stirred at room temperature for 1 hour. The reaction solution was concentrated under a reduced pressure. Residues were slurried with petroleum ether to obtain B6-7 (80 mg, 90.5%).

LC-MS: 1263.6[M+H]⁺.

### Step 5: synthesis of (4-{[(31S,34S,37S)-37-methyl-31-({2-[(3-{4-[5-(methyldioxo-λ6-sulfanyl)-1,3,4-oxadiazol-2-yl ]phenyl}prop-2-ynyl)oxy]acetyl}amino)-25,32,35,38-tetraoxo-34-(prop-2-yl)-2,5,8,11,14,17,2 0,23-octaoxa-26,33,36-triazaoctatriacontan-38-yl]amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B6

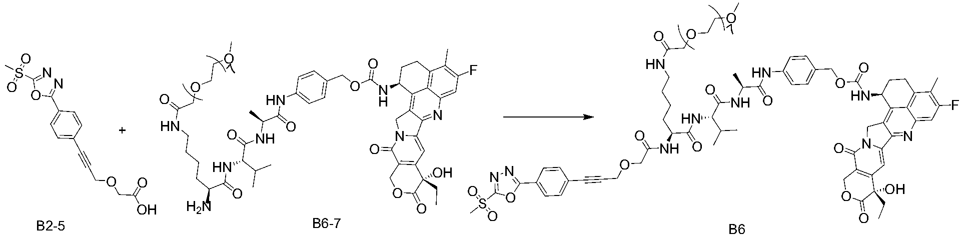

B6-7 (80.0 mg, 0.06 mmol) and 2-(7-azabenzotriazol-1-yl)-N,N,N',N'-tetramethyluronium hexafluorophosphate (22.8 mg, 0.06 mmol) were added into a 10 mL single-neck flask, and dissolved with N,N-dimethylformamide (2 mL). The obtained solution was added with diisopropylethylamine (0.15 mL, 1.2 mmol). The mixed solution was stirred at room temperature for 15 minutes. Then, B2-5 (20.2 mg, 0.06 mmol) was added into the mixed solution. The reaction solution was stirred at room temperature for 3 hours. The reaction solution was filtered and purified by preparative high performance liquid chromatography to obtain B6 (25 mg, 26.3 %).

LC-MS: 1581.6[M+H]⁺.

### Example B7: preparation of compound B7

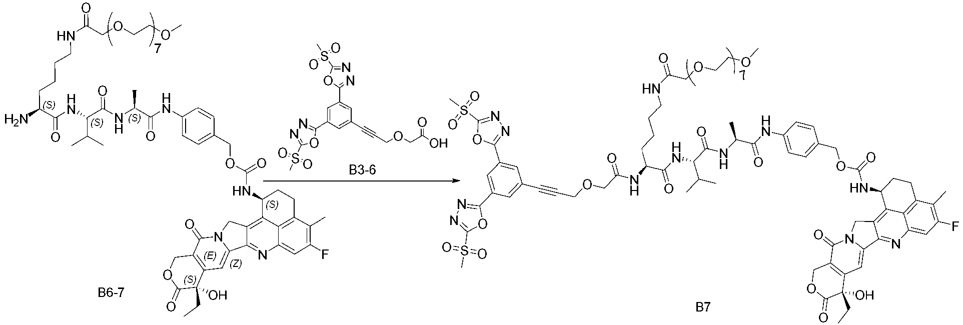

B6-7 (80.0 mg, 0.06 mmol) and 2-(7-azabenzotriazol-1-yl)-N,N,N',N'-tetramethyluronium hexafluorophosphate (22.8 mg, 0.06 mmol) were added into a 10 mL single-neck flask, and dissolved with N,N-dimethylformamide (2 mL). The obtained solution was added with diisopropylethylamine (0.15 mL, 1.2 mmol). The mixed solution was stirred at room temperature for 15 minutes. Then, B3-6 (28.9 mg, 0.06 mmol) was added into the mixed solution. The reaction solution was stirred at room temperature for 3 hours. The reaction solution was filtered and purified by preparative high performance liquid chromatography to obtain B7 (30 mg, 28.9 %).

LC-MS: 1727.6[M+H]⁺.

### Example B8: preparation of compound B8

### Step 1: synthesis of (4-{[(31S,34S,37S)-31-{[1-(9H-fluoren-9-yl)-3,18-dioxo-4-aza-2,7,10,13,16-pentaoxaoctadeca n-18-yl]amino}-37-methyl-25,32,35,38-tetraoxo-34-(prop-2-yl)-2,5,8,11,14,17,20,23-octaoxa-26,33,36-triazaoctatriacontan-38-yl]amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate (B8-1)

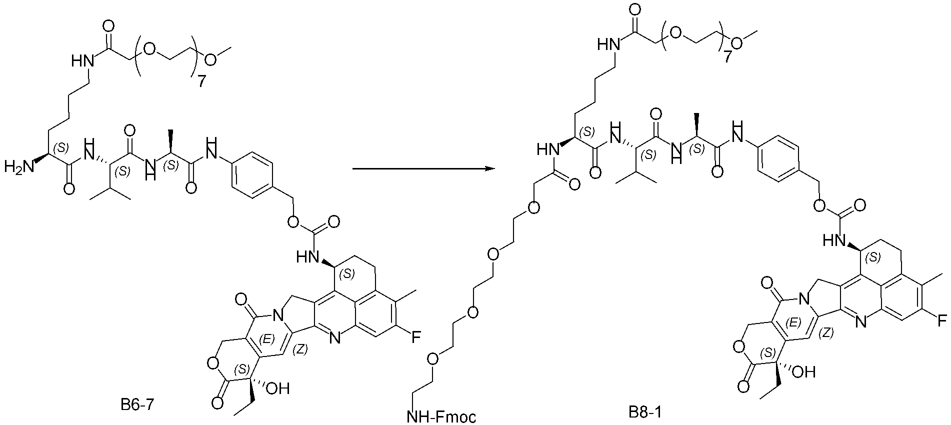

B6-7 (80.0 mg, 0.06 mmol) and 2-(7-azabenzotriazol-1-yl)-N,N,N',N'-tetramethyluronium hexafluorophosphate (22.8 mg, 0.06 mmol) were added into a 10 mL single-neck flask, and dissolved with N,N-dimethylformamide (2 mL). The obtained solution was added with diisopropylethylamine (0.15 mL, 1.2 mmol). The mixed solution was stirred at room temperature for 15 minutes. Then, {[1-(9H-fluoren-9-yl)-3-oxo-4-aza-2,7,10,13-tetraoxapentadecan-15-yl]oxy}acetic acid (28.4 mg, 0.06 mmol) was added into the mixed solution. The reaction solution was dried by rotary evaporation, eluted with 5% methanol in dichloromethane by silica gel column chromatography to obtain B8-1 (50 mg, 48.2%).

LC-MS: 1718.9 [M+H]⁺.

### Step 2: synthesis of (4-{[(31S,34S,37S)-31-[(14-amino-1-oxo-3,6,9,12-tetraoxatetradecan-1-yl)amino]-37-methyl-25,32,35,38-tetraoxo-34-(prop-2-yl)-2,5,8,11,14,17,20,23-octaoxa-26,33,36-triazaoctatriacont an-38-yl]amino}phenyl)methyl f [(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate (B8-2)

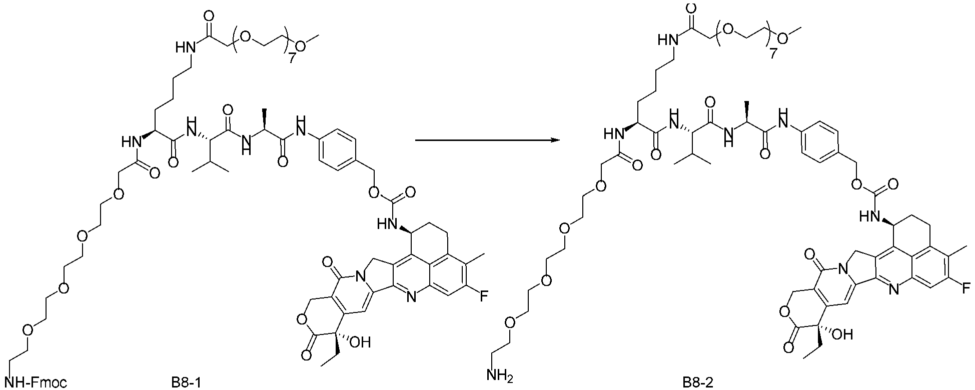

B8-1 (50.0 mg, 0.03 mmol) was added into a 10 mL single-neck flask and dissolved with N,N-dimethylformamide (1 mL). The obtained solution was added with diethylamine (0.45 mL, 0.3 mmol). The reaction solution was stirred at room temperature for 1 hour. The reaction solution was concentrated under a reduced pressure. Residues were slurried with petroleum ether to obtain B8-2 (40 mg, 89.1%).

LC-MS: 1496.8[M+H]⁺.

### Step 3: synthesis of (4-{[(31S,34S,37S)-37-methyl-31-[(21-{4-[5-(methyldioxo-λ⁶-sulfanyl)-1,3,4-oxadiazol-2-yl]p henyl}-1,16-dioxo-15-aza-3,6,9,12,18-pentaoxahenicos-20-yn-1-yl)amino]-25,32,35,38-tetrao xo-34-(prop-2-yl)-2,5,8,11,14,17,20,23-octaoxa-26,33,36-triazaoctatriacontan-38-yl]amino}p henyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B8

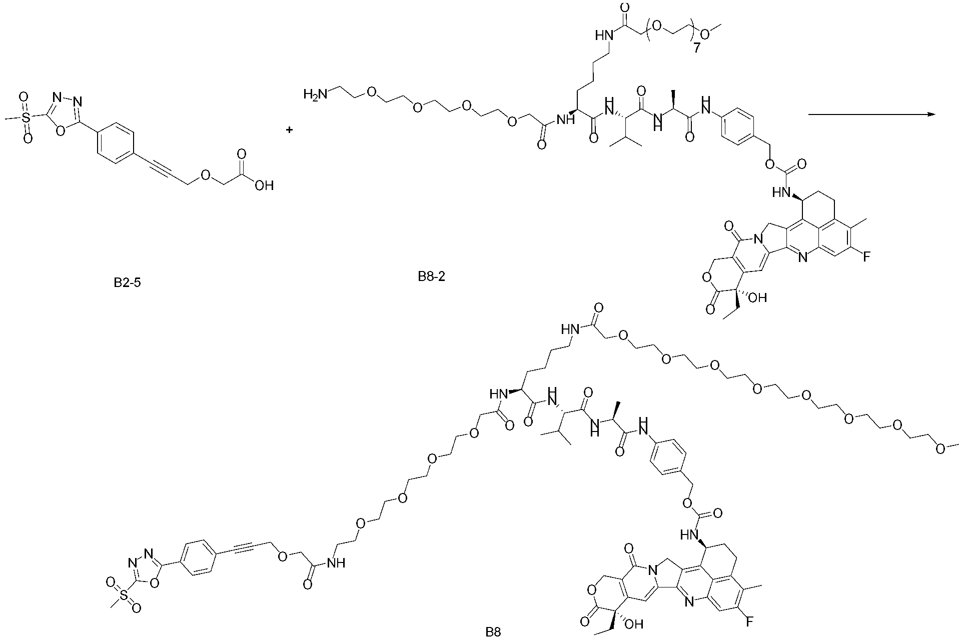

B8-2 (40.0 mg, 0.03 mmol) and 2-(7-azabenzotriazol-1-yl)-N,N,N',N'-tetramethyluronium hexafluorophosphate (11.4 mg, 0.03 mmol) were added into a 10 mL single-neck flask, and dissolved with N,N-dimethylformamide (1 mL). The obtained solution was added with diisopropylethylamine (0.03 mL, 0.06 mmol). The mixed solution was stirred at room temperature for 15 minutes. Then, B2-5 (10.1 mg, 0.03 mmol) was added into the mixed solution. The reaction solution was stirred at room temperature for 3 hours. The reaction solution was filtered and purified by preparative high performance liquid chromatography to obtain the compound B8 (15 mg, 27.6 %).

LC-MS: 1814.8[M+H]⁺.

1H NMR (400 MHz, DMSO) δ 9.99 (s, 1H), 8.17 (d, J = 6.7 Hz, 1H), 8.10 (d, J = 8.5 Hz, 2H), 8.05 (d, J = 8.8 Hz, 1H), 7.88 (d, J = 8.7 Hz, 1H), 7.82 (t, J = 5.7 Hz, 1H), 7.77 (d, J = 10.9 Hz, 1H), 7.72 (d, J = 8.5 Hz, 2H), 7.64 (dd, J = 10.5, 5.3 Hz, 2H), 7.59 (d, J = 8.4 Hz, 2H), 7.36 (d, J = 8.5 Hz, 2H), 7.31 (s, 1H), 6.51 (s, 1H), 5.44 (s, 2H), 5.34 - 5.21 (m, 3H), 5.07 (s, 2H), 4.53 (s, 2H), 4.38 (dd, J = 13.5, 6.5 Hz, 2H), 4.23 - 4.16 (m, 1H), 4.02 (s, 2H), 3.91 (s, 2H), 3.84 (s, 2H), 3.71 (s, 3H), 3.60 - 3.49 (m, 37H), 3.42 (dt, J = 6.7, 5.2 Hz, 4H), 3.29 - 3.25 (m, 4H), 3.23 (s, 3H), 3.13 (s, 1H), 3.07 - 3.02 (m, 2H), 2.37 (s, 3H), 2.19 (s, 2H), 2.00 - 1.93 (m, 1H), 1.92 - 1.81 (m, 2H), 1.65 (s, 1H), 1.54 (d, J = 8.8 Hz, 1H), 1.39 (s, 2H), 1.29 (d, J = 7.0 Hz, 3H), 1.24 (s, 2H), 0.90 - 0.81 (m, 9H).

### Example B9: preparation of compound B9

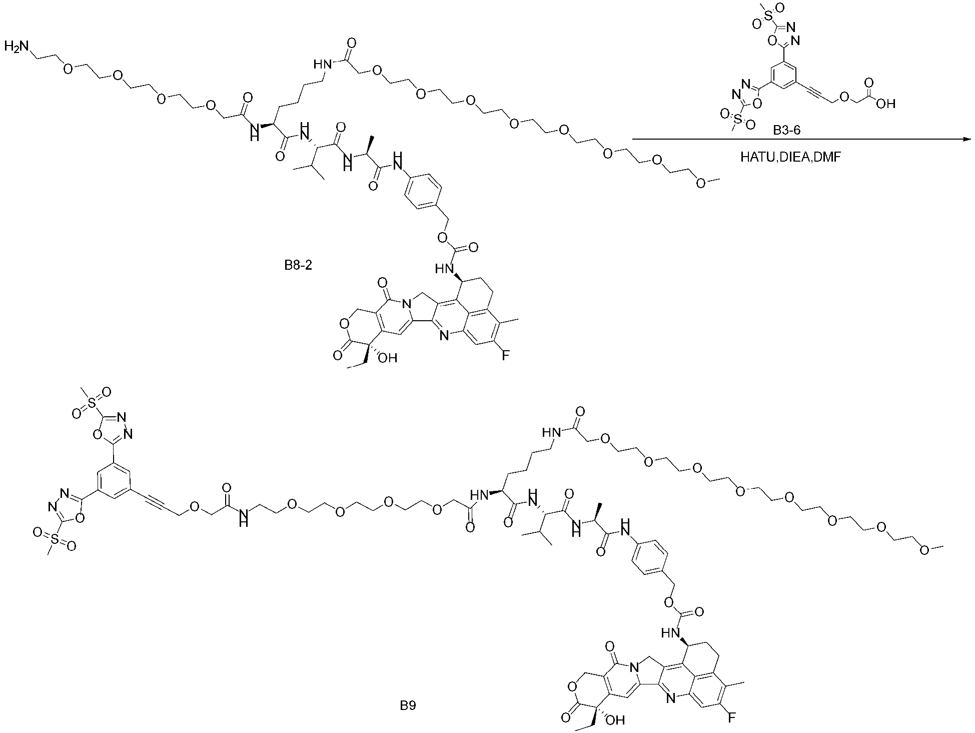

### Step 1: synthesis of (4-{[(31S,34S,37S)-31-[(21-{3,5-bis[5-(methyldioxo-λ⁶-sulfanyl)-1,3,4-oxadiazol-2-yl]phenyl}-1,16-dioxo-15-aza-3,6,9,12,18-pentaoxahenicos-20-yn-1-yl)amino]-37-methyl-25,32,35,38-tet raoxo-34-(prop-2-yl)-2,5,8,11,14,17,20,23-octaoxa-26,33,36-triazaoctatriacontan-38-yl]amin o}phenyl)methyl { [(1 S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B9

B8-2 (40.0 mg, 0.03 mmol) and 2-(7-azabenzotriazol-1-yl)-N,N,N',N'-tetramethyluronium hexafluorophosphate (11.4 mg, 0.03 mmol) were added into a 10 mL single-neck flask, and dissolved with N,N-dimethylformamide (1 mL). The obtained solution was added with diisopropylethylamine (0.03 mL, 0.06 mmol). The mixed solution was stirred at room temperature for 15 minutes. Then, B3-6 (14.5 mg, 0.03 mmol) was added into the mixed solution. The reaction solution was stirred at room temperature for 3 hours. The reaction solution was filtered and purified by preparative high performance liquid chromatography to obtain the compound B9 (15 mg, 25.5 %).

LC-MS: 1960.8[M+H]⁺.

### Example B10: preparation of compound B10

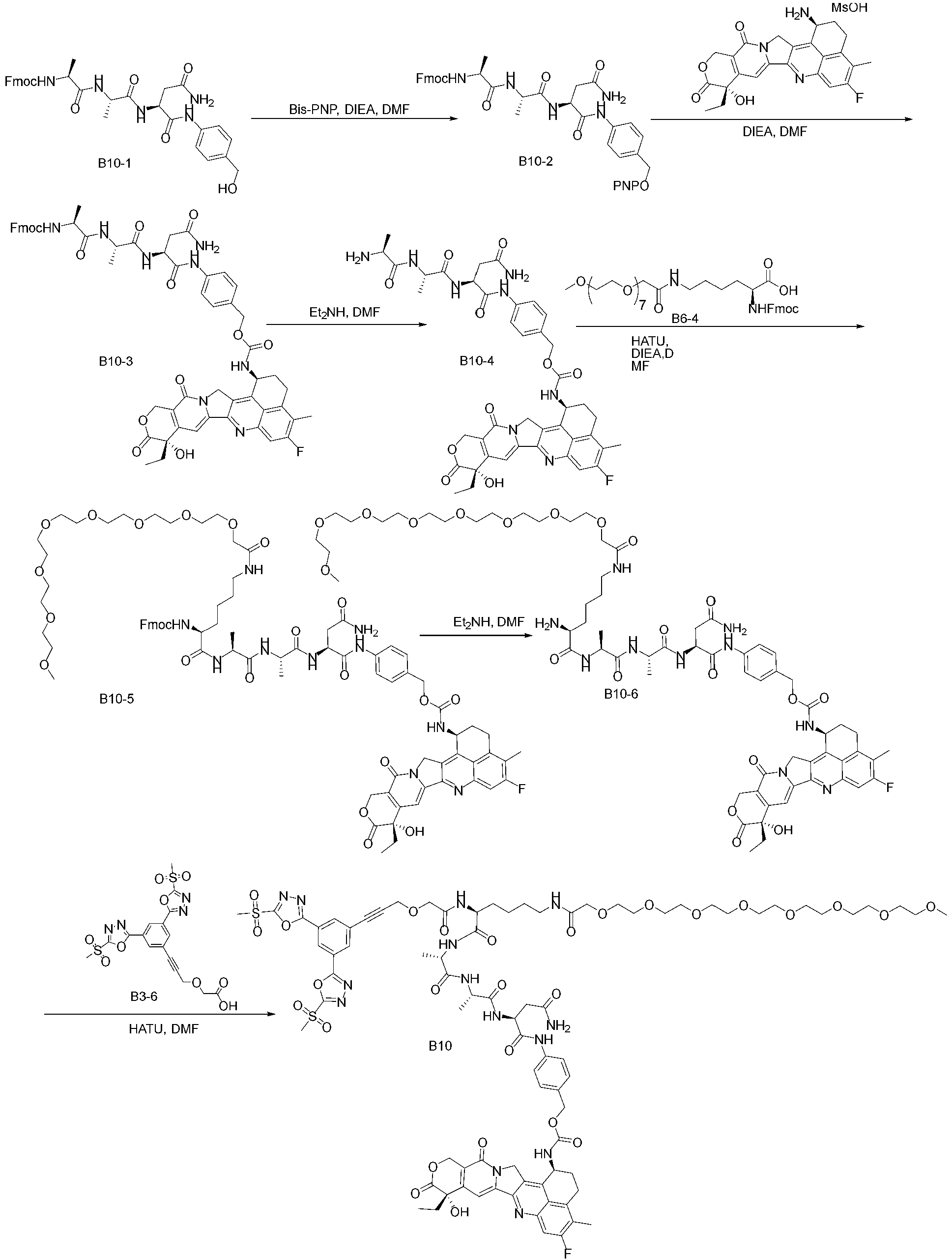

### Step 1: synthesis of 9H-fluoren-9-ylmethyl {[(2S,5S,8S)-2-(formamidomethyl)-5-methyl-1-({4-[({[(4-nitrophenyl)oxy]carbonyl}oxy)met hyl]phenyl}amino)-1,4,7-trioxo-3,6-diazanon-8-yl]amino}formate (B10-2)

9H-fluoren-9-ylmethyl {[(2S,5S,8S)-2-(formamidomethyl)-1-{[4-(hydroxymethyl)phenyl]amino}-5-methyl-1,4,7-trioxo-3,6-diazanon-8-yl]amino}formate (B10-1) (600.0 mg, 1.0 mmol) and bis(p-nitrophenyl)carbonate (608 mg, 2.0 mmol) were added into a 100 mL single-neck flask, and dissolved with N,N-dimethylformamide (6 mL). The obtained solution was added with diisopropylethylamine (0.4 mL, 2.55 mmol) and 4-dimethylaminopyridine (0.8 mg, 0.01 mmol). The reaction solution was stirred at room temperature overnight. The reaction solution was concentrated under a reduced pressure and slurried with methanol to obtain B10-2 (600.0 mg, 78.3 %).

LC-MS: 767.3[M+H]⁺.

### Step 2: synthesis of 9H-fluoren-9-ylmethyl {[(2S,5S,8S)-2-(formamidomethyl)-1-[(4-{[({[(1S,9S)-9-ethyl-5-fluoro-9-hydroxy-4-methyl-1 0,13-dioxo-2,3,9,10,12,15-hexahydro-1H-cyclohexo[1,2,3-de]pyrano[3',4':6,7]indolizino[1,2-b]quinolin-1-yl]amino}carbonyl)oxy)methyl}phenyl)amino]-5-methyl-1,4,7-trioxo-3,6-diaza non-8-yl]amino}formate (B10-3)

B10-2 (100.0 mg, 0.13 mmol), exatecan mesylate (69.3 mg, 0.13 mmol) and 1-hydroxybenzotriazole (17.6 mg, 0.13 mmol) were added into a 100 mL single-neck flask and dissolved with N,N-dimethylformamide (2 mL). The obtained solution was stirred at room temperature for 30 minutes, and then added with diisopropylethylamine (0.06 mL, 0.39 mmol). The reaction solution was stirred at room temperature for 3 hours, and the turbid solution became clear. The reaction solution was concentrated under a reduced pressure. Residues were purified by silica gel column chromatography (dichloromethane: methanol = 94: 6) to obtain B10-3 (110.0 mg, 79.6 %).

LC-MS: 1063.4[M+H]⁺.

### Step 3: synthesis of (4-1[(2S,5S,8S)-8-amino-2-(formamidomethyl)-5-methyl-1,4,7-trioxo-3,6-diazanon-1-yl]amin o}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate (B10-4)

B10-3 (110.0 mg, 0.10 mmol) was added into a 10 mL single-neck flask and dissolved with N,N-dimethylformamide (1 mL). The obtained solution was added with diethylamine (73 mg, 1.0 mmol). The reaction solution was stirred at room temperature for 1 hour. The reaction solution was concentrated under a reduced pressure. Residues were slurried with petroleum ether to obtain B10-4 (75 mg, 89.2%).

LC-MS: 841.3[M+H]⁺.

### Step 4: synthesis of (4-{[(31S,34S,37S,40S)-40-(formamidomethyl)-31-({[(9H-fluoren-9-ylmethyl)oxy]carbonyl}a mino)-34,37-dimethyl-25,32,35,38,41-pentaoxo-2,5,8,11,14,17,20,23-octaoxa-26,33,36,39-tetr aazahentetracontan-41-yl]amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate (B10-5)

B10-4 (40.0 mg, 0.03 mmol) and 2-(7-azabenzotriazol-1-yl)-N,N,N',N'-tetramethyluronium hexafluorophosphate (11.4 mg, 0.03 mmol) were added into a 10 mL single-neck flask, and dissolved with N,N-dimethylformamide (1 mL). The obtained solution was added with diisopropylethylamine (0.03 mL, 0.06 mmol). The mixed solution was stirred at room temperature for 15 minutes. Then, B6-4 (22.5 mg, 0.03 mmol) was added into the mixed solution. The reaction solution was stirred at room temperature for 3 hours. The reaction solution was filtered and purified by preparative high performance liquid chromatography to obtain B10-5 (35 mg, 74.2 %).

LC-MS: 1571.8[M+H]⁺.

### Step 5: synthesis of (4-{[(31S,34S,37S,40S)-31-amino-40-(formamidomethyl)-34,37-dimethyl-25,32,35,38,41-pent aoxo-2,5,8,11,14,17,20,23-octaoxa-26,33,36,39-tetraazahentetracontan-41-yl]amino}phenyl) methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate (B10-6)

B10-5 (35.0 mg, 0.02 mmol) was added into a 10 mL single-neck flask and dissolved with N,N-dimethylformamide (1 mL). The obtained solution was added with diethylamine (14.6 mg, 0.2 mmol). The reaction solution was stirred at room temperature for 1 hour. The reaction solution was concentrated under a reduced pressure. Residues were slurried with petroleum ether to obtain B10-6 (20 mg, 74.1%).

LC-MS: 1349.6[M+H]⁺.

### Step 6: synthesis of (4-{[(31S,34S,37S,40S)-31-({2-[(3-{3,5-bis[5-(methyldioxo-λ⁶-sulfanyl)-1,3,4-oxadiazol-2-yl]p henyl}prop-2-ynyl)oxy]acetyl}amino)-40-(formamidomethyl)-34,37-dimethyl-25,32,35,38,41 -pentaoxo-2,5,8,11,14,17,20,23-octaoxa-26,33,36,39-tetraazahentetracontan-41-yl]amino}phe nyl)methyl f [(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B10

B10-6 (20.0 mg, 0.015 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, B3-6 (7.3 mg, 0.015 mmol) was added into the mixed solution. The reaction solution was stirred at room temperature for 3 hours. The reaction solution was filtered and purified by preparative high performance liquid chromatography to obtain B10 (8 mg, 29.4 %).

LC-MS: 1813.6[M+H]⁺.

### Example B11: preparation of compound B11

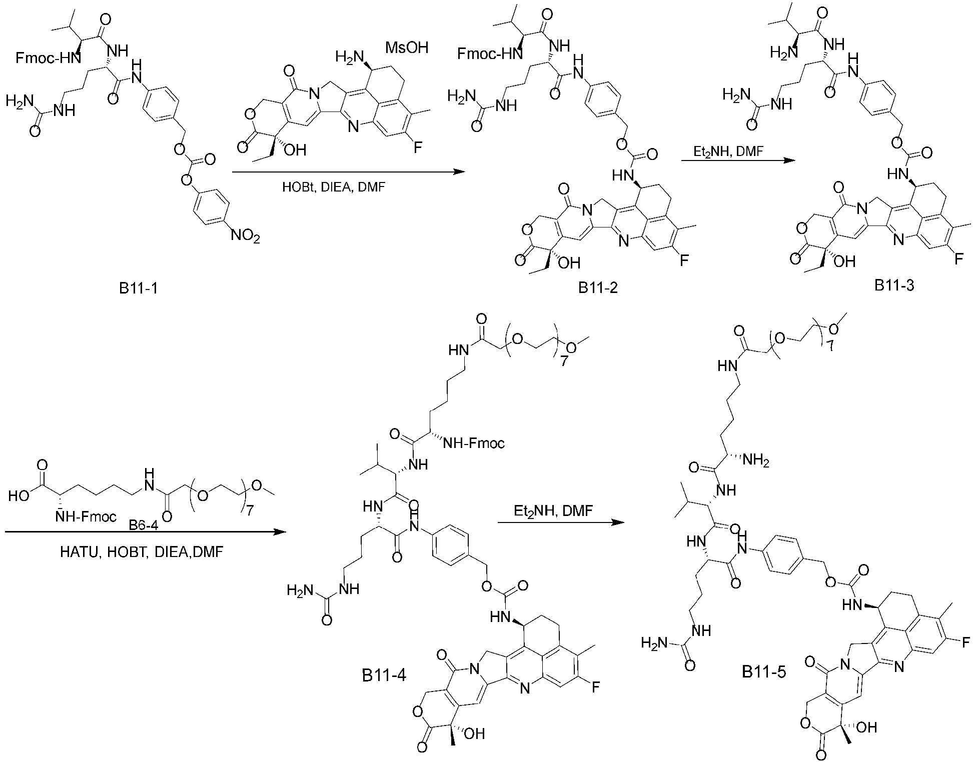

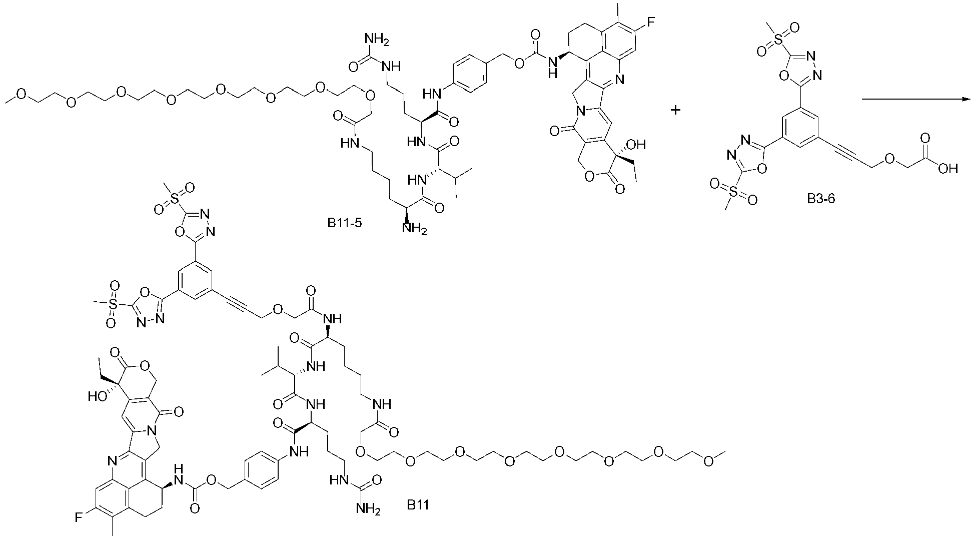

### Step 1: synthesis of (4-{[(5S,8S)-8-{3-[(aminocarbonyl)amino]propyl}-1-(9H-fluoren-9-yl)-3,6,9-trioxo-5-(prop-2 -yl)-4,7-diaza-2-oxanon-9-yl]amino}phenyl)methyl { [(1 S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate (B11-2)

B11-1 (70.0 mg, 0.09 mmol, CAS: 863971-53-3, purchased from Leyan), exatecan mesylate (48.0 mg, 0.09 mmol) and 1-hydroxybenzotriazole (12.2 mg, 0.09 mmol) were added into a 50 mL single-neck flask and dissolved with N,N-dimethylformamide (1 mL). The obtained solution was stirred at room temperature for 30 minutes, and then added with diisopropylethylamine (0.03 mL, 0.18 mmol). The reaction solution was stirred at room temperature for 3 hours, and the turbid solution became clear. The reaction solution was concentrated under a reduced pressure. Residues were purified by silica gel column chromatography (dichloromethane: methanol = 94: 6) to obtain B11-2 (85.0 mg, 88.9%).

LC-MS: 1063.1 [M+H]⁺.

### Step 2: synthesis of (4-{[(2S)-5-[(aminocarbonyl)amino]-2-{[(2S)-2-amino-3-methyl-1-oxobutyl]amino}-1-oxope ntyl]amino}phenyl)methyl f [(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B11-3

B11-2 (85.0 mg, 0.08 mmol) was added into a 25 mL single-neck flask and dissolved with N,N-dimethylformamide (2 mL). The obtained solution was added with diethylamine (0.07 mL, 0.71 mmol). The reaction solution was stirred at room temperature for 1 hour. The reaction solution was concentrated under a reduced pressure. Residues were slurried with petroleum ether to obtain B11-3 (60 mg, 89.5%).

LC-MS: 841.2[M+H]⁺.

### Step 3: synthesis of (4-{[(31S,34S,37S)]}-37-{3-[(aminocarbonyl)amino]propyl}-31-({[(9H-fluoren-9-ylmethyl)ox y]carbonyl}amino)-25,32,35,38-tetraoxo-34-(prop-2-yl)-2,5,8,11,14,17,20,23-octaoxa-26,33,3 6-triazaoctatriacontan-38-yl]amino}phenyl)methyl {[(1S,9S)-5-fluoro-9-hydroxy-4,9-dimethyl-10,13-dioxo-2,3,9,10,12,15-hexahydro-1H-cycloh exo[1,2,3-de]pyrano[3',4':6,7]indolizino[1,2-b]quinolin-1-yl]amino}formate B11-4

B11-3 (60 mg, 0.07 mmol), diisopropylethylamine (0.05 mL, 0.3 mmol) and B6-4 (74.9 mg, 0.10 mmol) were added into a 100 mL single-neck flask, and dissolved with N,N-dimethylformamide (2 mL). The obtained solution was added with HOBT (2 mg, 0.01 mmol) and HATU (38 mg, 0.10 mmol) in an ice bath. The reaction solution was stirred in an ice bath for 1 hour. The reaction solution was filtered and then purified by preparative high performance liquid chromatography to obtain B11-4 (86 mg, 78.9%).

LC-MS:1558.1 [M+H]⁺.

### Step 4: synthesis of (4-{[(31S,34S,37S)-31-amino-37-{3-[(aminocarbonyl)amino]propyl}-25,32,35,38-tetraoxo-34 -(prop-2-yl)-2,5,8,11,14,17,20,23-octaoxa-26,33,36-triazaoctatriacontan-38-yl]amino}phenyl) methyl {[(1S,9S)-5-fluoro-9-hydroxy-4,9-dimethyl-10,13-dioxo-2,3,9,10,12,15-hexahydro-1H-cycloh exo[1,2,3-de]pyrano[3',4':6,7]indolizino[1,2-b]quinolin-1-yl]amino}formate B11-5

B11-4 (86.0 mg, 0.055 mmol) was added into a 25 mL single-neck flask and dissolved with N,N-dimethylformamide (2 mL). The obtained solution was added with diethylamine (0.05 mL, 0.55 mmol). The reaction solution was stirred at room temperature for 1 hour. The reaction solution was concentrated under a reduced pressure. Residues were slurried with petroleum ether to obtain B11-5 (60 mg, 81.6%).

LC-MS:1335.8 [M+H]⁺.

### Step 5: synthesis of (4-{[(31S,34S,37S)-37-{3-[(aminocarbonyl)amino]propyl}-31-({2-[(1-{3,5-bis[5-(methyldioxo -λ6-sulfanyl)-1,3,4-oxadiazol-2-yl]phenyl}prop-1-yn-3-yl)oxy]acetyl}amino)-25,32,35,38-tetr aoxo-34-(prop-2-yl)-2,5,8,11,14,17,20,23-octaoxa-26,33,36-triazaoctatriacontan-38-yl]amino} phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B11

B11-5 (20.0 mg, 0.015 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, B3-6 (7.3 mg, 0.015 mmol) was added into the mixed solution. The reaction solution was stirred at room temperature for 3 hours. The reaction solution was filtered and purified by preparative high performance liquid chromatography to obtain B11 (8 mg, 29.4 %).

LC-MS: 1813.7[M+H]⁺.

### Example B12: preparation of compound B12

### Step 1: synthesis of 9H-fluoren-9-ylmethyl {[(31S,34S,37S)-38-[(4-{[({[(1S,9S)-9-ethyl-5-fluoro-9-hydroxy-4-methyl-10,13-dioxo-2,3,9,1 0,12,15-hexahydro-1H-cyclohexo[1,2,3-de]pyrano[3',4':6,7]indolizino[1,2-b]quinolin-1-yl]a mino}carbonyl)oxy)methyl}-3-(2,5,8,11,14,17,20,23,26-nonaoxaoctacosan-28-yloxy)phenyl)a mino]-37-methyl-25,32,35,38-tetraoxo-34-(prop-2-yl)-2,5,8,11,14,17,20,23-octaoxa-26,33,36-t riazaoctatriacontan-31-yl]amino}formate (B12-2)

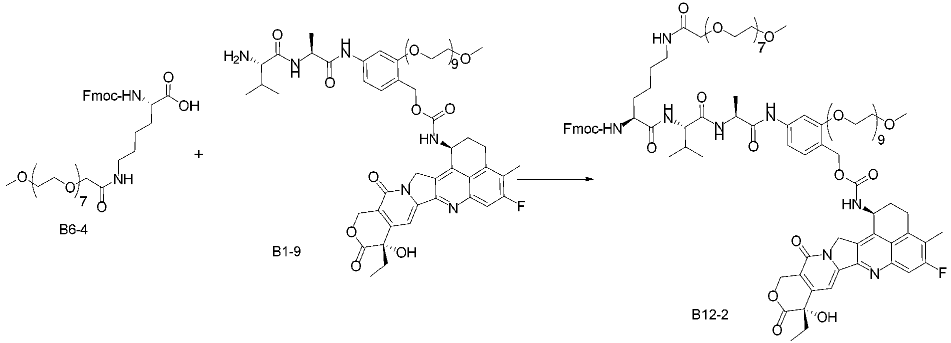

B1-9 (100 mg, 0.08 mmol), diisopropylethylamine (0.03 mL, 0.18 mmol) and B6-4 (60.0 mg, 0.08 mmol) were added into a 100 mL single-neck flask, and dissolved with N,N-dimethylformamide (2 mL). The obtained solution was added with 4-(4,6-dimethoxy-triazin-2-yl)-4-methylmorpholine hydrochloride (28.3 mg, 0.10 mmol) in an ice bath. The reaction solution was stirred in an ice bath for 1 hour. The reaction solution was filtered and then purified by preparative high performance liquid chromatography to obtain B12-2 (90 mg, 58.8 %).

LC-MS: 1913.0[M+H]⁺.

### Step 2: synthesis of (4-{[(31S,34S,37S)-31-amino-37-methyl-25,32,35,38-tetraoxo-34-(prop-2-yl)-2,5,8,11,14,17,2 0,23-octaoxa-26,33,36-triazaoctatriacontan-38-yl]amino}-2-(2,5,8,11,14,17,20,23,26-nonaoxa octacosan-28-yloxy)phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate (B12-3)

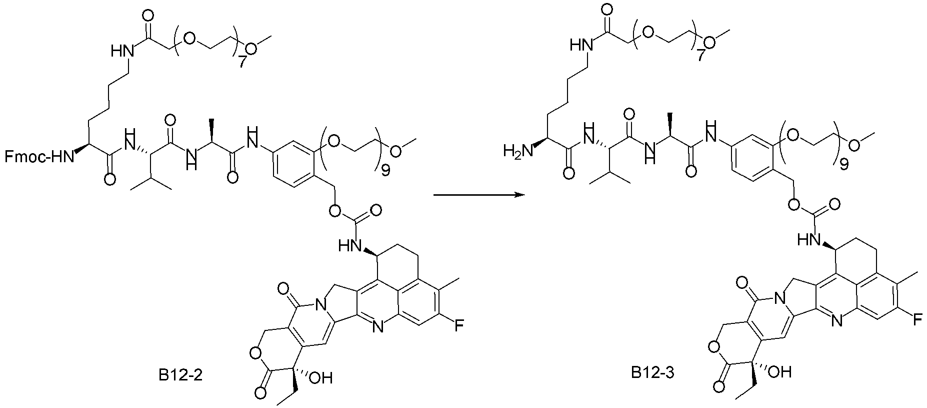

B12-2 (90.0 mg, 0.05 mmol) was added into a 10 mL single-neck flask and dissolved with N,N-dimethylformamide (1 mL). The obtained solution was added with diethylamine (36.5 mg, 0.5 mmol). The reaction solution was stirred at room temperature for 1 hour. The reaction solution was concentrated under a reduced pressure. Residues were slurried with petroleum ether to obtain B12-3 (70 mg, 82.8%).

LC-MS: 1690.1[M+H]⁺.

### Step 3: synthesis of (4-{[(31S,34S,37S)-37-methyl-31-({2-[(3-{4-[5-(methyldioxo-λ⁶-sulfanyl)-1,3,4-oxadiazol-2-yl ]phenyl}prop-2-ynyl)oxy]acetyl}amino)-25,32,35,38-tetraoxo-34-(prop-2-yl)-2,5,8,11,14,17,2 0,23-octaoxa-26,33,36-triazaoctatriacontan-38-yl]amino }-2-(2,5,8,11,14,17,20,23,26-nonaoxa octacosan-28-yloxy)phenyl)methyl { [(1 S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B12

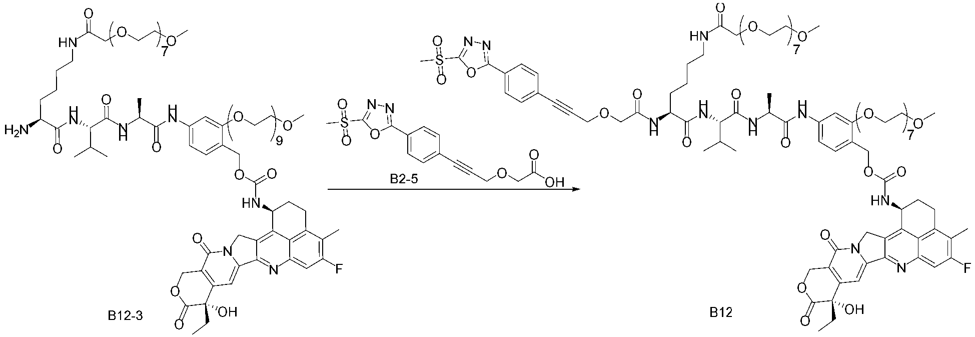

B12-3 (25.0 mg, 0.015 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, B2-5 (5.0 mg, 0.015 mmol) was added into the mixed solution. The reaction solution was stirred at room temperature for 3 hours. The reaction solution was filtered and purified by preparative high performance liquid chromatography to obtain B12 (5.2 mg, 17.3 %).

LC-MS: 2008.9[M+H]⁺.

### Example B13: preparation of compound B13

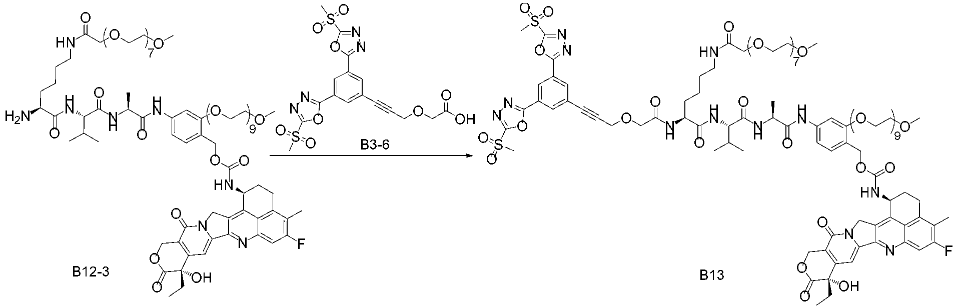

### Step 1: synthesis of (4-{[(31S,34S,37S)-31-({2-[(3-{3,5-bis[5-(methyldioxo-λ⁶-sulfanyl)-1,3,4-oxadiazol-2-yl]phen yl}prop-2-ynyl)oxy]acetyl}amino)-37-methyl-25,32,35,38-tetraoxo-34-(prop-2-yl)-2,5,8,11,14, 17,20,23-octaoxa-26,33,36-triazaoctatriacontan-38-yl]amino}-2-(2,5,8,11,14,17,20,23,26-non aoxaoctacosan-28-yloxy)phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B13

B12-3 (25.0 mg, 0.015 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, B3-6 (7.2 mg, 0.015 mmol) was added into the mixed solution. The reaction solution was stirred at room temperature for 3 hours. The reaction solution was filtered and purified by preparative high performance liquid chromatography to obtain B13 (8.5 mg, 26.3 %).

LC-MS: 2154.9[M+H]⁺.

### Example B14: preparation of compound B14

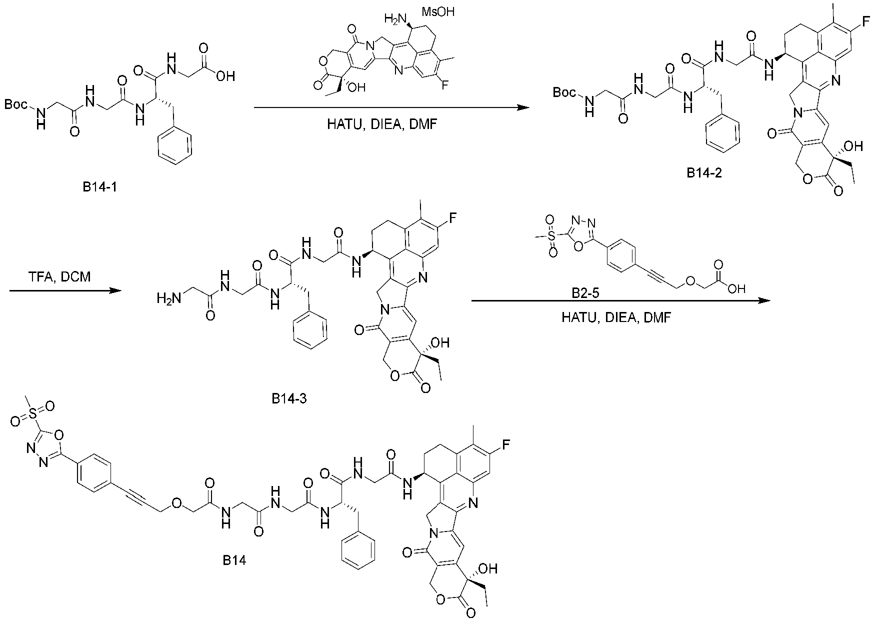

### Step 1: synthesis of 2-methylpropan-2-yl {[(7S)-7-benzyl-11-{[(1S,9S)-9-ethyl-5-fluoro-9-hydroxy-4-methyl-10,13-dioxo-2,3,9,10,12,15 -hexahydro-1H-cyclohexo[1,2,3-de]pyrano[3',4':6,7]indolizino[1,2-b]quinolin-1-yl]amino}-2, 5,8,11-tetraoxo-3,6,9-triazaaundecan-1-yl]amino}formate (B14-2)

N-[(12S)-12-benzyl-2,2-dimethyl-4,7,10,13-tetraoxo-3-oxa-5,8,11-triazatridecan-13-yl]glyci ne (B14-1) (100.0 mg, 0.23 mmol) and 2-(7-azabenzotriazol-1-yl)-N,N,N',N'-tetramethyluronium hexafluorophosphate (87.5 mg, 0.23 mmol) were added into a 100 mL single-neck flask, and dissolved with N,N-dimethylformamide (2 mL). The obtained solution was added with diisopropylethylamine (89 mg, 0.69 mmol). The mixed solution was stirred at room temperature for 15 minutes. Then, exatecan mesylate (122.2 mg, 0.23 mmol) was added into the mixed solution. The reaction solution was stirred at room temperature for 3 hours. The reaction solution was dried by evaporation and purified by silica gel column chromatography (dichloromethane: methanol = 94: 6) to obtain B14-2 (120 mg 61.2 %).

LC-MS: 854.3[M+H]⁺.

### Step 2: synthesis of N-[(1S,9S)-9-ethyl-5-fluoro-9-hydroxy-4-methyl-10,13-dioxo-2,3,9,10,12,15-hexahydro-1H-c yclohexo[1,2,3-de]pyrano[3',4':6,7]indolizino[1,2-b]quinolin-1-yl]-2-{[(2S)-8-amino-2-benzyl -1,4,7-trioxo-3,6-diazaoct-1-yl]amino}acetamide (B14-3)

B14-2 (120.0 mg, 0.14 mmol) was added into a 50 mL single-neck flask and dissolved with trifluoroacetic acid (1 mL) and dichloromethane (2 mL). The reaction solution was stirred at room temperature for 1 hour, and then heated to 30°C and stirred for 2 hours. The reaction solution was concentrated under a reduced pressure, dissolved with 10 mL of toluene, and concentrated under a reduced pressure again to obtain B14-3 (106.0 mg, 100%).

LC-MS: 754.3[M+H]⁺.

### Step 3: synthesis of N-[(7S)-7-benzyl-11-{[(1S,9S)-9-ethyl-5-fluoro-9-hydroxy-4-methyl-10,13-dioxo-2,3,9,10,12,1 5-hexahydro-1H-cyclohexo[1,2,3-de]pyrano[3',4':6,7]indolizino[1,2-b]quinolin-1-yl]amino}-2,5,8,11-tetraoxo-3,6,9-triazaaundecan-1-yl]-2-[(3-{4-[2-(methyldioxo-λ⁶-sulfanyl)-1,3,4-oxad iazol-5-yl]phenyl}prop-2-ynyl)oxy]acetamide B14

B14-3 (11.3 mg, 0.015 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, B2-5 (5.0 mg, 0.015 mmol) was added into the mixed solution. The reaction solution was stirred at room temperature for 3 hours. The reaction solution was filtered and purified by preparative high performance liquid chromatography to obtain the compound B14 (5.5 mg, 34.2 %).

LC-MS: 1072.3[M+H]⁺.

### Example B15: preparation of compound B15

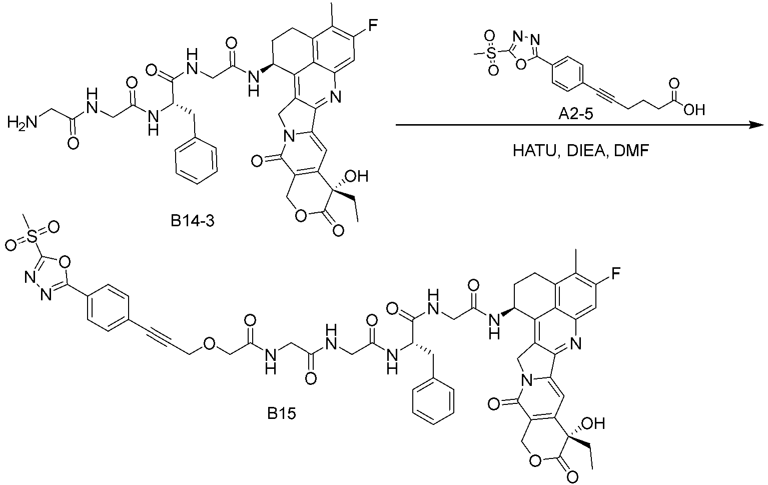

### Step 1: synthesis of N-[(7S)-7-benzyl-11-{[(1S,9S)-9-ethyl-5-fluoro-9-hydroxy-4-methyl-10,13-dioxo-2,3,9,10,12,1 5-hexahydro-1H-cyclohexo[1,2,3-de]pyrano[3',4':6,7]indolizino[1,2-b]quinolin-1-yl]amino}-2,5,8,11-tetraoxo-3,6,9-triazaaundecan-1-yl]-6-{4-[2-(methyldioxo-λ⁶-sulfanyl)-1,3,4-oxadiaz ol-5-yl]phenyl}hex-5-ynamide B15

B14-3 (11.3 mg, 0.015 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, A2-5 (5.0 mg, 0.015 mmol) was added into the mixed solution. The reaction solution was stirred at room temperature for 3 hours. The reaction solution was filtered and purified by preparative high performance liquid chromatography to obtain the compound B15 (4.8 mg, 30.0 %).

LC-MS: 1070.4[M+H]⁺.

### Example B16: preparation of compound B16

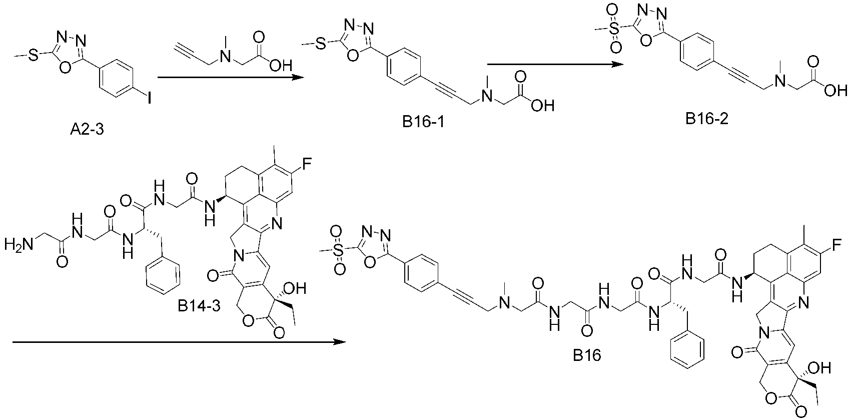

### Step 1: synthesis of N-methyl-N-(3-{4-[2-(methylthio)-1,3,4-oxadiazol-5-yl]phenyl}prop-2-ynyl)glycine (B16-1)

A2-3 (500.0 mg, 1.58 mmol) was added into a 10 mL single-neck flask and dissolved with diisopropylamine (3 mL). The obtained solution was added with bis(acetonitrile)palladium(II) chloride (12.7 mg, 0.05 mmol), triphenylphosphine (42.7 mg, 0.16 mmol) and cuprous iodide (10.3 mg, 0.03 mmol). The reaction solution was stirred at room temperature for 5 minutes, and then dropwise added with N-methyl-N-(prop-2-ynyl)glycine (301.0 mg, 2.37 mmol). The reaction solution was stirred at 50°C overnight. The reaction solution was concentrated under a reduced pressure. Residues were purified by silica gel column chromatography (dichloromethane: methanol = 93: 7) to obtain B16-1 (450.0 mg, 89.5%).

LC-MS:318.1[M+H]⁺.

### Step 2: synthesis of N-methyl-N-(3-{4-[2-(methyldioxo-λ⁶-sulfanyl)-1,3,4-oxadiazol-5-yl]phenyl}prop-2-ynyl)glyc ine (B16-2)

B16-1 (450.0 mg, 1.42 mmol) was added into a 50 mL single-neck flask and dissolved with dichloromethane (20 mL). The obtained solution was added with meta-chloroperoxybenzoic acid (888.3 mg, 5.15 mmol). The reaction solution was stirred at room temperature overnight. The reaction solution was concentrated under a reduced pressure to half of its original volume, and filtered. Then, the organic phase was concentrated under a reduced pressure. Residues were purified by preparative high performance liquid chromatography to obtain B16-2 (80.0 mg, 16.1 %).

LC-MS: 350.1[M+H]⁺.

### Step 3: synthesis of N-[(7S)-7-benzyl-11-{[(1S,9S)-9-ethyl-5-fluoro-9-hydroxy-4-methyl-10,13-dioxo-2,3,9,10,12,1 5-hexahydro-1H-cyclohexo[1,2,3-de]pyrano[3',4':6,7]indolizino[1,2-b]quinolin-1-yl]amino}-2,5,8,11-tetraoxo-3,6,9-triazaaundecan-1-yl]-2-[methyl(3-{4-[2-(methyldioxo-λ⁶-sulfanyl)-1,3 ,4-oxadiazol-5-yl]phenyl}prop-2-ynyl)amino]acetamide B16

B14-3 (11.3 mg, 0.015 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, B16-2 (5.2 mg, 0.015 mmol) was added into the mixed solution. The reaction solution was stirred at room temperature for 3 hours. The reaction solution was filtered and purified by preparative high performance liquid chromatography to obtain B16 (4.5 mg, 27.6 %).

LC-MS: 1085.4[M+H]⁺.

### Example B17: preparation of compound B17

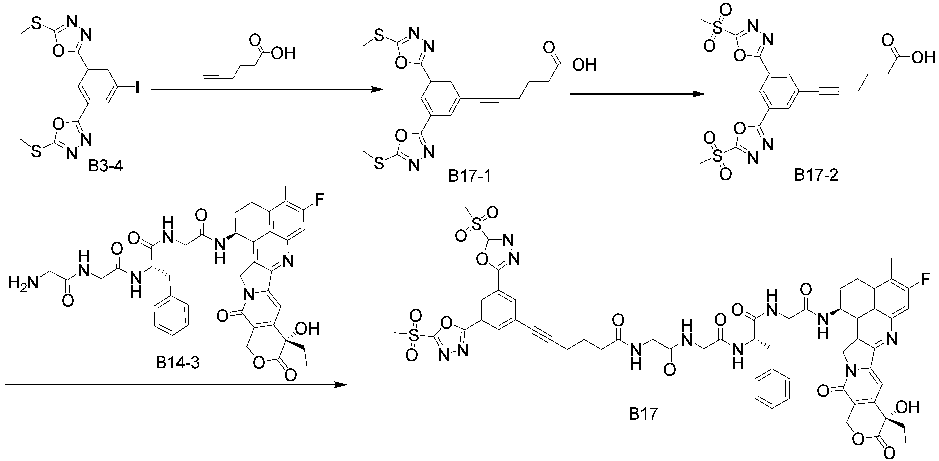

### Step 1: synthesis of 6-{3,5-bis[5-(methylthio)-1,3,4-oxadiazol-2-yl]phenyl}hex-5-ynoic acid B17-1

B3-4 (683.0 mg, 1.58 mmol) was added into a 10 mL single-neck flask and dissolved with diisopropylamine (3 mL). The obtained solution was added with bis(acetonitrile)palladium(II) chloride (12.7 mg, 0.05 mmol), triphenylphosphine (42.7 mg, 0.16 mmol) and cuprous iodide (10.3 mg, 0.03 mmol). The reaction solution was stirred at room temperature for 5 minutes, and then dropwise added with hex-5-ynoic acid (265.4 mg, 2.37 mmol). The reaction solution was stirred at 50°C overnight. The reaction solution was concentrated under a reduced pressure. Residues were purified by silica gel column chromatography (dichloromethane: methanol = 93: 7) to obtain B17-1 (550.0 mg, 83.5%).

LC-MS:417.1[M+H]⁺.

### Step 2: synthesis of 6-{3,5-bis[5-(methyldioxo-λ⁶-sulfanyl)-1,3,4-oxadiazol-2-yl]phenyl}hex-5-yne B17-2

B17-1 (417.0 mg, 1.0 mmol) was added into a 50 mL single-neck flask and dissolved with dichloromethane (20 mL). The obtained solution was added with meta-chloroperoxybenzoic acid (888.3 mg, 5.15 mmol). The reaction solution was stirred at room temperature overnight. The reaction solution was concentrated under a reduced pressure to half of its original volume, and filtered. Then, the organic phase was concentrated under a reduced pressure. Residues were purified by preparative high performance liquid chromatography to obtain B17-2 (90.0 mg, 18.8 %).

LC-MS: 481.1[M+H]⁺.

### Step 3: synthesis of N-[(1S,9S)-9-ethyl-5-fluoro-9-hydroxy-4-methyl-10,13-dioxo-2,3,9,10,12,15-hexahydro-1H-c yclohexo[1,2,3-de]pyrano[3',4':6,7]indolizino[1,2-b]quinolin-1-yl]-2-{[(2S)-2-benzyl-15-{3,5-bis[5-(methyldioxo-λ⁶-sulfanyl)-1,3,4-oxadiazol-2-yl]phenyl}-1,4,7,10-tetraoxo-3,6,9-triazape ntadec -14-yn-1-yl]amino]acetamide B17

B14-3 (11.3 mg, 0.015 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, B17-2 (7.2 mg, 0.015 mmol) was added into the mixed solution. The reaction solution was stirred at room temperature for 3 hours. The reaction solution was filtered and purified by preparative high performance liquid chromatography to obtain the compound B17 (6.4 mg, 35.1 %).

LC-MS: 1216.4[M+H]⁺.

### Example B18: preparation of compound B18

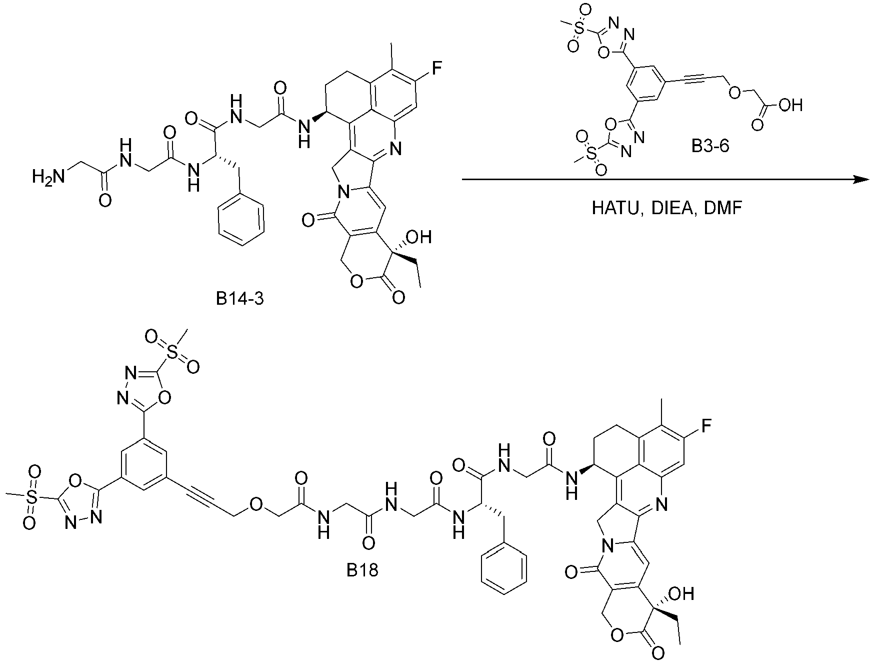

### Step 1: synthesis of N-[(7S)-7-benzyl-11-{[(1S,9S)-9-ethyl-5-fluoro-9-hydroxy-4-methyl-10,13-dioxo-2,3,9,10,12,1 5-hexahydro-1H-cyclohexo[1,2,3-de]pyrano[3',4':6,7]indolizino[1,2-b]quinolin-1-yl]amino}-2,5,8,11-tetraoxo-3,6,9-triazaaundecan-1-yl]-2-[(3-{3,5-bis[5-(methyldioxo-λ⁶-sulfanyl)-1,3,4-oxadiazol -2-yl]phenyl}prop-2-ynyl)oxy]acetamide B18

B14-3 (11.3 mg, 0.015 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, B3-6 (7.2 mg, 0.015 mmol) was added into the mixed solution. The reaction solution was stirred at room temperature for 3 hours. The reaction solution was filtered and purified by preparative high performance liquid chromatography to obtain the compound B18 (6.2 mg, 33.9 %).

LC-MS: 1218.3[M+H]⁺.

### Example B19: preparation of compound B19

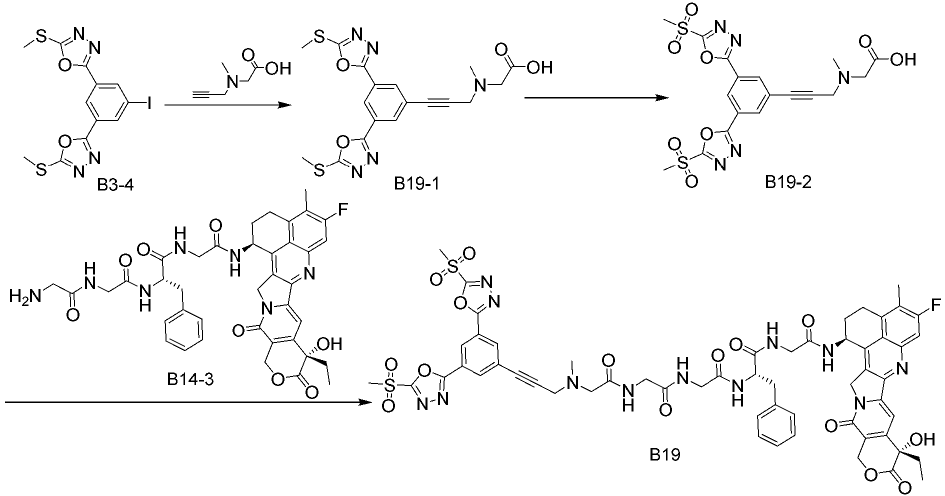

### Step 1: synthesis of N-(3-{3,5-bis[5-(methylthio)-1,3,4-oxadiazol-2-yl]phenyl}prop-2-ynyl)-N-methylglycine (B19-1)

B3-4 (683.0 mg, 1.58 mmol) was added into a 100 mL single-neck flask and dissolved with diisopropylamine (3 mL). The obtained solution was added with bis(acetonitrile)palladium(II) chloride (12.7 mg, 0.05 mmol), triphenylphosphine (42.7 mg, 0.16 mmol) and cuprous iodide (10.3 mg, 0.03 mmol). The reaction solution was stirred at room temperature for 5 minutes, and then dropwise added with N-methyl-N-(prop-2-ynyl)glycine (301.8 mg, 2.37 mmol). The reaction solution was stirred at 50°C overnight. The reaction solution was concentrated under a reduced pressure. Residues were purified by silica gel column chromatography (dichloromethane: methanol = 93: 7) to obtain B19-1 (600.0 mg, 87.8 %).

LC-MS:432.1[M+H]⁺.

### Step 2: synthesis of N-(3-{3,5-bis[5-(methyldioxo-λ⁶-sulfanyl)-1,3,4-oxadiazol-2-yl]phenyl}prop-2-ynyl)-N-methy lglycine (B19-2)

B19-1 (432.0 mg, 1.0 mmol) was added into a 50 mL single-neck flask and dissolved with dichloromethane (20 mL). The obtained solution was added with meta-chloroperoxybenzoic acid (888.3 mg, 5.15 mmol). The reaction solution was stirred at room temperature overnight. The reaction solution was concentrated under a reduced pressure to half of its original volume, and filtered. Then, the organic phase was concentrated under a reduced pressure. Residues were purified by reverse preparative high performance liquid chromatography to obtain B19-2 (80.0 mg, 16.2 %).

LC-MS: 496.1[M+H]⁺.

### Step 3: synthesis of N-[(7S)-7-benzyl-11-{[(1S,9S)-9-ethyl-5-fluoro-9-hydroxy-4-methyl-10,13-dioxo-2,3,9,10,12,1 5-hexahydro-1H-cyclohexo[1,2,3-de]pyrano[3',4':6,7]indolizino[1,2-b]quinolin-1-yl]amino}-2,5,8,11-tetraoxo-3,6,9-triazaaundecan-1-yl]-2-[(3-{3,5-bis[5-(methyldioxo-λ⁶-sulfanyl)-1,3,4-oxadiazol-2-yl]phenyl}prop-2-ynyl)(methyl)amino]acetamide B19

B14-3 (11.3 mg, 0.015 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, B19-2 (7.4 mg, 0.015 mmol) was added into the mixed solution. The reaction solution was stirred at room temperature for 3 hours. The reaction solution was filtered and purified by preparative high performance liquid chromatography to obtain B19 (5.2 mg, 28.2 %).

LC-MS: 1231.3[M+H]⁺.

### Example B20: preparation of compound B20

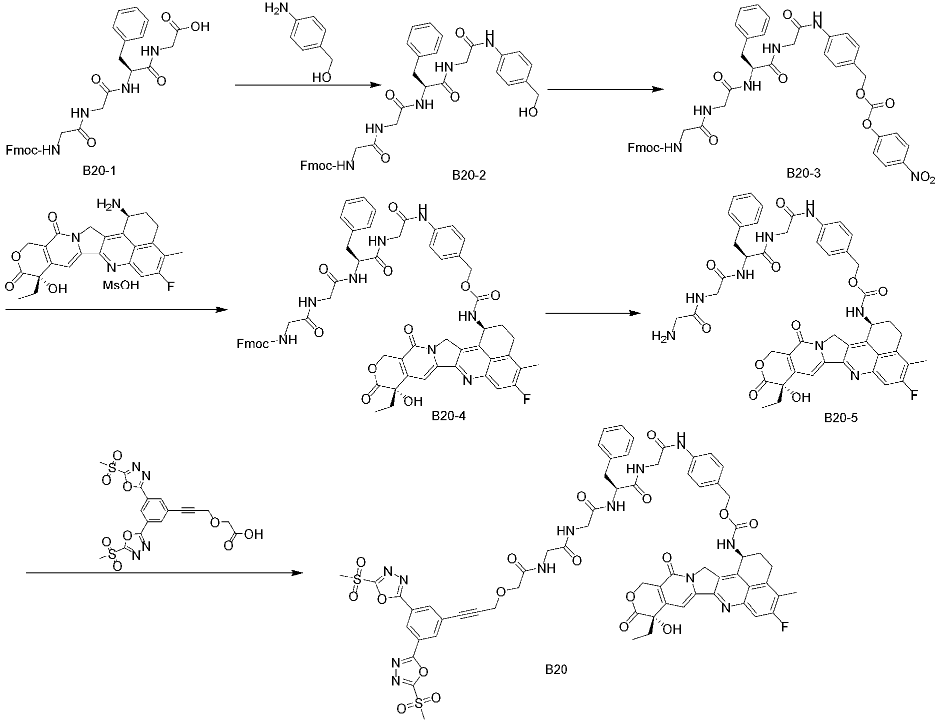

### Step 1: synthesis of 9H-fluoren-9-ylmethyl {[(7S)-7-benzyl-11-{[4-(hydroxymethyl)phenyl]amino}-2,5,8,11-tetraoxo-3,6,9-triazoundecan -1-yl]amino}formate (B20-2)

B20-1 (559.0 mg, 1.0mmol, prepared according to WO 2015155976) and (4-aminophenyl)methanol (123 mg, 1.0 mmol) were added into a 100 mL single-neck flask, dissolved with dichloromethane (5 mL) and methanol (5 mL), and dropwise added with 2-ethoxy-1-ethoxycarbonyl-1,2-dihydroquinoline (296.7 mg, 1.2 mmol). The reaction solution was stirred at room temperature overnight. The reaction solution was concentrated under a reduced pressure. Residues were purified by silica gel column chromatography (dichloromethane: methanol = 93: 7) to obtain B20-2 (550.0 mg, 82.8 %).

LC-MS:664.3[M+H]⁺.

### Step 2 to step 5: synthesis of B20-3 to B20

B20 to B20-3 were synthesized by reference to the synthetic methods for the compounds B10-2 to B10-5 to obtain 6.2 mg of the compound B20.

LC-MS:1367.4[M+H]⁺.

### Example B21: preparation of compound B21

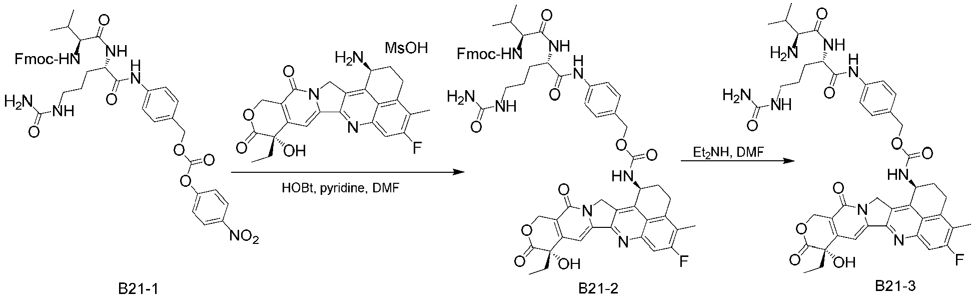

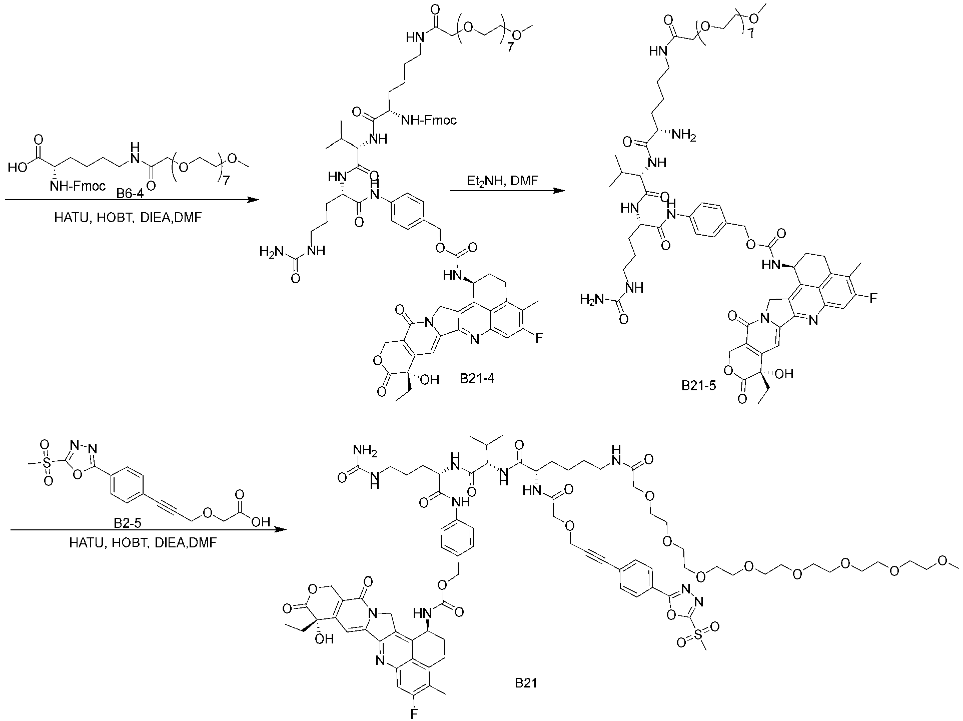

5 mg of the compound B21 was obtained through the preparative liquid chromatography by reference to the synthetic method for the compound B6.

LC-MS:1667.7[M+H]⁺.

### Example B22: preparation of compound B22

### Step 1: synthesis of 2-methylpropan-2-yl {[(22S)-22-benzyl-26-[(4-{[({[(1S,9S)-9-ethyl-5-fluoro-9-hydroxy-4-methyl-10,13-dioxo-2,3,9, 10,12,15-hexahydro-1H-cyclohexo[1,2,3-de]pyrano[3',4':6,7]indolizino[1,2-b]quinolin-1-yl]a mino}carbonyl}oxy]methyl}phenyl)amino]-14,17,20,23,26-pentaoxo-15,18,21,24-tetraaza-3,6 ,9,12-tetraoxahexacosan-1-yl]amino}formate (B22-1)

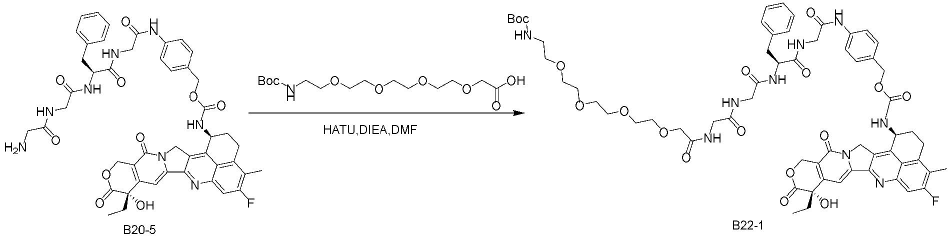

B20-5 (90.3 mg, 0.1 mmol) and 2-(7-azabenzotriazol-1-yl)-N,N,N',N'-tetramethyluronium hexafluorophosphate (57 mg, 0.15 mmol) were added into a 50 mL single-neck flask, and dissolved with N,N-dimethylformamide (1 mL). The obtained solution was added with diisopropylethylamine (25.8 mg, 0.2 mmol). The mixed solution was stirred at room temperature for 15 minutes. Then, [(2,2-dimethyl-4-oxo-5-aza-3,8,11,14-tetraoxahexadecan-16-yl)oxy]acetic acid (52.6 mg, 0.15 mmol) was added into the mixed solution. The reaction solution was stirred at room temperature for 3 hours. The reaction solution was dried by rotary evaporation and purified by preparative high performance liquid chromatography to obtain B22-1 (60 mg, 48.5 %).

LC-MS: 1236.5[M+H]⁺.

### Step 2: synthesis of (4-{[(22S)-1-amino-22-benzyl-14,17,20,23,26-pentaoxo-15,18,21,24-tetraaza-3,6,9,12-tetraox ahexacosan-26-yl]amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate (B22-2)

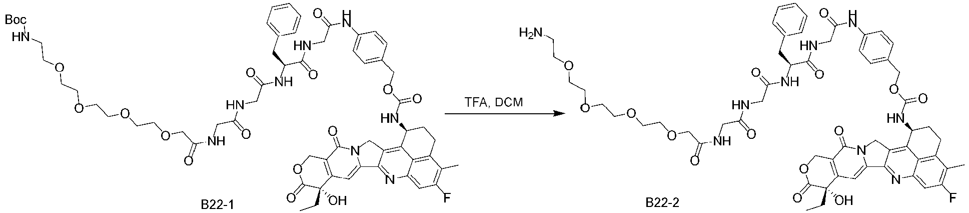

B22-1 (60 mg, 0.049 mmol) was added into a 50 mL single-neck flask and dissolved with dichloromethane (1 mL). The solution was added with trifluoroacetic acid (0.2 mL), and the mixed solution was stirred at room temperature for 30 minutes. The reaction solution was dried by rotary evaporation to obtain the crude product B22-2 (70 mg, 100 %).

LC-MS: 1136.5[M+H]⁺.

### Step 3: synthesis of (4-{[(31S,55S)-55-benzyl-31-({[(9H-fluoren-9-ylmethyl)oxy]carbonyl}amino)-25,32,47,50,53, 56,59-heptaoxo-2,5,8,11,14,17,20,23,36,39,42,45-dodecaoxa-26,33,48,51,54,57-hexaazanonap entacontan-59-yl]amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate (B22-3)

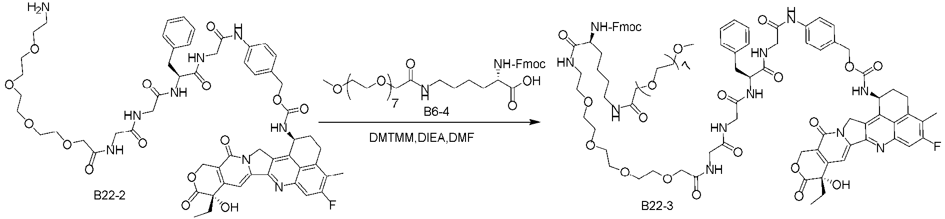

B22-2 (70 mg, 0.06 mmol) and 4-(4,6-dimethoxytriazin-2-yl)-4-methylmorpholine hydrochloride (26.5 mg, 0.09 mmol) were added into a 50 mL single-neck flask, and dissolved with N,N-dimethylformamide (1 mL). The obtained solution was added with diisopropylethylamine (25.8 mg, 0.2 mmol). The mixed solution was stirred at room temperature for 15 minutes. Then, B6-4 (67 mg, 0.09 mmol) was added into the mixed solution. The reaction solution was stirred at room temperature for 3 hours. The reaction solution was dried by rotary evaporation and purified by preparative high performance liquid chromatography to obtain B22-3 (55 mg, 49.1 %).

LC-MS: 1867.9[M+H]⁺.

### Step 4: synthesis of (4-{[(31S,55S)-31-amino-55-benzyl-25,32,47,50,53,56,59-heptaoxo-2,5,8,11,14,17,20,23,36,39, 42,45-dodecaoxa-26,33,48,51,54,57-hexaazanonapentacontan-59-yl]amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate (B22-4)

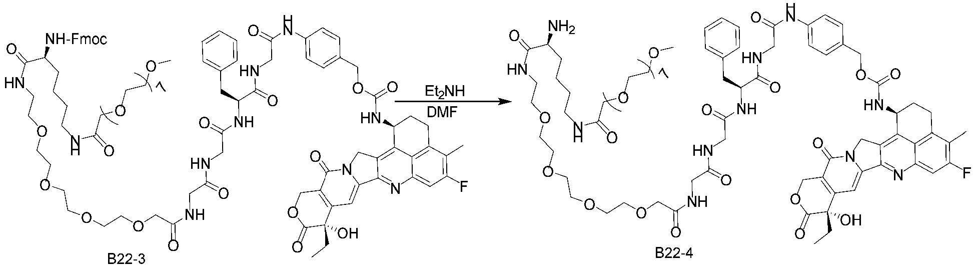

B22-3 (55.0 mg, 0.03 mmol) was added into a 50 mL single-neck flask and dissolved with N,N-dimethylformamide (1 mL). The obtained solution was added with diethylamine (22.2 mg, 0.3 mmol). The reaction solution was stirred at room temperature for 1 hour. The reaction solution was concentrated under a reduced pressure. Residues were slurried with petroleum ether to obtain B22-4 (55mg, 100%).

LC-MS: 1844.8[M+H]⁺.

### Step 5: synthesis of (4-{[(31S,55S)-55-benzyl-31-({2-[(3-{4-[5-(methyldioxo-λ6-sulfanyl)-1,3,4-oxadiazol-2-yl]phe nyl}prop-2-ynyl)oxy]acetyl}amino)-25,32,47,50,53,56,59-heptaoxo-2,5,8,11,14,17,20,23,36,39, 42,45-dodecaoxa-26,33,48,51,54,57-hexaazanonapentacontan-59-yl]amino}phenyl)methyl { [(1 S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B22

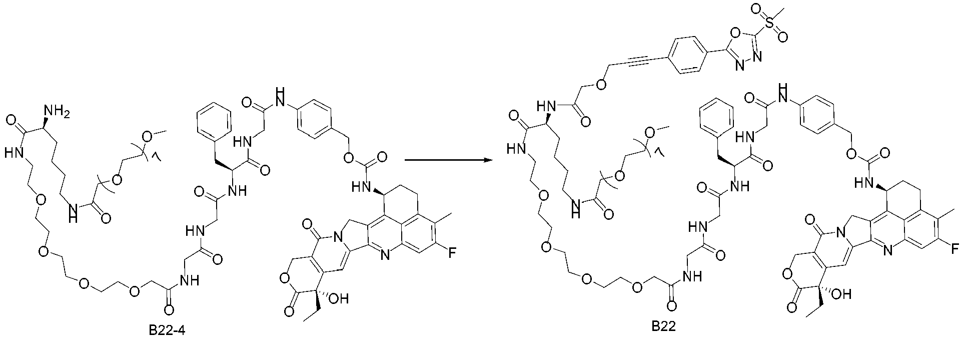

B22-4 (20 mg, 0.01 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, B2-5 (5.0 mg, 0.015 mmol) was added into the mixed solution. The reaction solution was stirred at room temperature for 3 hours. The reaction solution was filtered and purified by preparative high performance liquid chromatography to obtain B22 (7.2 mg, 36.7 %).

LC-MS: 1963.8[M+H]⁺.

### Example B23: preparation of compound B23

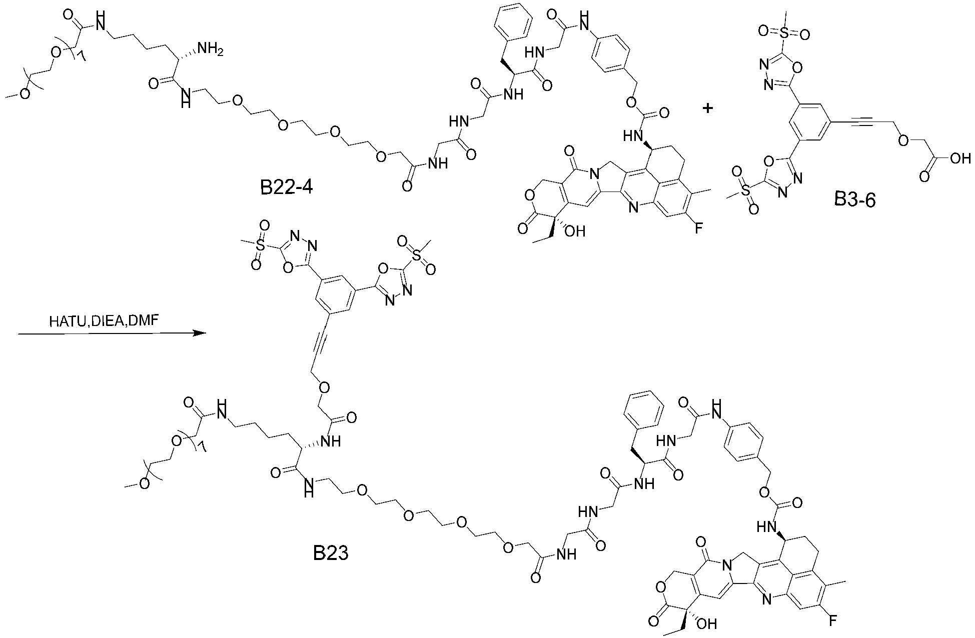

### Step 1: synthesis of (4-{[(31S,55S)-55-benzyl-31-({2-[(3-{3,5-bis[5-(methyldioxo-λ6-sulfanyl)-1,3,4-oxadiazol-2-yl ]phenyl}prop-2-ynyl)oxy]acetyl}amino)-25,32,47,50,53,56,59-heptaoxo-2,5,8,11,14,17,20,23,3 6,39,42,45-dodecaoxa-26,33,48,51,54,57-hexaazanonapentacontan-59-yl]amino}phenyl)meth yl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B23

B22-4 (20 mg, 0.01 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, B3-6 (7.2 mg, 0.015 mmol) was added into the mixed solution. The reaction solution was stirred at room temperature for 3 hours. The reaction solution was filtered and purified by preparative high performance liquid chromatography to obtain B23 (8.3 mg, 39.3 %).

LC-MS: 2109.8[M+H]⁺.

### Example B24: preparation of compound B24

### Step 1: synthesis of (4-{[(31S,34S,37S)-37-{3-[(aminocarbonyl)amino]propyl}-31-[(2,2-dimethyl-4,19-dioxo-5-az a-3,8,11,14,17-pentaoxanonadecan-19-yl)amino]-25,32,35,38-tetraoxo-34-(prop-2-yl)-2,5,8,11 ,14,17,20,23-octaoxa-26,33,36-triazaoctatriacontan-38-yl]amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate (B24-1)

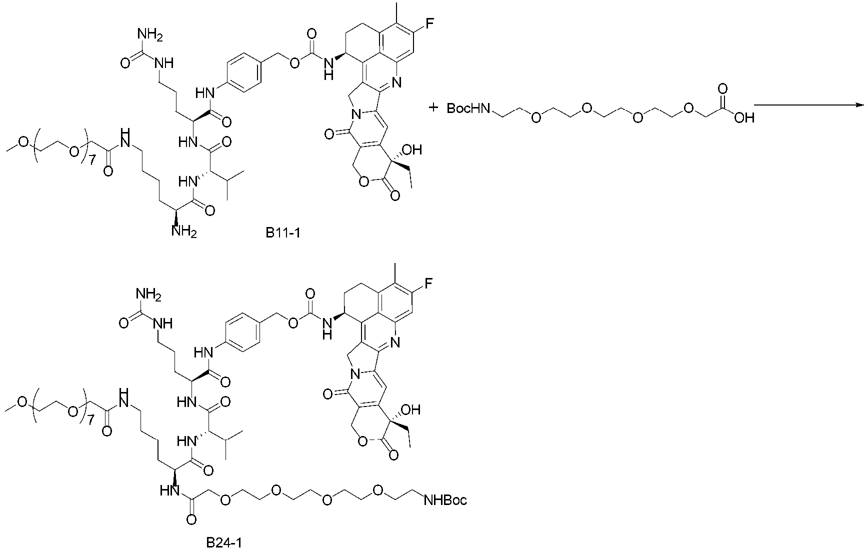

B11-1 (80.0 mg, 0.06 mmol) and 2-(7-azabenzotriazol-1-yl)-N,N,N',N'-tetramethyluronium hexafluorophosphate (22.8 mg, 0.06 mmol) were added into a 50 mL single-neck flask, and dissolved with N,N-dimethylformamide (2 mL). The obtained solution was added with diisopropylethylamine (0.15 mL, 1.2 mmol). Then, the mixed solution was added with [(2,2-dimethyl-4-oxo-5-aza-3,8,11,14-tetraoxahexadecan-16-yl)oxy]acetic acid (21.0 mg, 0.06 mmol), and stirred at room temperature for 1 hour. The reaction solution was dried by rotary evaporation and purified by silica gel column chromatography (DCM: MeOH=10: 1) to obtain B24-1 (50 mg, 49.5 %).

LC-MS: 1682.9[M+H]⁺.

### Step 2: synthesis of (4-{[(31S,34S,37S)-37-{3-[(aminocarbonyl)amino]propyl}-31-[(14-amino-1-oxo-3,6,9,12-tetr aoxatetradecan-1-yl)amino]-25,32,35,38-tetraoxo-34-(prop-2-yl)-2,5,8,11,14,17,20,23-octaox a-26,33,36-triazaoctatriacontan-38-yl]amino}phenyl)methyl { [(1 S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate (B24-2)

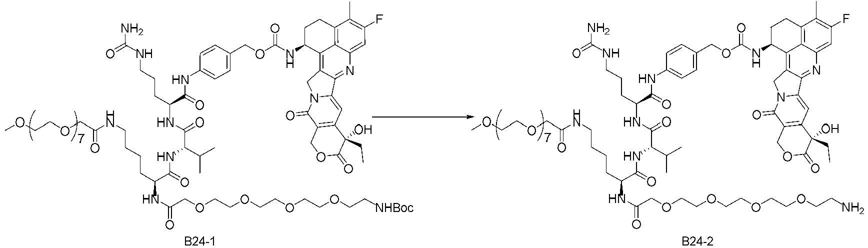

B24-1 (50 mg, 0.03 mmol) was added into a 50 mL single-neck flask and dissolved with dichloromethane (1 mL). The solution was added with trifluoroacetic acid (0.2 mL), and the mixed solution was stirred at room temperature for 30 minutes. The reaction solution was dried by rotary evaporation to obtain the crude product B24-2 (60 mg, 100 %). The crude product directly reacted in the next step without purification.

LC-MS: 1582.9[M+H]⁺.

### Step 3: synthesis of (4-{[(31S,34S,37S) -37-{3-[(aminocarbonyl)amino]propyl}-31-[(21-{3,5-bis[5-(methyldioxo-λ6-sulfanyl)-1,3,4-o xadiazol-2-yl]phenyl}-1,16-dioxo-15-aza-3,6,9,12,18-pentaoxahenicos-20-yn-1-yl)amino]-25, 32,35,38-tetraoxo-34-(prop-2-yl)-2,5,8,11,14,17,20,23-octaoxa-26,33,36-triazaoctatriacontan-38-yl]amino}phenyl)methyl{[(1S,9S)-9-ethyl-5-fluoro-9-hydroxy-4-methyl-10,13-dioxo-2,3,9, 10,12,15-hexahydro-1H-cyclohexo[1,2,3-de]pyrano[3',4':6,7]indolizino[1,2-b]quinolin-1-yl]a mino}formate B24

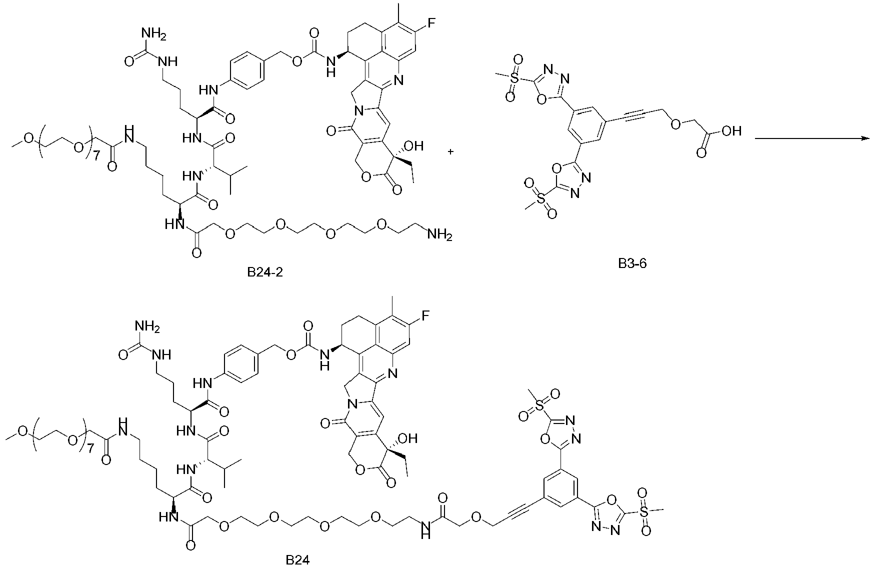

B24-2 (32 mg, 0.02 mmol) and 2-(7-azabenzotriazol-1-yl)-N,N,N',N'-tetramethyluronium hexafluorophosphate (11.4 mg, 0.03 mmol) were added into a 50 mL single-neck flask, and dissolved with N,N-dimethylformamide (1 mL). The obtained solution was added with diisopropylethylamine (0.2 mL, 0.06 mmol). The mixed solution was stirred at room temperature for 15 minutes. Then, B3-6 (14.5 mg, 0.03 mmol) was added into the mixed solution. The reaction solution was stirred at room temperature for 3 hours. The reaction solution was filtered and purified by preparative high performance liquid chromatography to obtain B24 (12 mg, 29.3 %).

LC-MS: 2046.8[M+H]⁺.

### Example B25: preparation of compound B25

### Step 1: synthesis of 1-[(25-oxo-2,5,8,11,14,17,20,23-octaoxapentacosan-25-yl)oxy]tetrahydropyrrole-2,5-dione B25-2

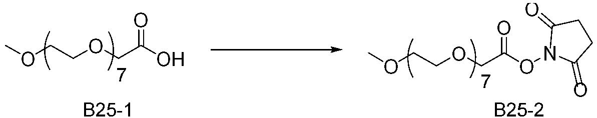

(2,5,8,11,14,17,20-heptaoxadocosan-22-yloxy)acetic acid (B25-1) (1 g, 2.5 mmol) was dissolved with anhydrous dichloromethane (10 mL), and 1-(3-dimethylaminopropyl)-3-ethylcarbodiimide hydrochloride (575 mg, 3 mmol) and N-hydroxysuccinimide (345 mg, 3 mmol) was added into the reaction system. The reaction solution was stirred for 3 hours, then dried by rotary evaporation, and purified by silica gel column chromatography to obtain B25-2 (1 g, 80.6%).

LC-MS: 496.2[M+H]⁺.

### Step 2: synthesis of (2S)-2-({[(9H-fluoren-9-ylmethyl)oxy]carbonyl}amino)-4-[(25-oxo-2,5,8,11,14,17,20,23-octao xapentacosan-25-yl)amino]butanoic acid B25-3

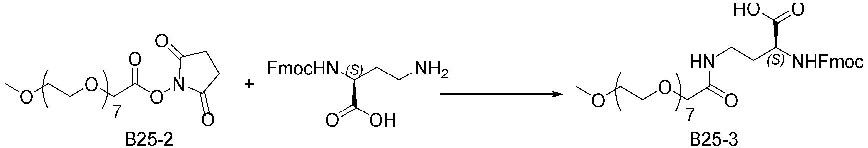

The compound B25-2 (500 mg, 1.0 mmol) and (2S)-4-amino-2-({[(9H-fluoren-9-ylmethyl)oxy]carbonyl}amino)butanoic acid (374 mg, 1.1 mmol) were dissolved with N,N-dimethylformamide (8 mL), and triethylamine (202 mg, 2 mmol) was added into the reaction system. Tthe reaction solution was stirred for 3 hours, then dried by rotary evaporation, and purified by preparative liquid chromatography to obtain the compound B25-3 (550 mg, 76.3%).

LC-MS: 721.5[M+H]⁺.

### Step 3: synthesis of 9H-fluoren-9-ylmethyl {[(29S,32S,35S)-36-[(4-{[({[(1S,9S)-9-ethyl-5-fluoro-9-hydroxy-4-methyl-10,13-dioxo-2,3,9,1 0,12,15-hexahydro-1H-cyclohexo[1,2,3-de]pyrano[3',4':6,7]indolizino[1,2-b]quinolin-1-yl]a mino}carbonyl)oxy)methyl}phenyl)amino]-35-methyl-25,30,33,36-tetraoxo-32-(prop-2-yl)-2, 5,8,11,14,17,20,23-octaoxa-26,31,34-triazahexatriacontan-29-yl]amino}formate B25-4

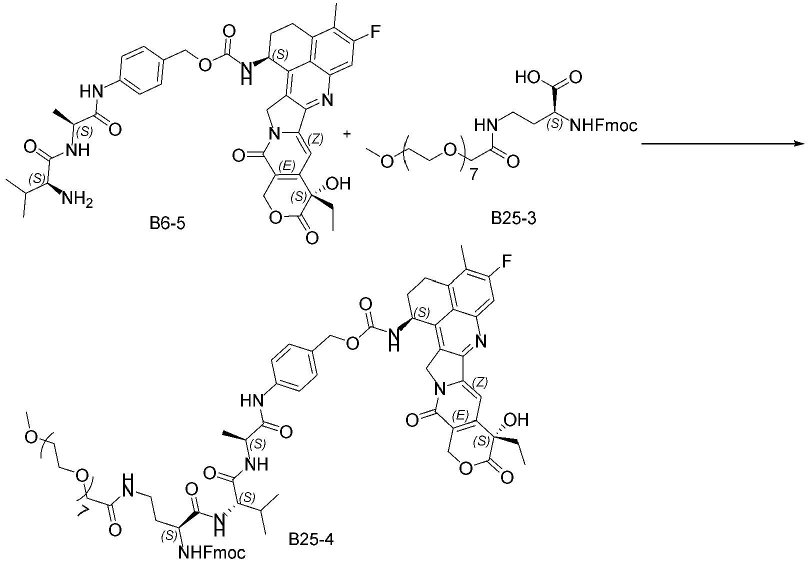

B6-5 (75.4 mg, 0.10 mmol), diisopropylethylamine (0.05 mL, 0.3 mmol) and B25-3 (72 mg, 0.10 mmol) were added into a 100 mL single-neck flask, and dissolved with N,N-dimethylformamide (2 mL). The obtained solution was added with 4-(4,6-dimethoxy-triazin-2-yl)-4-methylmorpholine hydrochloride (28.3 mg, 0.10 mmol) in an ice bath. The reaction solution was stirred in an ice bath for 1 hour. The reaction solution was filtered and then purified by preparative high performance liquid chromatography to obtain B25-4 (80 mg, 54.9%).

LC-MS: 1457.8[M+H]⁺.

### Step 4: synthesis of (4-{[(29S,32S,35S)-29-amino-35-methyl-25,30,33,36-tetraoxo-32-(prop-2-yl)-2,5,8,11,14,17,2 0,23-octaoxa-26,31,34-triazahexatriacontan-36-yl]amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate (B25-5)

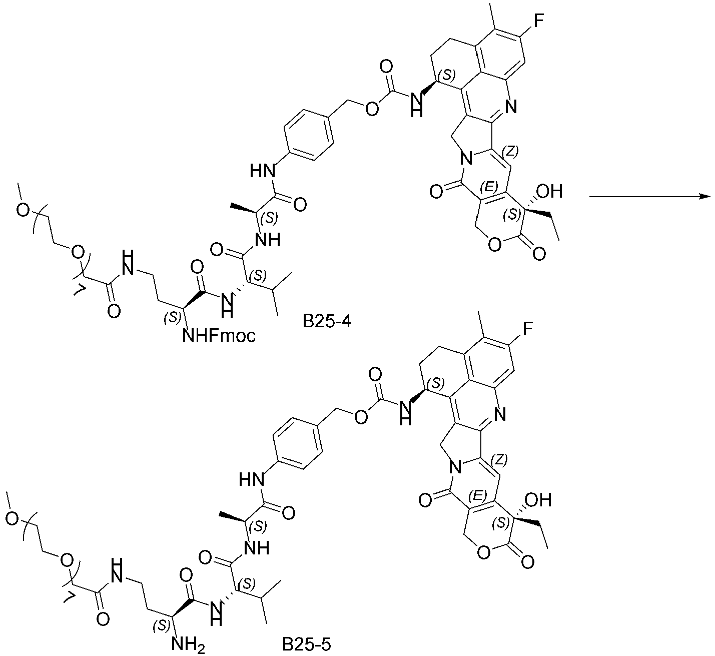

B25-4 (80.0 mg, 0.055 mmol) was added into a 50 mL single-neck flask and dissolved with N,N-dimethylformamide (2 mL). The obtained solution was added with diethylamine (40 mg, 0.55 mmol). The reaction solution was stirred at room temperature for 1 hour. The reaction solution was concentrated under a reduced pressure. Residues were slurried with petroleum ether to obtain B25-5 (60 mg, 88.3%).

LC-MS: 1235.6[M+H]⁺.

### Step 5: synthesis of 2-methylpropan-2-yl {[(29S)-29-[(3S,6S)-7-[(4-{[({[(1S,9S)-9-ethyl-5-fluoro-9-hydroxy-4-methyl-10,13-dioxo-2,3,9, 10,12,15-hexahydro-1H-cyclohexo[1,2,3-de]pyrano[3',4':6,7]indolizino[1,2-b]quinolin-1-yl]a mino}carbonyl)oxy]methyl}phenyl)amino]-6-methyl-1,4,7-trioxo-3-(prop-2-yl)-2,5-diazahep t-1-yl]-25,31-dioxo-26,30-diaza-2,5,8,11,14,17,20,23,33,36,39,42-dodecaoxatetratetracontan-44-yl]amino}formate B25-6

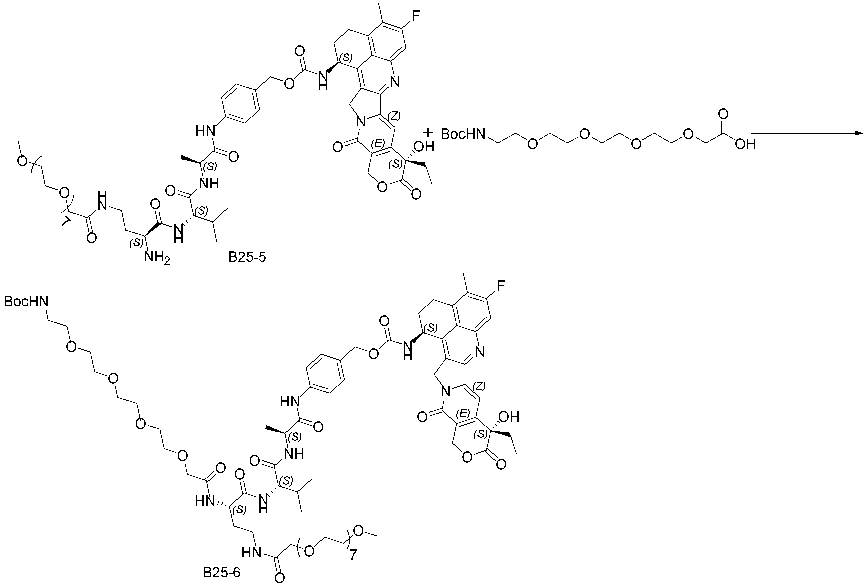

B25-5 (60.0 mg, 0.048 mmol) and 2-(7-azabenzotriazol-1-yl)-N,N,N',N'-tetramethyluronium hexafluorophosphate (18 mg, 0.048 mmol) were added into a 50 mL single-neck flask, and dissolved with N,N-dimethylformamide (2 mL). The obtained solution was added with diisopropylethylamine (0.15 mL, 1.2 mmol). Then, the mixed solution was added with [(2,2-dimethyl-4-oxo-5-aza-3,8,11,14-tetraoxahexadecan-16-yl)oxy]acetic acid (21.0 mg, 0.06 mmol), and stirred at room temperature for 1 hour. The reaction solution was dried by rotary evaporation and purified by silica gel column chromatography (DCM: MeOH=10: 1) to obtain B25-6 (40 mg, 53.1 %).

LC-MS: 1568.8[M+H]⁺.

### Step 6: synthesis of (4-1[(29S,32S,35S)-29-[(14-amino-1-oxo-3,6,9,12-tetraoxatetradecan-1-yl)amino]-35-methyl-25,30,33,36-tetraoxo-32-(prop-2-yl)-2,5,8,11,14,17,20,23-octaoxa-26,31,34-triazahexatriacont an-36-yl]amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B25-7

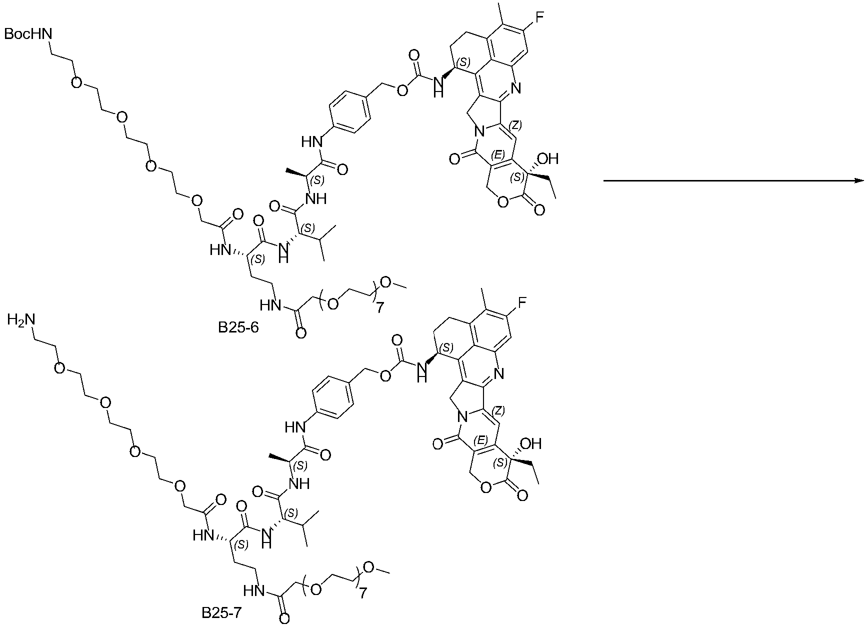

B25-6 (40 mg, 0.025 mmol) was added into a 50 mL single-neck flask and dissolved with dichloromethane (1 mL). The solution was added with trifluoroacetic acid (0.2 mL), and the mixed solution was stirred at room temperature for 30 minutes. The reaction solution was dried by rotary evaporation to obtain the crude product B25-7 (45 mg, 100 %). The crude product directly reacted in the next step without purification.

LC-MS: 1468.8[M+H]⁺.

### Step 7: synthesis of (4-{[(29S,32S,35S)-35-methyl-29-[(21-{4-[5-(methyldioxo-λ⁶-sulfanyl)-1,3,4-oxadiazol-2-yl]p henyl}-1,16-dioxo-15-aza-3,6,9,12,18-pentaoxahenicos-20-yn-1-yl)amino]-25,30,33,36-tetrao xo-32-(prop-2-yl)-2,5,8,11,14,17,20,23-octaoxa-26,31,34-triazahexatriacontan-36-yl]amino}p henyl)methyl { [(1 S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B25

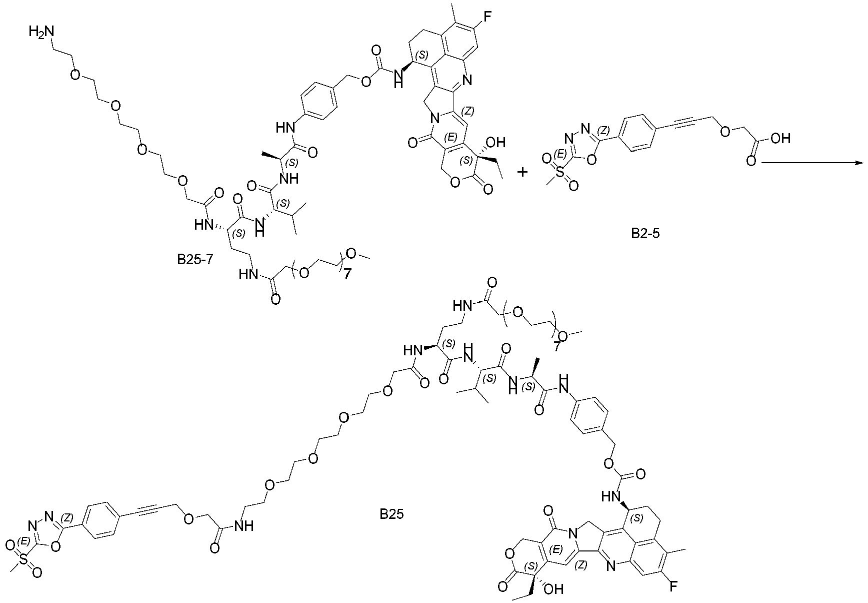

B25-7 (37 mg, 0.03 mmol) and 2-(7-azabenzotriazol-1-yl)-N,N,N',N'-tetramethyluronium hexafluorophosphate (11.4 mg, 0.03 mmol) were added into a 50 mL single-neck flask, and dissolved with N,N-dimethylformamide (1 mL). The obtained solution was added with diisopropylethylamine (0.03 mL, 0.06 mmol). The mixed solution was stirred at room temperature for 15 minutes. Then, B2-5 (10.1 mg, 0.03 mmol) was added into the mixed solution. The reaction solution was stirred at room temperature for 3 hours. The reaction solution was filtered and purified by preparative high performance liquid chromatography to obtain the compound B25 (18 mg, 33.6 %).

LC-MS: 1786.8[M+H]⁺.

### Example B26: preparation of compound B26

### Step 1: synthesis of (2S)-2-({[(9H-fluoren-9-ylmethyl)oxy]carbonyl}amino)-3-[(25-oxo-2,5,8,11,14,17,20,23-octao xapentacosan-25-yl)amino]propanoic acid B26-1

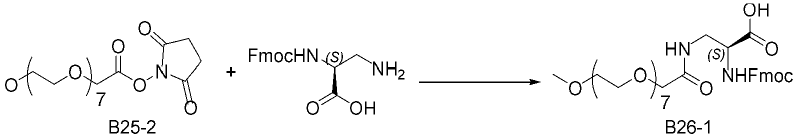

The compound B25-2 (500 mg, 1.0 mmol) and (2S)-3-amino-2-({[(9H-fluoren-9-ylmethyl)oxy]carbonyl}amino)propanoic acid (359 mg, 1.1 mmol) were dissolved with N,N-dimethylformamide (8 mL), and triethylamine (202 mg, 2 mmol) was added into the reaction system. The reaction solution was stirred for 3 hours, then dried by rotary evaporation, and purified by preparative liquid chromatography to obtain the compound B26-1 (520 mg, 73.6%).

LC-MS: 707.3[M+H]⁺.

### Step 2: synthesis of (4-{[(28S,31S,34S)-28-({[(9H-fluoren-9-ylmethyl)oxy]carbonyl}amino)-34-methyl-25,29,32,3 5-tetraoxo-31-(prop-2-yl)-2,5,8,11,14,17,20,23-octaoxa-26,30,33-triazapentatriacontan-35-yl] amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B26-2

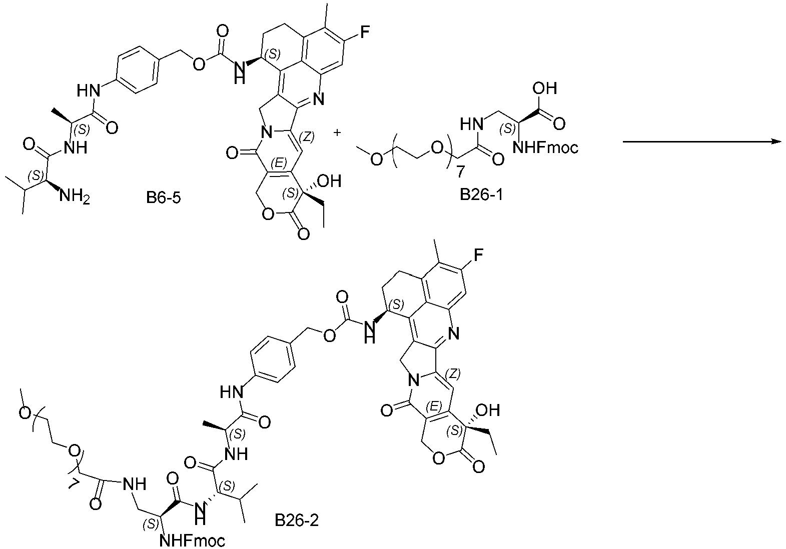

B6-5 (75.4 mg, 0.10 mmol), diisopropylethylamine (0.05 mL, 0.3 mmol) and B26-1 (70 mg, 0.10 mmol) were added into a 100 mL single-neck flask, and dissolved with N,N-dimethylformamide (2 mL). The obtained solution was added with 4-(4,6-dimethoxy-triazin-2-yl)-4-methylmorpholine hydrochloride (28.3 mg, 0.10 mmol) in an ice bath. The reaction solution was stirred in an ice bath for 1 hour. The reaction solution was filtered and then purified by preparative high performance liquid chromatography to obtain B26-2 (75 mg, 51.9%).

LC-MS: 1443.8[M+H]⁺.

### Step 3: synthesis of (4-{[(28S,31S,34S)-28-amino-34-methyl-25,29,32,35-tetraoxo-31-(prop-2-yl)-2,5,8,11,14,17,2 0,23-octaoxa-26,30,33-triazapentatriacontan-35-yl]amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B26-3

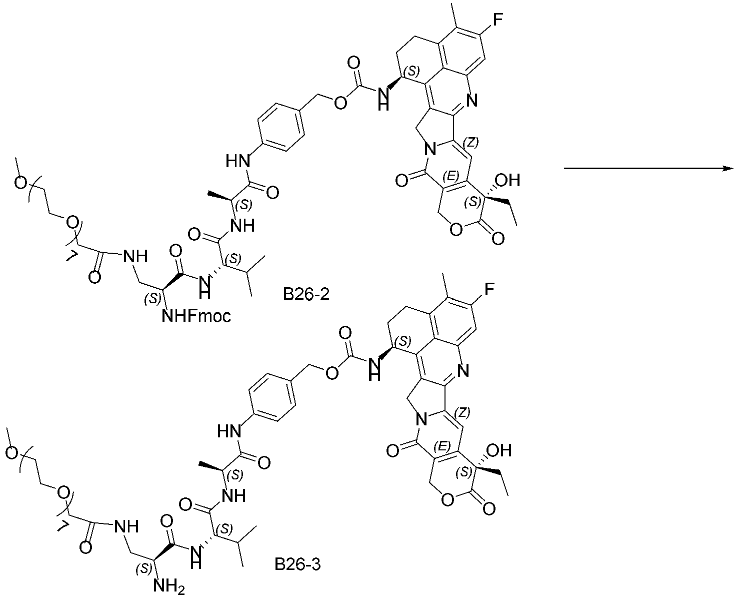

B26-2 (80.0 mg, 0.052 mmol) was added into a 50 mL single-neck flask and dissolved with N,N-dimethylformamide (2 mL). The obtained solution was added with diethylamine (40 mg, 0.55 mmol). The reaction solution was stirred at room temperature for 1 hour. The reaction solution was concentrated under a reduced pressure. Residues were slurried with petroleum ether to obtain B26-3 (55 mg, 87.3%).

LC-MS: 1221.6[M+H]⁺.

### Step 4: synthesis of (4-{[(28S,31S,34S)-28-[(2,2-dimethyl-4,19-dioxo-5-aza-3,8,11,14,17-pentaoxanonadecan-19-y l)amino]-34-methyl-25,29,32,35-tetraoxo-31-(prop-2-yl)-2,5,8,11,14,17,20,23-octaoxa-26,30,3 3-triazapentatriacontan-35-yl]amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B26-4

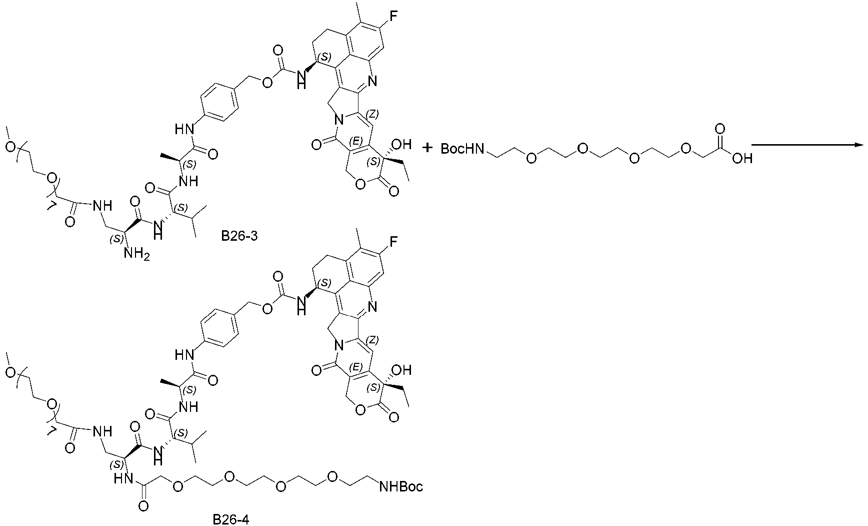

B26-3 (55.0 mg, 0.045 mmol) and 2-(7-azabenzotriazol-1-yl)-N,N,N',N'-tetramethyluronium hexafluorophosphate (18 mg, 0.048 mmol) were added into a 50 mL single-neck flask, and dissolved with N,N-dimethylformamide (2 mL). The obtained solution was added with diisopropylethylamine (0.15 mL, 1.2 mmol). Then, the mixed solution was added with [(2,2-dimethyl-4-oxo-5-aza-3,8,11,14-tetraoxahexadecan-16-yl)oxy]acetic acid (21.0 mg, 0.06 mmol), and stirred at room temperature for 1 hour. The reaction solution was dried by rotary evaporation and purified by silica gel column chromatography (DCM: MeOH=10: 1) to obtain B26-4 (40 mg, 57.2 %).

LC-MS: 1554.8[M+H]⁺.

### Step 5: synthesis of (4-{[(28S,31S,34S)-28-[(14-amino-1-oxo-3,6,9,12-tetraoxatetradecan-1-yl)amino]-34-methyl-25,29,32,35-tetraoxo-31-(prop-2-yl)-2,5,8,11,14,17,20,23-octaoxa-26,30,33-triazapentatriacon tan-35-yl]amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B26-5

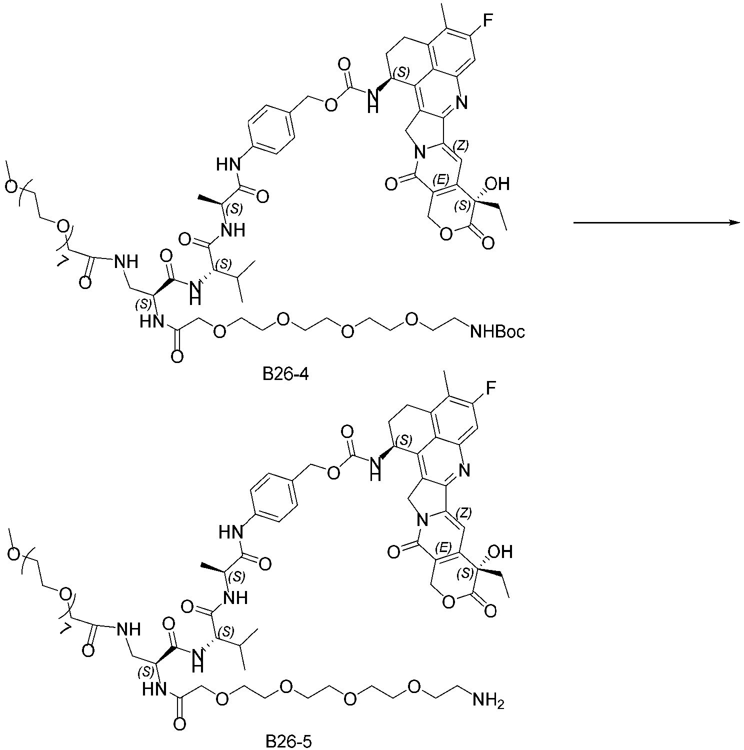

B26-4 (40 mg, 0.025 mmol) was added into a 50 mL single-neck flask and dissolved with dichloromethane (1 mL). The solution was added with trifluoroacetic acid (0.2 mL), and the mixed solution was stirred at room temperature for 30 minutes. The reaction solution was dried by rotary evaporation to obtain the crude product B26-5 (45 mg, 100 %). The crude product directly reacted in the next step without purification.

LC-MS: 1454.9[M+H]⁺.

### Step 6: synthesis of (4-{[(28S,31S,34S)-34-methyl-28-[(21-{4-[5-(methyldioxo-λ6-sulfanyl)-1,3,4-oxadiazol-2-yl]p henyl}-1,16-dioxo-15-aza-3,6,9,12,18-pentaoxahenicos-20-yn-1-yl)amino]-25,29,32,35-tetrao xo-31-(prop-2-yl)-2,5,8,11,14,17,20,23-octaoxa-26,30,33-triazapentatriacontan-35-yl]amino} phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B26

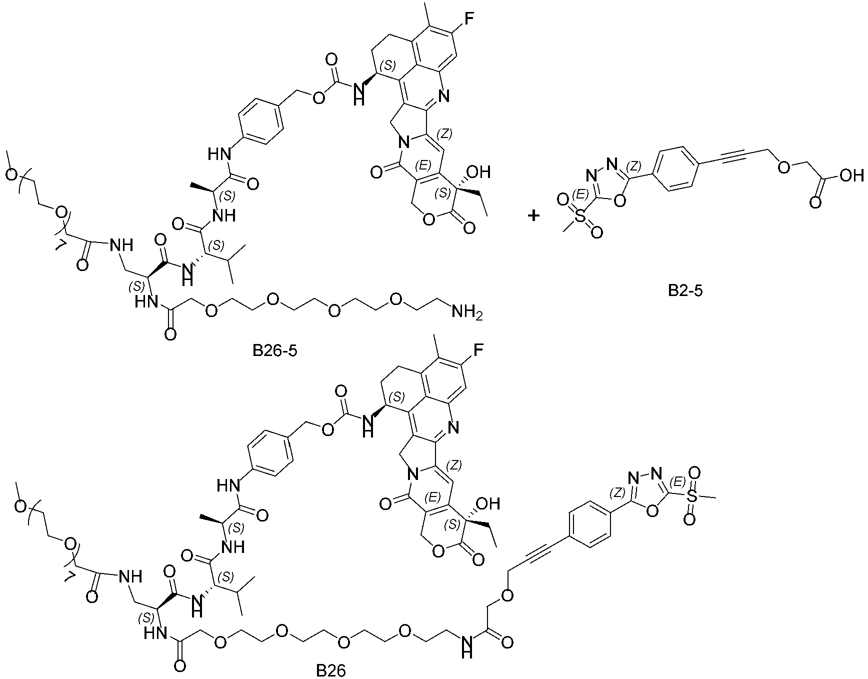

B26-5 (45 mg, 0.03 mmol) and 2-(7-azabenzotriazol-1-yl)-N,N,N',N'-tetramethyluronium hexafluorophosphate (11.4 mg, 0.03 mmol) were added into a 50 mL single-neck flask, and dissolved with N,N-dimethylformamide (1 mL). The obtained solution was added with diisopropylethylamine (0.045 mL, 0.09 mmol). The mixed solution was stirred at room temperature for 15 minutes. Then, B2-5 (10.1 mg, 0.03 mmol) was added into the mixed solution. The reaction solution was stirred at room temperature for 3 hours. The reaction solution was filtered and purified by preparative high performance liquid chromatography to obtain the compound B26 (15 mg, 28.2%).

LC-MS: 1772.8[M+H]⁺.

### Example B27: preparation of compound B27

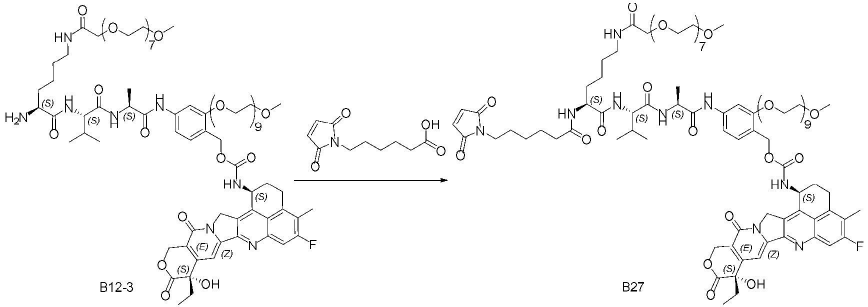

### Step 1: synthesis of (4-{[(31S,34S,37S)-31-{[6-(2,5-dioxo-2,5-dihydro-1H-pyrrol-1-yl)-1-oxohexyl]amino}-37-met hyl-25,32,35,38-tetraoxo-34-(prop-2-yl)-2,5,8,11,14,17,20,23-octaoxa-26,33,36-triazaoctatriac ontan-38-yl]amino}-2-(2,5,8,11,14,17,20,23,26-nonaoxaoctacosan-28-yloxy)phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B27

B12-3 (25.0 mg, 0.015 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, 6-(2,5-dioxo-2,5-dihydro-1H-pyrrol-1-yl)hexanoic acid (3.0 mg, 0.015 mmol) was added into the mixed solution. The reaction solution was stirred at room temperature for 3 hours. The reaction solution was filtered and purified by preparative high performance liquid chromatography to obtain B27 (8.3 mg, 29.4 %).

LC-MS: 1883.1[M+H]⁺.

### Example B28: preparation of compound B28

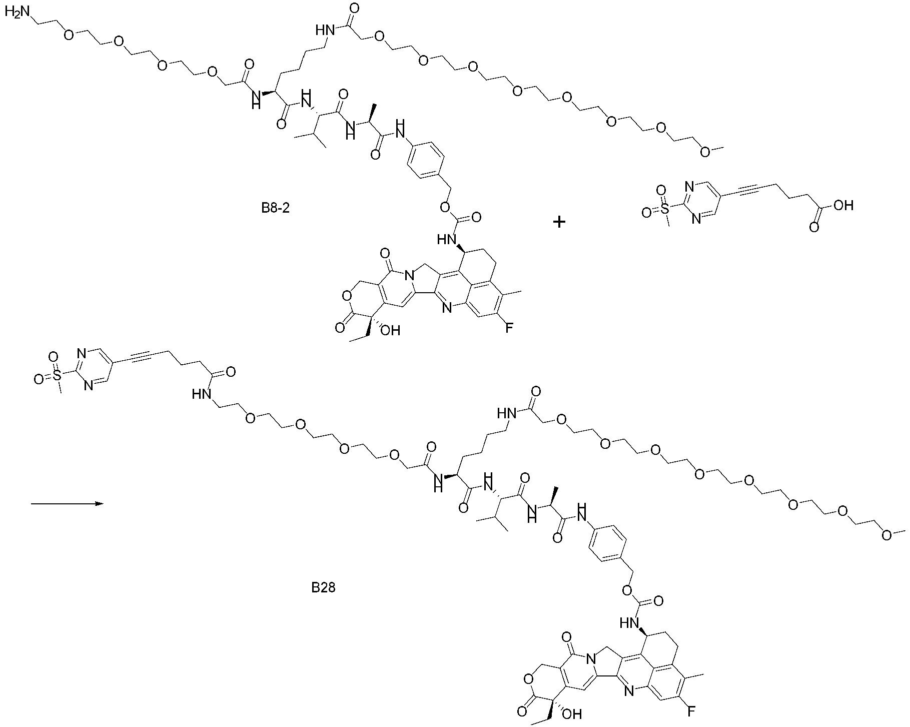

### Step 1: synthesis of (4-{[(31S,34S,37S)-37-methyl-31-({21-[2-(methyldioxo-λ6-sulfanyl)pyrimidin-5-yl]-1,16-diox o-15-aza-3,6,9,12-tetraoxahenicos-20-yn-1-yl}amino)-25,32,35,38-tetraoxo-34-(prop-2-yl)-2,5, 8,11,14,17,20,23-octaoxa-26,33,36-triazaoctatriacontan-38-yl]amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B28

B8-2 (40.0 mg, 0.03 mmol) and 2-(7-azabenzotriazol-1-yl)-N,N,N',N'-tetramethyluronium hexafluorophosphate (11.4 mg, 0.03 mmol) were added into a 10 mL single-neck flask, and dissolved with N,N-dimethylformamide (1 mL). The obtained solution was added with diisopropylethylamine (0.03 mL, 0.06 mmol). The mixed solution was stirred at room temperature for 15 minutes. Then, 6-[2-(methyldioxo-λ⁶-sulfanyl)pyrimidin-5-yl]hex-5-ynoic acid (8 mg, 0.03 mmol) was added into the mixed solution. The reaction solution was stirred at room temperature for 3 hours. The reaction solution was filtered and purified by preparative high performance liquid chromatography to obtain the compound B28 (14.8 mg, 28.2 %).

LC-MS: 1746.8[M+H]⁺.

¹H NMR (400 MHz, DMSO) δ 9.99 (s, 1H), 9.11 (s, 2H), 8.17 (d, *J =* 6.7 Hz, 1H), 8.05 (d, *J* = 8.1 Hz, 1H), 7.95 - 7.85 (m, 2H), 7.78 (d, *J =* 11.1 Hz, 1H), 7.64 (d, *J =* 7.8 Hz, 2H), 7.58 (d, *J* = 8.4 Hz, 2H), 7.36 (d, *J =* 8.3 Hz, 2H), 7.31 (s, 1H), 6.51 (s, 1H), 5.45 (s, 2H), 5.29 (s, 3H), 5.07 (s, 2H), 4.38 (d, *J =* 6.6 Hz, 2H), 4.24 - 4.15 (m, 1H), 3.91 (s, 2H), 3.84 (s, 2H), 3.60 - 3.48 (m, 40H), 3.41 (d, *J =* 3.6 Hz, 6H), 3.23 (s, 3H), 3.20 (d, *J =* 5.7 Hz, 2H), 3.14 (s, 1H), 3.05 (d, *J* = 6.1 Hz, 2H), 2.55 (d, *J=* 7.1 Hz, 2H), 2.38 (s, 3H), 2.26 (t, *J=* 7.3 Hz, 2H), 2.19 (s, 2H), 1.98 (dd, *J=* 13.5, 6.4 Hz, 1H), 1.91 - 1.83 (m, 2H), 1.83 - 1.76 (m, 2H), 1.65 (s, 1H), 1.55 (s, 1H), 1.40 (s, 2H), 1.29 (d, *J=* 7.1 Hz, 3H), 1.23 (s, 2H), 0.89 - 0.81 (m, 9H).

### Example B29: preparation of compound B29

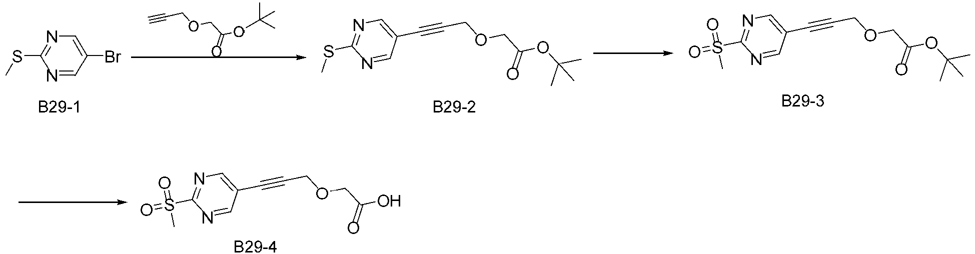

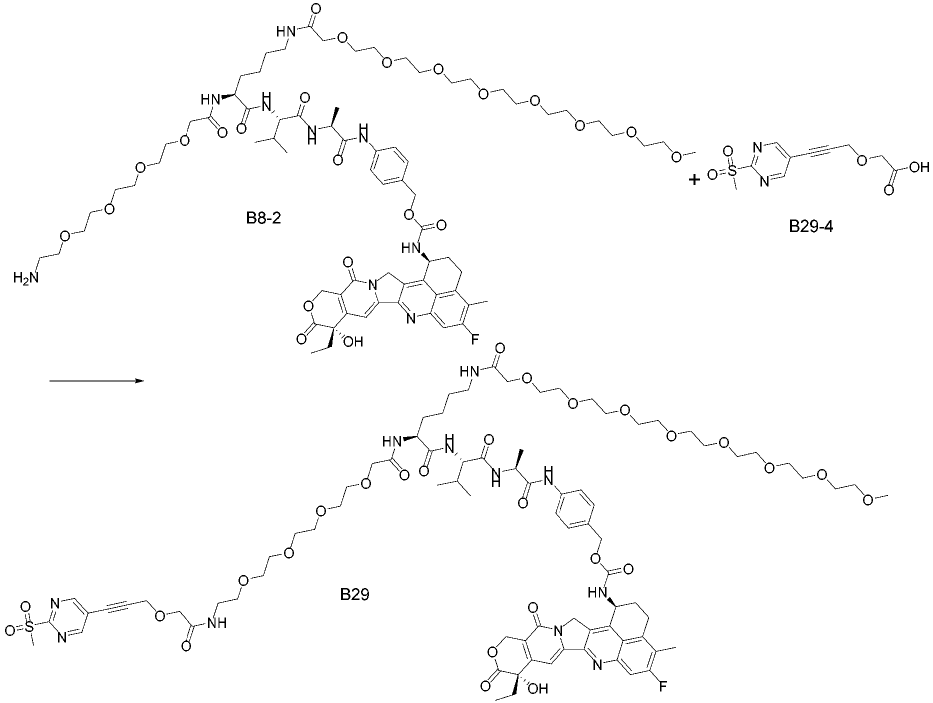

### Step 1: synthesis of 2-methylpropan-2-yl ({3-[2-(methylthio)pyrimidin-5-yl]prop-2-ynyl}oxy)acetate (B29-2)

B29-1 (2.05 g, 10 mmol) and toluene (30 mL) were added into a 250 mL three-neck flask, added with 2-methylpropan-2-yl (prop-2-ynyloxy)acetate (2.04 g, 12 mmol), cuprous iodide (38 mg, 0.2 mmol), triphenylphosphine (262 mg, 1 mmol), bis(acetonitrile)palladium(II) chloride (77.7 mg, 0.3 mmol) and diisopropylamine (2.02 g, 20 mmol) at room temperature, subjected to nitrogen replacement for 3 times, and stirred at room temperature overnight. The reaction solution was filtered, concentrated and then purified by column chromatography (eluting agent: petroleum ether/ethyl acetate) to obtain B29-2 (2.0 g, 67.8%).

LC-MS:295.1 [M+H]⁺.

### Step 2: synthesis of 2-methylpropan-2-yl ({3-[2-(methyldioxo-λ⁶-sulfanyl)pyrimidin-5-yl]prop-2-ynyl}oxy)acetate (B29-3)

B29-2 (2.0 g, 6.79 mmol) and dichloromethane (30 mL) were added into a 100 mL three-neck flask, added with meta-chloroperbenzoic acid (5.86 g, 33.95 mmol) at room temperature, subjected to nitrogen replacement for 3 times, and stirred at room temperature overnight. The reaction solution was filtered, and the filtrate was concentrated and then purified by column chromatography (eluting agent: petroleum ether/ethyl acetate) to obtain B29-3 (1.5 g, 67.6%).

LC-MS: 327.1[M+H]⁺.

### Step 3: synthesis of ({3-[2-(methyldioxo-λ⁶-sulfanyl)pyrimidin-5-yl]prop-2-ynyl}oxy)acetic acid (B29-4)

B29-3 (1.5 g, 4.58 mmol) was added into a 100 mL single-neck flask and dissolved with trifluoroacetic acid (4 mL) and dichloromethane (20 mL). The reaction solution was stirred at room temperature for 1 hour. The reaction solution was concentrated under a reduced pressure. The crude product was purified by preparative high performance liquid chromatography to obtain B29-4 (900 mg, 72.5%).

LC-MS 271.0[M+H]⁺.

### Step 4: synthesis of (4-{[(31S,34S,37S)-37-methyl-31-({21-[2-(methyldioxo-λ6-sulfanyl)pyrimidin-5-yl]-1,16-diox o-15-aza-3,6,9,12,18-pentaoxahenicos-20-yn-1-yl}amino)-25,32,35,38-tetraoxo-34-(prop-2-yl) -2,5,8,11,14,17,20,23-octaoxa-26,33,36-triazaoctatriacontan-38-yl]amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B29

B8-2 (40.0 mg, 0.03 mmol) and 2-(7-azabenzotriazol-1-yl)-N,N,N',N'-tetramethyluronium hexafluorophosphate (11.4 mg, 0.03 mmol) were added into a 10 mL single-neck flask, and dissolved with N,N-dimethylformamide (1 mL). The obtained solution was added with diisopropylethylamine (0.03 mL, 0.06 mmol). The mixed solution was stirred at room temperature for 15 minutes. Then, B29-4 (8 mg, 0.03 mmol) was added into the mixed solution. The reaction solution was stirred at room temperature for 3 hours. The reaction solution was filtered and purified by preparative high performance liquid chromatography to obtain the compound B29 (13.6 mg, 25.9 %).

LC-MS: 1748.8[M+H]⁺.

¹H NMR (400 MHz, DMSO) δ 9.99 (s, 1H), 9.20 (s, 2H), 8.17 (d, *J=* 6.6 Hz, 1H), 8.05 (d, *J* = 8.5 Hz, 1H), 7.89 (d, *J* = 8.8 Hz, 1H), 7.83 (t, *J =* 5.8 Hz, 1H), 7.78 (d, *J =* 10.9 Hz, 1H), 7.64 (t, *J=* 7.0 Hz, 2H), 7.59 (d, *J=* 8.4 Hz, 2H), 7.36 (d, *J =* 8.5 Hz, 2H), 7.31 (s, 1H), 6.51 (s, 1H), 5.45 (s, 2H), 5.28 (s, 3H), 5.07 (s, 2H), 4.59 (s, 2H), 4.38 (dd, *J =* 13.6, 6.7 Hz, 2H), 4.23 - 4.15 (m, 1H), 4.04 (s, 2H), 3.91 (s, 2H), 3.84 (s, 2H), 3.59 - 3.47 (m, 39H), 3.45 - 3.40 (m, 7H), 3.28 - 3.24 (m, 2H), 3.23 (s, 3H), 3.14 (s, 1H), 3.05 (dd, *J* = 13.5, 7.0 Hz, 2H), 2.38 (s, 3H), 2.19 (s, 2H), 1.98 (dd, *J=* 13.7, 6.9 Hz, 1H), 1.87 (tt, *J =* 14.1, 7.1 Hz, 2H), 1.65 (s, 1H), 1.54 (d, *J =* 9.6 Hz, 1H), 1.40 (s, 2H), 1.29 (d, *J=* 7.1 Hz, 3H), 1.23 (s, 2H), 0.91 - 0.79 (m, 9H).

### Example B30: preparation of compound B30

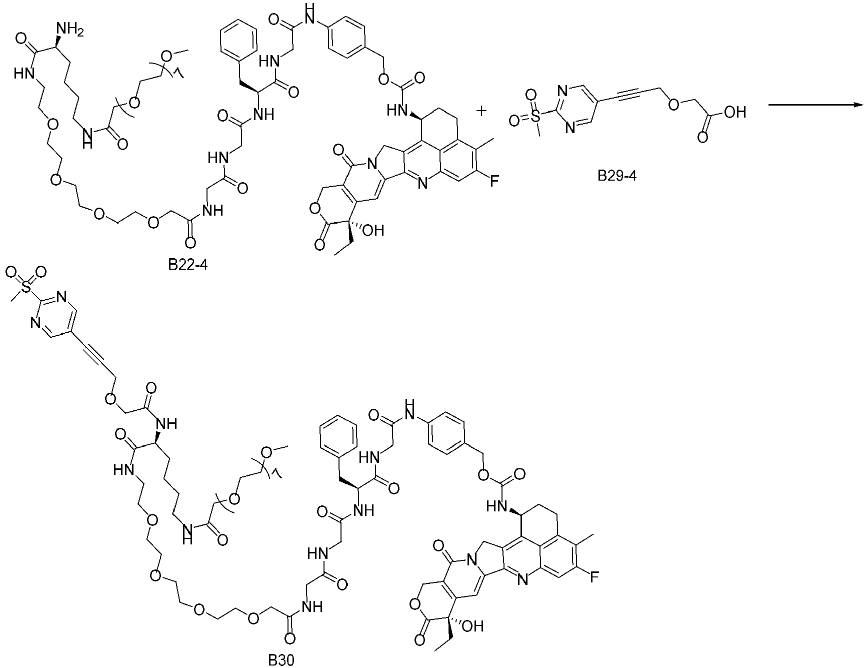

### Step 1: synthesis of (4-{[(31S,55S)-55-benzyl-31-{[2-({3-[2-(methyldioxo-λ6-sulfanyl)pyrimidin-5-yl]prop-2-ynyl }oxy)acetyl]amino}-25,32,47,50,53,56,59-heptaoxo-2,5,8,11,14,17,20,23,36,39,42,45-dodecaox a-26,33,48,51,54,57-hexaazanonapentacontan-59-yl]amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B30

B22-4 (49.0 mg, 0.03 mmol) and 2-(7-azabenzotriazol-1-yl)-N,N,N',N'-tetramethyluronium hexafluorophosphate (11.4 mg, 0.03 mmol) were added into a 10 mL single-neck flask, and dissolved with N,N-dimethylformamide (1 mL). The obtained solution was added with diisopropylethylamine (0.03 mL, 0.06 mmol). The mixed solution was stirred at room temperature for 15 minutes. Then, B29-4 (8 mg, 0.03 mmol) was added into the mixed solution. The reaction solution was stirred at room temperature for 3 hours. The reaction solution was filtered and purified by preparative high performance liquid chromatography to obtain the compound B30 (14.8 mg, 26.0 %).

LC-MS: 1896.8[M+H]⁺.

### Example B31: preparation of compound B31

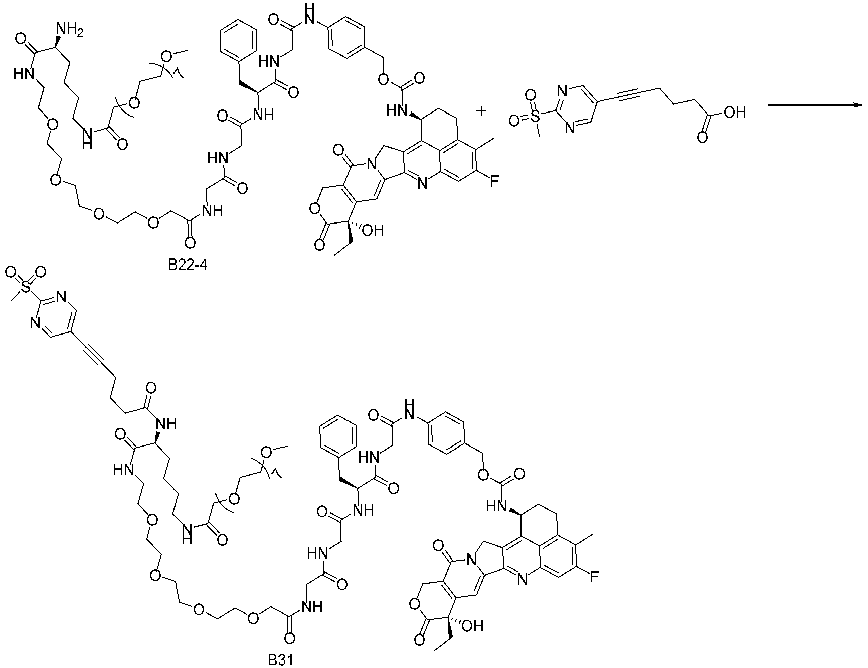

### Step 1: synthesis of (4-{[(31S,55S)-55-benzyl-31-({6-[2-(methyldioxo-λ6-sulfanyl)pyrimidin-5-yl]-1-oxohex-5-yny l}amino)-25,32,47,50,53,56,59-heptaoxo-2,5,8,11,14,17,20,23,36,39,42,45-dodecaoxa-26,33,48, 51,54,57-hexaazanonapentacontan-59-yl]amino}phenyl)methyl {(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B31

B22-4 (49.0 mg, 0.03 mmol) and 2-(7-azabenzotriazol-1-yl)-N,N,N',N'-tetramethyluronium hexafluorophosphate (11.4 mg, 0.03 mmol) were added into a 10 mL single-neck flask, and dissolved with N,N-dimethylformamide (1 mL). The obtained solution was added with diisopropylethylamine (0.03 mL, 0.06 mmol). The mixed solution was stirred at room temperature for 15 minutes. Then, 6-[2-(methyldioxo-λ⁶-sulfanyl)pyrimidin-5-yl]hex-5-ynoic acid (8 mg, 0.03 mmol) was added into the mixed solution. The reaction solution was stirred at room temperature for 3 hours. The reaction solution was filtered and purified by preparative high performance liquid chromatography to obtain the compound B31 (16.2 mg, 29.5 %).

LC-MS: 1894.8[M+H]⁺.

### Example B32: preparation of compound B32

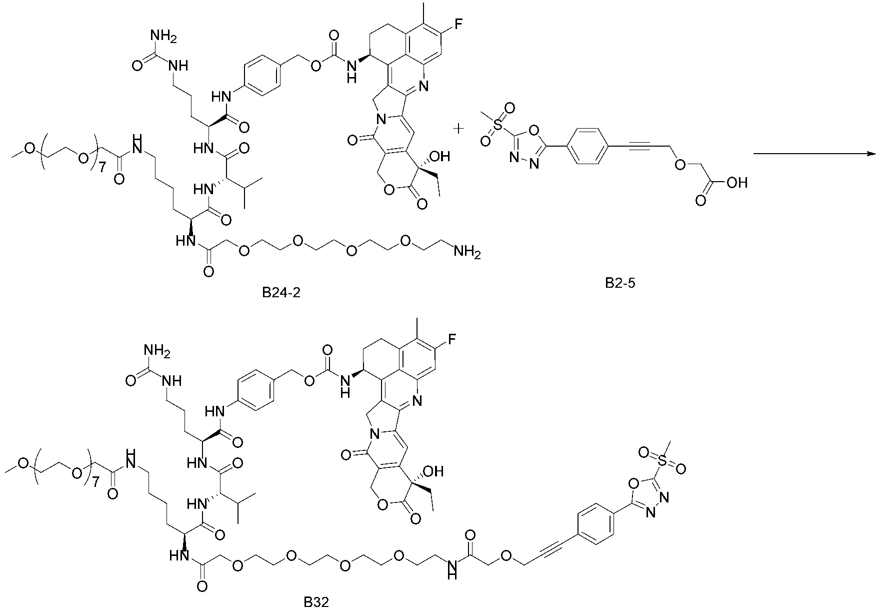

### Step 1: synthesis of (4-{[(31S,34S,37S)-37-{3-[(aminocarbonyl)amino]propyl}-31-[(21-{4-[5-(methyldioxo-λ6-sulf anyl)-1,3,4-oxadiazol-2-yl]phenyl}-1,16-dioxo-15-aza-3,6,9,12,18-pentaoxahenicos-20-yn-1-yl )amino]-25,32,35,38-tetraoxo-34-(prop-2-yl)-2,5,8,11,14,17,20,23-octaoxa-26,33,36-triazaocta triacontan-38-yl]amino}phenyl)methyl { [(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B32

B24-2 (32 mg, 0.02 mmol) and 2-(7-azabenzotriazol-1-yl)-N,N,N',N'-tetramethyluronium hexafluorophosphate (11.4 mg, 0.03 mmol) were added into a 50 mL single-neck flask, and dissolved with N,N-dimethylformamide (1 mL). The obtained solution was added with diisopropylethylamine (0.2 mL, 0.06 mmol). The mixed solution was stirred at room temperature for 15 minutes. Then, B2-5 (10 mg, 0.03 mmol) was added into the mixed solution. The reaction solution was stirred at room temperature for 3 hours. The reaction solution was filtered and purified by preparative high performance liquid chromatography to obtain B32 (10 mg, 26.3 %).

LC-MS: 1900.8[M+H]⁺.

### Example B33: preparation of compound B33

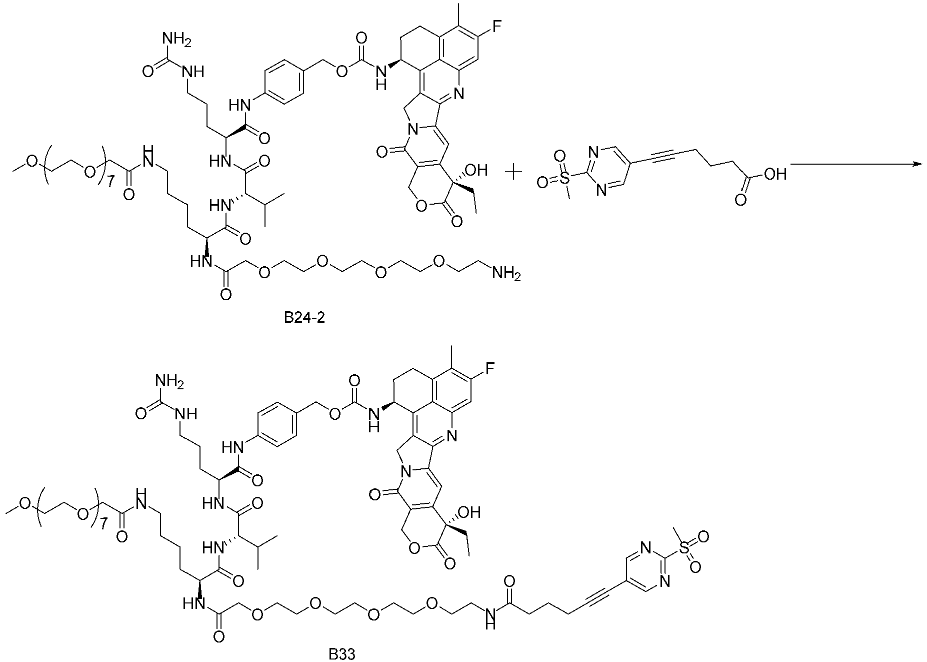

### Step 1: synthesis of (4-{[(31S,34S,37S)-37-{3-[(aminocarbonyl)amino]propyl}-31-({21-[2-(methyldioxo-λ6-sulfan yl)pyrimidin-5-yl]-1,16-dioxo-15-aza-3,6,9,12-tetraoxahenicos-20-yn-1-yl}amino)-25,32,35,3 8-tetraoxo-34-(prop-2-yl)-2,5,8,11,14,17,20,23-octaoxa-26,33,36-triazaoctatriacontan-38-yl]a mino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B33

B24-2 (32 mg, 0.02 mmol) and 2-(7-azabenzotriazol-1-yl)-N,N,N',N'-tetramethyluronium hexafluorophosphate (11.4 mg, 0.03 mmol) were added into a 50 mL single-neck flask, and dissolved with N,N-dimethylformamide (1 mL). The obtained solution was added with diisopropylethylamine (0.2 mL, 0.06 mmol). The mixed solution was stirred at room temperature for 15 minutes. Then, 6-[2-(methyldioxo-λ⁶-sulfanyl)pyrimidin-5-yl]hex-5-ynoic acid (8 mg, 0.03 mmol) was added into the mixed solution. The reaction solution was stirred at room temperature for 3 hours. The reaction solution was filtered and purified by preparative high performance liquid chromatography to obtain B33 (9.8 mg, 26.8 %).

LC-MS: 1832.8[M+H]⁺.

### Example B34: preparation of compound B34

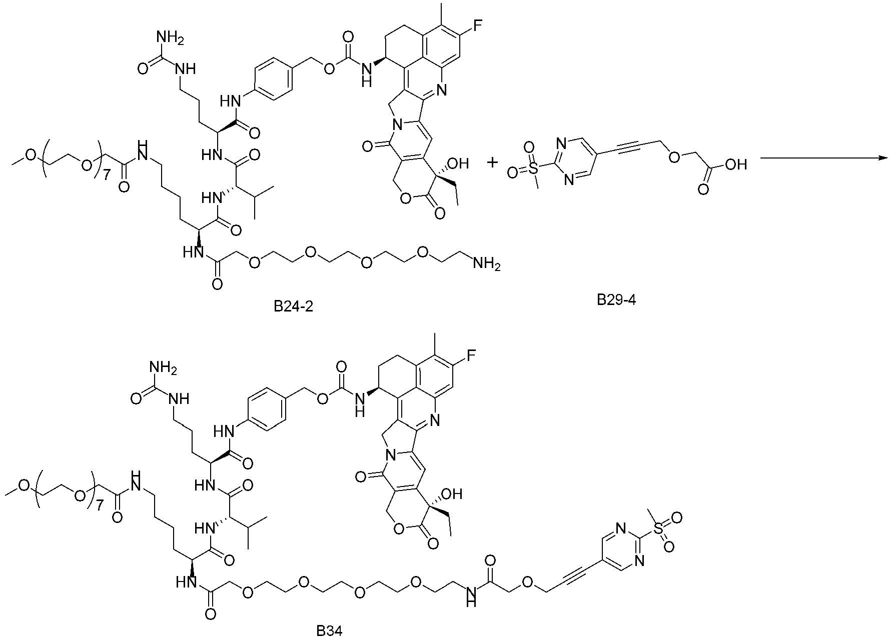

### Step 1: synthesis of (4-{[(31S,34S,37S)-37-{3-[(aminocarbonyl)amino]propyl}-31-({21-[2-(methyldioxo-λ6-sulfan yl)pyrimidin-5-yl]-1,16-dioxo-15-aza-3,6,9,12,18-pentaoxahenicos-20-yn-1-yl}amino)-25,32,3 5,38-tetraoxo-34-(prop-2-yl)-2,5,8,11,14,17,20,23-octaoxa-26,33,36-triazaoctatriacontan-38-y l]amino}phenyl)methyl {[(1S,9S)-9-ethyl-5-fluoro-9-hydroxy-4-methyl-10,13-dioxo-2,3,9,10,12,15-hexahydro-1H-cyc lohexo[1,2,3-de]pyrano[3',4':6,7]indolizino[1,2-b]quinolin-1-yl]amino}formate B34

B24-2 (32 mg, 0.02 mmol) and 2-(7-azabenzotriazol-1-yl)-N,N,N',N'-tetramethyluronium hexafluorophosphate (11.4 mg, 0.03 mmol) were added into a 50 mL single-neck flask, and dissolved with N,N-dimethylformamide (1 mL). The obtained solution was added with diisopropylethylamine (0.2 mL, 0.06 mmol). The mixed solution was stirred at room temperature for 15 minutes. Then, B29-4 (8 mg, 0.03 mmol) was added into the mixed solution. The reaction solution was stirred at room temperature for 3 hours. The reaction solution was filtered and purified by preparative high performance liquid chromatography to obtain B34 (11.5 mg, 31.3 %).

LC-MS: 1834.8[M+H]⁺.

### Example B35: preparation of compound B35

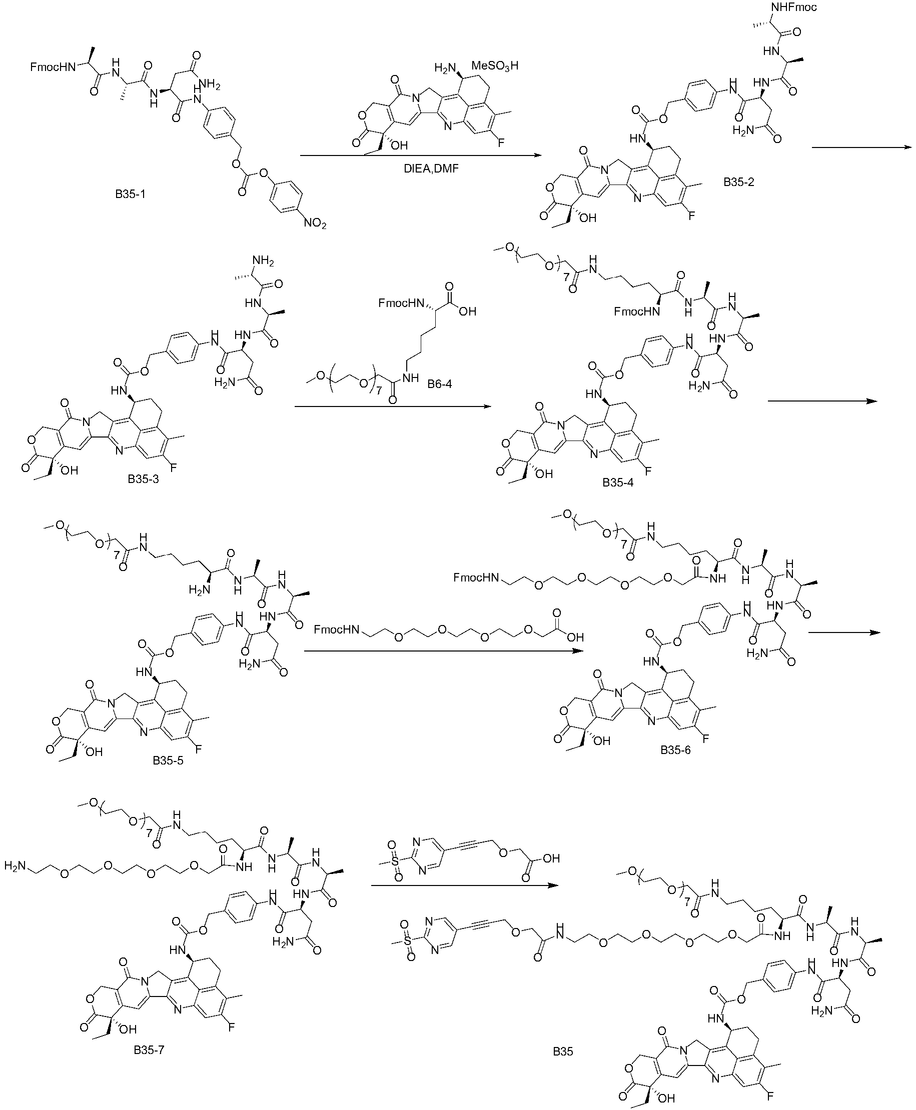

The compound B35 was obtained by reference to the preparation methods for the compounds B20 and B24.

LC-MS: 1834.8[M+H]⁺.

### Example B36: preparation of compound B36

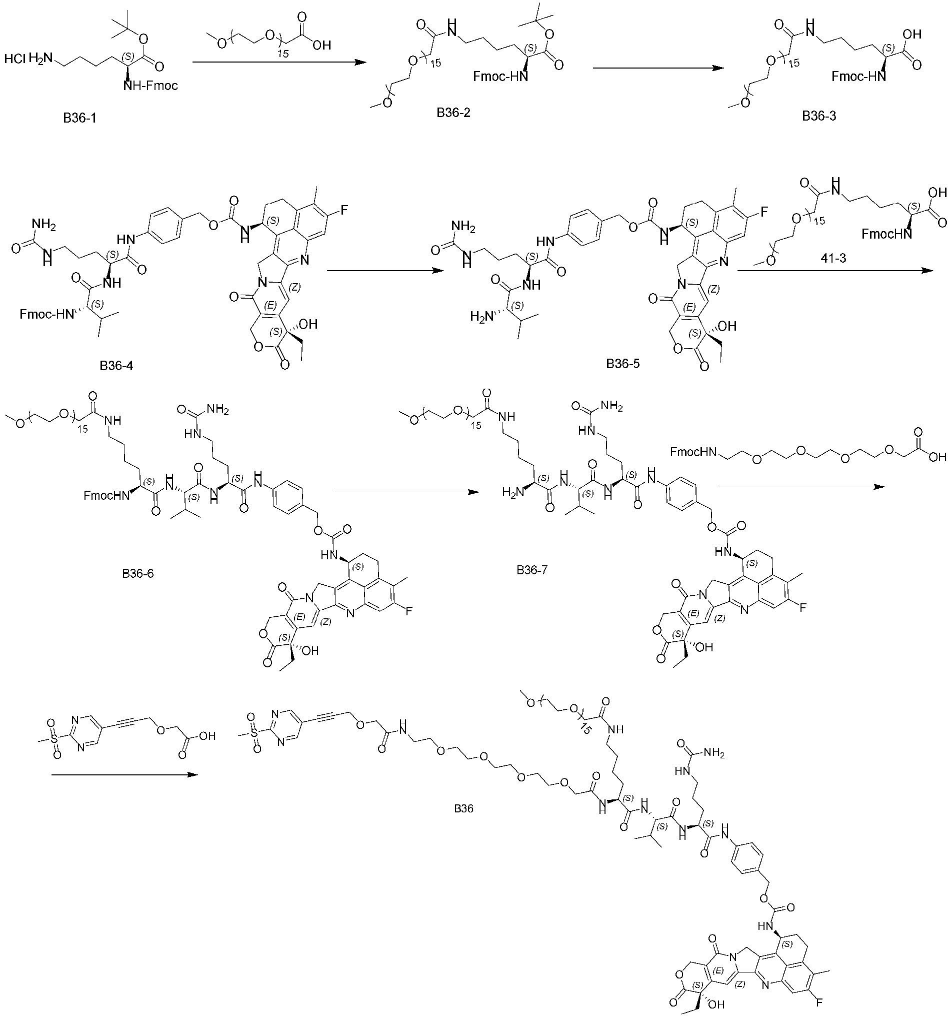

Compound B36-3 was synthesized by reference to the synthetic method for the compound B6-4, and the compound B36 was overall synthesized by reference to the synthetic method for the compound B8. Finally, 20 mg of the compound B36 was obtained through purification by preparative liquid chromatography.

LC-MS: 1094.1[M/2+H]+.

¹H NMR (400 MHz, DMSO-d₆) δ 10.04 (s, 1H), 9.20 (s, 2H), 8.12 (d, J = 7.3 Hz, 1H), 8.05 (d, J = 8.8 Hz, 1H), 7.90 (d, J = 8.7 Hz, 1H), 7.83 (t, J = 5.7 Hz, 1H), 7.78 (d, J = 10.9 Hz, 1H), 7.70 - 7.63 (m, 2H), 7.60 (d, J = 8.3 Hz, 2H), 7.36 (d, J = 8.4 Hz, 2H), 7.31 (s, 1H), 6.52 (s, 1H), 5.98 (t, J = 5.9 Hz, 1H), 5.45 (s, 2H), 5.41 (s, 2H), 5.29 (s, 3H), 5.07 (s, 2H), 4.59 (s, 2H), 4.38 (d, J = 6.4 Hz, 2H), 4.27 - 4.15 (m, 1H), 4.04 (s, 2H), 3.91 (s, 2H), 3.84 (s, 2H), 3.62 - 3.54 (m, 8H), 3.53 - 3.45 (m, 57H), 3.43 (d, J = 5.0 Hz, 8H), 3.27 (t, J = 6.1 Hz, 5H), 3.23 (s, 5H), 3.05 (p, J = 7.4 Hz, 4H), 2.97 - 2.89 (m, 1H), 2.38 (d, J = 1.7 Hz, 3H), 2.17 (d, J = 13.1 Hz, 2H), 2.03 - 1.77 (m, 3H), 1.73 - 1.50 (m, 4H), 1.40 (dt, J = 12.6, 8.1 Hz, 4H), 1.29 - 1.14 (m, 2H), 0.94 - 0.73 (m, 9H).

### Example B37: preparation of compound B37

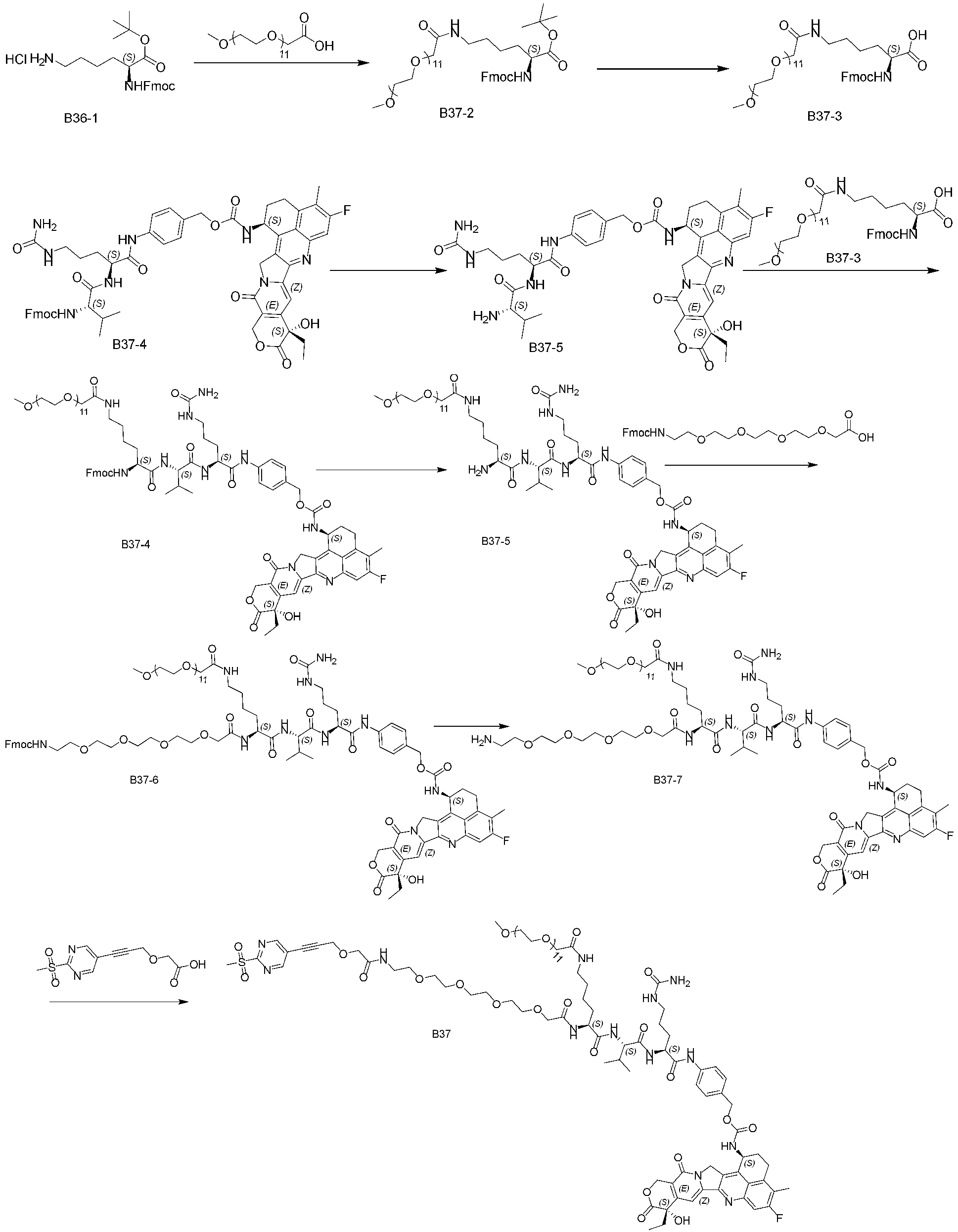

The compound B37 was overall synthesized by reference to the synthetic method for the compound B36. Finally, 30 mg of the compound B37 was obtained through purification by preparative liquid chromatography.

LC-MS: 1006.6 [M/2+H]⁺.

¹H NMR (400 MHz, DMSO-d₆) δ 10.04 (s, 1H), 9.20 (s, 2H), 8.12 (d, J = 7.2 Hz, 1H), 8.05 (d, J = 8.8 Hz, 1H), 7.90 (d, J = 8.6 Hz, 1H), 7.83 (t, J = 5.8 Hz, 1H), 7.77 (d, J = 10.9 Hz, 1H), 7.65 (dd, J = 7.3, 3.8 Hz, 2H), 7.60 (d, J = 8.3 Hz, 2H), 7.36 (d, J = 8.3 Hz, 2H), 7.31 (s, 1H), 6.51 (s, 1H), 5.97 (t, J = 5.9 Hz, 1H), 5.45 (s, 2H), 5.41 (s, 2H), 5.29 (d, J = 4.1 Hz, 3H), 5.07 (s, 2H), 4.59 (s, 2H), 4.38 (q, J = 7.5, 7.1 Hz, 2H), 4.21 (dd, J = 8.7, 6.8 Hz, 1H), 4.04 (s, 2H), 3.91 (s, 2H), 3.84 (s, 2H), 3.60 - 3.49 (m, 52H), 3.43 (d, J = 4.2 Hz, 8H), 3.27 (q, J = 5.8 Hz, 3H), 3.23 (s, 3H), 3.18 - 2.86 (m, 6H), 2.42 - 2.34 (m, 3H), 2.27 - 2.08 (m, 2H), 1.98 (q, J = 6.8 Hz, 1H), 1.87 (dt, J = 16.7, 7.0 Hz, 2H), 1.74 - 1.49 (m, 4H), 1.40 (s, 4H), 1.23 (d, J = 8.1 Hz, 2H), 0.91 - 0.77 (m, 9H).

### Example B38: preparation of compound B38

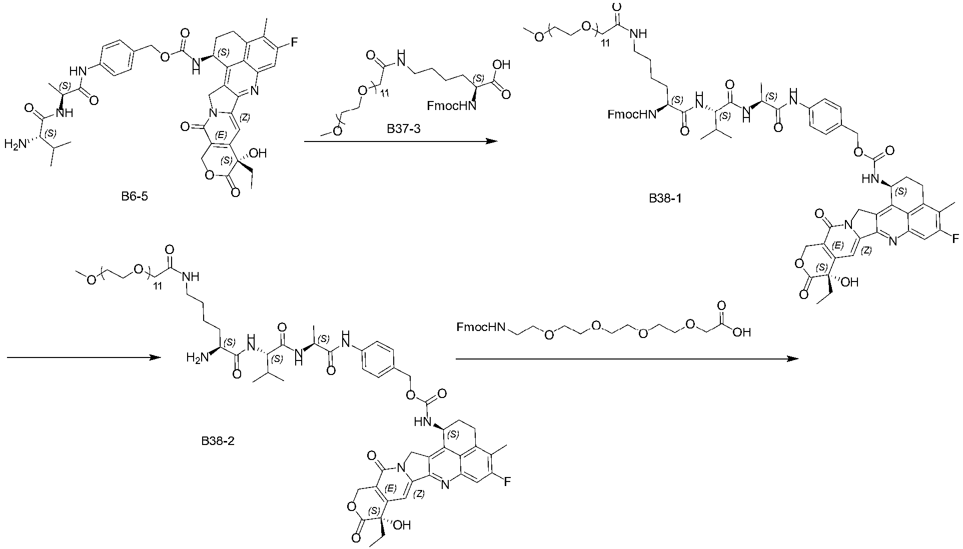

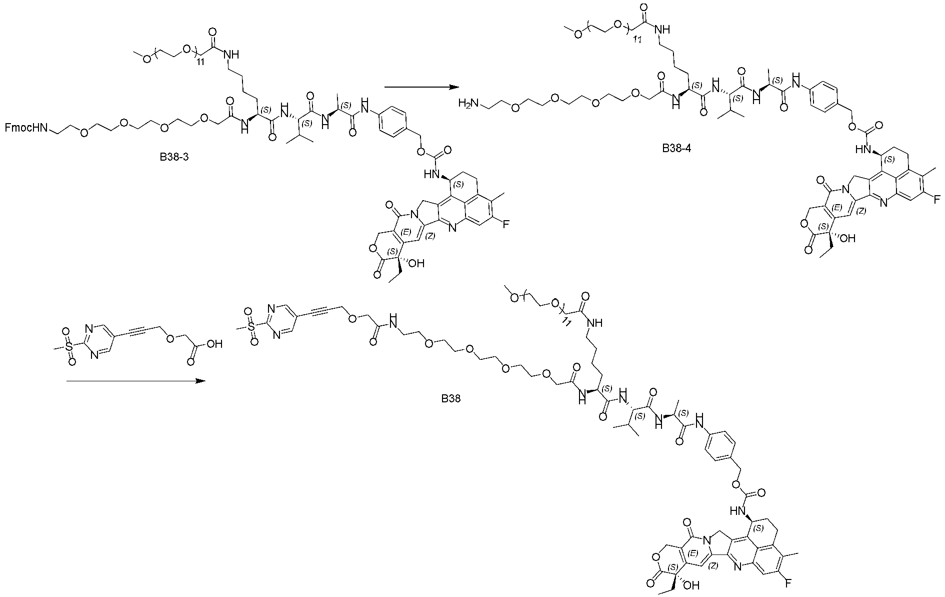

The compound B38 was overall synthesized by reference to the synthetic method for the compound B36. Finally, 12.5 mg of the compound B38 was obtained through purification by preparative liquid chromatography.

LC-MS: 963.0 [M/2+H]⁺.

¹H NMR (400 MHz, DMSO-d₆) δ 9.99 (s, 1H), 9.20 (s, 2H), 8.17 (d, J = 6.8 Hz, 1H), 8.05 (d, J = 8.8 Hz, 1H), 7.89 (d, J = 8.7 Hz, 1H), 7.82 (t, J = 5.7 Hz, 1H), 7.78 (d, J = 10.9 Hz, 1H), 7.65 (d, J = 7.9 Hz, 2H), 7.59 (d, J = 8.2 Hz, 2H), 7.36 (d, J = 8.3 Hz, 2H), 7.31 (s, 1H), 6.51 (s, 1H), 5.45 (s, 2H), 5.29 (s, 3H), 5.07 (s, 2H), 4.59 (s, 2H), 4.44 - 4.31 (m, 2H), 4.25 - 4.15 (m, 1H), 4.04 (s, 2H), 3.91 (s, 2H), 3.84 (s, 2H), 3.59 - 3.54 (m, 7H), 3.53 - 3.49 (m, 42H), 3.46 - 3.37 (m, 8H), 3.27 (t, J = 5.9 Hz, 4H), 3.23 (s, 3H), 3.05 (q, J = 6.8 Hz, 2H), 2.38 (s, 3H), 2.19 (s, 2H), 1.98 (q, J = 7.1 Hz, 2H), 1.86 (dq, J = 14.1, 6.9 Hz, 2H), 1.65 (s, 1H), 1.55 (d, J = 9.6 Hz, 1H), 1.40 (s, 2H), 1.33 - 1.11 (m, 8H), 0.86 (dt, J = 15.7, 7.1 Hz, 9H).

### Example C1: preparation of compound C1

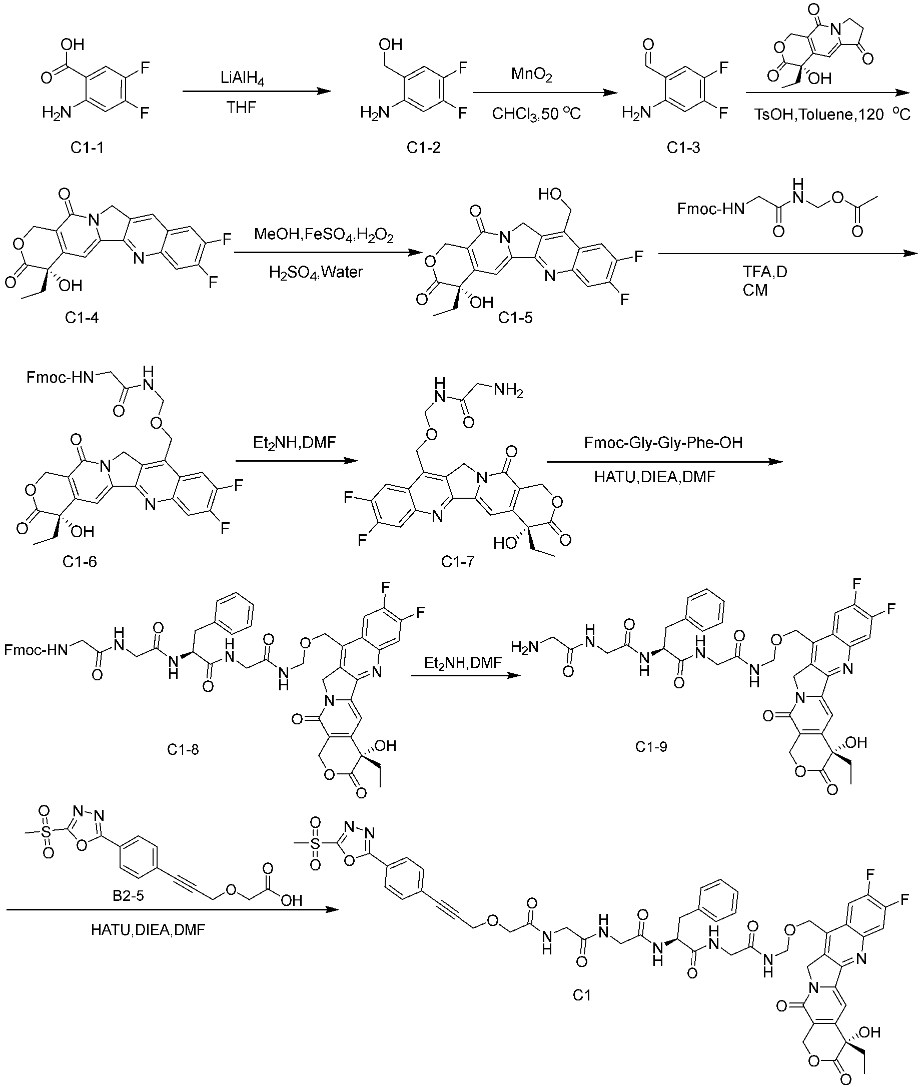

### Step 1: synthesis of (2-amino-4,5-difluorophenyl)methanol (C1-2)

C1-1 (3 g, 17.3 mmol) was added into a 100 mL three-neck flask and dissolved with anhydrous tetrahydrofuran (30 mL). The solution was stirred at 0°C for 15 minutes. Then, lithium aluminum hydride (10.38 mL, 2.5 mol/L in THF) was dropwise added into the mixed solution. The reaction solution was stirred at 0°C for 30 minutes, and then reacted at room temperature overnight. The reaction solution was quenched with sodium sulfate decahydrate at 0°C, extracted with ethyl acetate, and dried with anhydrous sodium sulfate. The organic phase was dried by rotary evaporation, and purified by silica gel column chromatography (petroleum ether: ethyl acetate = 5: 1) to obtain C1-2 (2.5 g, 90.9%).

LC-MS: 160.1[M+H]⁺.

### Step 2: synthesis of 2-amino-4,5-difluorobenzene-1-carbaldehyde (C1-3)

C1-2 (2.5 g, 15.6 mmol) was added into a 100 mL three-neck flask and dissolved with trichloromethane (30 mL). The reaction solution was added with 2 g of activated manganese dioxide, and reacted at 50°C overnight. The solution was filtered, dried by rotary evaporation, and purified by silica gel column chromatography (petroleum ether: ethyl acetate = 8: 1) to obtain C1-3 (2.2 g, 89.4%).

LC-MS: 158.0[M+H]⁺.

### Step 3: synthesis of (4S)-4-ethyl-8,9-difluoro-4-hydroxy-3,4,12,14-tetrahydro-1H-pyrano[3',4':6,7]indolizino[1,2 -b]quinolin-3,14-dione (C1-4)

(4S)-4-ethyl-4-hydroxy-3,4,6,7,8,10-hexahydro-1H-pyrano[3,4-f]indolizine-3,6,10-trione (3 g, 11.4 mmol), C1-3 (2.2 g, 13.7 mmol) and p-toluenesulfonic acid (189.4 mg, 1.1 mmol) were added into a 250 mL single-neck flask, and dissolved with anhydrous toluene (50 mL). The reaction solution reacted at 120°C overnight, dried by rotary evaporation to remove the solvent, then slurried with methanol, and filtered to obtain C1-4 (2.2 g, 50.3%).

LC-MS: 385.1[M+H]⁺.

### Step 4: synthesis of (4S)-4-ethyl-8,9-difluoro-4-hydroxy-11-(hydroxymethyl)-3,4,12,14-tetrahydro-1H-pyrano[3', 4':6,7]indolizino[1,2-b]quinolin-3,14-dione (C1-5)

C1-4 (500 mg, 1.3 mmol) was added into a 100 mL single-neck flask, dissolved with 15 mL of methanol and 12.5 mL of water, dropwise added with 6.5 mL of concentrated sulfuric acid at 0°C, added with 400 mg of ferrous sulfate heptahydrate, and finally dropwise added with 1.2 mL of 30% hydrogen peroxide. The reaction solution was stirred at room temperature overnight, and the reaction system was added with 50 mL of ice water, and filtered to obtain C1-5 (400 mg, 74.1%).

LC-MS: 415.1[M+H]⁺.

### Step 5: synthesis of 9H-fluoren-9-ylmethyl ({1-[(4S)-4-ethyl-8,9-difluoro-4-hydroxy-3,14-dioxo-1,3,4,12-tetrahydro-pyrano[3',4':6,7]ind olizino[1,2-b]quinolin-11-yl]-5-oxo-4-aza-2-oxahex-6-yl}amino)formate (C1-6)

C1-5 (380 mg, 0.92 mmol) and 1-(9H-fluoren-9-yl)-3,6-dioxo-4,7-diaza-2-oxaoctan-8-yl acetate (169 mg, 0.46 mmol) were added into a 100 mL single-neck flask, and dissolved with a mixed solution of 1 mL of trifluoroacetic acid and 4 mL of dichloromethane. The reaction solution was stirred at room temperature overnight, then dried by rotary evaporation to remove the solvent, and purified by preparative liquid chromatography to obtain C1-6 (180 mg, 54.2%).

LC-MS:723.2[M+H]⁺.

### Step 6: synthesis of 2-amino-N-[({[(4S)-4-ethyl-8,9-difluoro-4-hydroxy-3,14-dioxo-1,3,4,12-tetrahydropyrano[3', 4':6,7]indolizino[1,2-b]quinolin-11-yl]methyl}oxy)methyl]acetamide (C1-7)

C1-6 (180.0 mg, 0.25 mmol) was added into a 50 mL single-neck flask and dissolved with N,N-dimethylformamide (2 mL). The obtained solution was added with diethylamine (182 mg, 2.5 mmol). The reaction solution was stirred at room temperature for 1 hour. The reaction solution was concentrated under a reduced pressure. Residues were slurried with petroleum ether to obtain C1-7 (110 mg, 88.0 %).

LC-MS: 501.2[M+H]⁺.

### Step 7: synthesis of 9H-fluoren-9-ylmethyl {[(9S)-9-benzyl-1-[(4S)-4-ethyl-8,9-difluoro-4-hydroxy-3,14-dioxo-1,3,4,12-tetrahydropyran o[3',4':6,7]indolizino[1,2-b]quinolin-11-yl]-5,8,11,14-tetraoxo-2-oxa-4,7,10,13-tetraazapenta decan-15-yl]amino}formate (C1-8)

C1-7 (110 mg, 0.22 mmol) and 2-(7-azabenzotriazol-1-yl)-N,N,N',N'-tetramethyluronium hexafluorophosphate (91 mg, 0.24 mmol) were added into a 100 mL single-neck flask, and dissolved with N,N-dimethylformamide (2 mL). The obtained solution was added with diisopropylethylamine (57 mg, 0.44 mmol). The mixed solution was stirred at room temperature for 15 minutes. Then, N-[1-(9H-fluoren-9-yl)-3,6,9-trioxo-4,7-diaza-2-oxanonan-9-yl]-L-phenylalanine (120 mg, 0.24 mmol) was added into the mixed solution. The reaction solution was stirred at room temperature for 3 hours. The solution was dried by rotary evaporation and purified by silica gel column chromatography (methanol: dichloromethane = 1: 15) to obtain C1-8 (169 mg, 78.2 %).

LC-MS: 984.3[M+H]⁺.

### Step 8: synthesis of 2-amino-N-[(9S)-9-benzyl-1-[(4S)-4-ethyl-8,9-difluoro-4-hydroxy-3,14-dioxo-1,3,4,12-tetrahy dropyrano[3',4':6,7]indolizino[1,2-b]quinolin-11-yl]-5,8,11-trioxo-2-oxa-4,7,10-triazadodeca n-12-yl]acetamide (C1-9)

C1-8 (169.0 mg, 0.17mmol) was added into a 50 mL single-neck flask and dissolved with N,N-dimethylformamide (2 mL). The obtained solution was added with diethylamine (124 mg, 1.7 mmol). The reaction solution was stirred at room temperature for 1 hour. The reaction solution was concentrated under a reduced pressure. Residues were slurried with petroleum ether to obtain C1-9 (115 mg, 89.1 %).

LC-MS: 762.2[M+H]⁺.

### Step 9: synthesis of N-[(9S)-9-benzyl-1-[(4S)-4-ethyl-8,9-difluoro-4-hydroxy-3,14-dioxo-1,3,4,12-tetrahydropyra no[3',4':6,7]indolizino[1,2-b]quinolin-11-yl]-5,8,11-trioxo-2-oxa-4,7,10-triazadodecan-12-yl]-2-({2-[(3-{4-[5-(methyldioxo-λ⁶-sulfanyl)-1,3,4-oxadiazol-2-yl]phenyl}prop-2-ynyl)oxy]acetyl

### }amino)acetamide C1

C1-9 (11.4 mg, 0.015 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, B2-5 (5.0 mg, 0.015 mmol) was added into the mixed solution. The reaction solution was stirred at room temperature for 3 hours. The reaction solution was filtered and purified by preparative high performance liquid chromatography to obtain C1 (5.6 mg, 34.6 %).

LC-MS: 1080.3[M+H]⁺.

### Example C2: preparation of compound C2

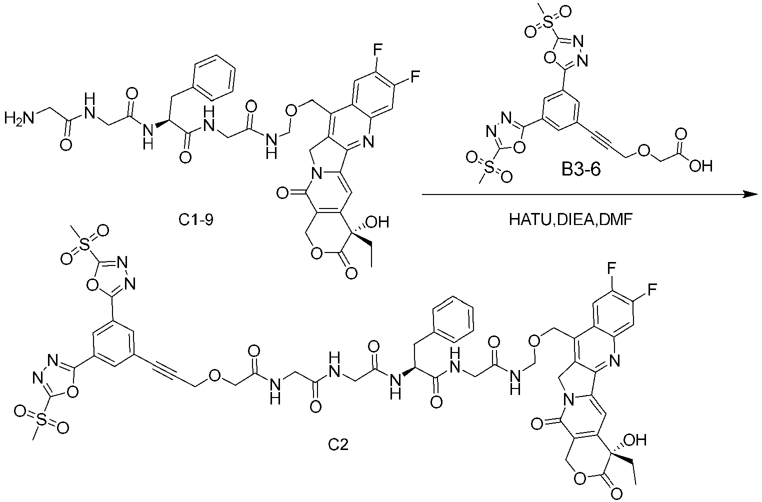

### Step 1: synthesis of N-[(9S)-9-benzyl-1-[(4S)-4-ethyl-8,9-difluoro-4-hydroxy-3,14-dioxo-1,3,4,12-tetrahydropyra no[3',4':6,7]indolizino[1,2-b]quinolin-11-yl]-5,8,11-trioxo-2-oxa-4,7,10-triazadodecan-12-yl]-2-({2-[(3-{3,5-bis[5-(methyldioxo-λ⁶-sulfanyl)-1,3,4-oxadiazol-2-yl]phenyl}prop-2-ynyl)oxy]a cetyl}amino)acetamide C2

C1-9 (11.4 mg, 0.015 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, B3-6 (7.2 mg, 0.015 mmol) was added into the mixed solution. The reaction solution was stirred at room temperature for 3 hours. The reaction solution was filtered and purified by preparative high performance liquid chromatography to obtain C2 (4.5 mg, 24.5 %).

LC-MS: 1226.3[M+H]⁺.

### Example C3: preparation of compound C3

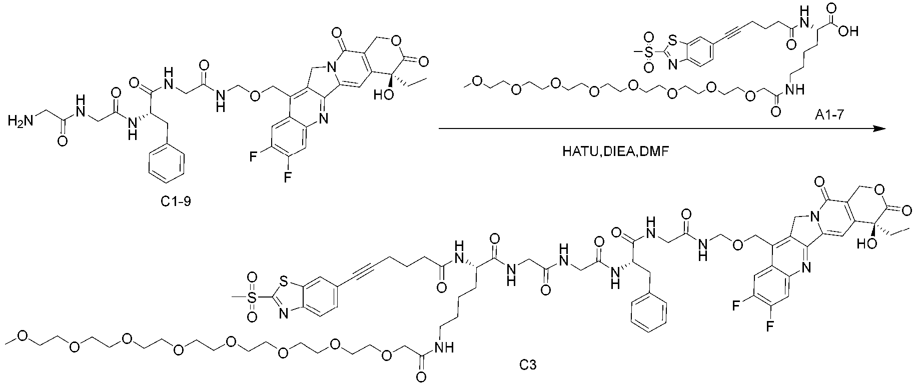

### Step 1: synthesis of N-[(9S)-9-benzyl-1-[(4S)-4-ethyl-8,9-difluoro-4-hydroxy-3,14-dioxo-1,3,4,12-tetrahydropyra no[3',4':6,7]indolizino[1,2-b]quinolin-11-yl]-5,8,11-trioxo-2-oxa-4,7,10-triazadodecan-12-yl]-2-{[(31S)-31-({6-[2-(methyldioxo-λ⁶-sulfanyl)benzo[d][1,3]thiazol-6-yl]-1-oxohex-5-ynyl]ami no)-25,32-dioxo-26-aza-2,5,8,11,14,17,20,23-octaoxadotriacontan-32-yl]amino}acetamide C3

C1-9 (11.4 mg, 0.015 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, A1-7 (12.5 mg, 0.015 mmol) was added into the mixed solution. The reaction solution was stirred at room temperature for 3 hours. The reaction solution was filtered and purified by preparative high performance liquid chromatography to obtain the compound C3 (7.6 mg, 32.2 %).

LC-MS: 1575.6[M+H]⁺.

### Example C4: preparation of compound C4

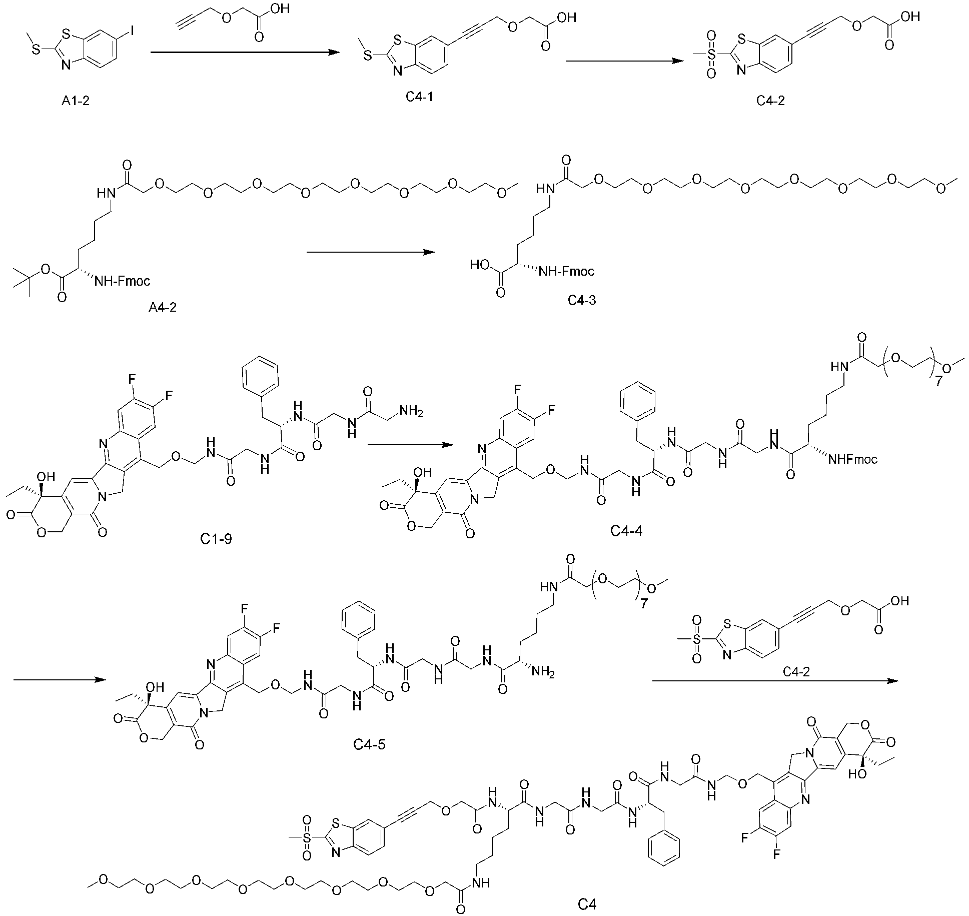

### Step 1: synthesis of ({3-[2-(methylthio)benzo[d][1,3]thiazol-6-yl]prop-2-ynyl}oxy)acetic acid (C4-1)

A1-2 (485.0 mg, 1.58 mmol) was added into a 50 mL single-neck flask and dissolved with diisopropylamine (3 mL). The obtained solution was added with bis(acetonitrile)palladium(II) chloride (12.7 mg, 0.05 mmol), triphenylphosphine (42.7 mg, 0.16 mmol) and cuprous iodide (10.3 mg, 0.03 mmol). The reaction solution was stirred at room temperature for 5 minutes, and then dropwise added with (prop-2-ynyloxy)acetic acid (270.0 mg, 2.37 mmol). The reaction solution was stirred at 50°C overnight. The reaction solution was concentrated under a reduced pressure. Residues were purified by silica gel column chromatography (dichloromethane: methanol = 93: 7) to obtain C4-1 (430.0 mg, 92.9 %).

LC-MS:294.1[M+H]⁺.

### Step 2: synthesis of ({3-[2-(methyldioxo-λ⁶-sulfanyl)benzo[d][1,3]thiazol-6-yl]prop-2-ynyl}oxy)acetic acid (C4-2)

C4-1 (416.0 mg, 1.42 mmol) was added into a 50 mL single-neck flask and dissolved with dichloromethane (20 mL). The obtained solution was added with meta-chloroperoxybenzoic acid (888.3 mg, 5.15 mmol). The reaction solution was stirred at room temperature overnight. The reaction solution was concentrated under a reduced pressure to half of its original volume, and filtered. Then, the organic phase was concentrated under a reduced pressure. Residues were purified by preparative liquid chromatography to obtain C4-2 (70.0 mg, 15.2 %).

LC-MS: 326.1[M+H]⁺.

### Step 3: synthesis of (2S)-2-({[(9H-fluoren-9-ylmethyl)oxy]carbonyl}amino)-6-[(25-oxo-2,5,8,11,14,17,20,23-octao xapentacosan-25-yl)amino]hexanoic acid (C4-3)

A4-2 (200.0 mg, 0.25 mmol) was added into a 50 mL single-neck flask and dissolved with trifluoroacetic acid (1 mL) and dichloromethane (2 mL). The reaction solution was stirred at room temperature for 1 hour, and then heated to 30°C and stirred for 2 hours. The reaction solution was concentrated under a reduced pressure, dissolved with 10 mL of toluene, and concentrated under a reduced pressure again to obtain C4-3 (106.0 mg, 100%).

LC-MS: 749.4[M+H]⁺.

### Step 4: synthesis of 9H-fluoren-9-ylmethyl {[(9S,18S)-9-benzyl-1-[(4S)-4-ethyl-8,9-difluoro-4-hydroxy-3,14-dioxo-1,3,4,12-tetrahydropy rano[3',4':6,7]indolizino[1,2-b]quinolin-11-yl]-5,8,11,14,17,24-hexaoxo-4,7,10,13,16,23-hexaa za-2,26,29,32,35,38,41,44,47-nonaoxaoctatetracontan-18-yl]amino}formate (C4-4)

C1-9 (11.4 mg, 0.015 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, C4-3 (11.2 mg, 0.015 mmol) was added into the mixed solution. The reaction solution was stirred at room temperature for 3 hours. The reaction solution was filtered and purified by preparative high performance liquid chromatography to obtain C4-4 (10 mg, 44.7 %).

LC-MS: 1492.6[M+H]⁺.

### Step 5: synthesis of N-[(9S)-9-benzyl-1-[(4S)-4-ethyl-8,9-difluoro-4-hydroxy-3,14-dioxo-1,3,4,12-tetrahydropyra no[3',4':6,7]indolizino[1,2-b]quinolin-11-yl]-5,8,11-trioxo-2-oxa-4,7,10-triazadodecan-12-yl]-2-{[(31S)-31-amino-25,32-dioxo-26-aza-2,5,8,11,14,17,20,23-octaoxadotriacontan-32-yl]amin o}acetamide (C4-5)

C4-4 (10.0 mg, 0.007 mmol) was added into a 50 mL single-neck flask and dissolved with N,N-dimethylformamide (2 mL). The obtained solution was added with diethylamine (5 mg, 0.07 mmol). The reaction solution was stirred at room temperature for 1 hour. The reaction solution was concentrated under a reduced pressure. Residues were slurried with petroleum ether to obtain C4-5 (7 mg, 78.7 %).

LC-MS: 1270.6[M+H]⁺.

### Step 6: synthesis of N-[(9S)-9-benzyl-1-[(4S)-4-ethyl-8,9-difluoro-4-hydroxy-3,14-dioxo-1,3,4,12-tetrahydropyra no[3',4':6,7]indolizino[1,2-b]quinolin-11-yl]-5,8,11-trioxo-2-oxa-4,7,10-triazadodecan-12-yl]-2-{ [(31S)-31-{[2-({3-[2-(methyldioxo-λ⁶-sulfanyl)benzo[d][1,3]thiazol-6-yl]prop-2-ynyl}oxy)a cetyl]amino}-25,32-dioxo-26-aza-2,5,8,11,14,17,20,23-octaoxadotriacontan-32-yl]amino}acet amide C4

C4-5 (19 mg, 0.015 mmol) and 2-(7-azabenzotriazol-1-yl)-N,N,N',N'-tetramethyluronium hexafluorophosphate (5.7 mg, 0.015 mmol) were added into a 10 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, C4-2 (5.1 mg, 0.015 mmol) was added into the mixed solution. The reaction solution was stirred at room temperature for 3 hours. The reaction solution was filtered and purified by preparative high performance liquid chromatography to obtain C4 (8.8 mg, 37.2 %).

LC-MS: 1577.6[M+H]⁺.

### Example C5: preparation of compound C5

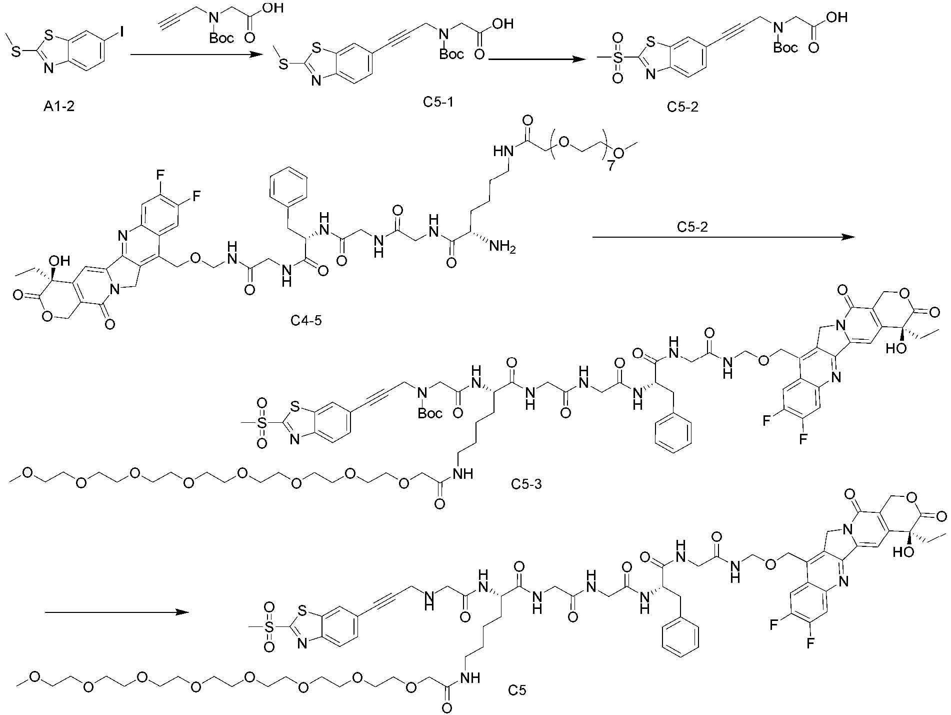

### Step 1: synthesis of N-{[(2-methylpropan-2-yl)oxy]carbonyl}-N-{3-[2-(methylsulfanyl)benzo[d][1,3]thiazol-6-yl] prop-2-ynyl}glycine (C5-1)

A1-2 (485.0 mg, 1.58 mmol) was added into a 50 mL single-neck flask and dissolved with diisopropylamine (3 mL). The obtained solution was added with bis(acetonitrile)palladium(II) chloride (12.7 mg, 0.05 mmol), triphenylphosphine (42.7 mg, 0.16 mmol) and cuprous iodide (10.3 mg, 0.03 mmol). The reaction solution was stirred at room temperature for 5 minutes, and then dropwise added with N-{[(2-methylpropan-2-yl)oxy]carbonyl}-N-(prop-2-ynyl)glycine (505.0 mg, 2.37 mmol). The reaction solution was stirred at 50°C overnight. The reaction solution was concentrated under a reduced pressure. Residues were purified by silica gel column chromatography (dichloromethane: methanol = 93: 7) to obtain C5-1 (550.0 mg, 88.8 %).

LC-MS:393.1[M+H]⁺.

### Step 2: synthesis of N-{3-[2-(methyldioxo-λ⁶-sulfanyl)benzo[d][1,3]thiazol-6-yl]prop-2-ynyl}-N-{[(2-methylprop an-2-yl)oxy]carbonyl}glycine (C5-2)

C5-1 (550.0 mg, 1.42 mmol) was added into a 50 mL single-neck flask and dissolved with dichloromethane (20 mL). The obtained solution was added with meta-chloroperoxybenzoic acid (888.3 mg, 5.15 mmol). The reaction solution was stirred at room temperature overnight. The reaction solution was concentrated under a reduced pressure to half of its original volume, and filtered. Then, the organic phase was concentrated under a reduced pressure. Residues were purified by preparative liquid chromatography to obtain C5-2 (90.0 mg, 14.9 %).

LC-MS: 425.1[M+H]⁺.

### Step 3: synthesis of 2-methylpropan-2-yl [(31S)-31-{(9S)-9-benzyl-1-[(4S)-4-ethyl-8,9-difluoro-4-hydroxy-3,14-dioxo-1,3,4,12-tetrahyd ropyrano[3',4':6,7]indolizino[1,2-b]quinolin-11-yl]-5,8,11,14,17-pentaoxo-2-oxa-4,7,10,13,16-pentaazaheptadecan-17-yl]-38-[2-(methyldioxo-λ⁶-sulfanyl)benzo[d][1,3]thiazol-6-yl]-25,33-dioxo-2,5,8,11,14,17,20,23-octaoxa-26,32,35-triazaoctatriacont-37-yn-35-yl]formate (C5-3)

C4-5 (19 mg, 0.015 mmol) and 2-(7-azabenzotriazol-1-yl)-N,N,N',N'-tetramethyluronium hexafluorophosphate (5.7 mg, 0.015 mmol) were added into a 50 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, C5-2 (6.3 mg, 0.015 mmol) was added into the mixed solution. The reaction solution was stirred at room temperature for 3 hours. The reaction solution was filtered and purified by preparative high performance liquid chromatography to obtain C5-3 (10 mg, 39.8 %).

LC-MS: 1676.6[M+H]⁺.

### Step 4: synthesis of N-[(9S)-9-benzyl-1-[(4S)-4-ethyl-8,9-difluoro-4-hydroxy-3,14-dioxo-1,3,4,12-tetrahydropyra no[3',4':6,7]indolizino[1,2-b]quinolin-11-yl]-5,8,11-trioxo-2-oxa-4,7,10-triazadodecan-12-yl]-2-{[(31S)-31-{[2-({3-[2-(methyldioxo-λ⁶-sulfanyl)benzo[d][1,3]thiazol-6-yl]prop-2-ynyl}amin o)acetyl]amino}-25,32-dioxo-26-aza-2,5,8,11,14,17,20,23-octaoxadotriacontan-32-yl]amino}a cetamide C5

C5-3 (10.0 mg, 0.006mmol) was added into a 50 mL single-neck flask and dissolved with trifluoroacetic acid (0.2 mL) and dichloromethane (0.4 mL). The reaction solution was stirred at room temperature for 1 hour, and then heated to 30°C and stirred for 2 hours. The reaction solution was concentrated under a reduced pressure and purified by preparative high performance liquid chromatography to obtain C5 (3 mg, 31.7 %).

LC-MS: 1576.6[M+H]⁺.

### Example C6: preparation of compound C6

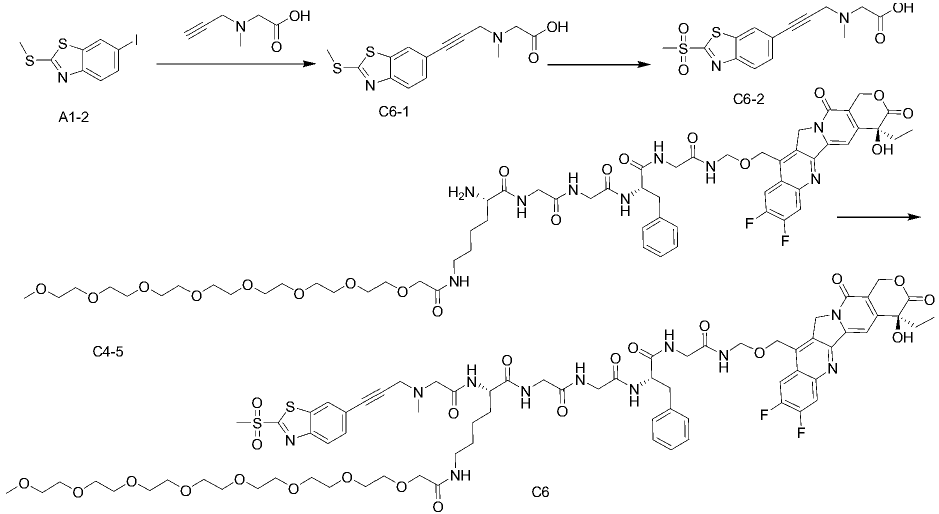

### Step 1: synthesis of N-methyl-N-{3-[2-(methylsulfanyl)benzo[d][1,3] thiazol-6-yl] prop-2-ynyl} glycine (C6-1)

A1-2 (485.0 mg, 1.58 mmol) was added into a 50 mL single-neck flask and dissolved with diisopropylamine (3 mL). The obtained solution was added with bis(acetonitrile)palladium(II) chloride (12.7 mg, 0.05 mmol), triphenylphosphine (42.7 mg, 0.16 mmol) and cuprous iodide (10.3 mg, 0.03 mmol). The reaction solution was stirred at room temperature for 5 minutes, and then dropwise added with N-methyl-N-(prop-2-ynyl)glycine (301.0 mg, 2.37 mmol). The reaction solution was stirred at 50°C overnight. The reaction solution was concentrated under a reduced pressure. Residues were purified by silica gel column chromatography (dichloromethane: methanol = 93: 7) to obtain C6-1 (435.0 mg, 90.1 %).

LC-MS:307.1[M+H]⁺.

### Step 2: synthesis of N-methyl-N-{3-[2-(methyldioxo-λ⁶-sulfanyl)benzo[d][1,3]thiazol-6-yl]prop-2-ynyl}glycine (C6-2)

C6-1 (435.0 mg, 1.42 mmol) was added into a 50 mL single-neck flask and dissolved with dichloromethane (20 mL). The obtained solution was added with meta-chloroperoxybenzoic acid (888.3 mg, 5.15 mmol). The reaction solution was stirred at room temperature overnight. The reaction solution was concentrated under a reduced pressure to half of its original volume, and filtered. Then, the organic phase was concentrated under a reduced pressure. Residues were purified by preparative liquid chromatography to obtain C6-2 (85.0 mg, 17.7 %).

LC-MS: 339.1[M+H]⁺.

### Step 3: synthesis of N-[(9S)-9-benzyl-1-[(4S)-4-ethyl-8,9-difluoro-4-hydroxy-3,14-dioxo-1,3,4,12-tetrahydropyra no[3',4':6,7]indolizino[1,2-b]quinolin-11-yl]-5,8,11-trioxo-2-oxa-4,7,10-triazadodecan-12-yl]-2-{[(31S)-31-{[2-(methyl{3-[2-(methyldioxo-λ⁶-sulfanyl)benzo[d][1,3]thiazol-6-yl]prop-2-yny l}amino)acetyl]amino}-25,32-dioxo-26-aza-2,5,8,11,14,17,20,23-octaoxadotriacontan-32-yl]a mino}acetamide C6

C4-5 (19 mg, 0.015 mmol) and 2-(7-azabenzotriazol-1-yl)-N,N,N',N'-tetramethyluronium hexafluorophosphate (5.7 mg, 0.015 mmol) were added into a 50 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, C6-2 (5.1 mg, 0.015 mmol) was added into the mixed solution. The reaction solution was stirred at room temperature for 3 hours. The reaction solution was filtered and purified by preparative high performance liquid chromatography to obtain C6 (7.8 mg, 32.7 %).

LC-MS: 1590.6[M+H]⁺.

### Example C7: preparation of compound C7

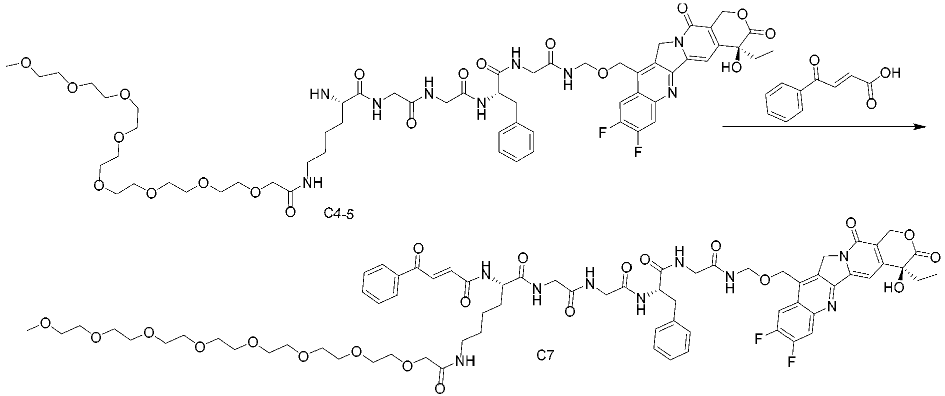

### Step 1: synthesis of (2S)-N-{(9S)-9-benzyl-1-[(4S)-4-ethyl-8,9-difluoro-4-hydroxy-3,14-dioxo-1,3,4,12-tetrahydro pyrano[3',4':6,7]indolizino[1,2-b]quinolin-11-yl]-5,8,11,14-tetraoxo-2-oxa-4,7,10,13-tetraaza pentadecan-15-yl]-2-{[(2E)-1,4-dioxo-4-phenylbut-2-enyl]amino}-6-{[(25-oxo-2,5,8,11,14,17, 20,23-octaoxapentacosan-25-yl)amino]hexanamide C7

C4-5 (19 mg, 0.015 mmol) and 2-(7-azabenzotriazol-1-yl)-N,N,N',N'-tetramethyluronium hexafluorophosphate (5.7 mg, 0.015 mmol) were added into a 50 mL single-neck flask, and dissolved with N,N-dimethylformamide (0.5 mL). The obtained solution was added with diisopropylethylamine (0.1 mL, 0.03 mmol). The mixed solution was stirred at room temperature for 15 minutes. Then, (2E)-4-oxo-4-phenylbut-2-enoic acid (2.7 mg, 0.015 mmol) was added into the mixed solution. The reaction solution was stirred at room temperature for 3 hours. The reaction solution was filtered and purified by preparative high performance liquid chromatography to obtain C7 (6.3 mg, 29.4 %).

LC-MS: 1428.6[M+H]⁺.

### Example D1: preparation of compound D1

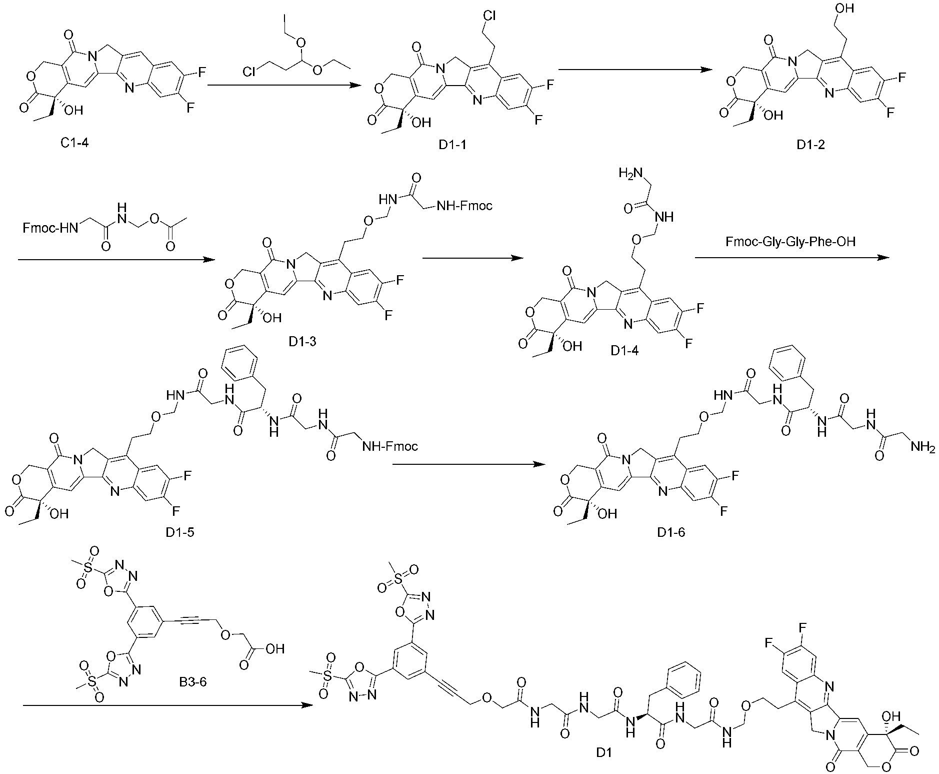

### Step 1: synthesis of (4S)-11-(2-chloroethyl)-4-ethyl-8,9-difluoro-4-hydroxy-3,4,12,14-tetrahydro-1H-pyrano[3',4' :6,7]indolizino[1,2-b]quinolin-3,14-dione (D1-1)

C1-4 (200 mg, 0.52 mmol) was added into a 100 mL single-neck flask, dissolved with 5 mL of water, dropwise added with 2.6 mL of concentrated sulfuric acid at 0°C, added with 3 mL of 3-chloro-1,1-diethoxypropane and 160 mg of ferrous sulfate heptahydrate, and finally dropwise added with 0.48 mL of 30% hydrogen peroxide. The reaction solution was stirred at room temperature overnight, and the reaction system was added with 20 mL of ice water for precipitation, and filtered to obtain D1-1 (100 mg, 43.0 %).

LC-MS: 447.1[M+H]⁺.

### Step 2: synthesis of (4S)-4-ethyl-8,9-difluoro-4-hydroxy-11-(2-hydroxyethyl)-3,4,12,14-tetrahydro-1H-pyrano[3', 4':6,7]indolizino[1,2-b]quinolin-3,14-dione (D1-2)

D1-1 (100 mg, 0.22 mmol) was added into a 50 mL single-neck flask, dissolved with 5 mL of 10% sulfuric acid, and reacted at 100°C for 48 hours. The reaction system was added with 10 mL of ice water for precipitation, and filtered to obtain D1-2 (40 mg, 42.5%).

LC-MS: 429.2[M+H]⁺.

### Steps 3 to 7: synthesis of compounds D1-3 to D1

The compounds were synthesized by reference to the synthetic methods for the compounds C1-5 to C1. Finally, 5.5 mg of the compound D1 was obtained by purification through preparative liquid chromatography.

LC-MS: 1240.3[M+H]⁺.

### Example D2: preparation of compound D2

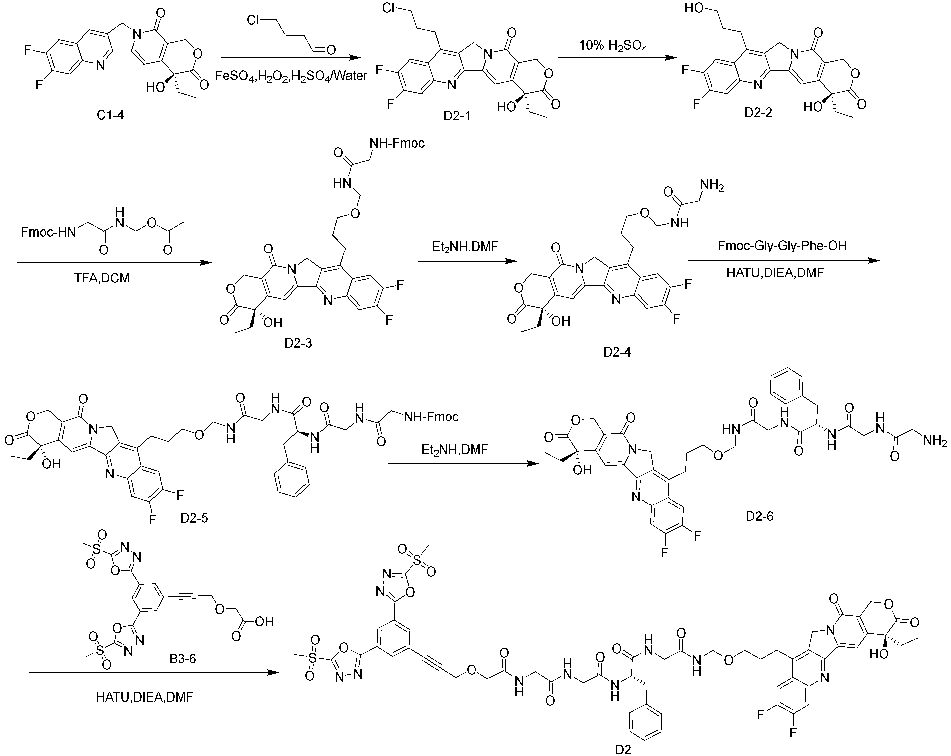

The compound was synthesized by reference to the synthetic method for the compound D1. Finally, 6.3 mg of the compound D2 was obtained by purification through preparative liquid chromatography.

LC-MS: 1254.3[M+H]⁺.

### Example D3: preparation of compound D3

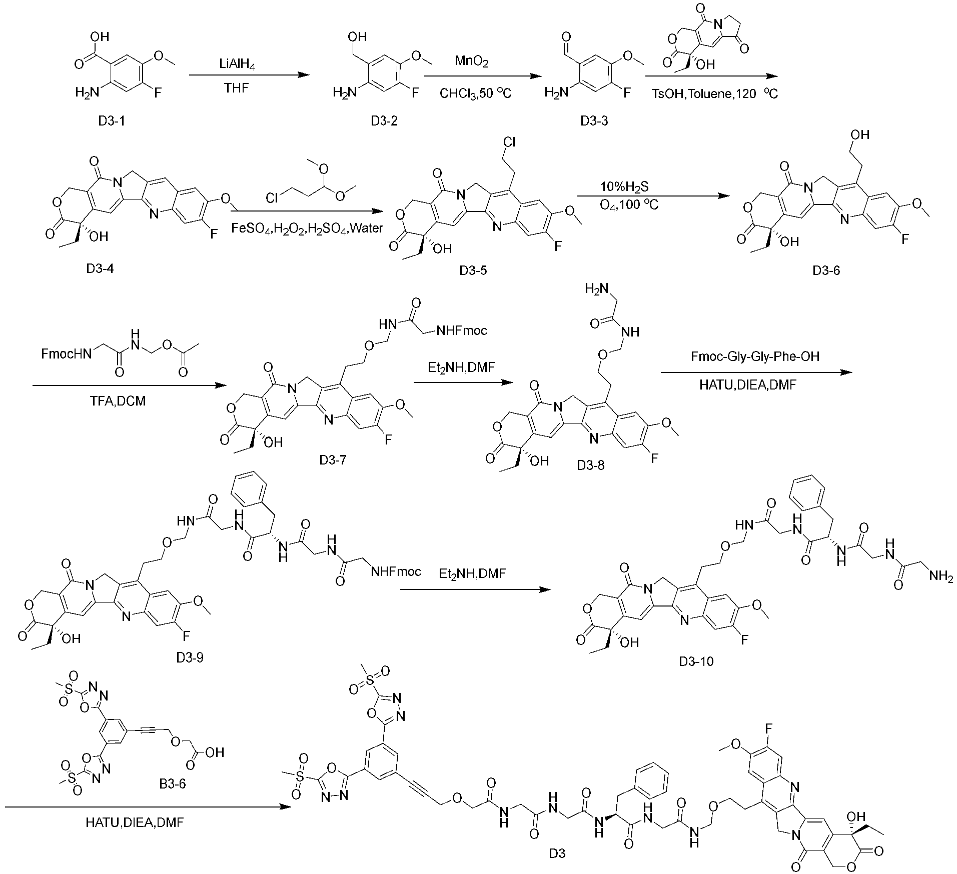

The compound was synthesized by reference to the synthetic method for the compound C1. Finally, 5.3 mg of the compound D3 was obtained by purification through preparative liquid chromatography.

LC-MS: 1252.3[M+H]⁺.

### Example D4: preparation of compound D4

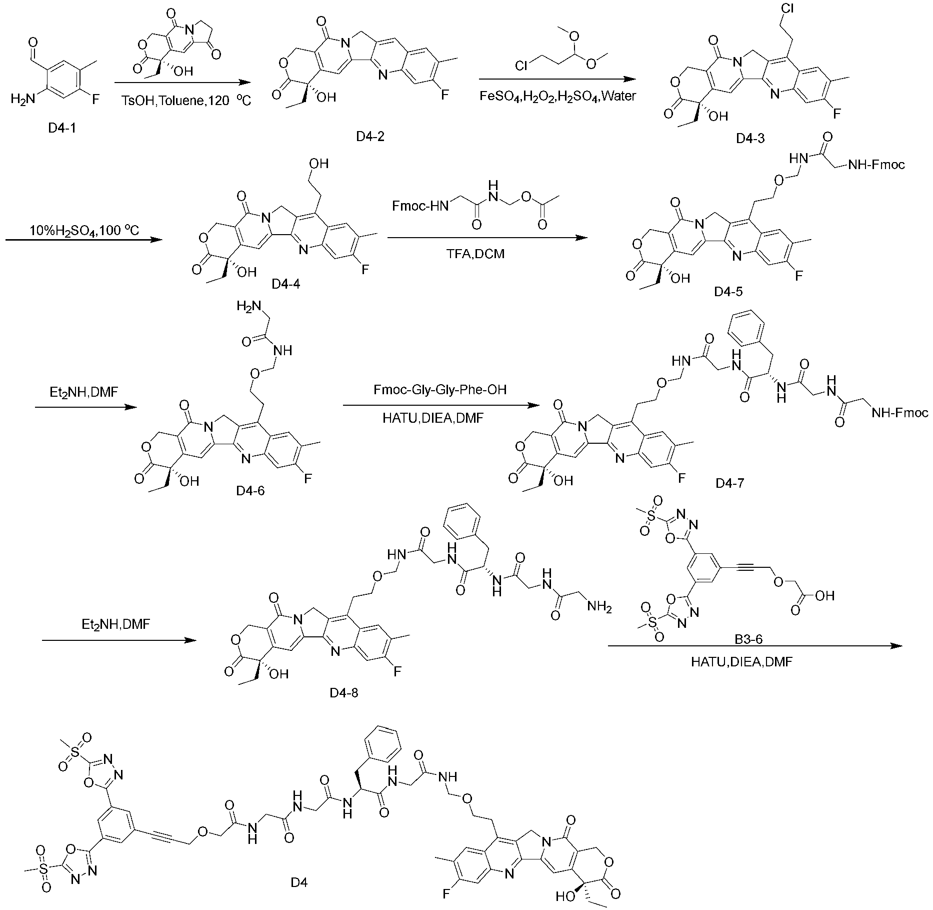

The compound was synthesized by reference to the synthetic method for the compound D1. Finally, 6.8 mg of the compound D4 was obtained by purification through preparative liquid chromatography.

LC-MS: 1236.3[M+H]⁺.

### Example D5: preparation of compound D5

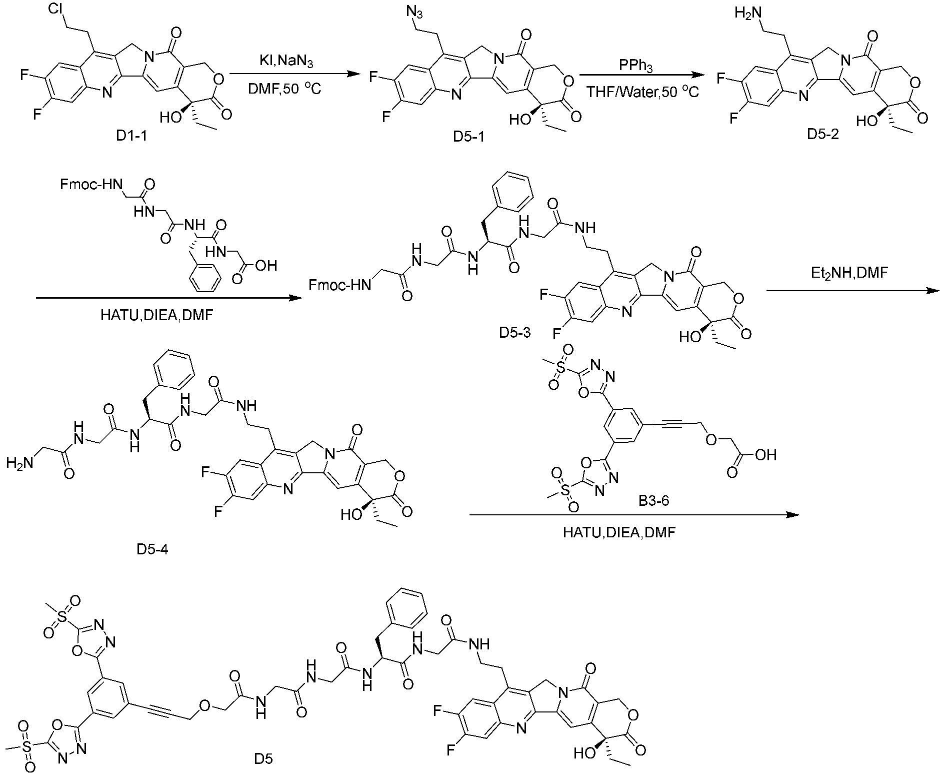

### Step 1: synthesis of (4S)-11-(2-azidoethyl)-4-ethyl-8,9-difluoro-4-hydroxy-3,4,12,14-tetrahydro-1H-pyrano[3',4': 6,7]indolizino[1,2-b]quinolin-3,14-dione (D5-1)

D1-1 (100 mg, 0.22 mmol) was added into a 50 mL single-neck flask, dissolved with 5 mL of N,N-dimethylformamide, and added with sodium azide (17 mg, 0.26 mmol) and potassium iodide (3.6 mg, 0.022 mmol). The reaction solution was stirred at 50°C overnight, and the reaction system was added with 20 mL of ice water for precipitation, and filtered to obtain D5-1 (80 mg, 80.3 %).

LC-MS: 454.1[M+H]⁺.

### Step 2: synthesis of (4S)-11-(2-aminoethyl)-4-ethyl-8,9-difluoro-4-hydroxy-3,4,12,14-tetrahydro-1H-pyrano[3',4' :6,7]indolizino[1,2-b]quinolin-3,14-dione (D5-2)

D5-1 (80 mg, 0.18 mmol) was added into a 50 mL single-neck flask, dissolved with 4 mL of tetrahydrofuran and 1 mL of water, and added with triphenylphosphine (57 mg, 0.22 mmol). The reaction solution was stirred at 50°C overnight, and dried by rotary evaporation to prepare D5-2 (60 mg, 77.9 %).

LC-MS: 428.1[M+H]⁺.

### Steps 3 to 5: synthesis of compounds D5-3 to D5

The compound was synthesized by reference to the synthetic method for the compound C1. Finally, 7.2 mg of the compound D5 was obtained by purification through preparative liquid chromatography.

LC-MS: 1210.3[M+H]⁺.

### Example D6: preparation of compound D6

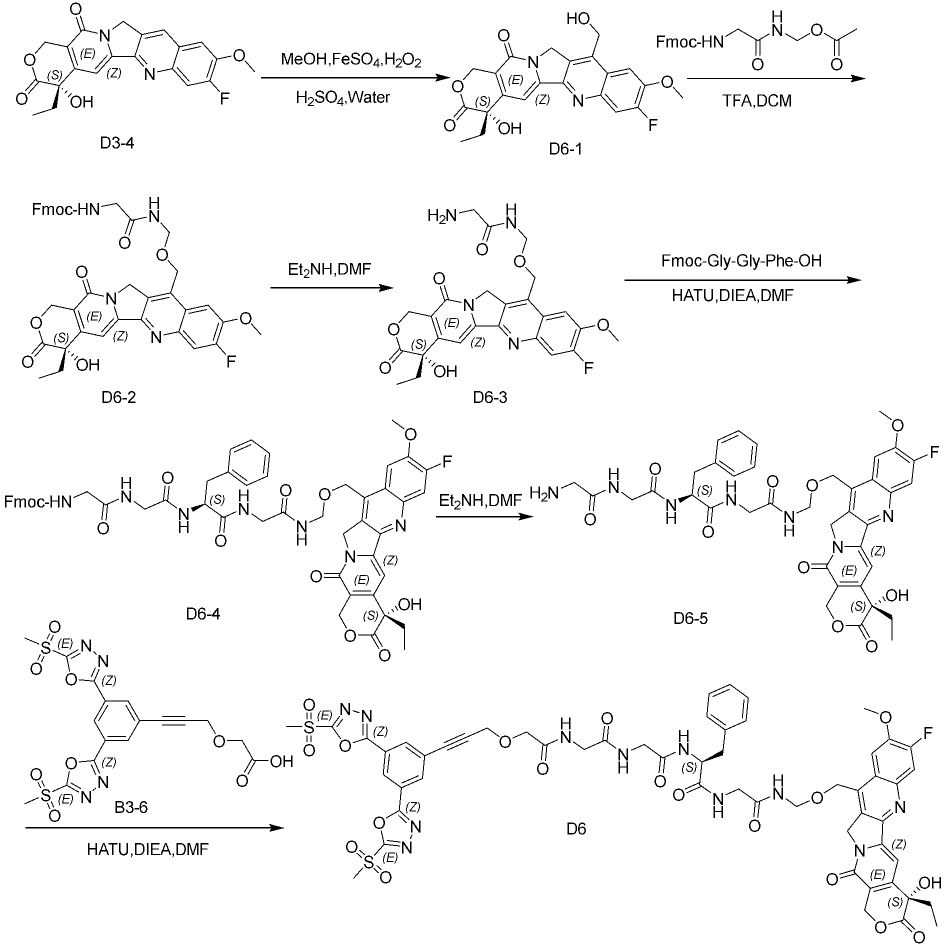

The compound was synthesized by reference to the synthetic method for the compound C1. Finally, 6.5 mg of the compound D6 was obtained by purification through preparative liquid chromatography.

LC-MS: 1238.3[M+H]⁺.

### Example D7: preparation of compound D7

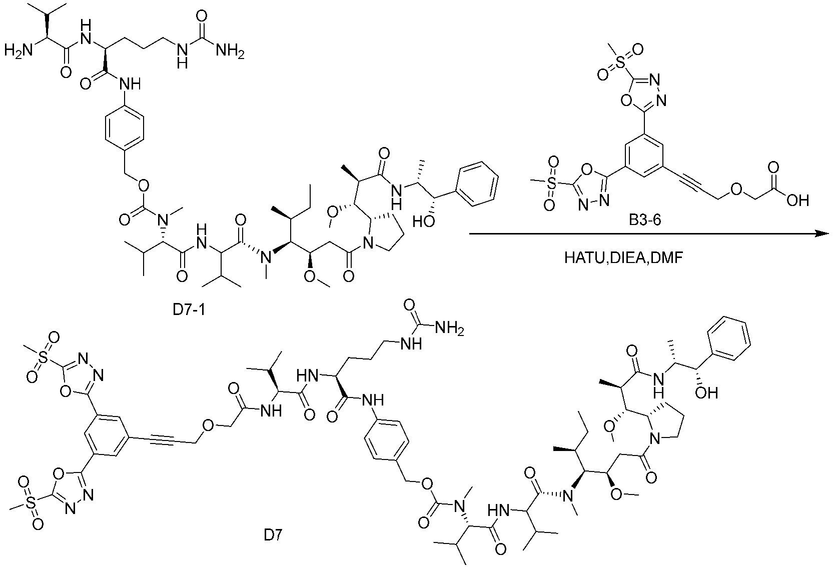

D7-1 (22.5 mg, 0.02 mmol) and 2-(7-azabenzotriazol-1-yl)-N,N,N',N'-tetramethyluronium hexafluorophosphate (7.6 mg, 0.02 mmol) were added into a 50 mL single-neck flask, and dissolved with N,N-dimethylformamide (0.5 mL). The obtained solution was added with diisopropylethylamine (5 mg, 0.04 mmol). The mixed solution was stirred at room temperature for 15 minutes. Then, B3-6 (10.0 mg, 0.02 mmol) was added into the mixed solution. The reaction solution was stirred at room temperature for 3 hours. The reaction solution was filtered and purified by preparative high performance liquid chromatography to obtain the compound D7 (8.5 mg, 26.8 %).

LC-MS: 1587.7[M+H]⁺.

### Example D8: preparation of compound D8

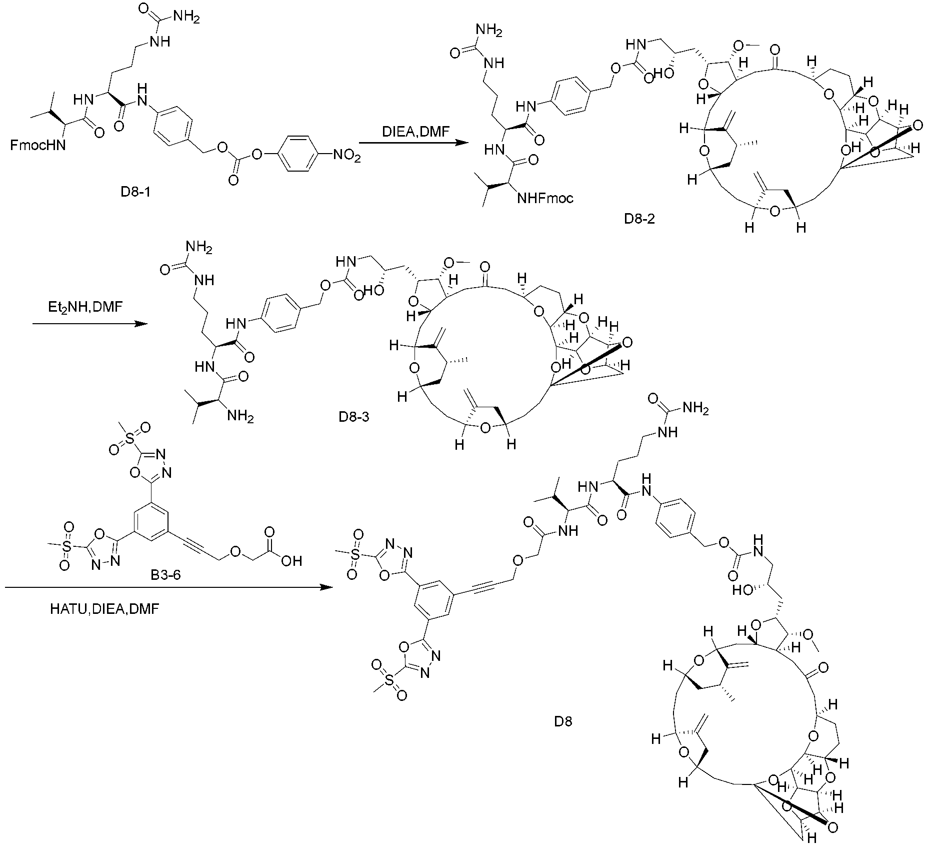

### Step 1: synthesis of (4-{[(5S,8S)-8-{3-[(aminocarbonyl)amino]propyl]}-1-(9H-fluoren-9-yl)-3,6,9-trioxo-5-(prop-2-yl)-4,7-diaza-2-oxanonan-9-yl]amino}phenyl)methyl {[(2S)-2-hydroxy-3-[(1S,4S,7S,10S,12R,14R,16S,18R,19R,20S,24R,27S,28S,29S,30R,31R,32S, 33R)-19-methoxy-12-methyl-6,13-dimethylidene-22-oxo-10,14-epoxy-4,7-epoxy-30,33-epoxy-24,28-epoxy-17,35,36,37-tetraoxapentacyclo[27.5.1.1^{1,32}.1^{27,31}.0^{16,20}]heptatriacontan-18-yl]pr opyl]amino}formate (D8-2)

D8-1 (100.0 mg, 0.13 mmol, prepared according to WO 2004010957) and eribulin (CAS No.: 253128-41-5, 95 mg, 0.13 mmol) were added into a 50 mL single-neck flask, and dissolved with N,N-dimethylformamide (2 mL). The obtained solution was stirred at room temperature for 30 minutes, and then added with diisopropylethylamine (0.06 mL, 0.39 mmol). The reaction solution was stirred at room temperature for 3 hours, and the turbid solution became clear. The reaction solution was concentrated under a reduced pressure. Residues were purified by silica gel column chromatography (dichloromethane: methanol = 94: 6) to obtain D8-2 (110.0 mg, 79.6 %).

LC-MS: 1357.7[M+H]⁺.

### Step 2: synthesis of (4-{[(2S)-5-[(aminocarbonyl)amino]-2-{[(2S)-2-amino-3-methyl-1-oxobutyl]amino}-1-oxope ntyl]amino}phenyl)methyl {[(2S)-2-hydroxy-3-[(1S,4S,7S,10S,12R,14R,16S,18R,19R,20S,24R,27S,28S,29S,30R,31R,32S, 33R)-19-methoxy-12-methyl-6,13-dimethylidene-22-oxo-10,14-epoxy-4,7-epoxy-30,33-epoxy-24,28-epoxy-17,35,36,37-tetraoxapentacyclo[27.5.1.1^{1,32}.1^{27,31}.0^{16,20}]heptatriacontan-18-yl]pr opyl]amino}formate (D8-3)

D8-2 (100.0 mg, 0.07 mmol) was added into a 50 mL single-neck flask and dissolved with N,N-dimethylformamide (2 mL). The obtained solution was added with diethylamine (51 mg, 0.7 mmol). The reaction solution was stirred at room temperature for 1 hour. The reaction solution was concentrated under a reduced pressure. Residues were slurried with petroleum ether to obtain D8-3 (70 mg, 88.6 %).

LC-MS: 1135.6[M+H]⁺.

### Step 3: synthesis of (4-{[(8S,11S)-11-{3-[(aminocarbonyl)amino]propyl]}-1-{3,5-bis[5-(methyldioxo-λ⁶-sulfanyl)-1,3,4-oxadiazol-2-yl]phenyl}-6,9,12-trioxo-8-(prop-2-yl)-7,10-diaza-4-oxadodec-1-yn-12-yl]a mino}phenyl)methyl {[(2S)-2-hydroxy-3-[(1S,4S,7S,10S,12R,14R,16S,18R,19R,20S,24R,27S,28S,29S,30R,31R,32S, 33R)-19-methoxy-12-methyl-6,13-dimethylidene-22-oxo-10,14-epoxy-4,7-epoxy-30,33-epoxy-24,28-epoxy-17,35,36,37-tetraoxapentacyclo[27.5.1.1^{1,32}.1^{27,31}.0^{16,20}]heptatriacontan-18-yl]pr opyl]amino}formate D8

D8-2 (22.7 mg, 0.02 mmol) and 2-(7-azabenzotriazol-1-yl)-N,N,N',N'-tetramethyluronium hexafluorophosphate (7.6 mg, 0.02 mmol) were added into a 50 mL single-neck flask, and dissolved with N,N-dimethylformamide (0.5 mL). The obtained solution was added with diisopropylethylamine (5 mg, 0.04 mmol). The mixed solution was stirred at room temperature for 15 minutes. Then, B3-6 (10.0 mg, 0.02 mmol) was added into the mixed solution. The reaction solution was stirred at room temperature for 3 hours. The reaction solution was filtered and purified by preparative high performance liquid chromatography to obtain D8 (7.5 mg, 23.4 %).

LC-MS: 1599.6[M+H]⁺.

### Example E Sequence expression and purification of antibody hLIV22

The Antibody hLIV22 could be prepared according to WO2012078668, or prepared by reference to the following method: cells were diluted to a density of 6×10⁶ viable cells/mL with fresh ExpiCHO expression medium (purchased from Thermo Fisher, Cat. No.: A29133), and a viability rate of the cells was greater than 95%. An ExpiFectamine CHO reagent: DNA complex was prepared according to a ratio of 4:1 and incubated at room temperature for 3 minutes, and then the complex was added into the cells to be transfected and mixed evenly. The cells were incubated in a 37°C and 80% humidity environment containing 8% CO₂. The cells were transfected for 18-22 hours, and then added with an ExpiFectamine CHO enhancer and an ExpiCHO feed. The culture flask was transferred to a 32°C and 80% humidity environment containing 5% CO₂ for further cultivation. The supernatant was collected on the 10^{th} day, incubated with Protein A MagBEADS (Genscript) at 4°C overnight, washed with PBS for 4 times, and then eluted with a glycine solution at pH 3.2 to obtain the hLIV22 antibody, and finally, the yield was calculated.

### Example F Preparation of ADC conjugation

### 1. Conditions of ADC conjugation

DAR8 ADC: in the present invention, a LIV1-targeting antibody hLIV22 (comprising a heavy chain as shown in SEQ ID NO: 5 and a light chain as shown in SEQ ID NO: 10) or a Her2-targeting antibody Trastuzumab (purchased from Sanyou Biopharmaceuticals (Shanghai) Co., Ltd., comprising a heavy chain as shown in SEQ ID NO: 15 and a light chain as shown in SEQ ID NO: 20) was reduced with 6-20 equivalents of tris(2-carboxyethyl)phosphine in 25 mM histidine buffer (pH 6.5) at a temperature of 4-40°C for 0.5-6 hours. After reduction, the reaction solution was added with 5-30% DMSO (DMF or DMAC could also be used as the organic solvent), and reacted with 6-30 equivalents of linker-payload compound at 4-40 °C for 0.5-24 hours, and the product was quenched with excess NAC. The product was purified and then subjected to buffer exchange into 25 mM histidine buffer (pH 5.5) for storage.

DAR4 ADC: in the present invention, the LIV1-targeting antibody hLIV22 was reduced with 2-6 equivalents of tris(2-carboxyethyl)phosphine in 25 mM histidine buffer (pH 6.5) at a temperature of 4-40 °C for 0.5-2 hours. After reduction, the reaction solution was added with 5-30% DMSO (DMF or DMAC could also be used as the organic solvent), and reacted with 4-12 equivalents of linker-payload compound at 4-40 °C for 0.5-12 hours, and the product was quenched with excess N-acetylcysteine. The product was purified and then subjected to buffer exchange into 25 mM histidine buffer (pH 5.5) for storage.

The ADC products conjugated by this method were analyzed by RP-UPLC and SEC-HPLC respectively to determine RP DAR values and SEC purities of the products, wherein the DAR values were shown in Table 1; and the SEC purities of the ADC compounds obtained in the examples of the present invention were all ≥ 99.5%.

### 2. ADC analysis method:

PB used in the present invention referred to a sodium phosphate buffer comprising a main ingredient of disodium hydrogen phosphate - sodium dihydrogen phosphate. Disodium hydrogen phosphate - sodium dihydrogen phosphate buffers with different pH values were generally prepared by mixing a sodium dihydrogen phosphate solution and a disodium hydrogen phosphate solution, both of which were at the same concentration.
DAR value of the conjugated drug was determined by reversed-phase ultra-performance liquid chromatography (RP-UPLC) using hydrophobic interaction chromatography;
sample processing: sample concentration 1.0-5 mg/mL, and filtration with 0.22 µm filter membrane;
general detection methods as follows.
   (1) Size-exclusion chromatography (SEC-HPLC)
      sample processing: sample concentration 1.0-5 mg/mL, and filtration with 0.22 µm filter membrane;
      chromatographic column: TOSOH, TSKgel G3000SWxL, 5 µm, 7.8 mm × 300 mm;
      mobile phase: 0.2 M PB containing 5-15% isopropanol at pH 7.0;
      flow rate: 0.5-1 mL/min;
      detection wavelength: 280 nm & 248 nm (or 360 nm);
      column temperature: RT;
      injection volume: 30 µg;
      SEC elution method: isocratic elution.
   (2) Reversed-phase chromatography (RP-UPLC)
      sample processing: sample concentration 0.1- 0.5 mg/mL, and full reduction with excess DTT;
      chromatographic column: Waters BioResolve^{™} RP mAb polyphenyl, 2.7 µm, 4.6 × 100 mm;
      mobile phase A: 0.1 TFA % water;
      mobile phase B: 0.1 TFA % acetonitrile;
      flow rate: 0.3 mL/min;
      column temperature: 60-90°C;
      injection volume: 5 µL;
      RP chromatography elution method: increase of mobile phase B from ~30% to ~50% within 30 minutes under equilibrium condition.

**Table 1 ADC compounds in the present invention and properties of these compounds**

| **ADC** | **Antibody** | **Linker-payload** | **DAR** |
|---|---|---|---|
| ADC1 | hLIV22 | Deruxtecan | 7.59 |
| ADC2 | hLIV22 | Compound A | 7.61 |
| ADC3 | hLIV22 | Compound B6 | 7.86 |
| ADC4 | hLIV22 | Compound B21 | 7.62 |
| ADC5 | hLIV22 | Compound B8 | 7.77 |
| ADC5.1 | hLIV22 | Compound B8 | 4.42 |
| ADC6 | hLIV22 | Compound B22 | 7.86 |
| ADC7 | hLIV22 | Compound B28 | 7.70 |
| ADC8 | hLIV22 | Compound B29 | 7.64 |
| ADC9 | hLIV22 | Compound B35 | 7.67 |
| ADC10 | Trastuzumab | Compound B29 | 7.81 |
| ADC11 | Trastuzumab | Deruxtecan | 7.77 |

### Biological activity experiment

### 1. Efficacy evaluation on compounds in HCC1806 tumor-bearing mice

BALB/c Nude mice (purchased from GemPharmatech Co., Ltd., and grouped according to 6 mice per group) were used as test animals, and a therapeutic efficacy of hLIV1-ADC administered via tail vein injection in the nude mice bearing xenografts of human breast cancer cells HCC1806 was evaluated.

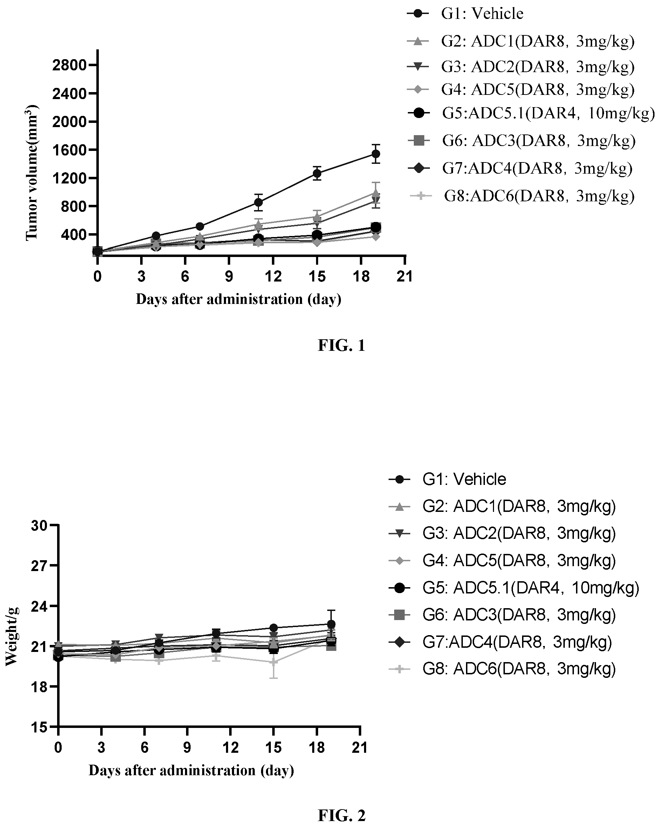

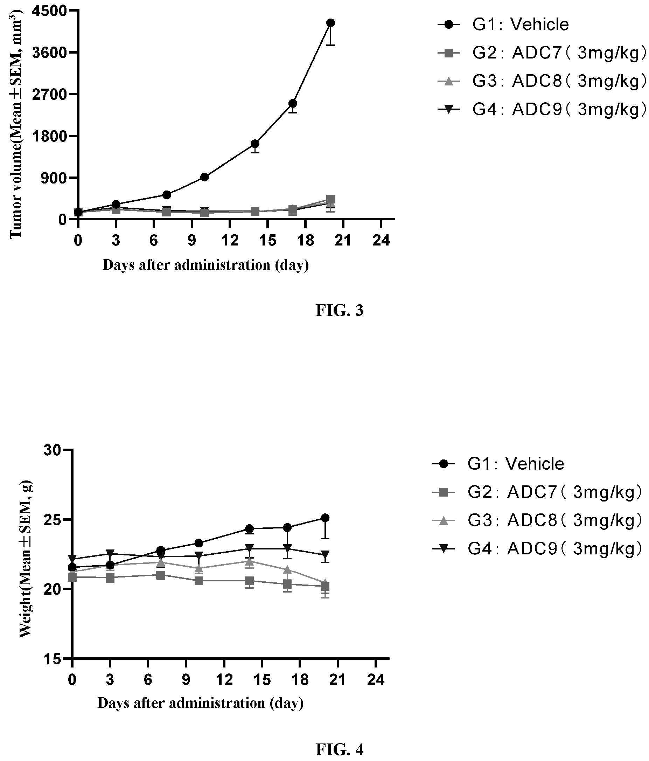

Under sterile conditions, in vitro-cultured HCC1806 cell (purchased from ATCC, passaged and preserved by the Non-clinical R&D Department of the Pharmaceutical R&D Center of Qilu Pharmaceutical Co., Ltd., the tumor cells expanded through in-vitro culture were prepared into the cell suspension for inoculation.) suspension was collected. The cell suspension was centrifuged, then added with 0.9% NaCl injection to adjust the cell concentration to 4×10⁷ cells/mL, and added with matrigel according to a ratio of 1: 1 to achieve a final concentration of 2×10⁷ cells/mL. The cells were subcutaneously inoculated into the right axilla of mice (0.1 mL/mouse). The mice were administered with a single dose of drug via tail vein injection, and the day of first administration was designated as Day 0 (D0). Tumor volume and body weight were measured twice a week, and data recording continued until Day 19 after administration. Tumor inhibition rate (%)=(Crtv-Trtv)/ Crtv(%), wherein Crtv and Trtv respectively represented relative tumor volumes of the blank control group (Vehicle, PBS) and the experimental group at the end of the experiment. Results of tumor inhibition rate determination were shown in Table 2, FIG. 1 and FIG. 3, and results of body weight determination were shown in FIG. 2 and FIG. 4.

It could be seen from the above data that the ADC conjugates of the present invention had better tumor inhibition rates, and there were small changes in body weights of the mice in the experiments of the ADC conjugates of the present invention, which indicated that the ADC conjugates of the present invention had excellent safety.

**Table 2 Conjugates obtained by the present invention and properties of these conjugates**

| ADC | DAR value | Dose (mg/kg) | Tumor inhibition rate |
|---|---|---|---|
| ADC1 | 8 | 3 | 29.9% |
| ADC2 | 8 | 3 | 48.8% |
| ADC3 | 8 | 3 | 75.8% |
| ADC4 | 8 | 3 | 81.1% |
| ADC5 | 8 | 3 | 85.5% |
| ADC5.1 | 4 | 10 | 75.3% |
| ADC6 | 8 | 3 | 79.3% |
| ADC7* | 8 | 3 | 93.1% |
| ADC8* | 8 | 3 | 94.7% |
| ADC9* | 8 | 3 | 95.2% |

| | | | |
|---|---|---|---|
| Note: * indicates that this data is calculated based on tumor volumes recorded up to Day 20 after administration. | | | |

### 2. In-vivo PK study of ADC compounds in mice

**PK protocol:** 9 B-hFcRn mice (Biocytogen, product No.: 110001), with 3 mice per cage, were respectively administered with Trastuzumab, ADC10 and ADC11 via tail vein injection (IV, n=3, free access to food and water), in which vehicles were all normal saline, doses were 10 mg/kg and dosing volumes were 10 mL/kg. Blood samples were collected from retro-orbital veins of the mice at 5 minutes, 8 hours, 1 day, 2 days, 4 days, 7 days, 10 days, 14 days and 21 days after administration. The collected blood samples were allowed to stand at room temperature for approximately half an hour until blood agglutination, then 1000 g of the blood agglutinate was centrifuged at 4°C for 15 minutes to obtain approximately 25 µL of serum samples. The serum samples were immediately stored at -80°C for subsequent analysis and detection of serum antibody concentrations by a LBA method (HTFR).

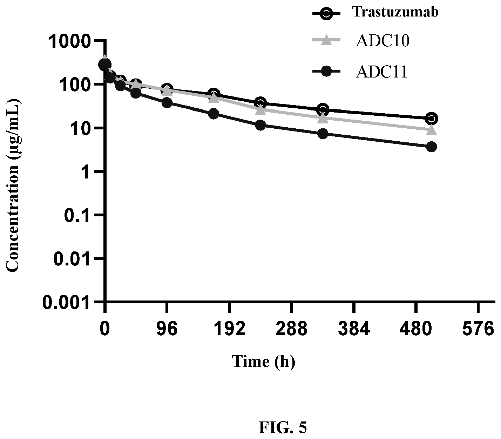

**Determination method for ADC concentration:** a reagent mixture PAb Anti Human IgG-Tb cryptate (1: 200, Cisbio-61HFCTAB) & Streptavidin-XL665 (1: 200, cisbio - 610SAXLB) & Goat Anti-Human IgG (Fab')2 (Biotin) (2 µg/mL, Abcam - ab64666) was added into a HTRF 96-well low-volume plate (Cisbio-66PL96100) according to 10 µL per well and mixed at 1000 rpm for 1 minute, and then added with 10 µL of diluted standard-curve and test serum samples (MRD = 50) and mixed at 1000 rpm for 1 minute. The plate was incubated at room temperature for 1-2 hours, and read by a HTRF module of an EnVision microplate reader (laser excitation), and fluorescence signals at 620 nm and 665 nm were read to calculate a signal ratio of 665 nm to 620 nm. Pharmacokinetic parameters were calculated through a non-compartmental model method by using WinNonlin statistical software. Results were shown in Table 3 and FIG. 5.

**Table 3 Pharmacokinetic parameters of partial ADC compounds determined in the present invention**

| | 10mpk IV | ADC10 | ADC11 |
|---|---|---|---|
| Parameter | AUC 0-t (µg/mL*h) | 22814.0 | 13062.8 |
| | CL (mL/day/kg) | 9.7 | 17.3 |

The experimental data in Table 3 indicated that the ADC10 using B29 showed a higher in-vivo exposure and a lower metabolic clearance rate.

### 3. Cell inhibitory activity experiment of payload compound C1-5

| **Serial number of cell** | **Name of cell** | **Source of cell** | **Complete medium** |
|---|---|---|---|
| KC-0300 | COLO205 | Cell Bank of the Institute of Basic Medical Sciences, CAMS & PUMC | RPMI1640+10%FBS |
| KC-0911 | SK-OV-3 | Cell Bank of the Institute of Basic Medical Sciences, CAMS & PUMC | McCoy's 5a+10%FBS |
| KC-0328 | HCT15 | Cell Bank of Chinese Academy of Sciences | RPMI1640+10%FBS |
| KC-0671 | COLO320DM | Cell Bank of the Institute of Basic Medical Sciences, CAMS & PUMC | RPMI1640+10%FBS |
| KC-0166 | AsPC-1 | Cell Bank of Chinese Academy of Sciences | RPMI1640+10%FBS |
| KC-0351 | HT29 | Cell Bank of Chinese Academy of Sciences | DMEM+10%FBS |
| KC-2471 | A549 | Cell Bank of Chinese Academy of Sciences | F12K+10%FBS |

a) All cell lines were cultured in complete medium at 37 °C under 5% CO₂.
b) Cells in logarithmic growth phase were collected and counted by a platelet counter. The cell viability was detected by trypan blue exclusion method, and guaranteed to be over 90%.
c) The cell density was adjusted with a complete medium. Subsequently, 80 µL of cell suspension was seeded into each well of a 96-well cell culture plate according to 3000 cells per well.
d) The cells in the 96-well plate were cultured at 37°C under 5% CO₂.
e) 10× drug solution was prepared. The test compound had a maximum working concentration of 10 µM and a total of 8 concentrations, and was diluted 4-fold. The working concentration of the test compound was 5 µM. For the test group, 10 µL of serially diluted test compound was transferred into the corresponding experimental well of 96-well cell culture plate, and added with 10 µL of diluent containing an equal amount of DMSO (1%), and three replicate wells were set up for each drug concentration. Control wells were added with corresponding volumes of diluent.
f) The cells in the 96-well plate added with the drug were continuously cultured at 37°C under 5% CO₂ for 72 hours.
g) CTG reagent (CellTiter-Glo^{®} Luminescent Cell Viability Assay, purchased from Promega, Cat. No. G7571) was thawed, and the cell culture plate was equilibrated to room temperature for 30 minutes.
h) 100 µL of CTG solution was added into each well.
i) The plate was shaken on an orbital shaker for 5 minutes to induce cell lysis.
j) The cell culture plate was placed at room temperature for 20 minutes to stabilize luminescent signals.
k) Luminescence intensities were read, and data were collected.
l) The data were analyzed by using GraphPad Prism 7.0 software, non-linear S-curve regression was applied to fit the data for generating dose-response curves, and IC₅₀ values were calculated accordingly. Specific experimental results were shown in Table 4. 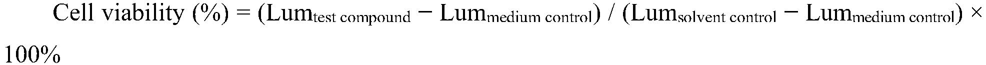
Cell viability (%) = (Lumtest compound - Lummedium control) / (Lumsolvent control - Lummedium control) × 100%

**Table 4 Cell inhibitory activities of payload compounds in the present invention**

| Compound | **IC₅₀ (nM)** | | | | | | |
|---|---|---|---|---|---|---|---|
| | **AsPC-1** | **COLO205** | **COLO320DM** | **HCT15** | **HT29** | **A549** | **SK-OV-3** |
| C1-5 | 1116.82 | 24.26 | 195.71 | 141.50 | 143.52 | 21.07 | 79.49 |
| DXd | 3098.41 | 95.54 | 574.93 | 889.02 | 343.93 | 104.21 | 287.61 |
| SN-38 | >10000 | 89.64 | 1421.84 | 312.79 | 286.88 | 66.55 | 167.89 |

## Claims

1. An antibody drug conjugate as shown in formula I or a pharmaceutically acceptable salt thereof:
**Ab-[-L₁-L₂-L₃-L₄-D]_{q}** **formula** I
wherein, Ab is an antibody part or an antigen-binding fragment; and D is a drug molecule, -L₁-L₂-L₃-L₄- is a fragment linking the antibody Ab to the drug molecule D, and q is selected from 2-9;
L₁ is a linker linked to the antibody, which has a structure of -La-Lb-; wherein La is selected from the following structures: 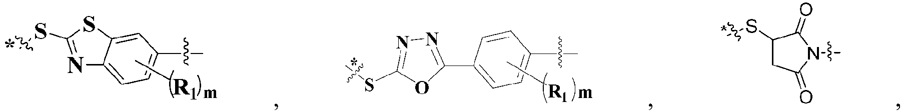 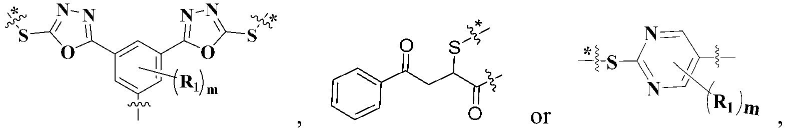 wherein R₁ is respectively independently selected from H, halogen atom, -CN, -OH, -NH₂, C₁₋₄ alkyl, C₁₋₄ haloalkyl, C₁₋₄ alkoxy, C₁₋₄ haloalkoxy or hydroxy-C₁₋₄ alkyl, and m is selected from 0, 1, 2 or 3; and the terminus of La marked with an asterisk * represents that the position is linked to the antibody Ab; and Lb is selected from 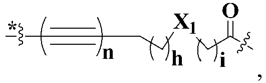 or Lb is a chemical bond, wherein n is selected from 0 or 1; h and i are respectively independently selected from 0, 1, 2, 3, 4 or 5, X₁ is selected from CH₂, N-Ra or O, and Ra is selected from H, C₁₋₄ alkyl, C₁₋₄ haloalkyl or C₁₋₄ alkyl-carbonyl; and the terminus of Lb marked with an asterisk * represents that the position is linked to La;
L₂ is a chemical bond, or L₂ is selected from 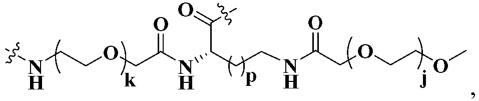 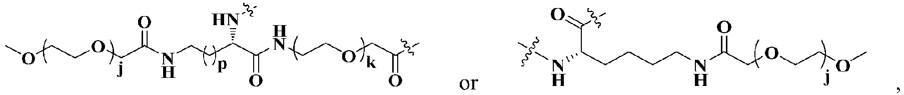 wherein k is selected from 0-8, j is selected from 0-20, and p is selected from 0, 1, 2, 3 or 4; and the -NH- terminus of L₂ is linked to L₁;
L₃ is selected from a peptide group consisting of 2-10 amino acid residues; and the amino acids are selected from natural amino acids or unnatural amino acids; and
L₄ is a chemical bond, or L₄ is selected from the following groups: -NH-CH₂- or 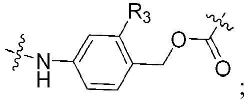
wherein, R₃ is selected from 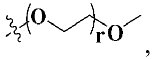
or R₃ is H, and r is selected from 0-10; and the -NH- terminus of L₄ is linked to L₃.

2. The antibody drug conjugate or the pharmaceutically acceptable salt thereof according to claim 1, wherein q is selected from 2-6, preferably 3-5, and more preferably 3.5-4.5.

3. The antibody drug conjugate or the pharmaceutically acceptable salt thereof according to claim 1, wherein q is selected from 7-9, preferably 7.5-8.5, and more preferably 7.5-8.0.

4. The antibody drug conjugate or the pharmaceutically acceptable salt thereof according to claim 1, wherein R₁ is selected from H.

5. The antibody drug conjugate or the pharmaceutically acceptable salt thereof according to claim 1, wherein n is 1; h is 0, and i is 1; k is selected from 2-6, preferably 4; and j is selected from 4-18, or j is selected from 6-16, or j is selected from 7-15, or j is selected from 11 or 15, or j is selected from7 or 8.

6. The antibody drug conjugate or the pharmaceutically acceptable salt thereof according to claim 1, wherein L₂ is a chemical bond, or L₂ is selected from 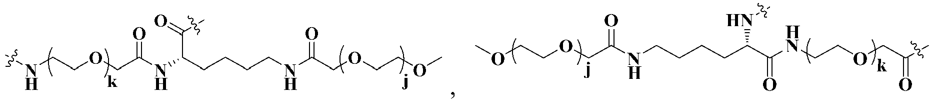 or 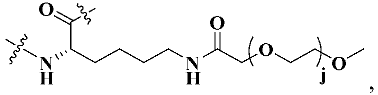 and k and j are as defined in claim 1 or 5.

7. The antibody drug conjugate or the pharmaceutically acceptable salt thereof according to claim 1 or 6, wherein L₂ is selected from 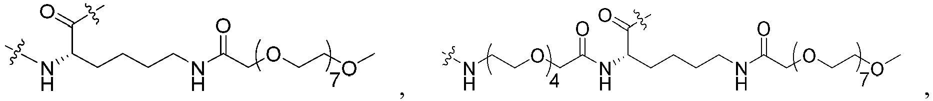 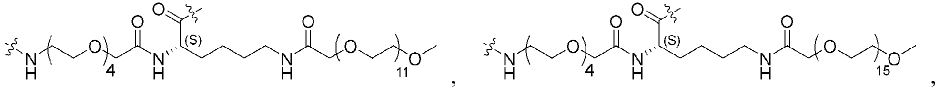 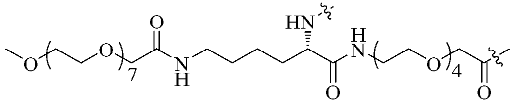 or a chemical bond.

8. The antibody drug conjugate or the pharmaceutically acceptable salt thereof according to claim 1, wherein L₃ is selected from the following peptide fragments: -Gly-Gly-Phe-Gly-, -Val-Ala-, -Ala-Ala-, -Ala-Ala-Asn- or -Val-Cit-, preferably -Val-Ala-, -Val-Cit- or -Gly-Gly-Phe-Gly-.

9. The antibody drug conjugate or the pharmaceutically acceptable salt thereof according to claim 1, wherein r is selected from 6-10, or r is selected from 9.

10. The antibody drug conjugate or the pharmaceutically acceptable salt thereof according to claim 1, wherein L₄ is a chemical bond, or L₄ is selected from 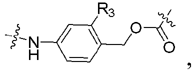 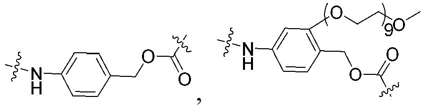 or -NH-CH₂-.

11. The antibody drug conjugate or the pharmaceutically acceptable salt thereof according to claim 1, wherein La is selected from the following structures;

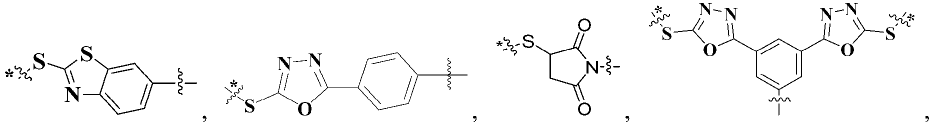

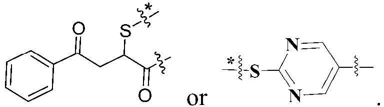

12. The antibody drug conjugate or the pharmaceutically acceptable salt thereof according to claim 1, wherein Lb is selected from the following fragment:

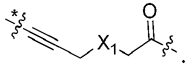

13. The antibody drug conjugate or the pharmaceutically acceptable salt thereof according to claim 1, wherein Lb is selected from the following fragments:

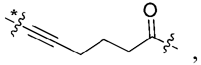

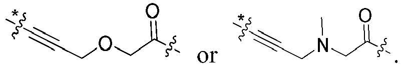

14. The antibody drug conjugate or the pharmaceutically acceptable salt thereof according to claim 1, wherein L₁ is selected from the following fragments:

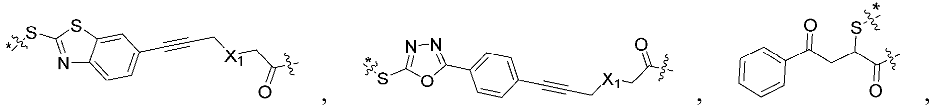

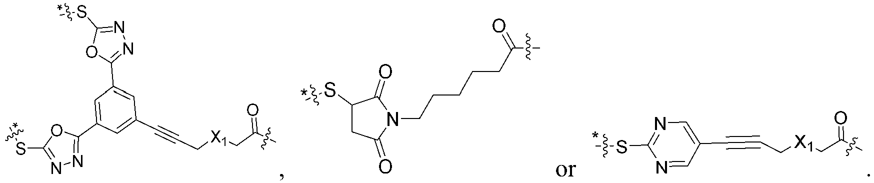

15. The antibody drug conjugate or the pharmaceutically acceptable salt thereof according to claim 1, wherein L₁ is selected from the following fragments:

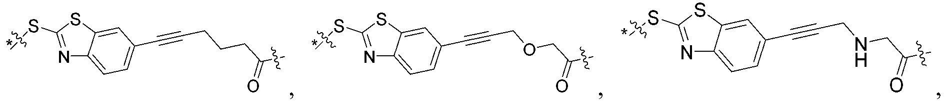

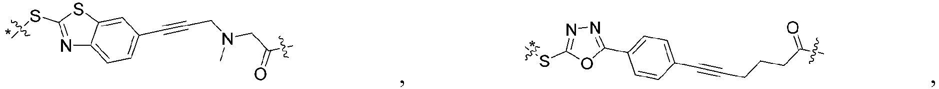

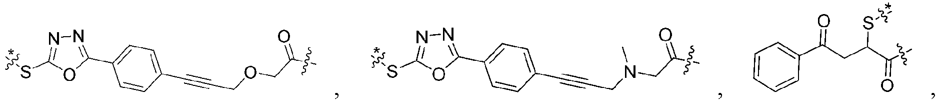

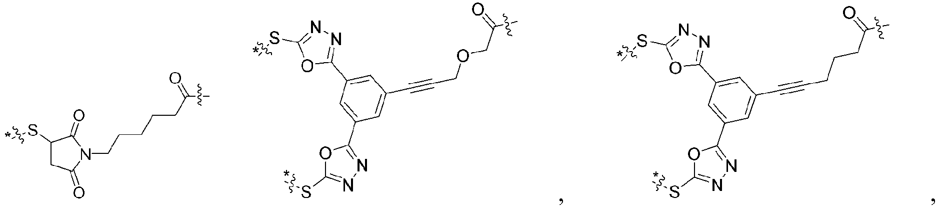

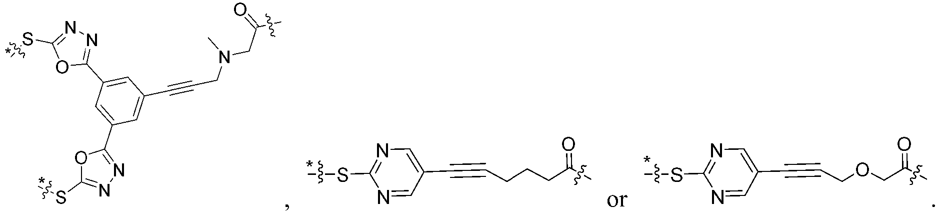

16. The antibody drug conjugate or the pharmaceutically acceptable salt thereof according to claim 1, wherein -L₁-L₂-L₃-L₄- is selected from the following fragments:

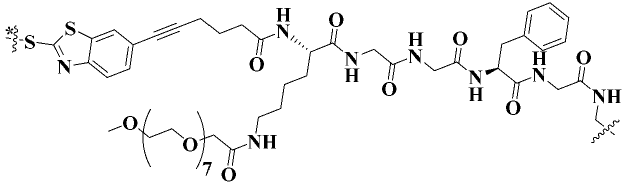

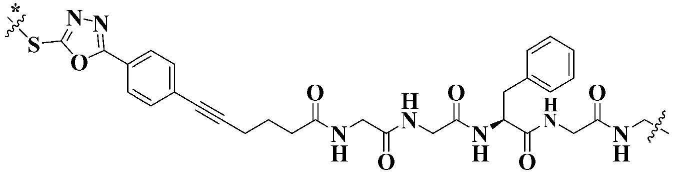

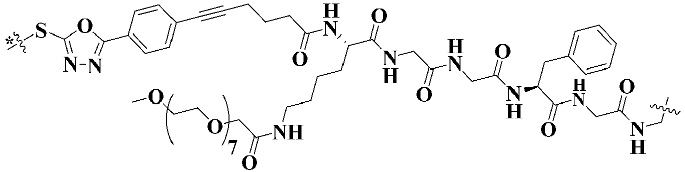

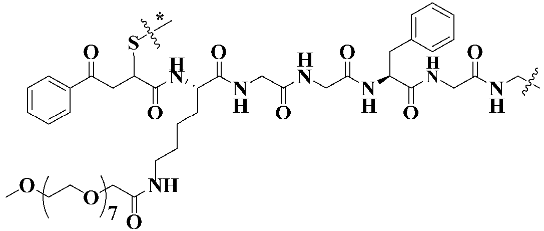

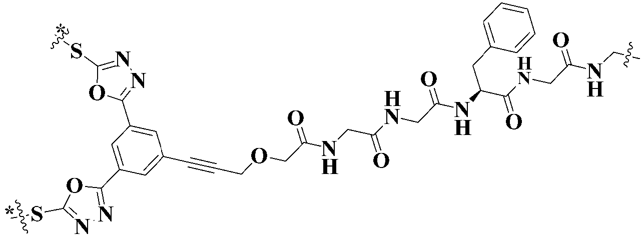

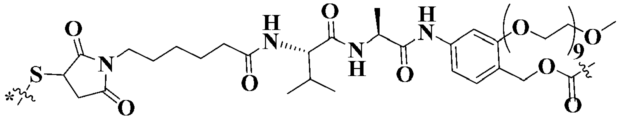

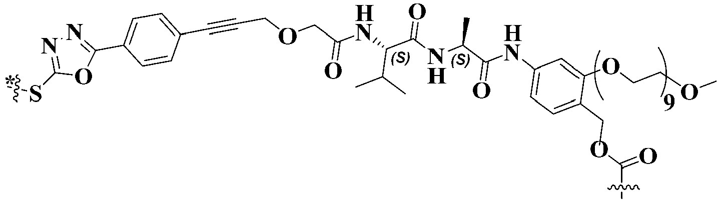

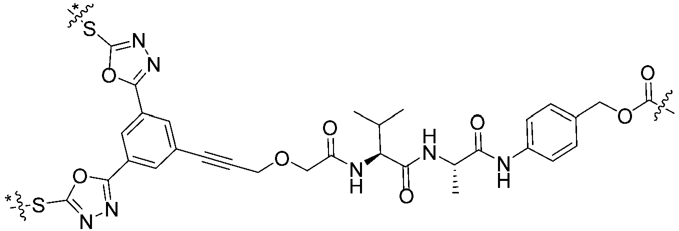

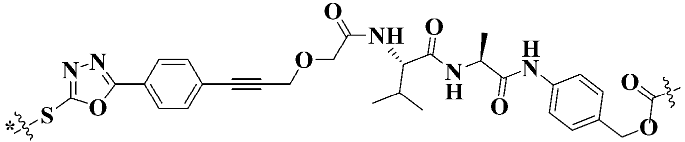

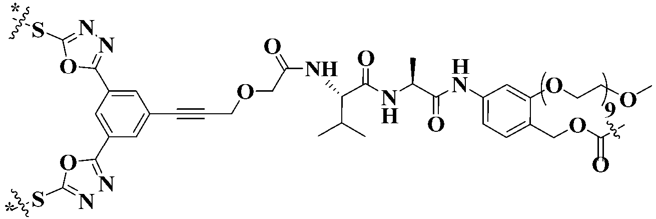

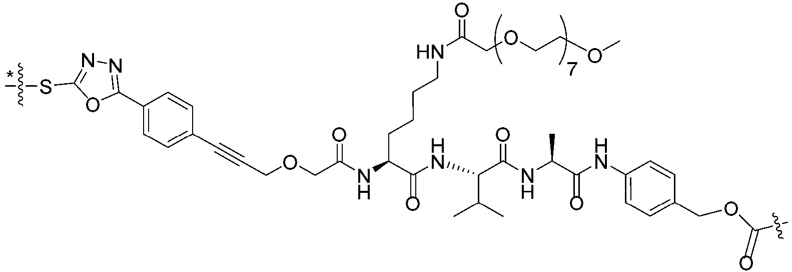

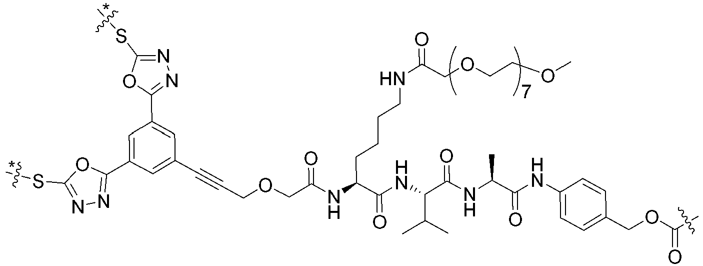

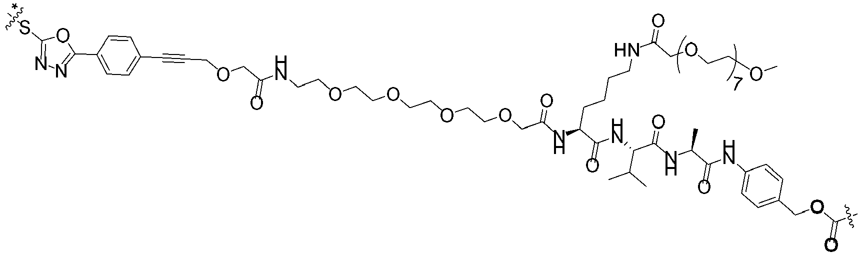

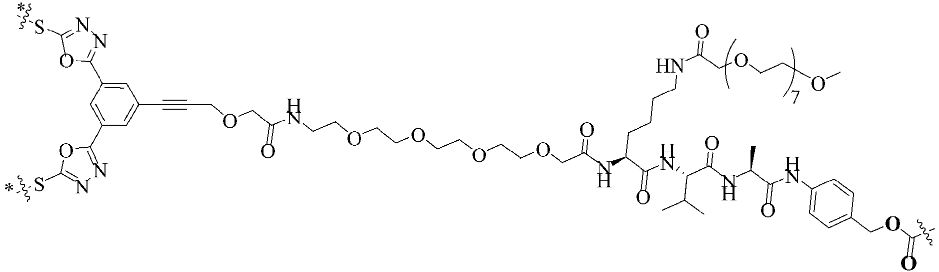

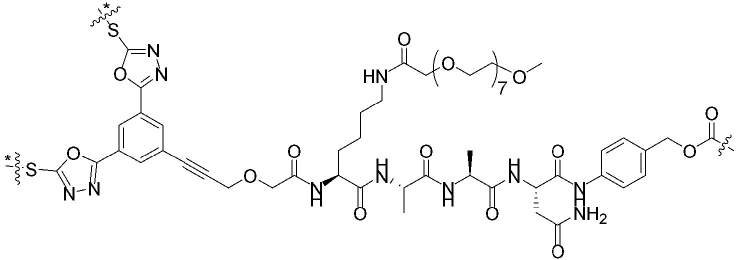

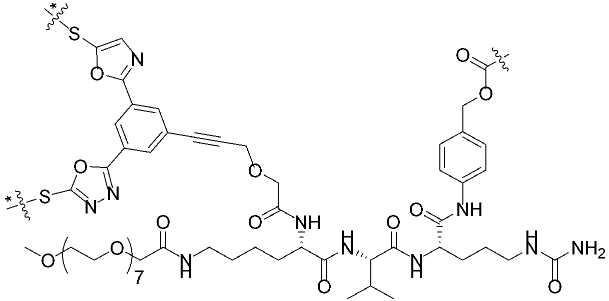

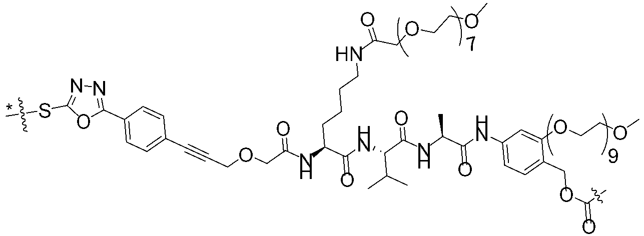

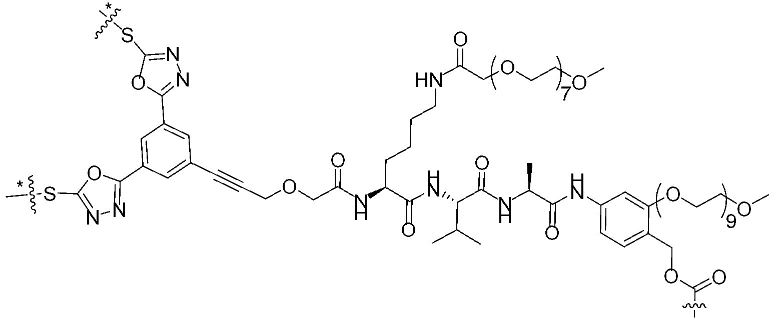

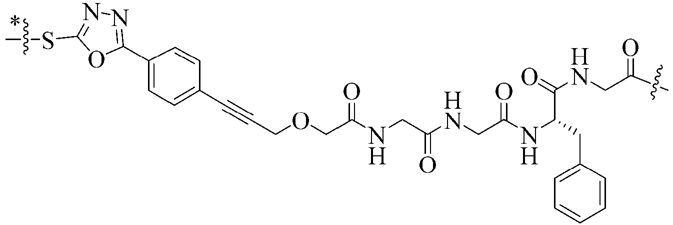

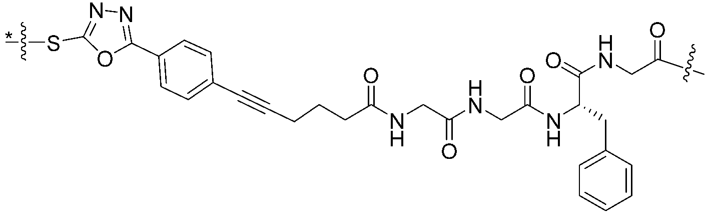

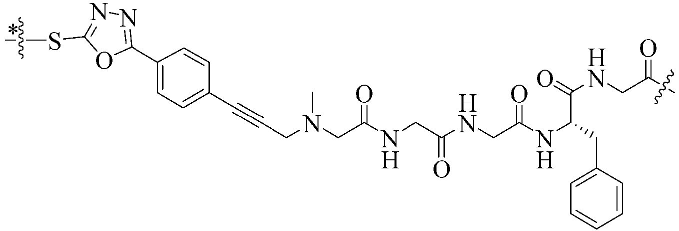

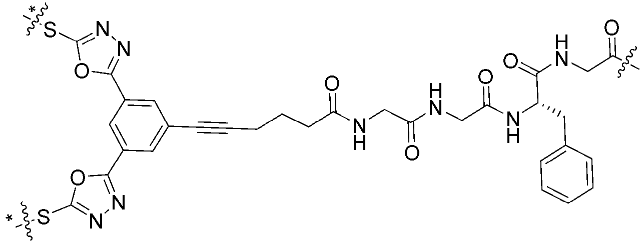

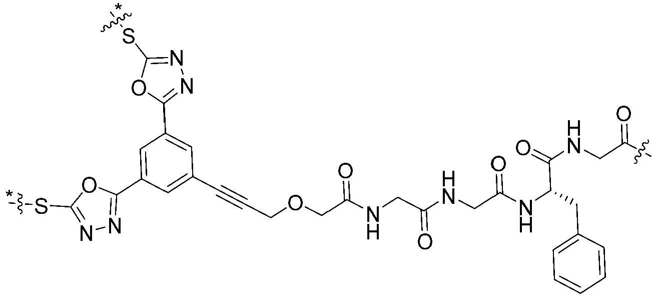

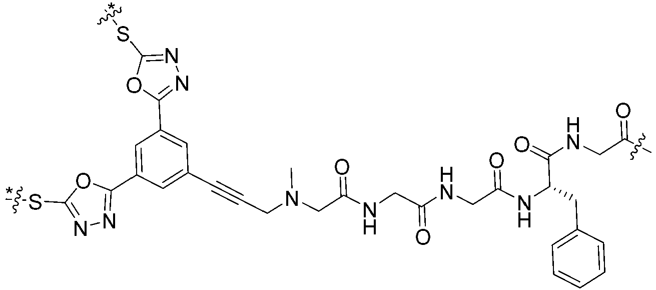

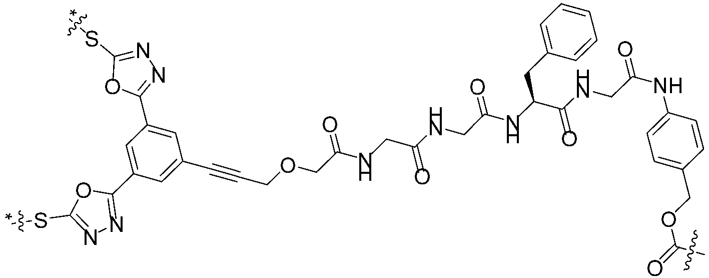

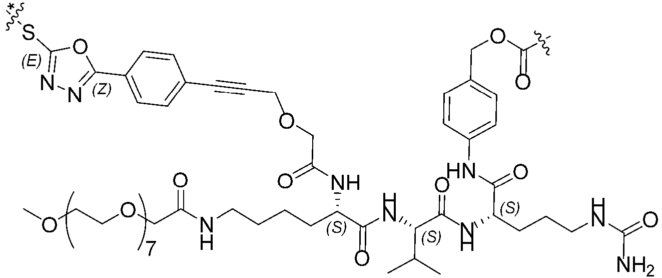

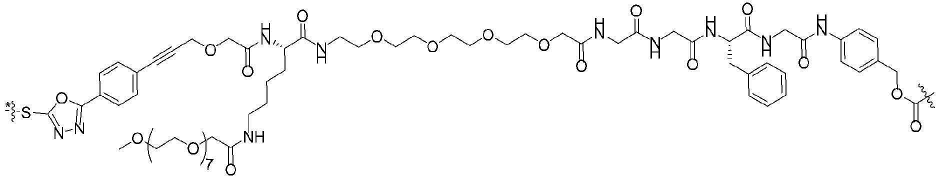

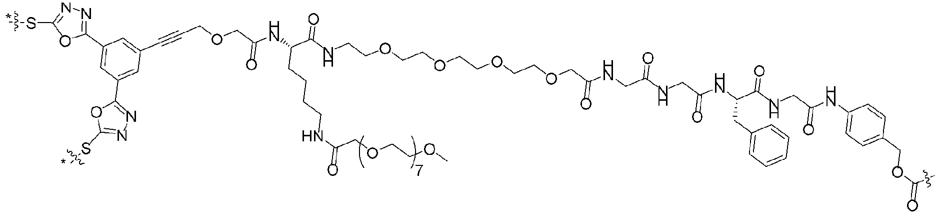

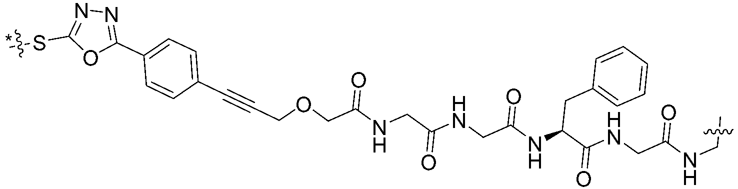

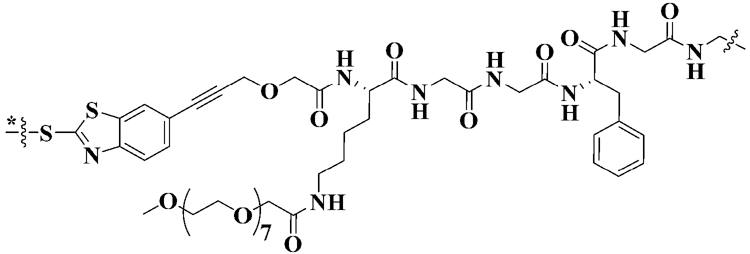

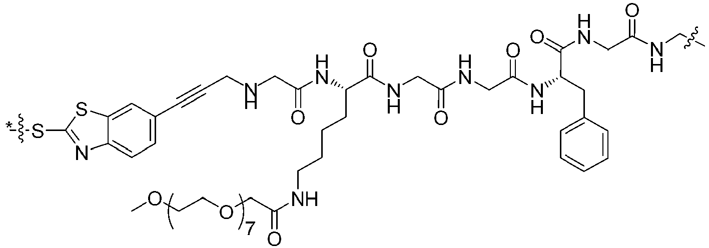

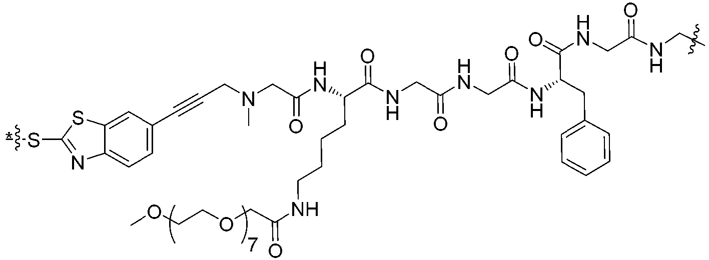

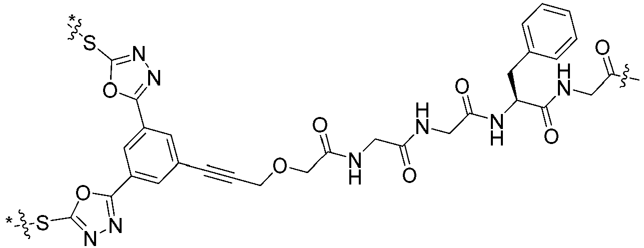

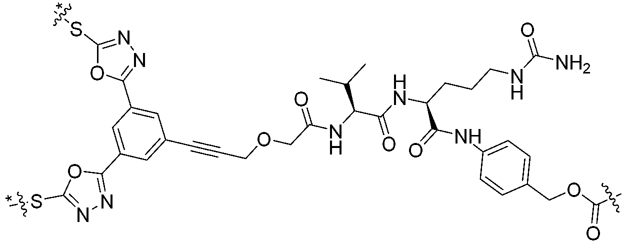

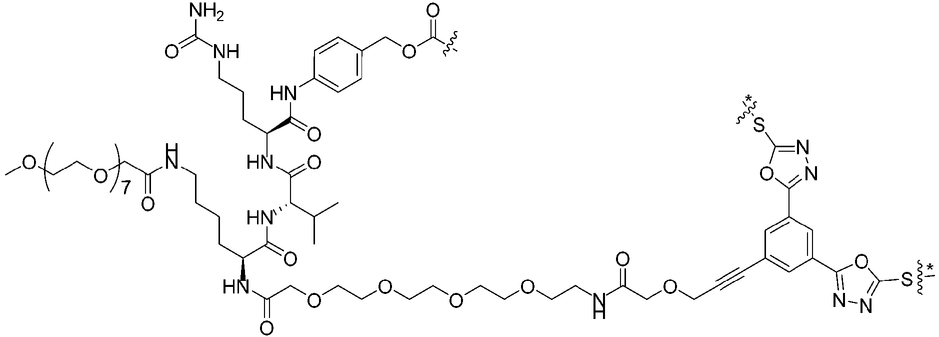

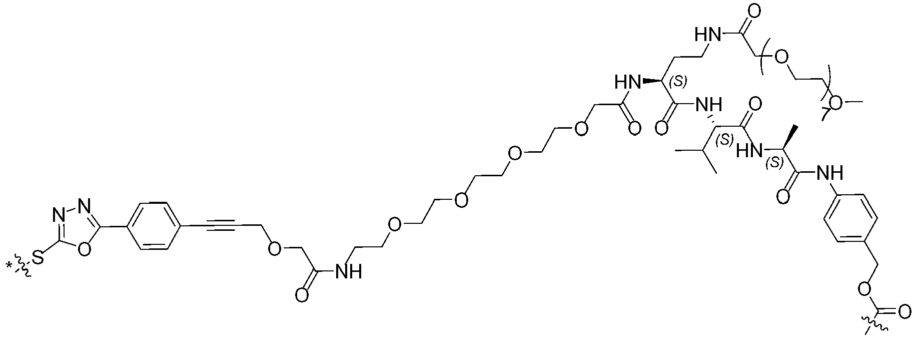

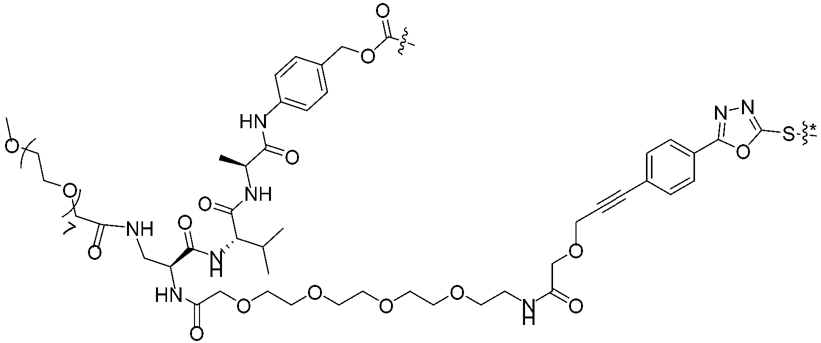

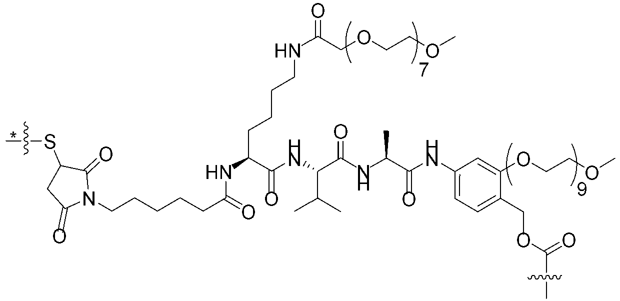

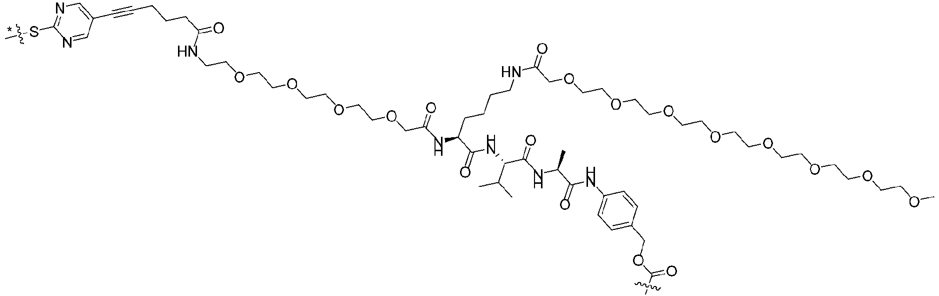

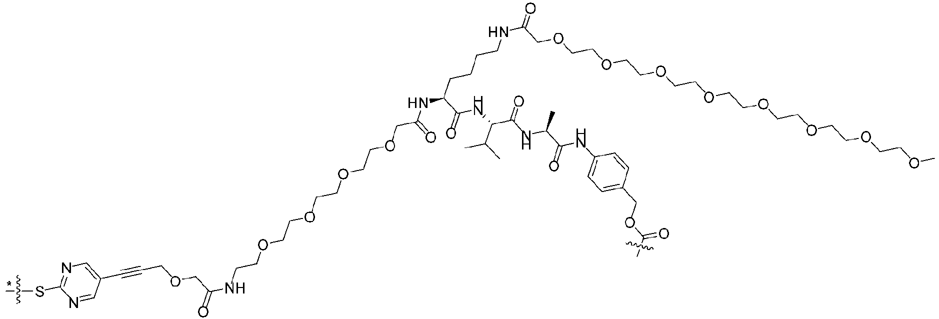

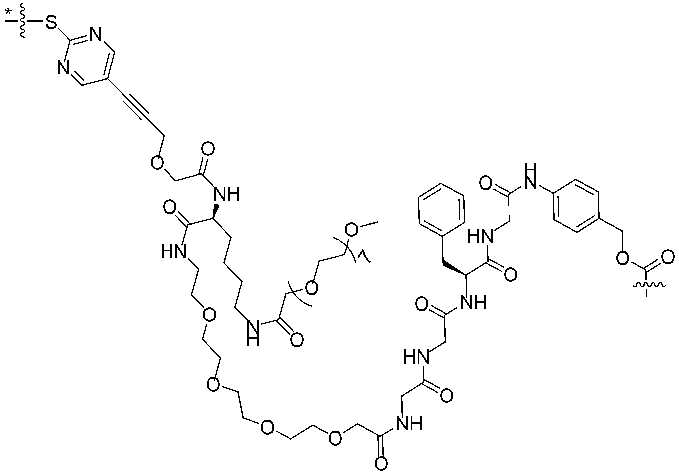

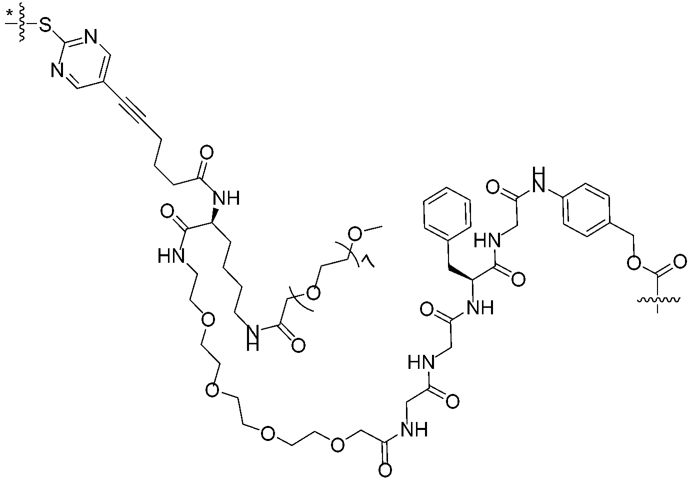

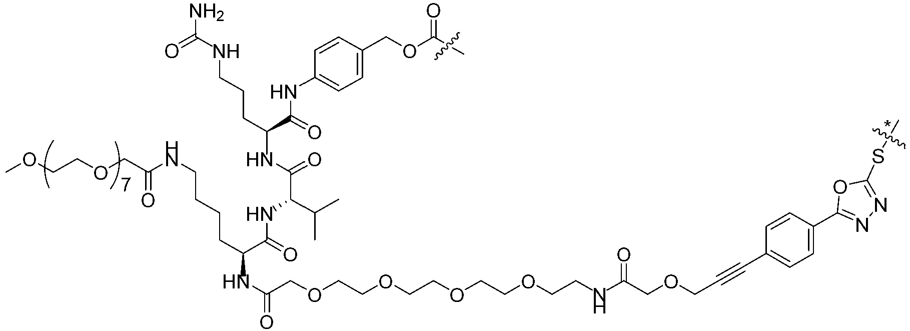

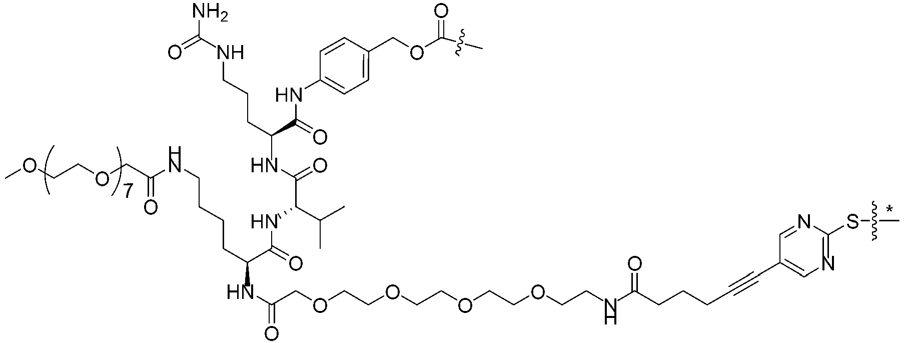

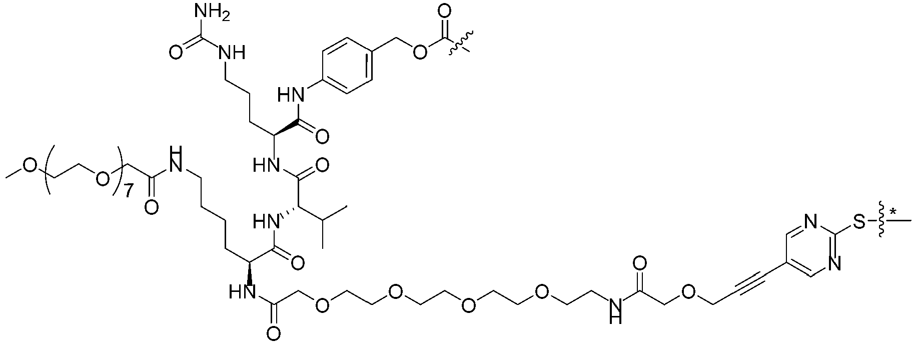

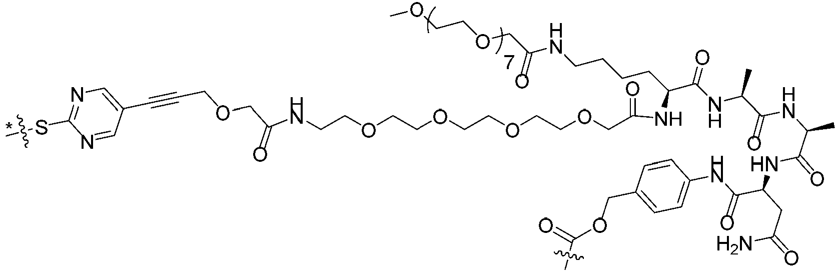

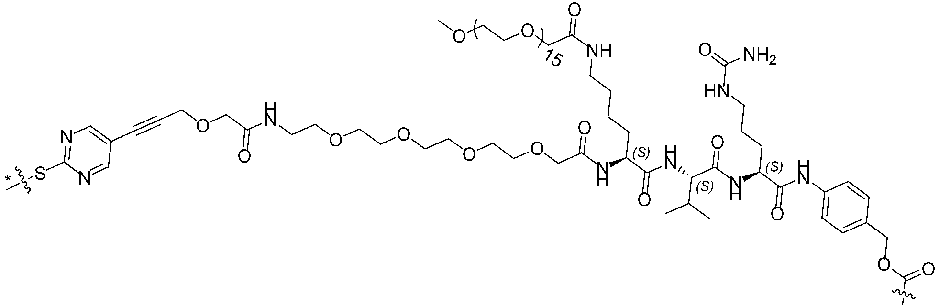

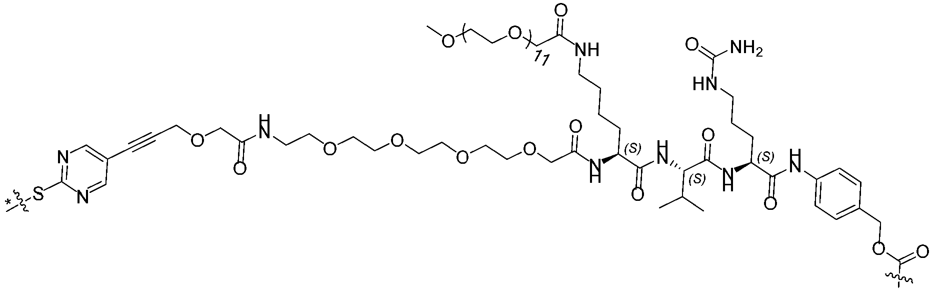

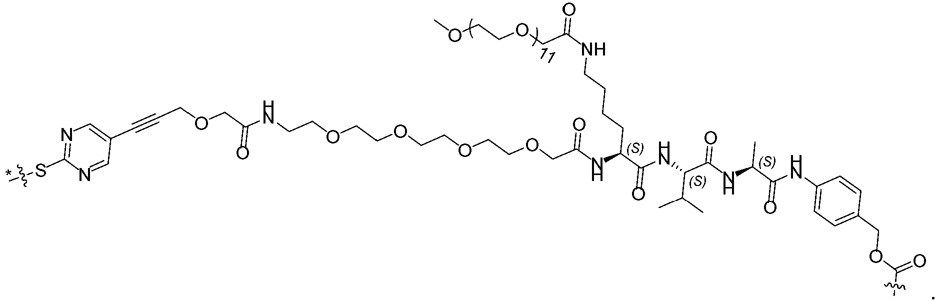

17. The antibody drug conjugate or the pharmaceutically acceptable salt thereof according to any one of claims 1 to 16, wherein Ab is selected from an anti-Her2 antibody, an anti-Trop2 antibody, an anti-Claudin18.2 antibody, an anti-LIV-1 antibody, an anti-5T4 antibody, an anti-CLDN6 antibody, an anti-CDH6 antibody or an anti-folate receptor antibody; preferably, the anti-Her2 antibody comprises HCDR1, HCDR2 and HCDR3 as shown in SEQ ID NO: 11, SEQ ID NO: 12 and SEQ ID NO: 13, and LCDR1, LCDR2 and LCDR3 as shown in SEQ ID NO: 16, SEQ ID NO: 17 and SEQ ID NO: 18; or, the anti-Her2 antibody comprises a heavy chain variable region as shown in SEQ ID NO: 14 and a light chain variable region as shown in SEQ ID NO: 19; or, the anti-Her2 antibody comprises a heavy chain as shown in SEQ ID NO: 15 and a light chain as shown in SEQ ID NO: 20; or, the anti-Her2 antibody is Trastuzumab.

18. The antibody drug conjugate or the pharmaceutically acceptable salt thereof according to claim 17, wherein Ab is the anti-LIV-1 antibody.

19. The antibody drug conjugate or the pharmaceutically acceptable salt thereof according to claim 18, wherein the anti-LIV-1 antibody comprises HCDR1, HCDR2 and HCDR3 as shown in SEQ ID NO: 1, SEQ ID NO: 2 and SEQ ID NO: 3; and LCDR1, LCDR2 and LCDR3 as shown in SEQ ID NO: 6, SEQ ID NO: 7 and SEQ ID NO: 8.

20. The antibody drug conjugate or the pharmaceutically acceptable salt thereof according to claim 19, wherein the anti-LIV-1 antibody comprises a heavy chain variable region as shown in SEQ ID NO: 4 and a light chain variable region as shown in SEQ ID NO: 9; and preferably, the anti-LIV-1 antibody comprises a heavy chain as shown in SEQ ID NO: 5 and a light chain as shown in SEQ ID NO: 10.

21. The antibody drug conjugate or the pharmaceutically acceptable salt thereof according to any one of claims 1 to 20, wherein the drug molecule D is selected from a camptothecin compound, an auristatin compound, an eribulin compound, a maytansine compound, a calicheamicin compound, or an anthramycin compound; and preferably, the drug molecule D is selected from a fragment with a structure of formula IIa or a pharmaceutically acceptable salt thereof: 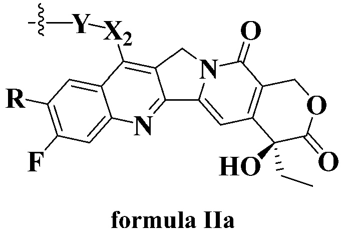 wherein, R is selected from F, C₁₋₄ alkoxy, C₁₋₄ haloalkyl or C₁₋₄ haloalkoxy; X₂ is selected from -(CH₂)s-, and s is selected from 0, 1, 2, 3 or 4; Y is selected from -O- or -N(Rc)-, and Rc is selected from H, C₁₋₄ alkyl, 3-5 membered cycloalkyl or 3-5 membered heterocycloalkyl; and when s=1, Rc is not H.

22. The antibody drug conjugate or the pharmaceutically acceptable salt thereof according to claim 21, wherein R is F, or R is methoxy.

23. The antibody drug conjugate or the pharmaceutically acceptable salt thereof according to claim 21, wherein s is 1, or s is 2, or s is 3.

24. The antibody drug conjugate or the pharmaceutically acceptable salt thereof according to claim 21, wherein Rc is selected from H, methyl or cyclopropyl.

25. The antibody drug conjugate or the pharmaceutically acceptable salt thereof according to claim 21, wherein Y is selected from -O-.

26. The antibody drug conjugate or the pharmaceutically acceptable salt thereof according to claim 21, wherein Y is -N(Rc)-, and Rc is methyl or cyclopropyl.

27. The antibody drug conjugate or the pharmaceutically acceptable salt thereof according to claim 1, wherein the drug molecule D is selected from the following structural fragments or pharmaceutically acceptable salts thereof:

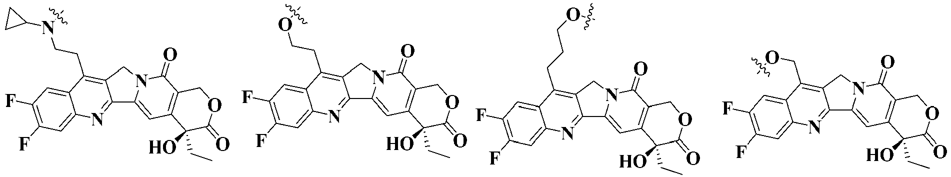

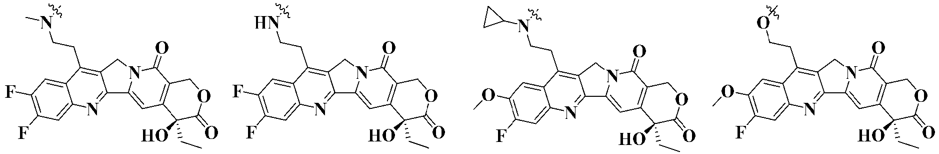

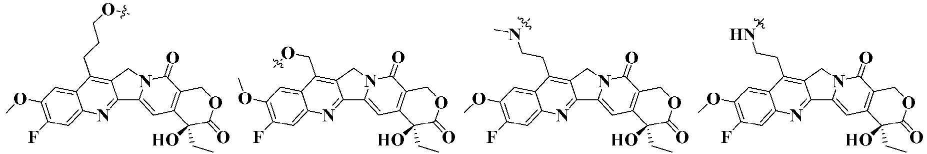

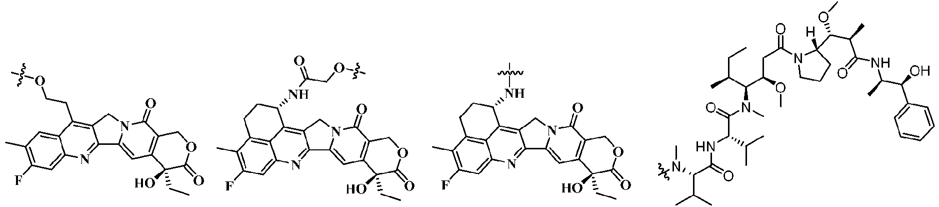

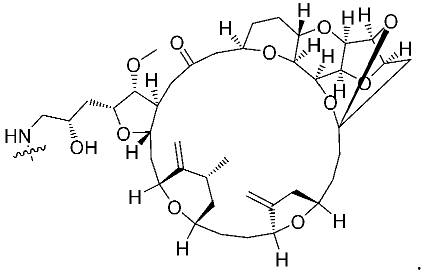

28. The antibody drug conjugate or the pharmaceutically acceptable salt thereof according to claim 1, wherein -L₁-L₂-L₃-L₄-D is selected from the following structural fragments or pharmaceutically acceptable salts thereof:

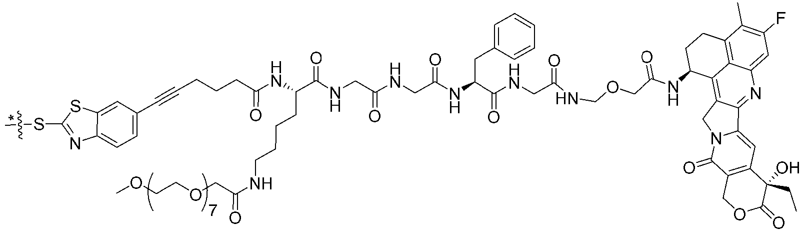

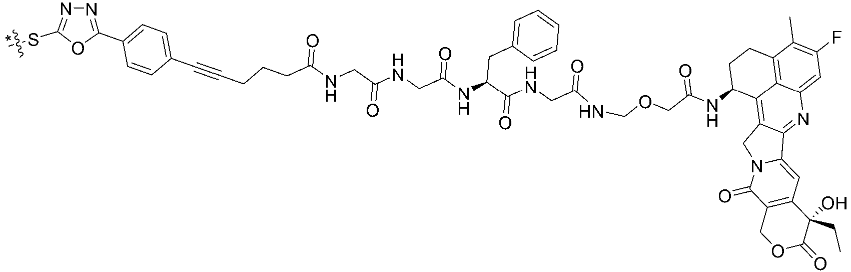

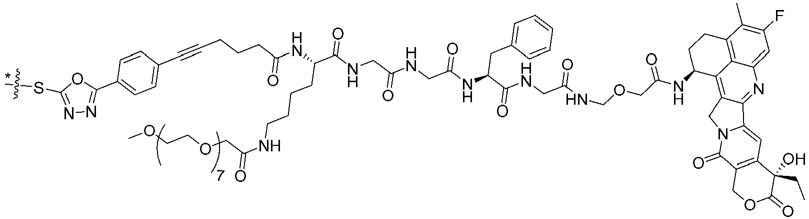

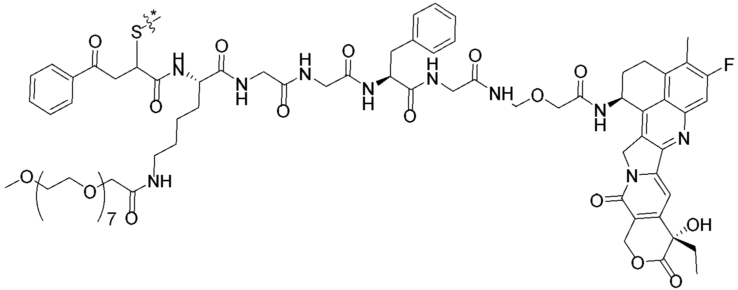

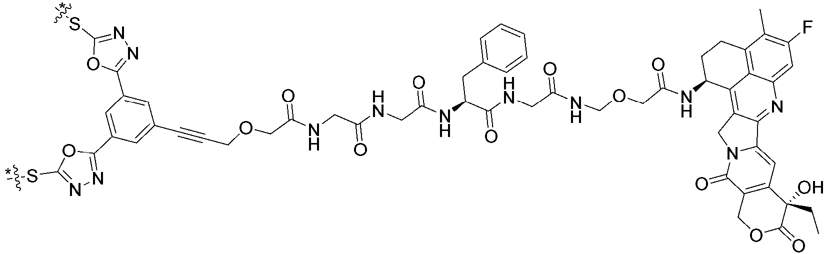

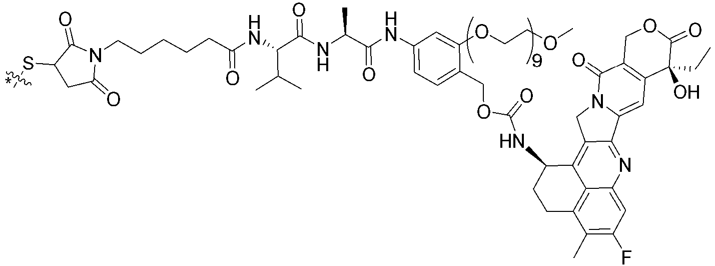

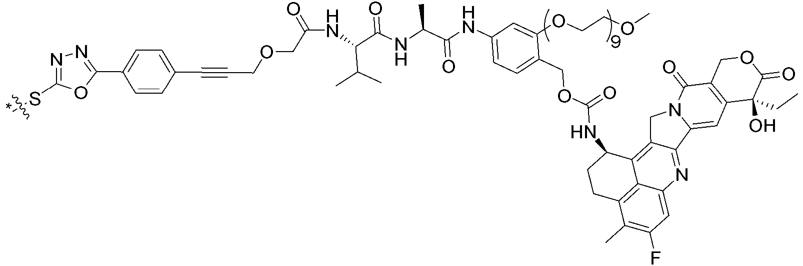

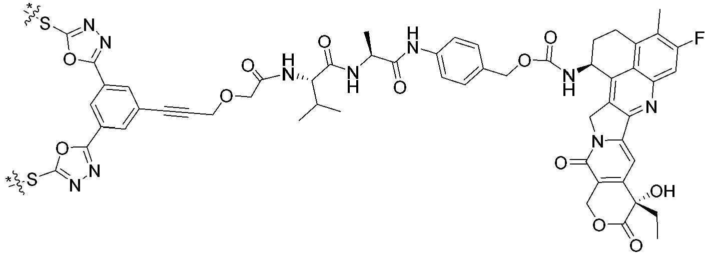

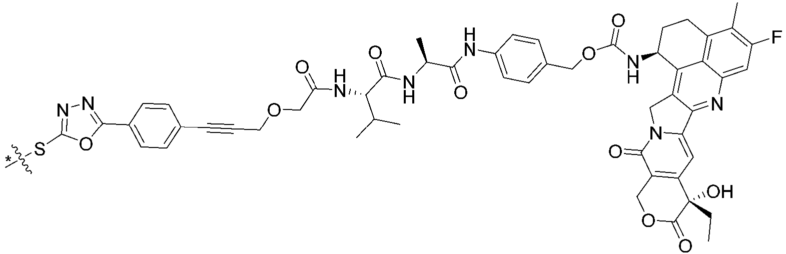

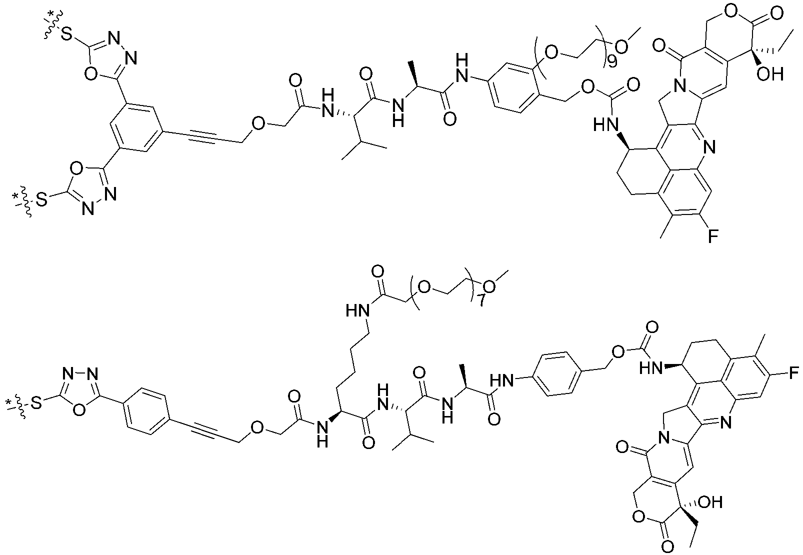

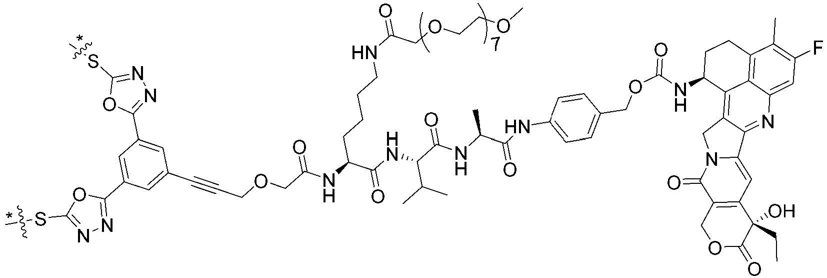

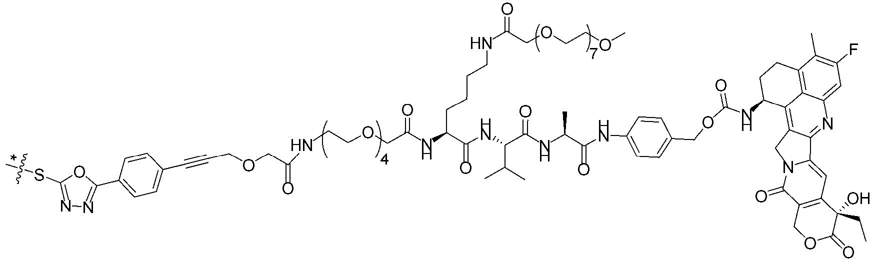

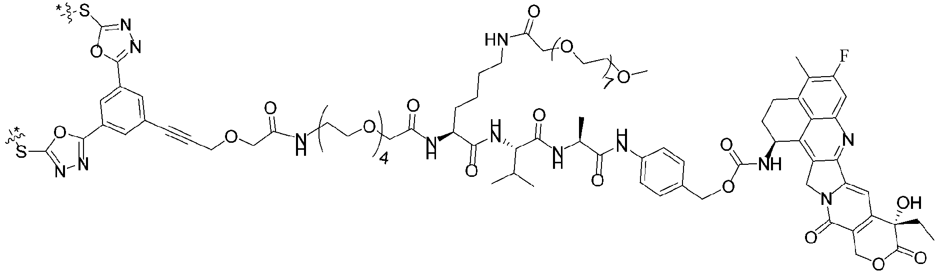

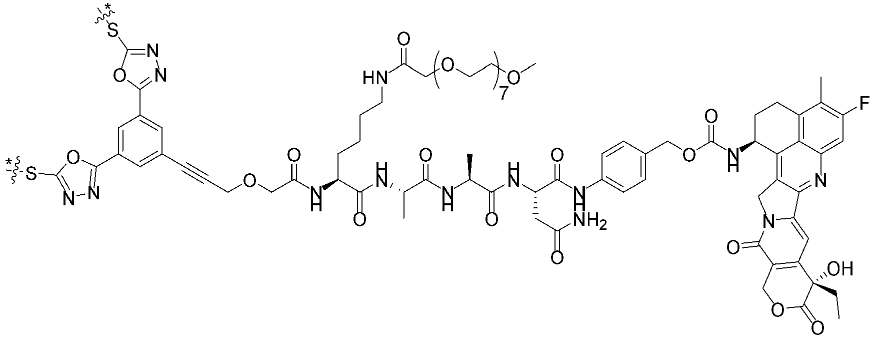

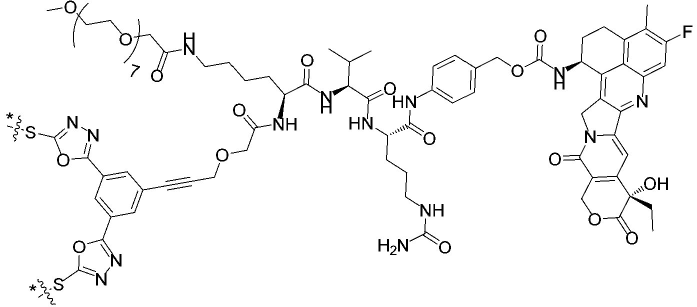

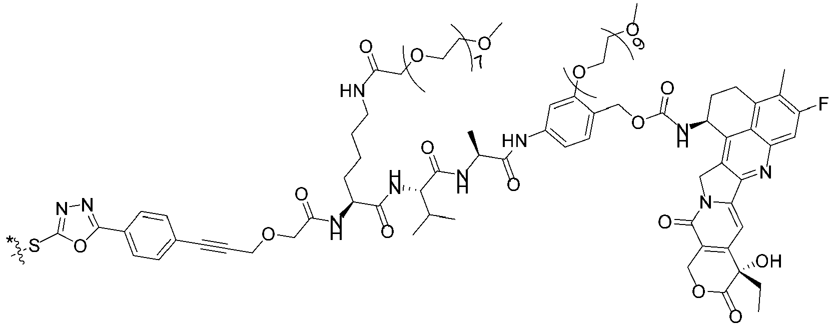

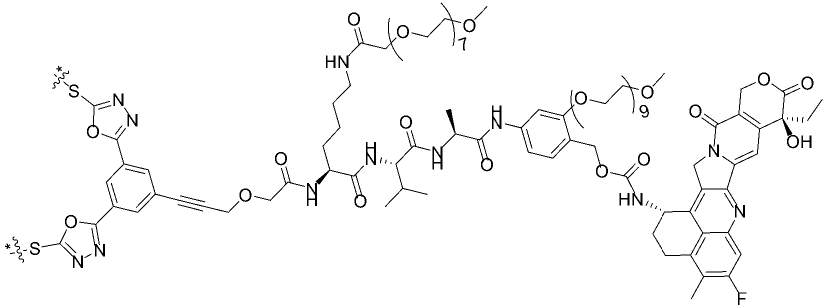

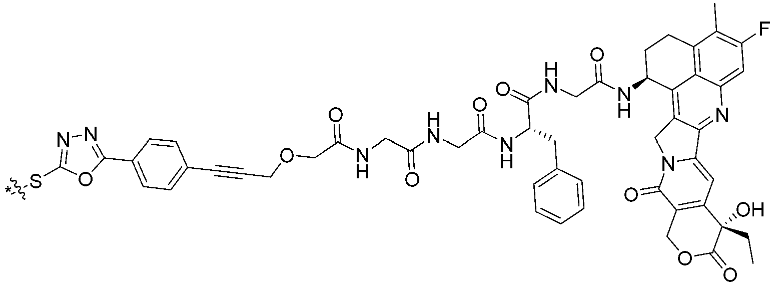

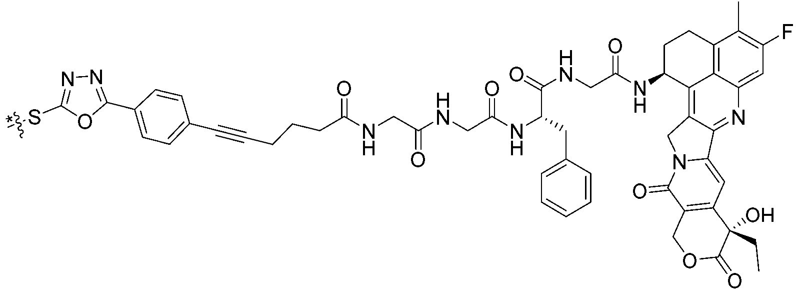

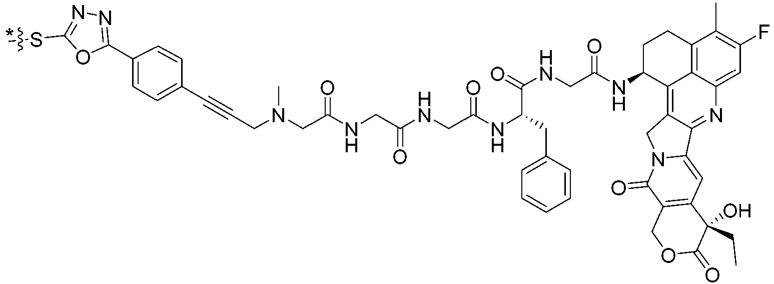

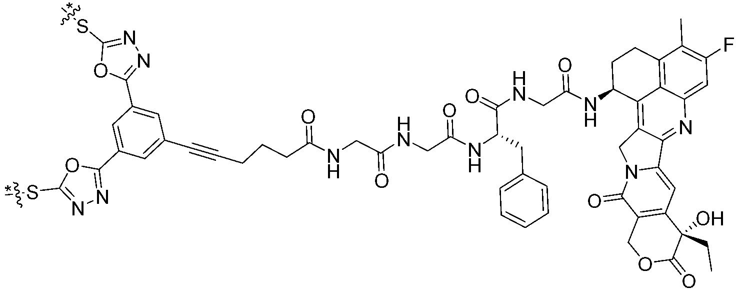

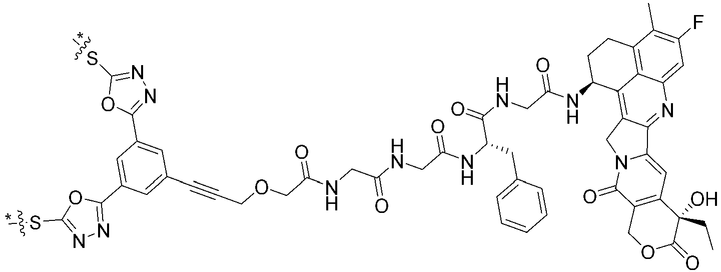

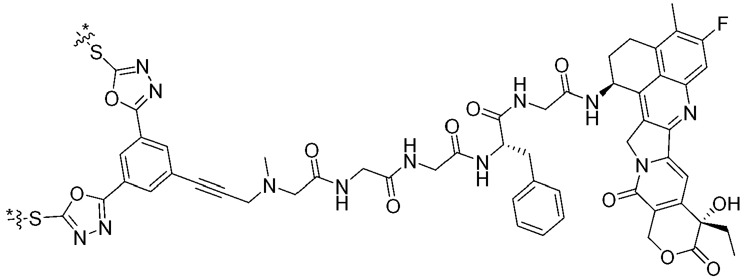

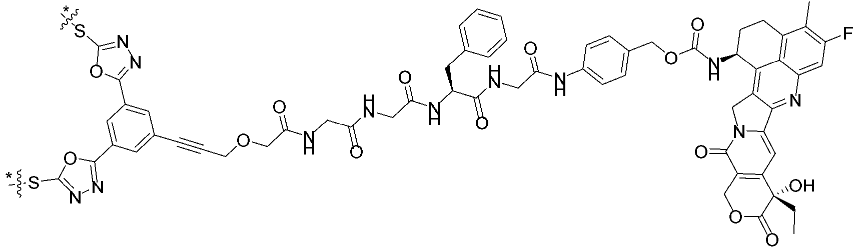

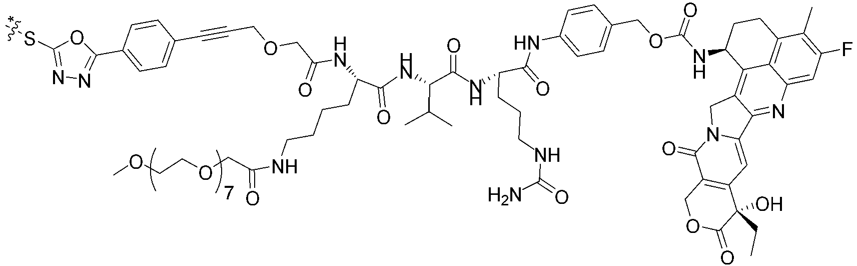

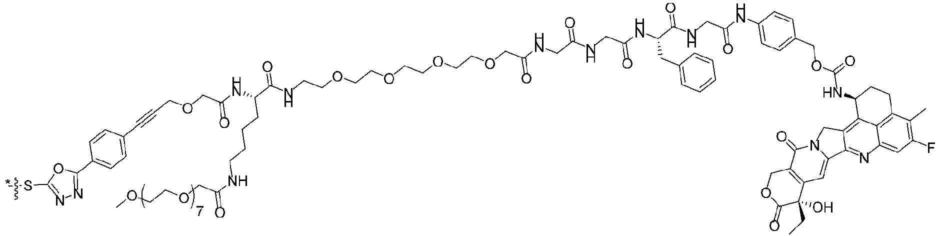

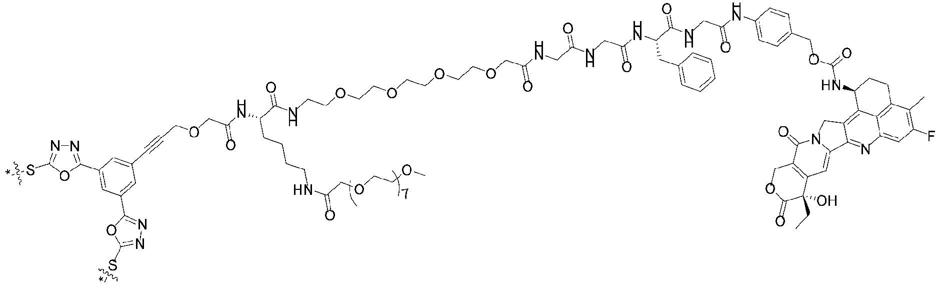

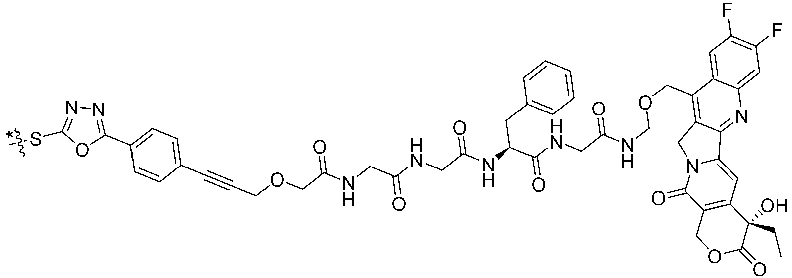

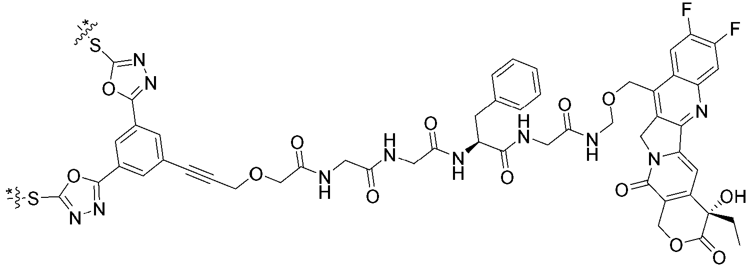

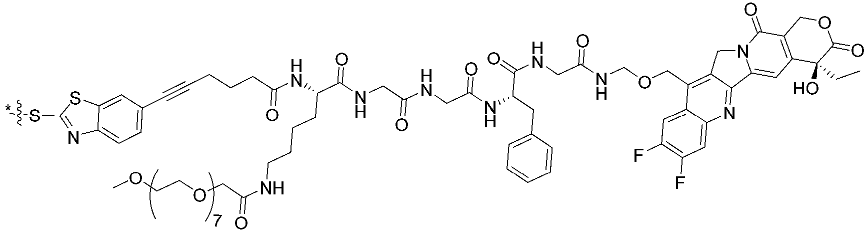

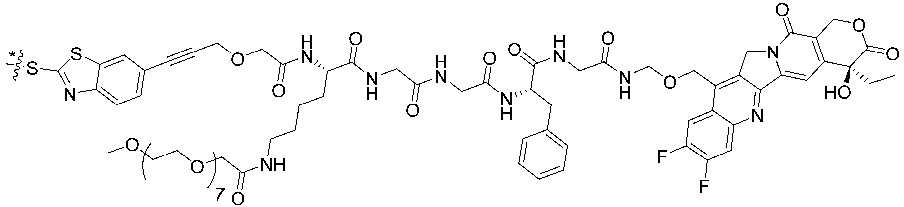

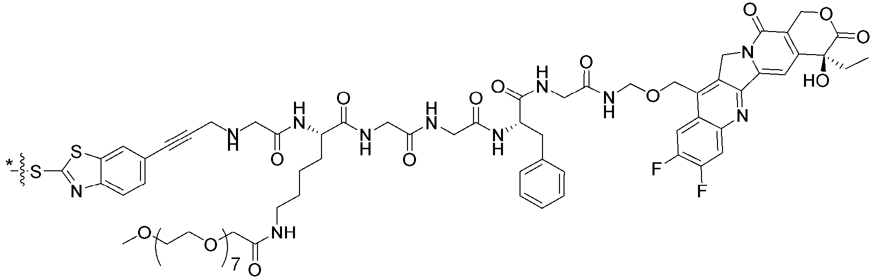

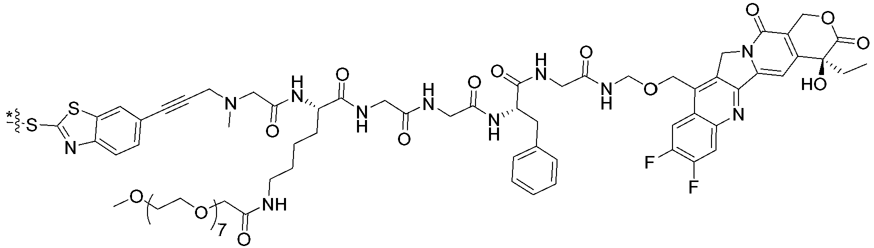

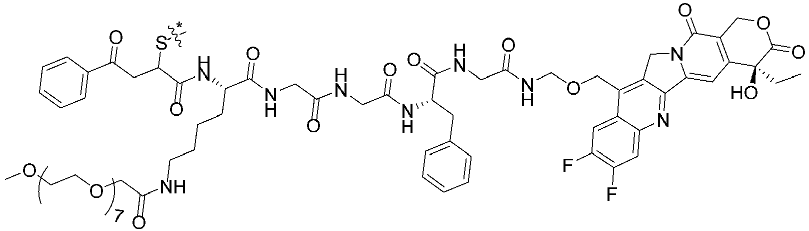

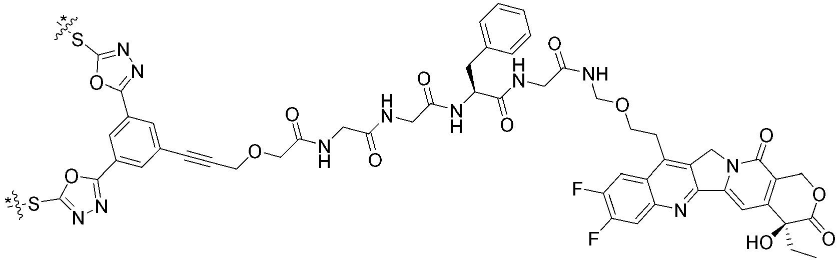

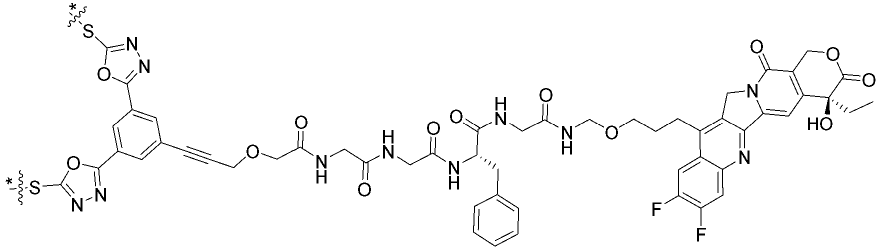

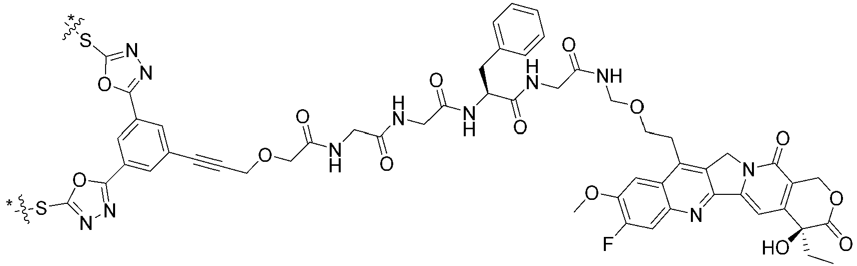

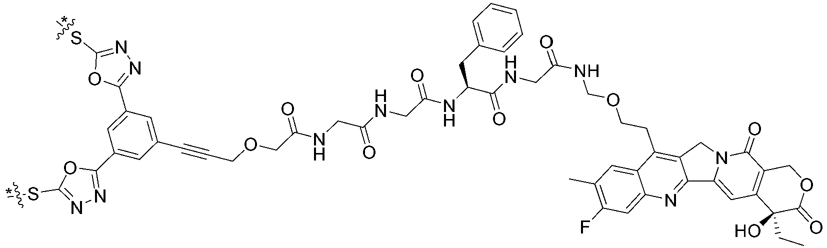

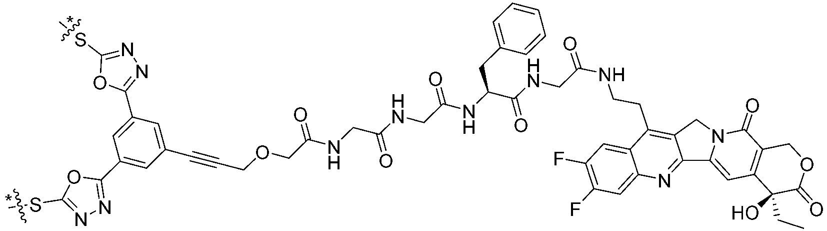

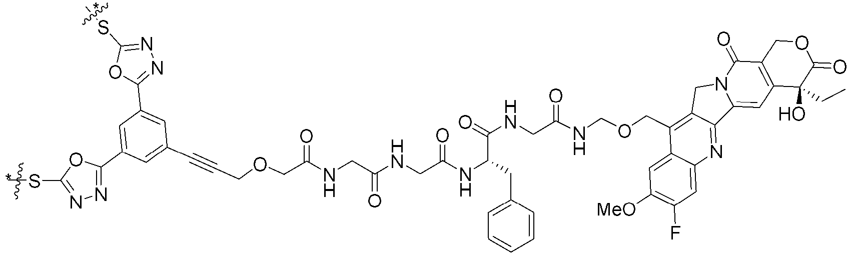

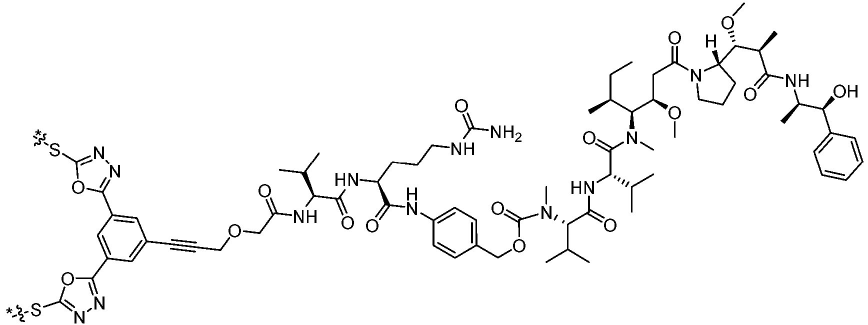

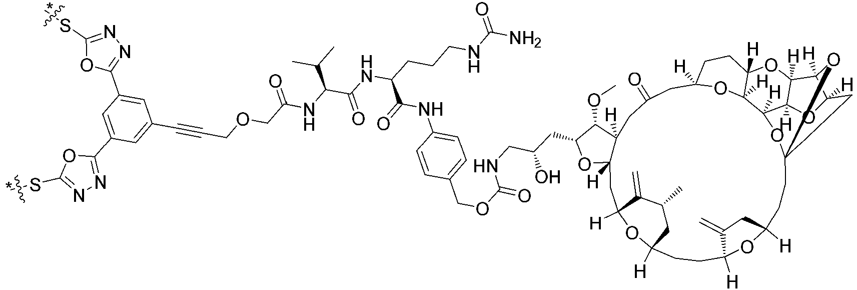

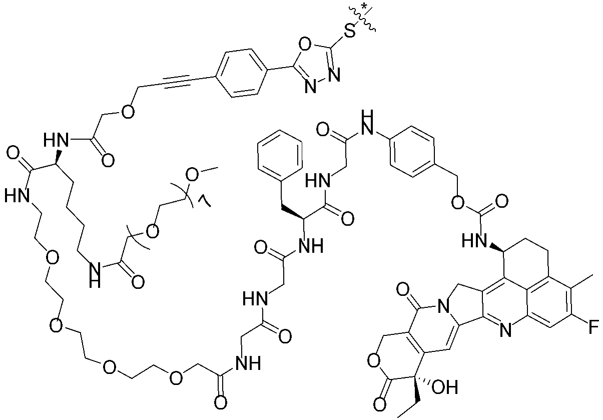

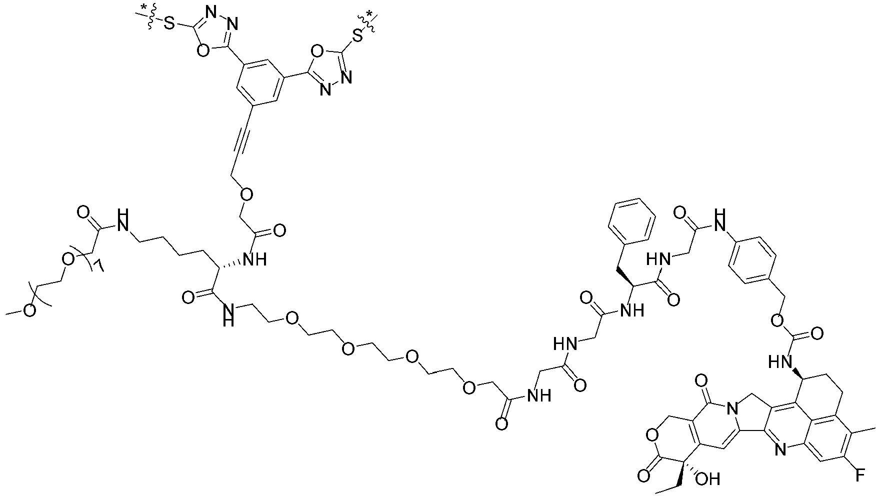

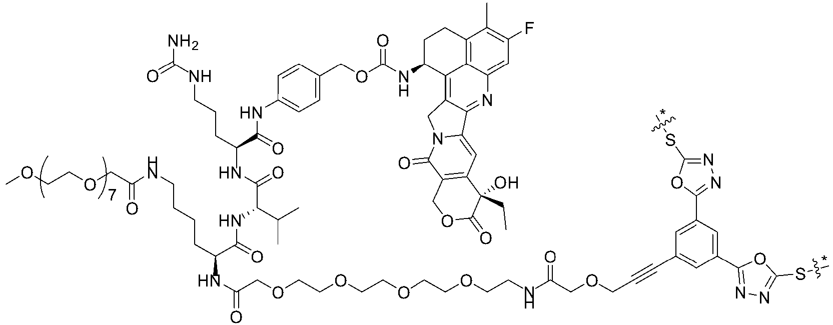

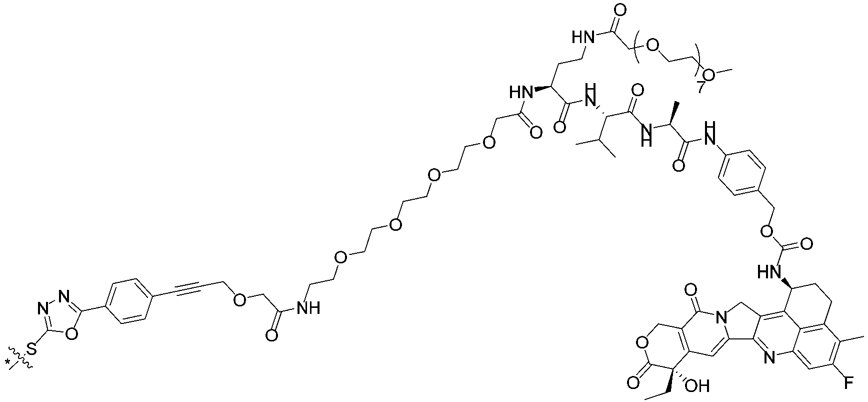

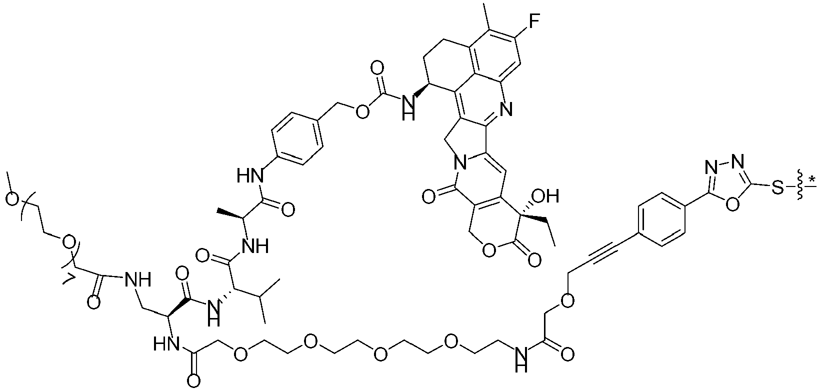

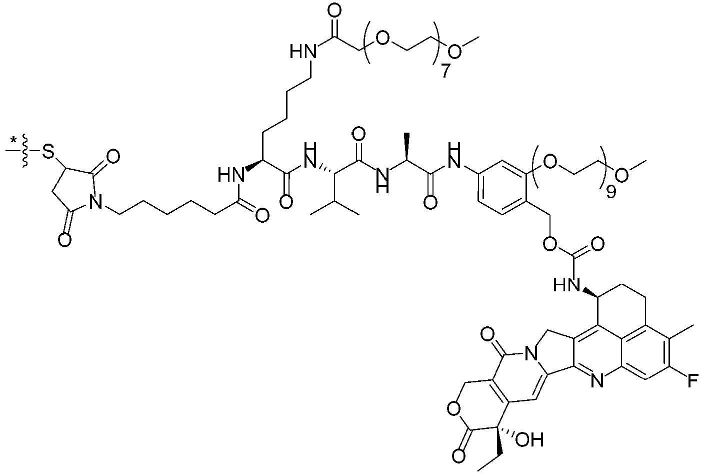

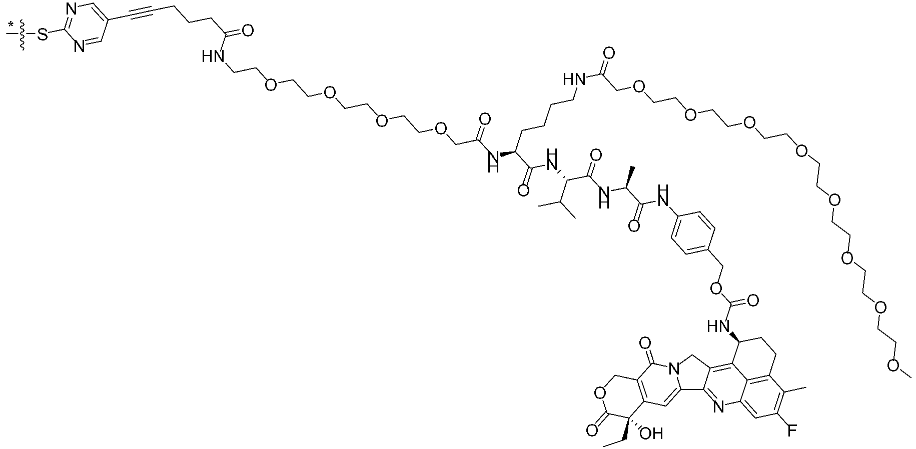

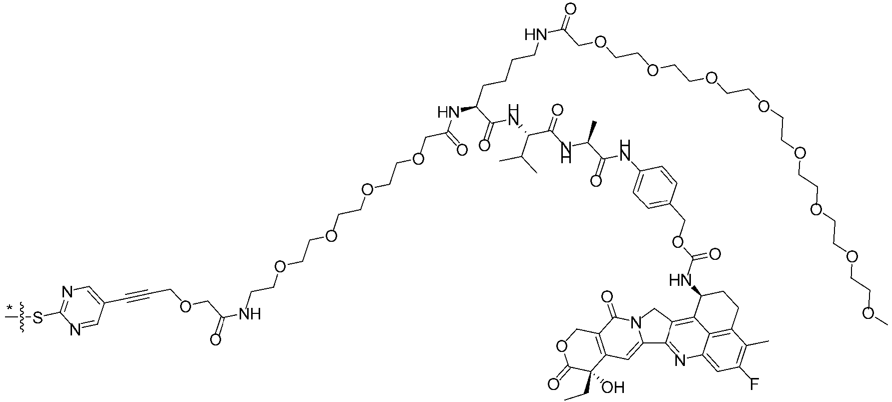

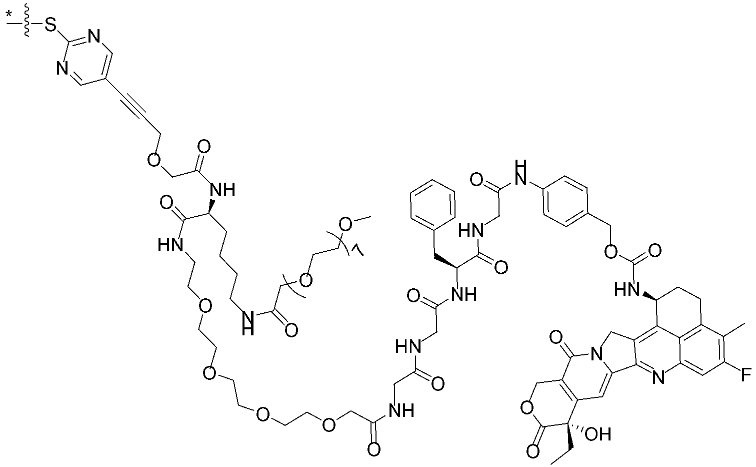

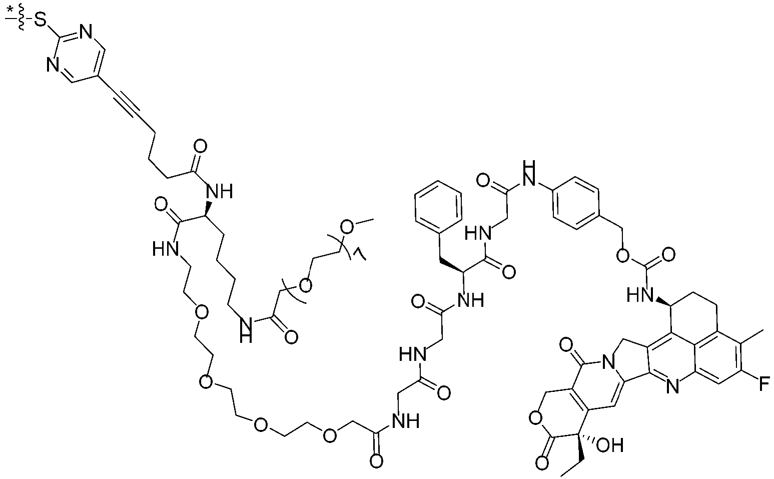

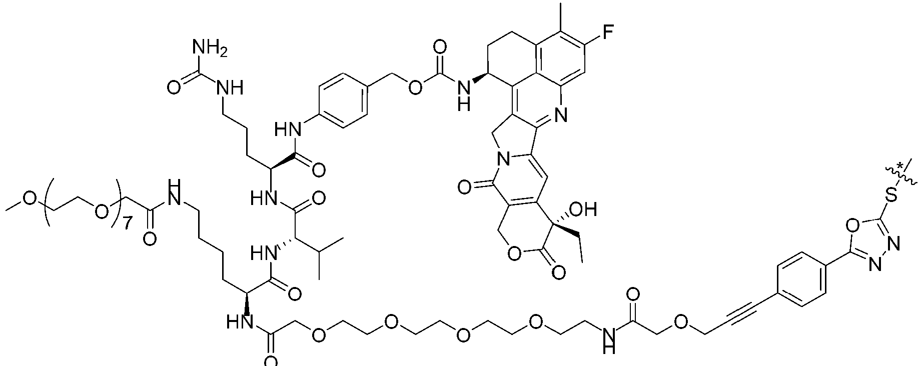

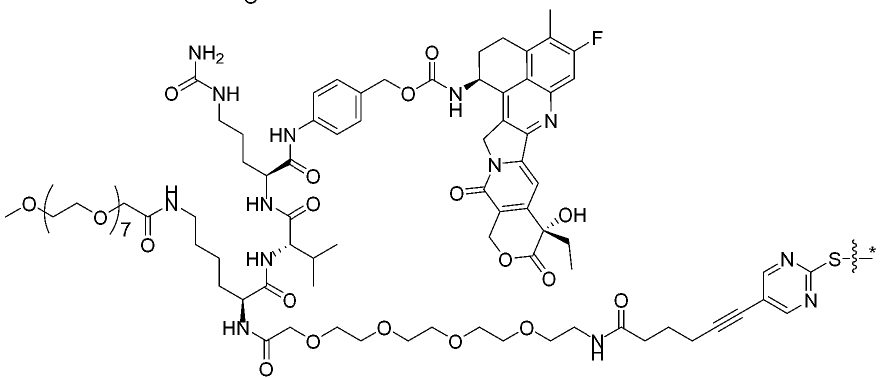

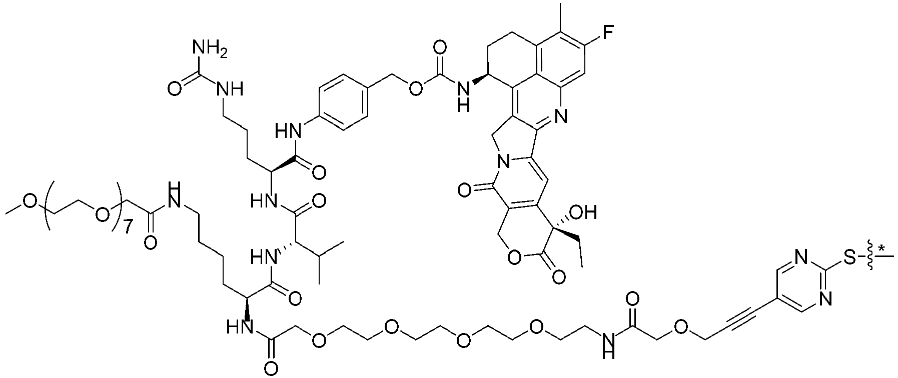

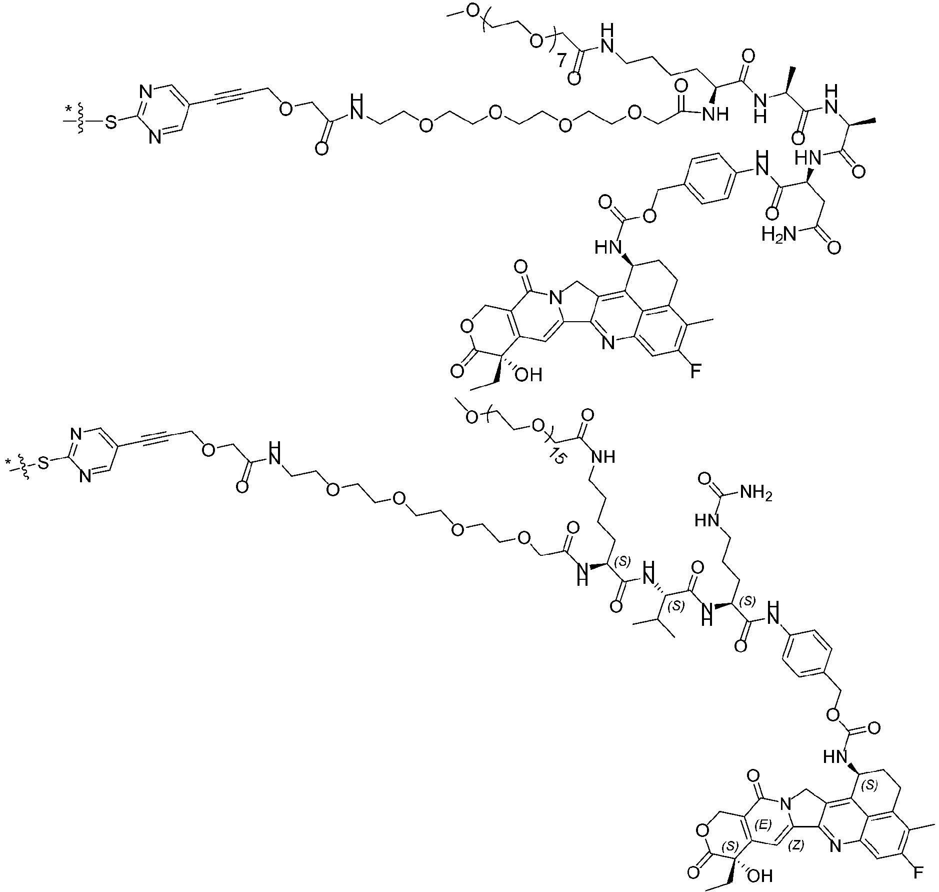

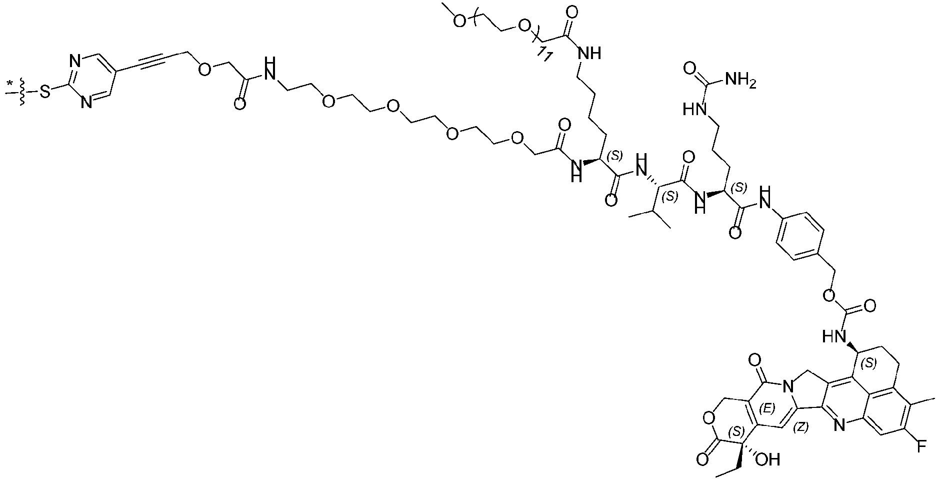

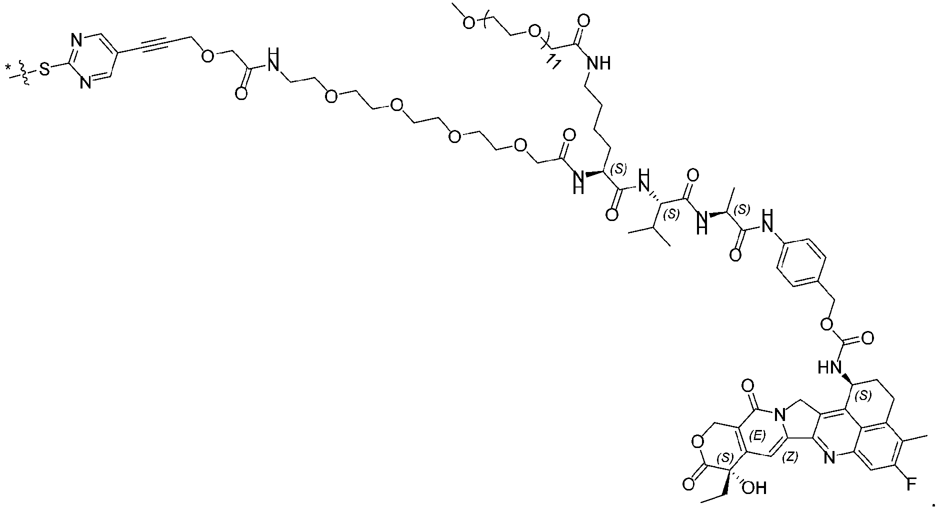

29. The antibody drug conjugate or the pharmaceutically acceptable salt thereof according to claim 1, wherein the antibody drug conjugate as shown in formula I or the pharmaceutically acceptable salt thereof is an antibody drug conjugate as shown in the following formula III or a pharmaceutically acceptable salt thereof, 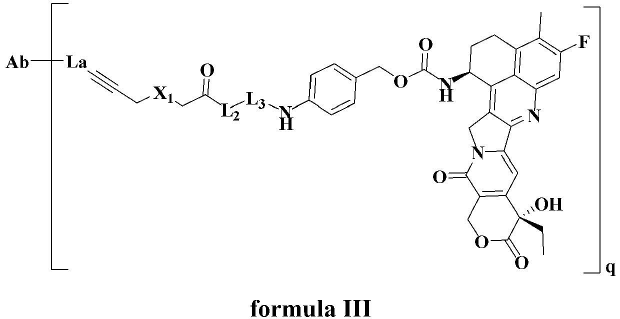 wherein, Ab, La, X₁, L₂, L₃ and q have the same meanings as defined in any one of claims 1 to 27.

30. The antibody drug conjugate or the pharmaceutically acceptable salt thereof according to claim 29, wherein La is selected from 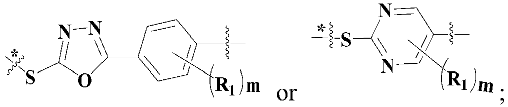 and R₁ and m have the same meanings as defined in claim 1.

31. The antibody drug conjugate or the pharmaceutically acceptable salt thereof according to claim 30, wherein La is selected from

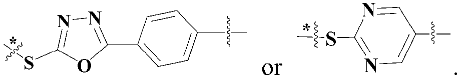

32. The antibody drug conjugate or the pharmaceutically acceptable salt thereof according to claim 29, wherein X₁ is selected from O or CH₂.

33. The antibody drug conjugate or the pharmaceutically acceptable salt thereof according to claim 29, wherein L₂ is selected from a chemical bond,

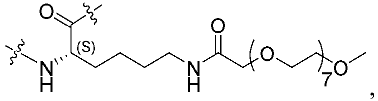

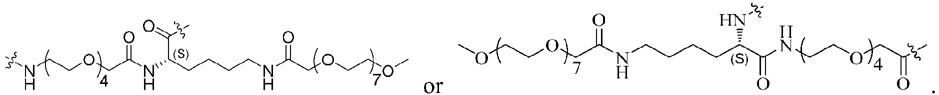

34. The antibody drug conjugate or the pharmaceutically acceptable salt thereof according to claim 29, wherein L₃ is a peptide fragment consisting of 2-4 amino acids selected from glycine, D-phenylalanine, D-valine, D-alanine, D-asparagine and citrulline, and the -NH- terminus of L₃ is linked to L₂.

35. The antibody drug conjugate or the pharmaceutically acceptable salt thereof according to claim 34, wherein L₃ is selected from -Val-Ala-, -Val-Cit- or -Gly-Gly-Phe-Gly-, wherein the -NH- terminus of L₃ is linked to L₂.

36. The antibody drug conjugate or the pharmaceutically acceptable salt thereof according to claim 29, wherein q is selected from 7.5-8.5.

37. The antibody drug conjugate or the pharmaceutically acceptable salt thereof according to claim 29, wherein La is selected from 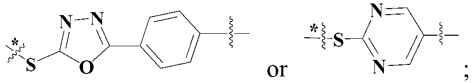 X₁ is selected from O or CH₂; L₂ is selected from a chemical bond, 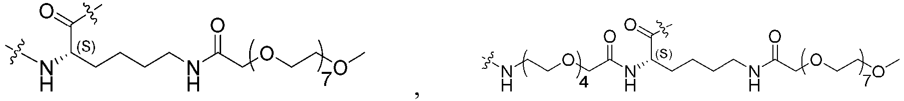 or 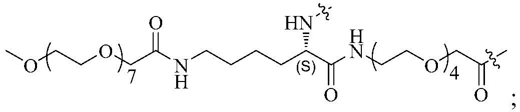 L₃ is selected from -Val-Ala-, -Val-Cit- or -Gly-Gly-Phe-Gly-, and the -NH- terminus of L₃ is linked to L₂; and q is selected from 7.5-8.5, preferably 7.5-8.0.

38. The antibody drug conjugate or the pharmaceutically acceptable salt thereof according to claim 1 or 29, wherein the antibody drug conjugate as shown in formula I or III or the pharmaceutically acceptable salt thereof is an antibody drug conjugate as shown in the following formula IV or a pharmaceutically acceptable salt thereof, 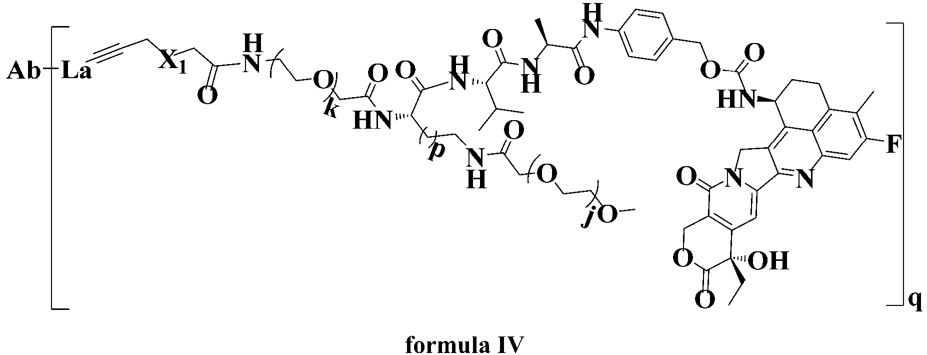 wherein, Ab, La, X₁, k, p, j and q have the same meanings as defined in claim 1 or 29.

39. The antibody drug conjugate or the pharmaceutically acceptable salt thereof according to claim 38, wherein k is selected from 3, 4 or 5, preferably 4; p is selected from 2, 3 or 4, preferably 3; and j is selected from 6, 7 or 8, preferably 7.

40. The antibody drug conjugate or the pharmaceutically acceptable salt thereof according to claim 38, wherein La is selected from 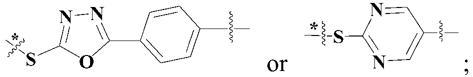 X₁ is selected from CH₂ or O; k is 4, p is 3, j is 7, and q is 7.5-8.0.

41. The antibody drug conjugate or the pharmaceutically acceptable salt thereof according to claim 1, which is selected from the following antibody drug conjugates or pharmaceutically acceptable salts thereof: 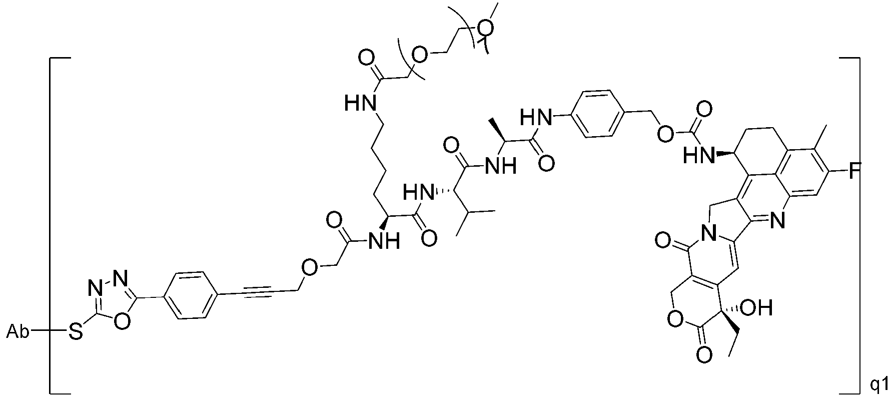 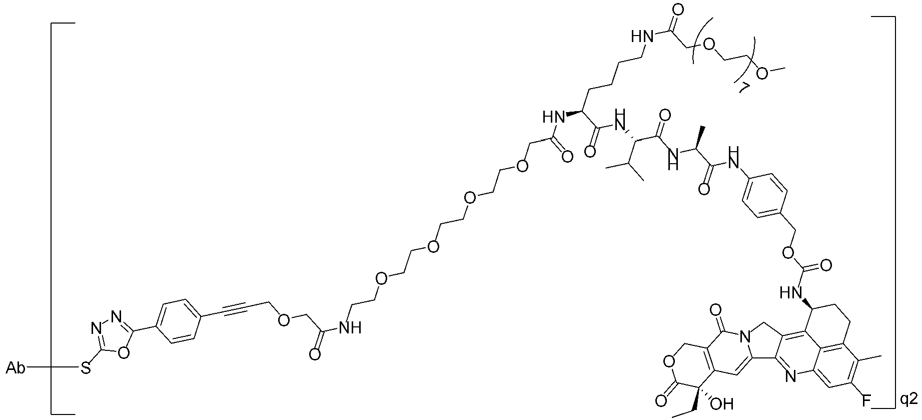 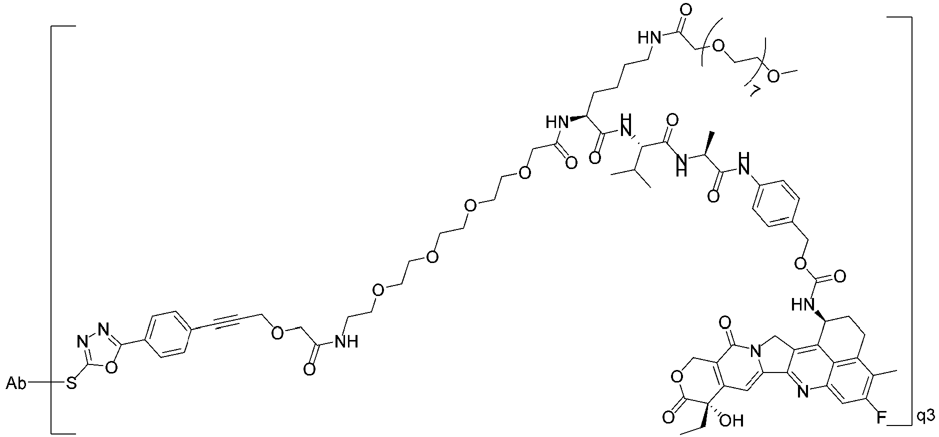 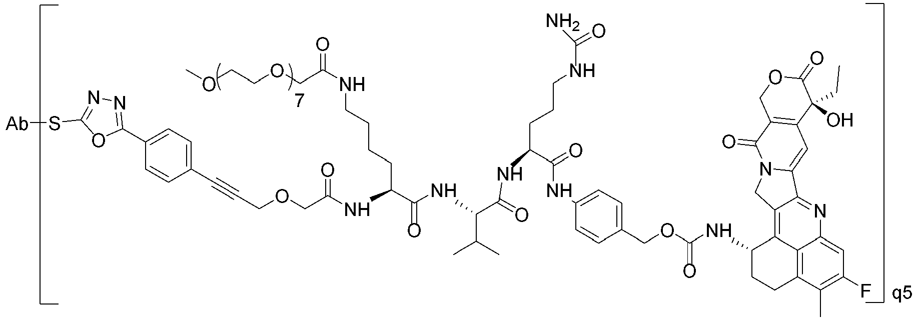 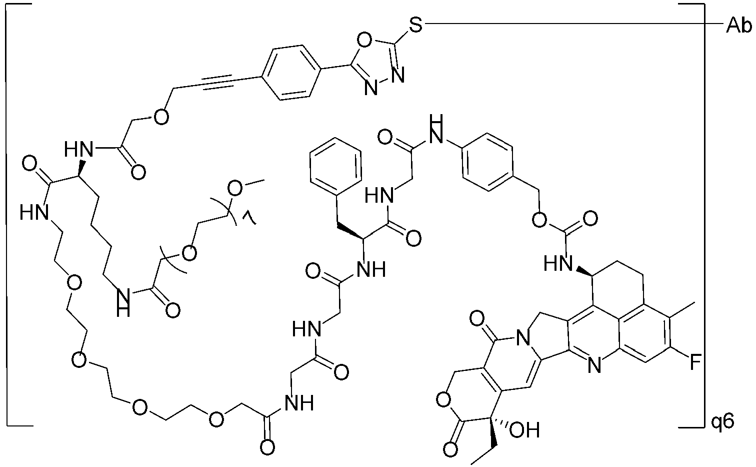 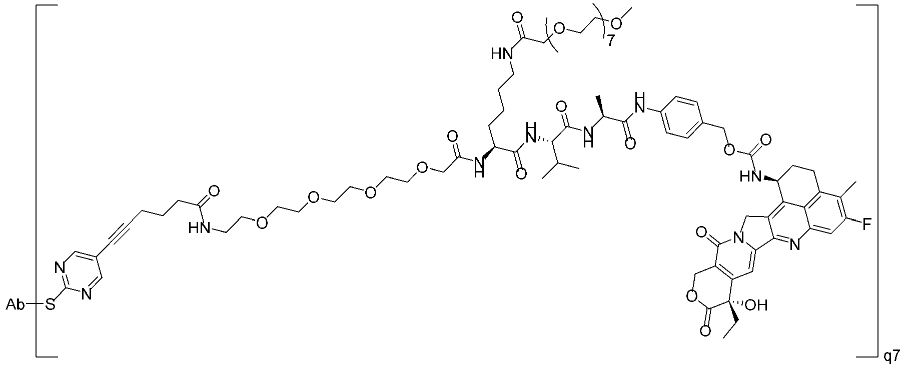 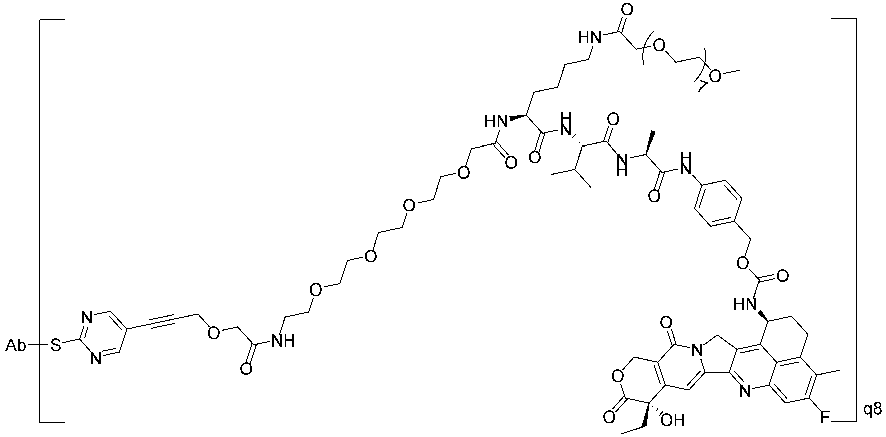 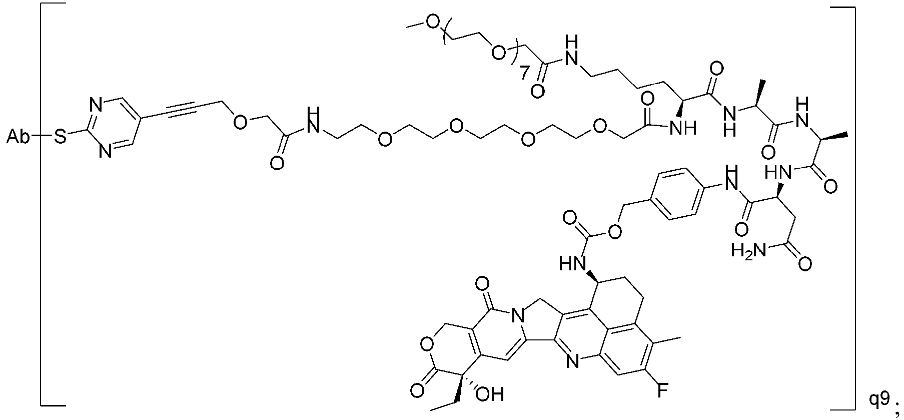 wherein, q1, q2, q4, q5, q6, q7, q8 and q9 are respectively independently selected from 7.5-8.0, preferably 7.6-7.7; q3 is selected from 4.0-5.0, preferably 4.2-4.6, and Ab is respectively independently selected from Ladiratuzumab or Trastuzumab.

42. A compound as shown in formula II or a pharmaceutically acceptable salt thereof, 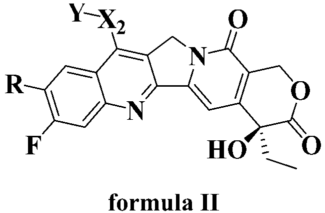 wherein, R is selected from F, C₁₋₄ alkoxy, C₁₋₄ haloalkyl or C₁₋₄ haloalkoxy; X₂ is selected from -(CH₂)s-, and s is selected from 0, 1, 2, 3 or 4; Y is selected from -OH or -NH-Rc, and Rc is selected from H, C₁₋₄ alkyl, 3-5 membered cycloalkyl or 3-5 membered heterocycloalkyl; and when s=1, Rc is not H.

43. The compound or the pharmaceutically acceptable salt thereof according to claim 42, wherein R is F, or R is methoxy.

44. The compound or the pharmaceutically acceptable salt thereof according to claim 42, wherein s is 1, or s is 2, or s is 3.

45. The compound or the pharmaceutically acceptable salt thereof according to claim 42, wherein Rc is selected from H, methyl or cyclopropyl.

46. The compound or the pharmaceutically acceptable salt thereof according to claim 42, wherein Y is selected from -OH or -NH-Rc; and Rc is methyl or cyclopropyl.

47. Compounds as follows or pharmaceutically acceptable salts thereof;

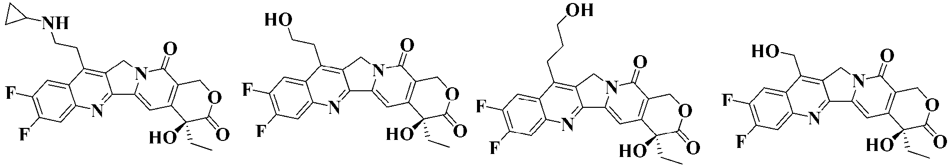

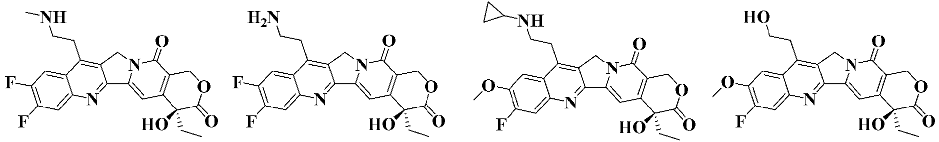

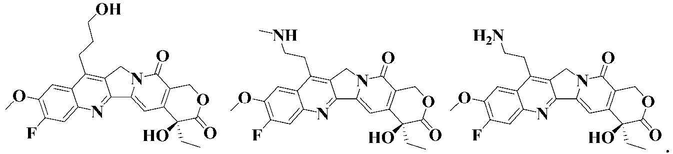

48. Compounds as follows or pharmaceutically acceptable salts thereof;

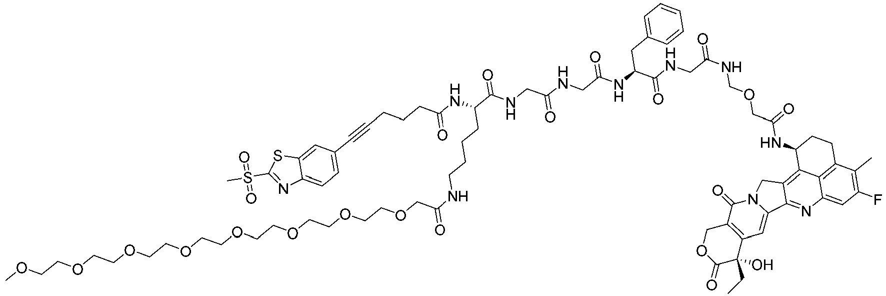

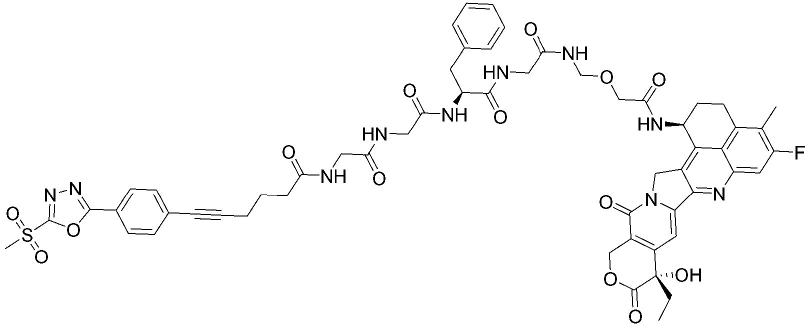

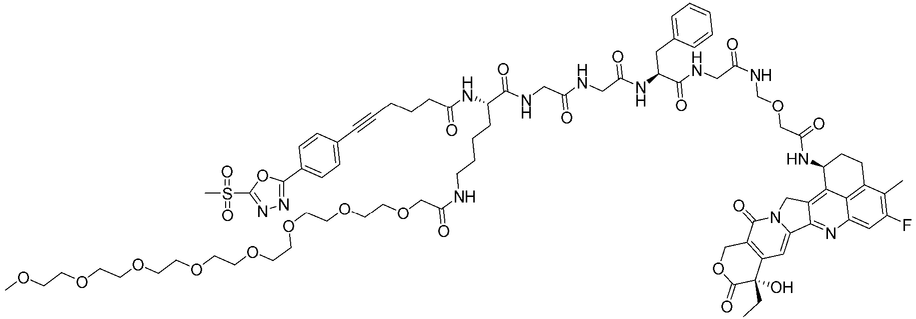

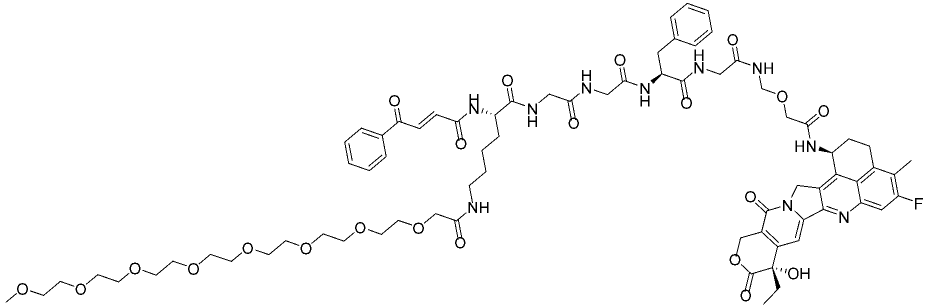

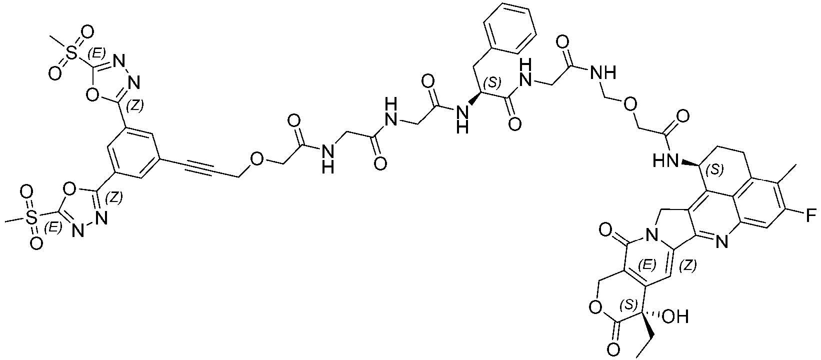

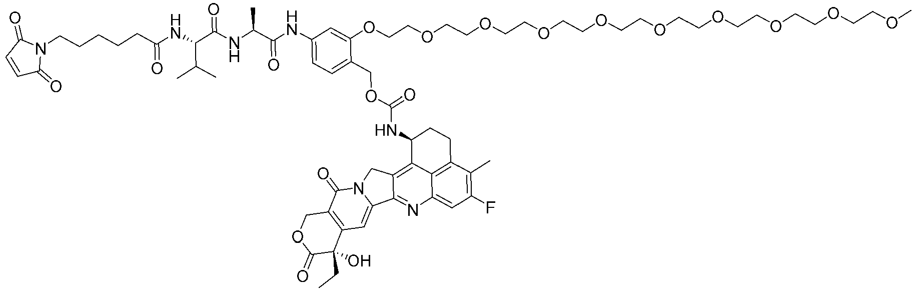

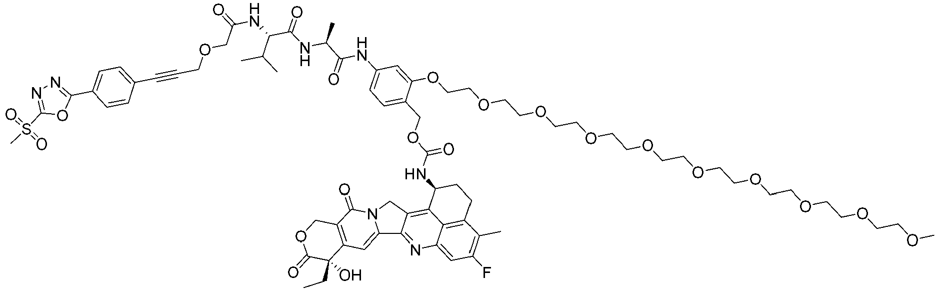

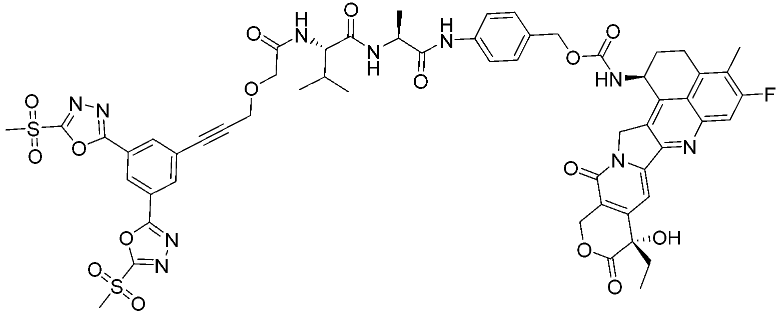

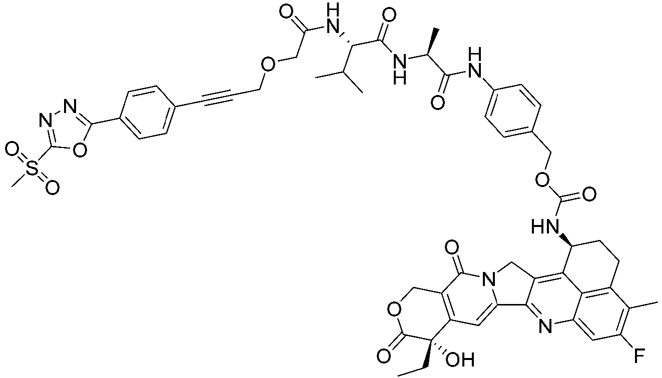

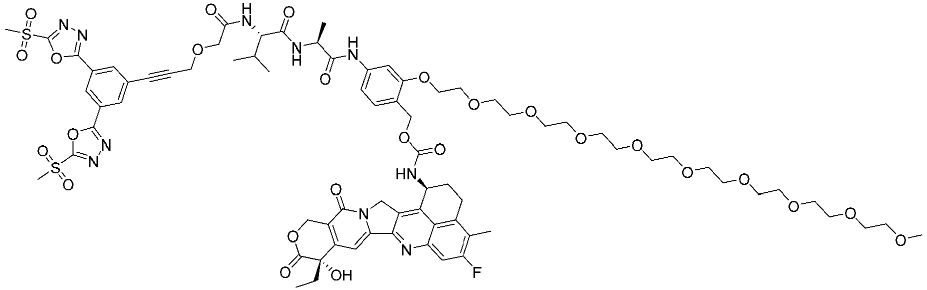

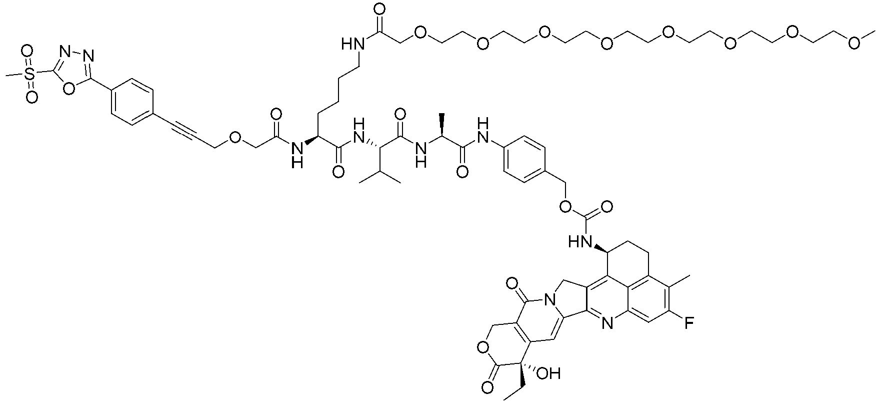

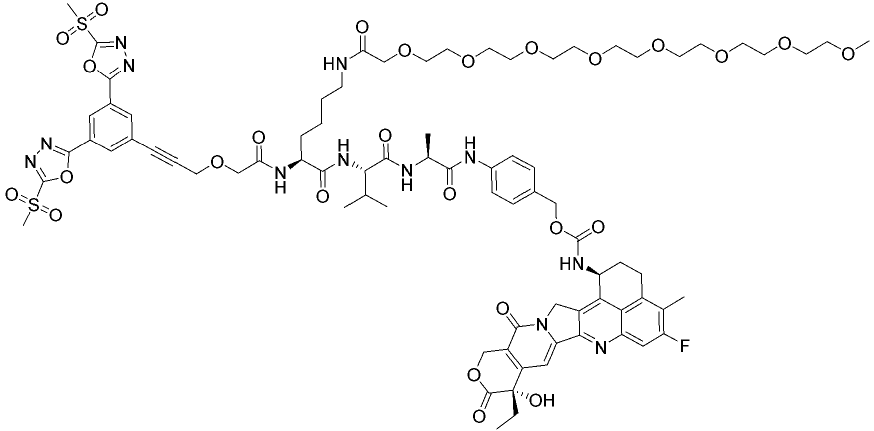

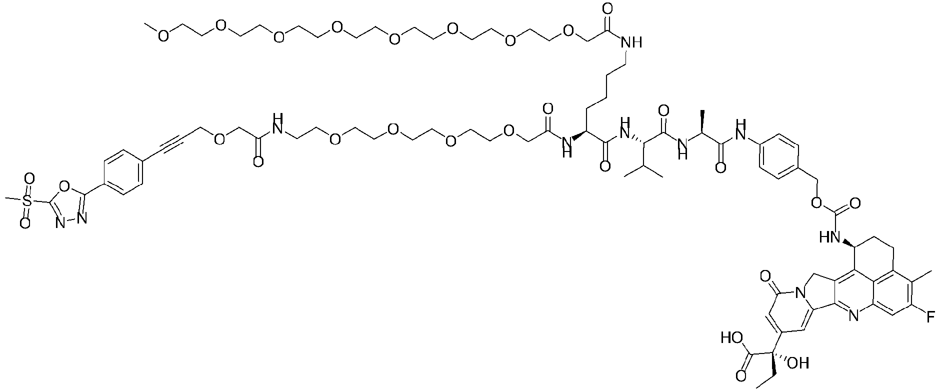

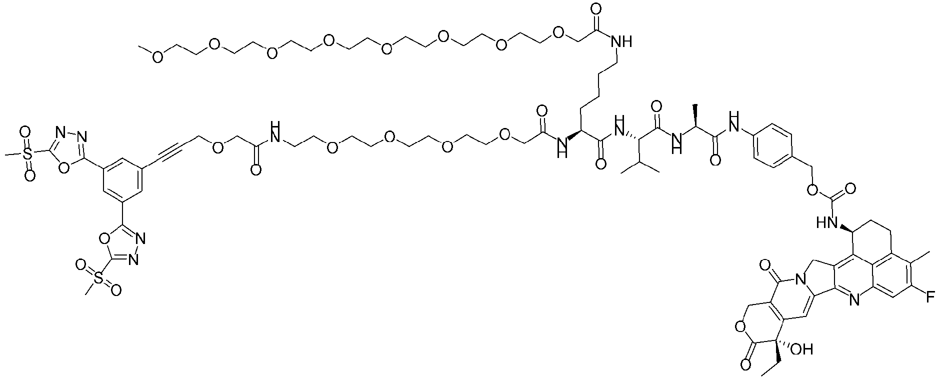

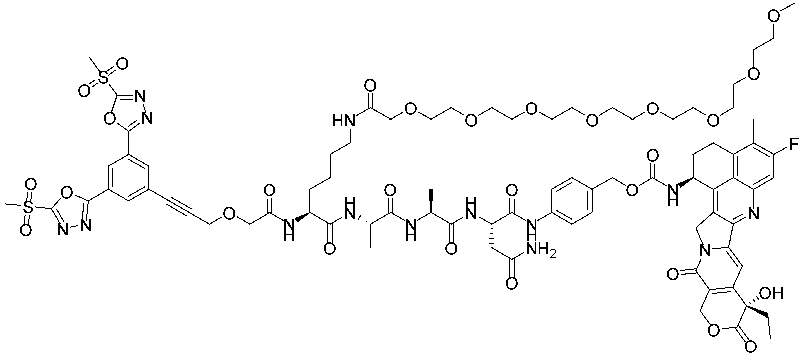

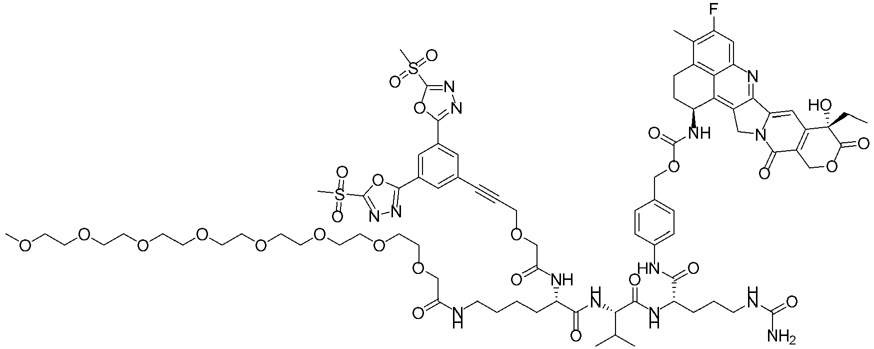

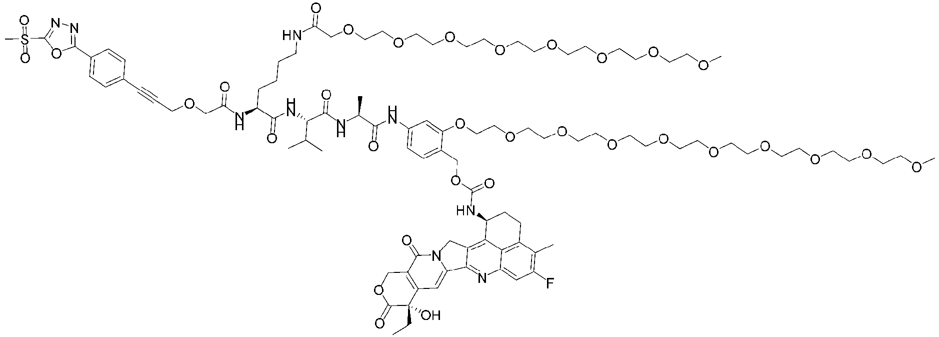

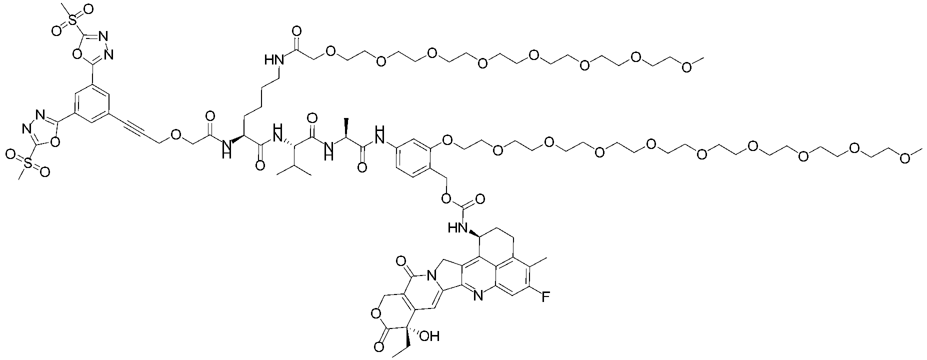

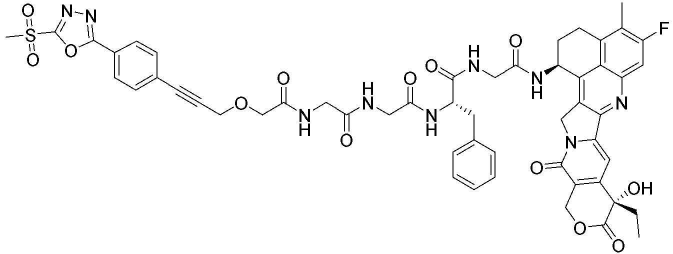

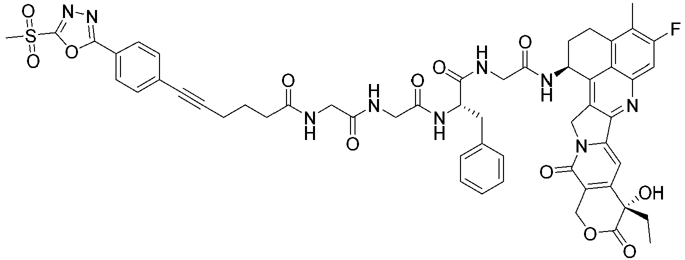

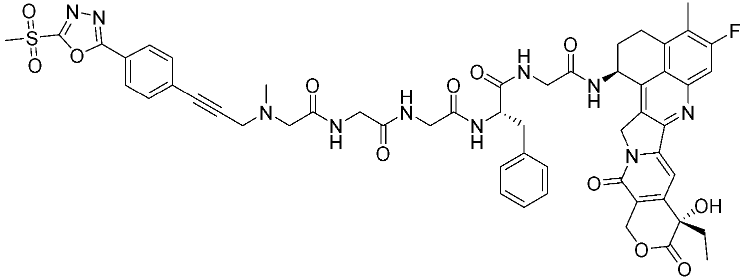

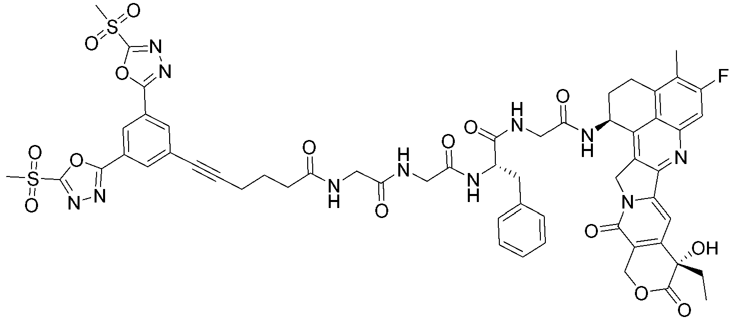

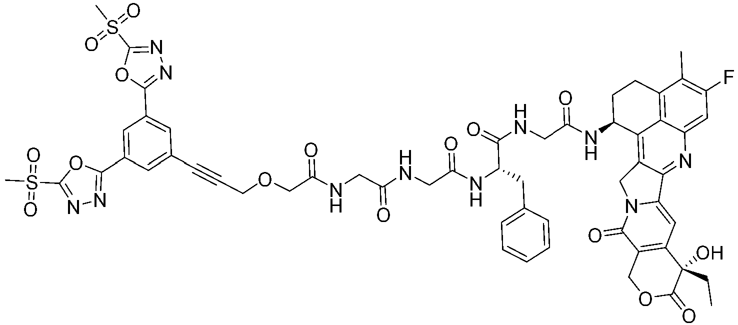

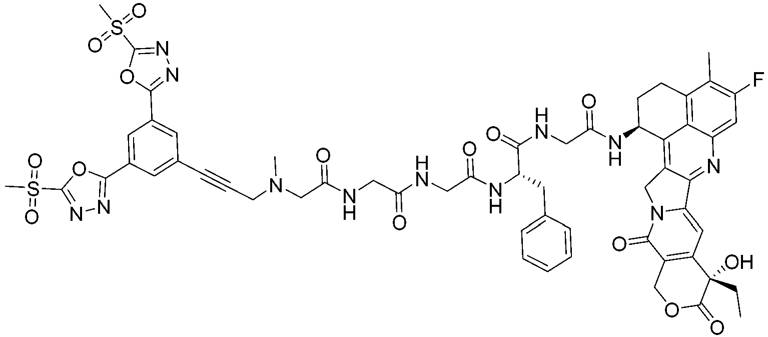

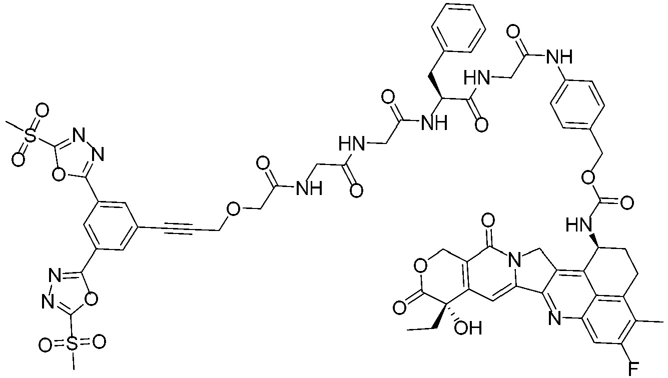

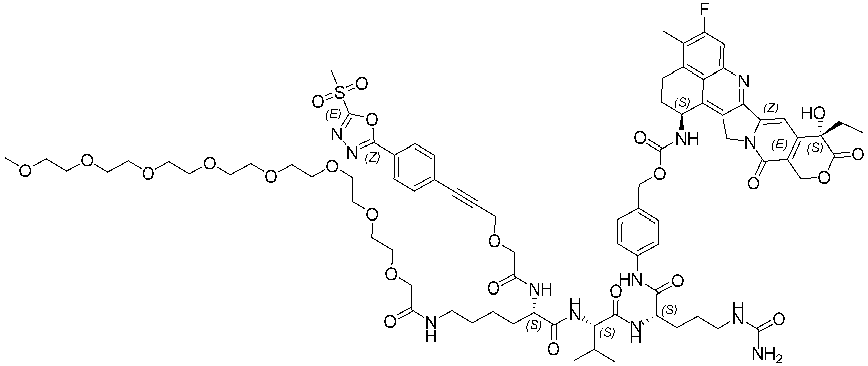

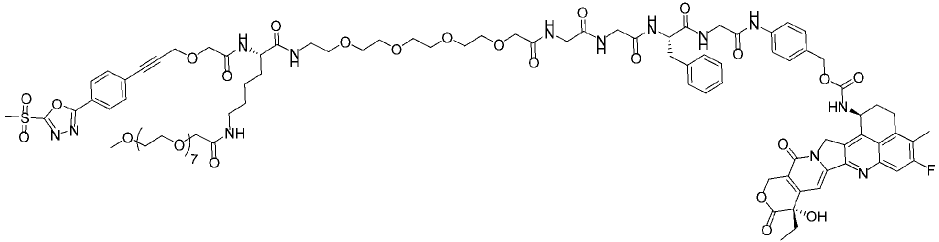

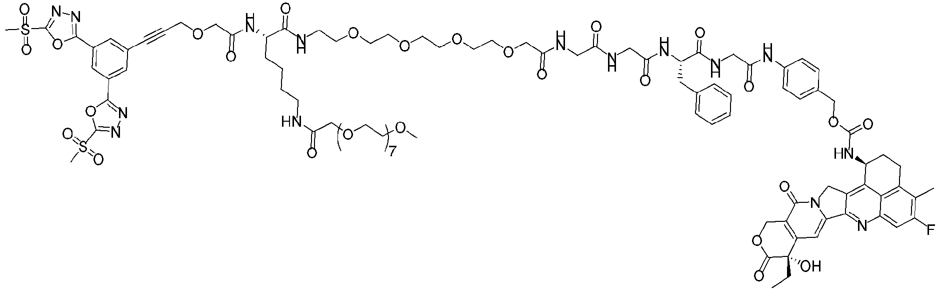

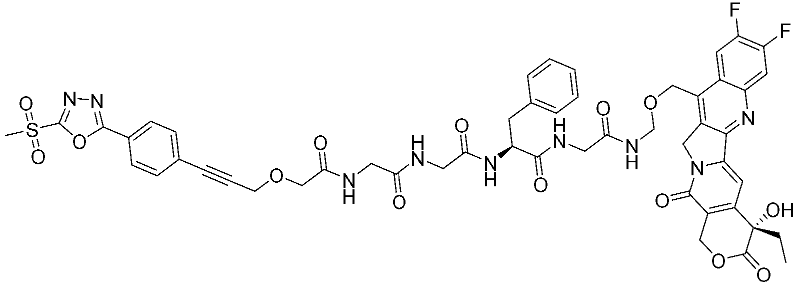

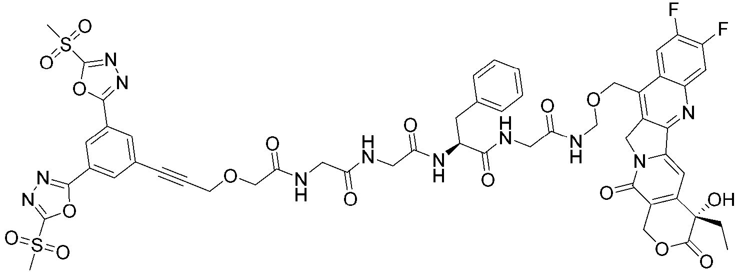

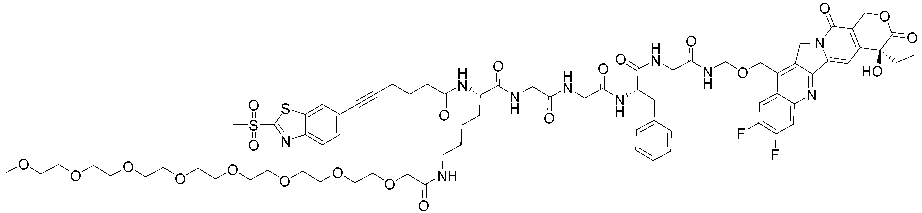

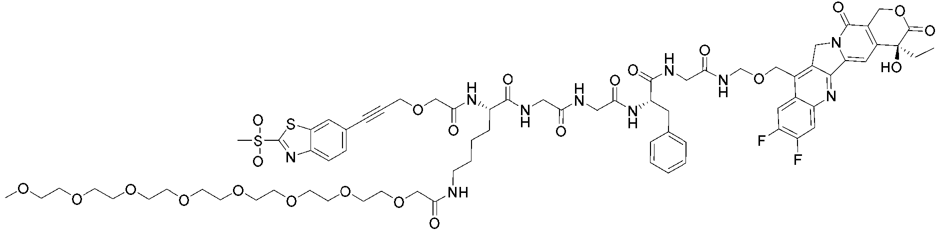

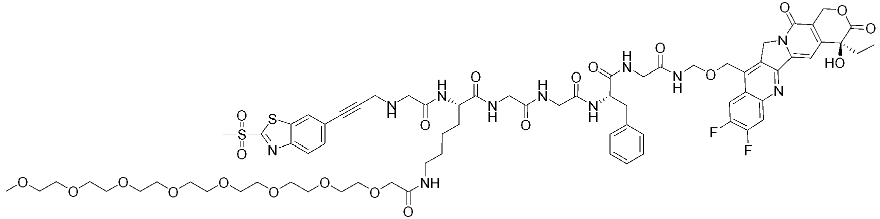

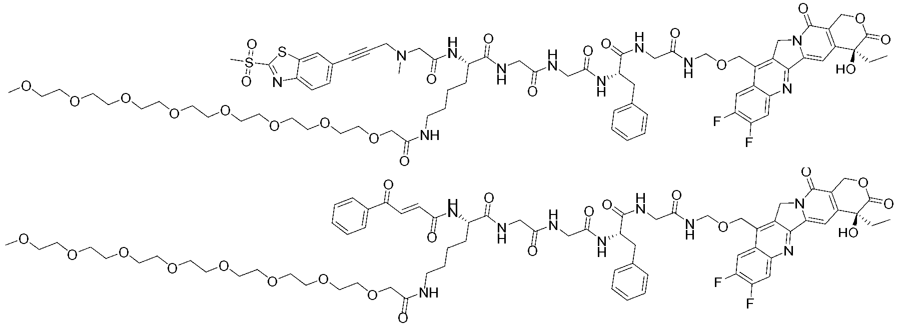

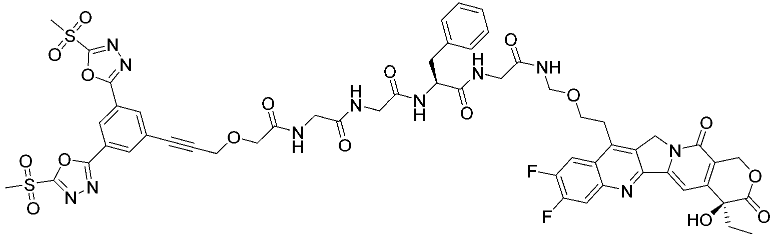

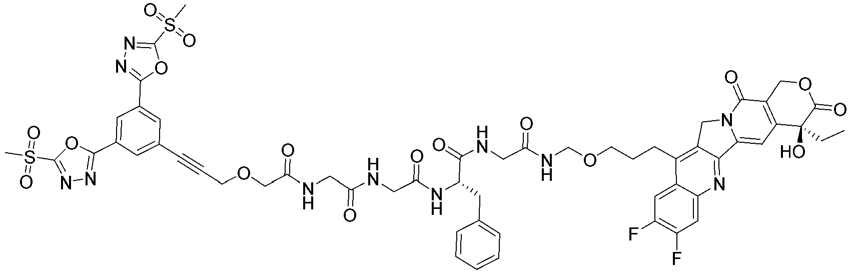

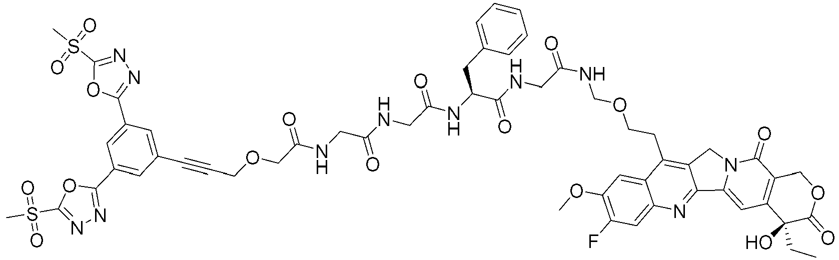

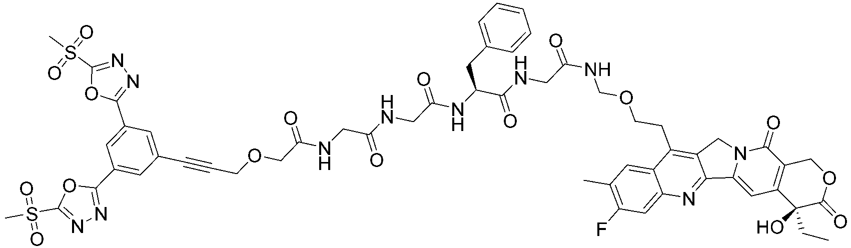

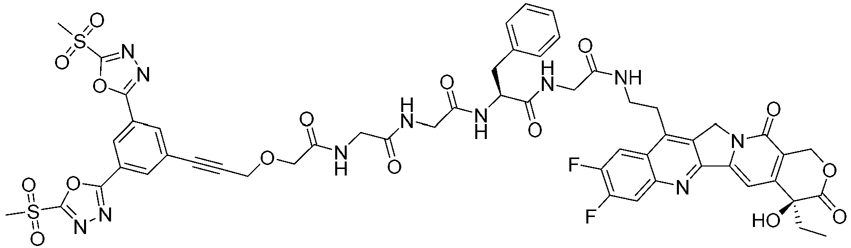

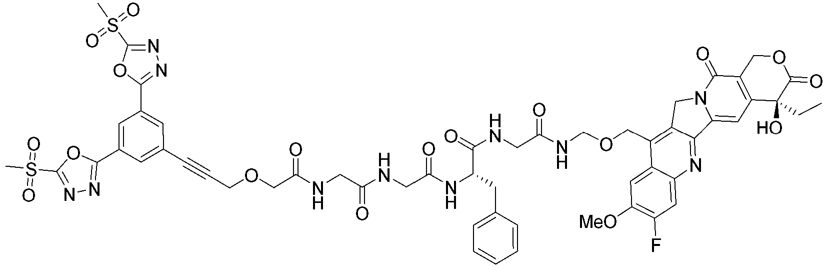

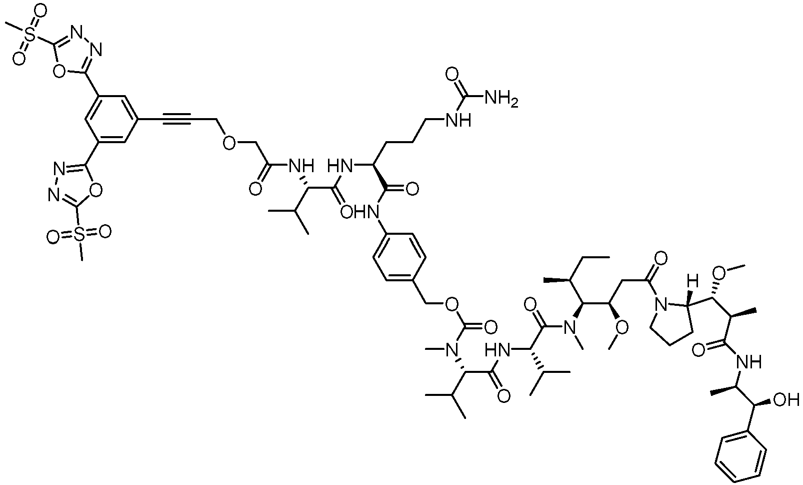

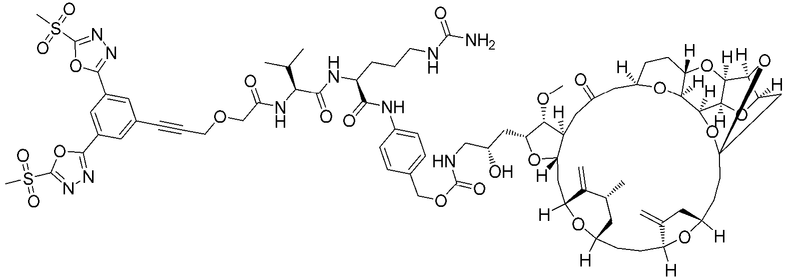

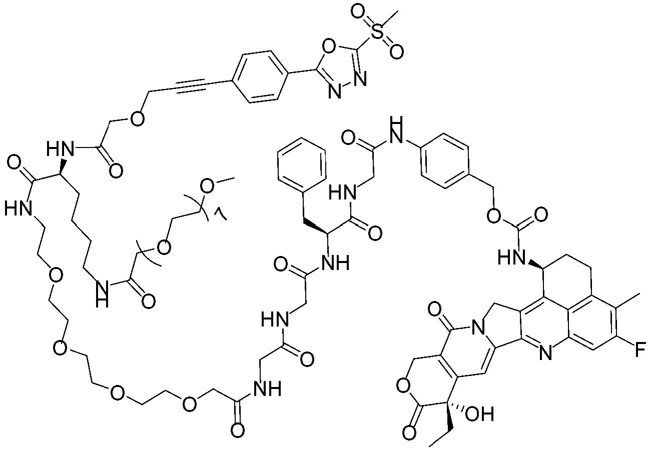

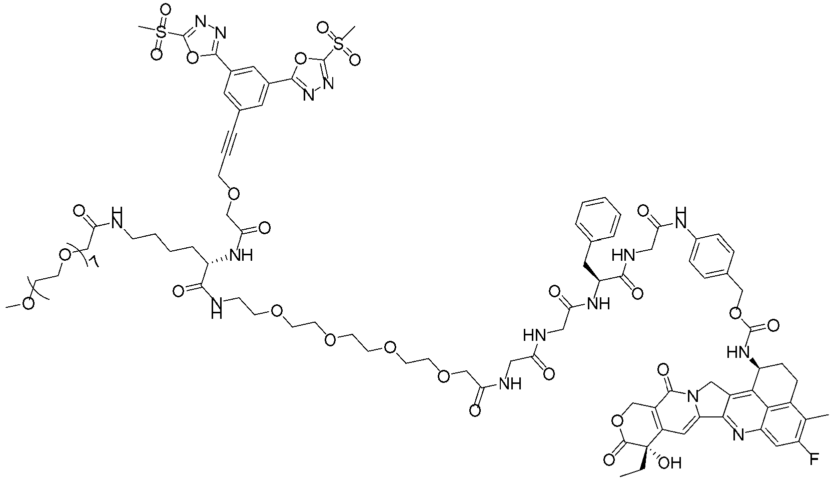

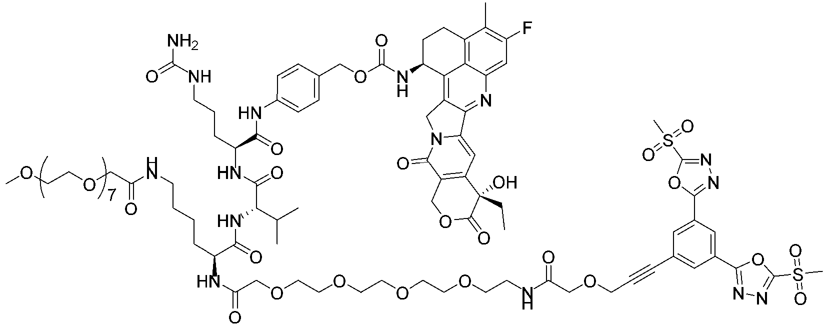

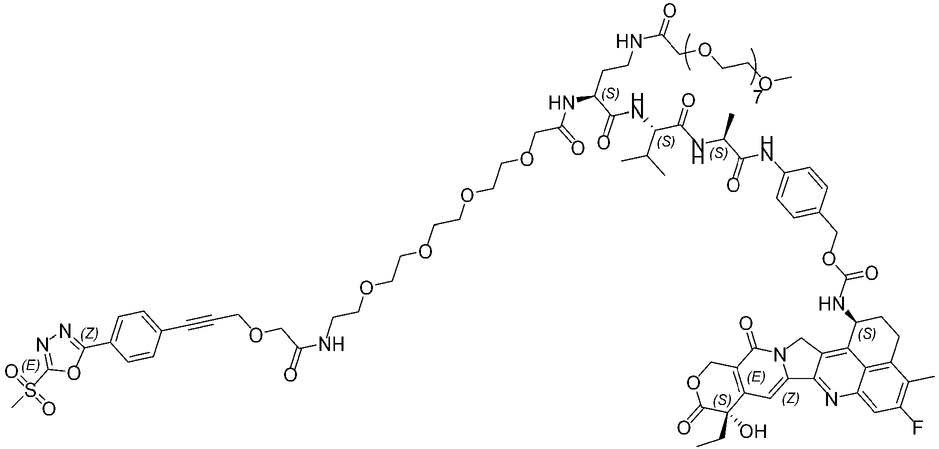

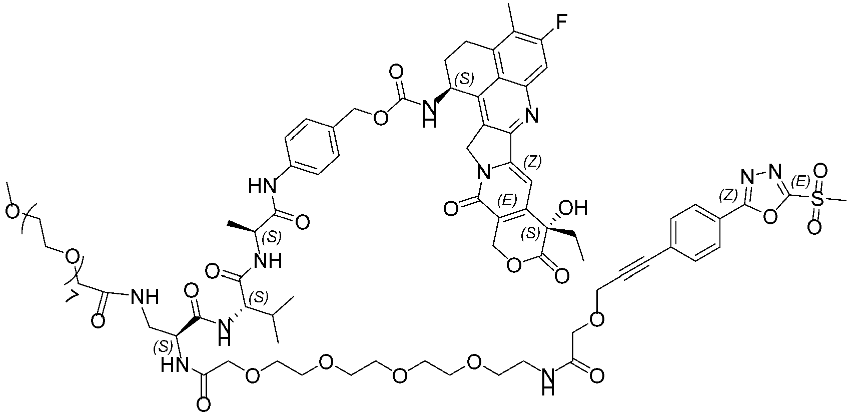

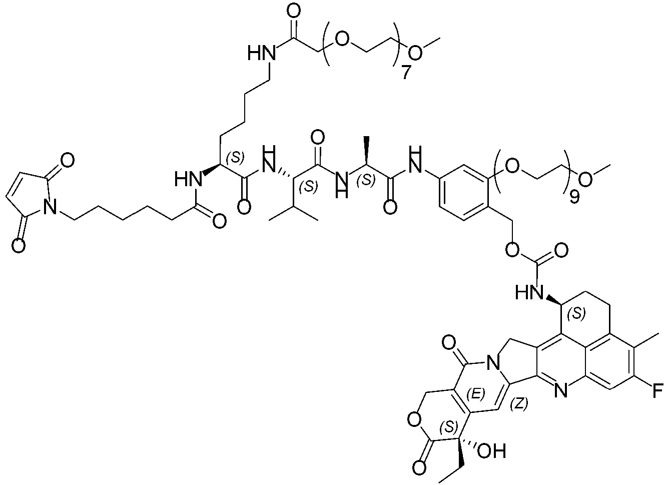

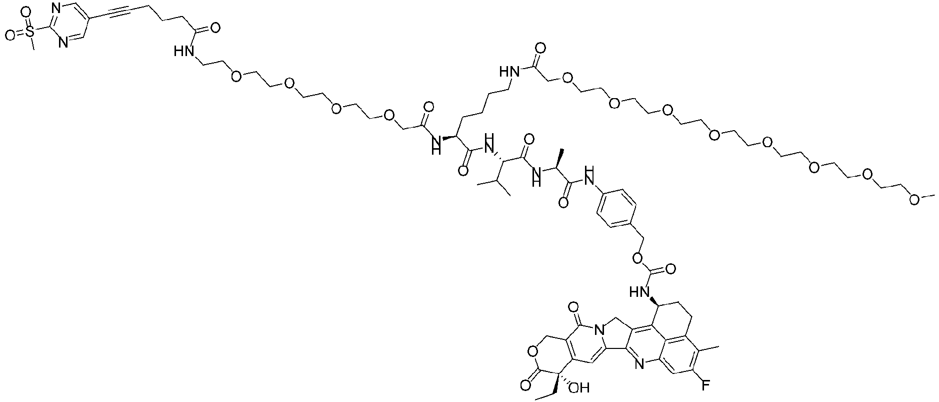

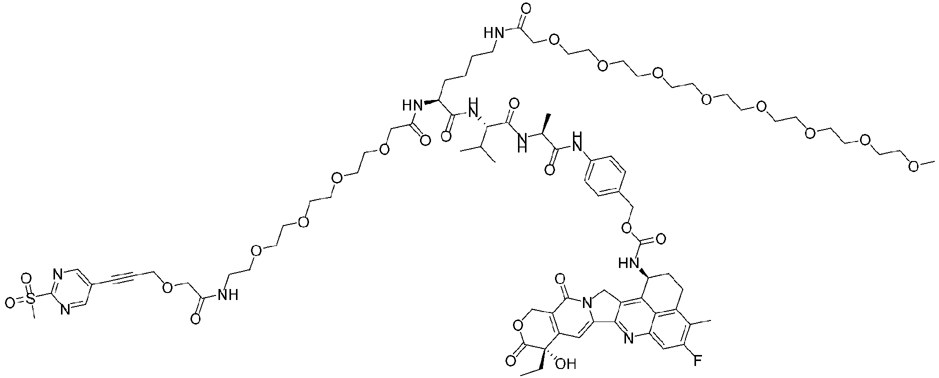

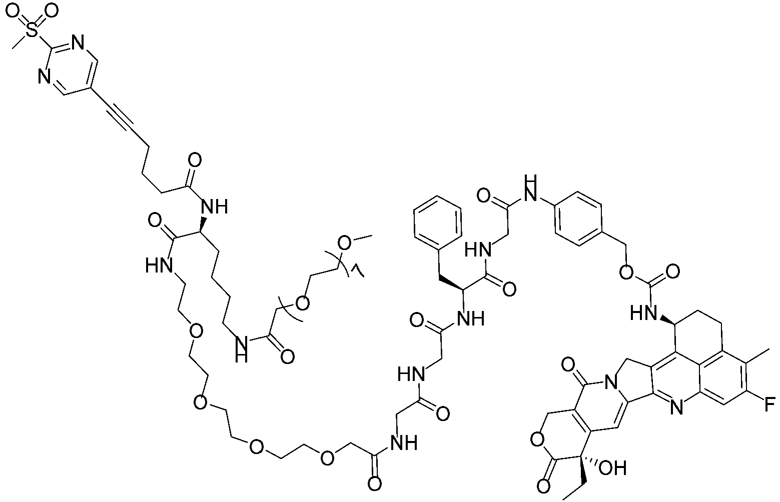

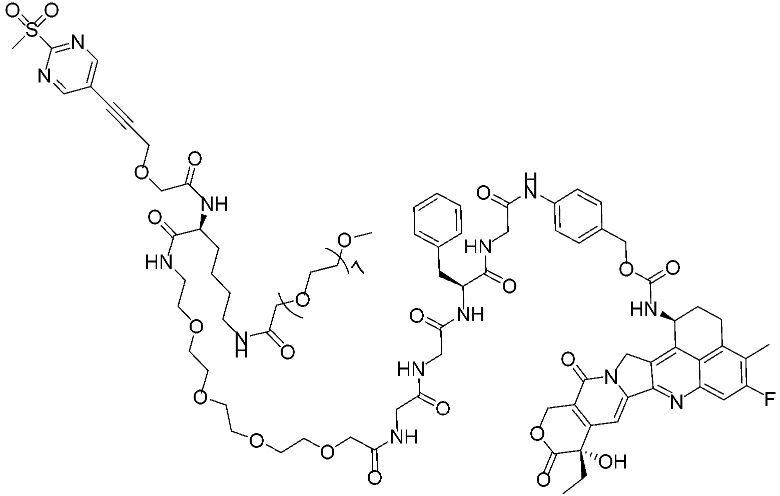

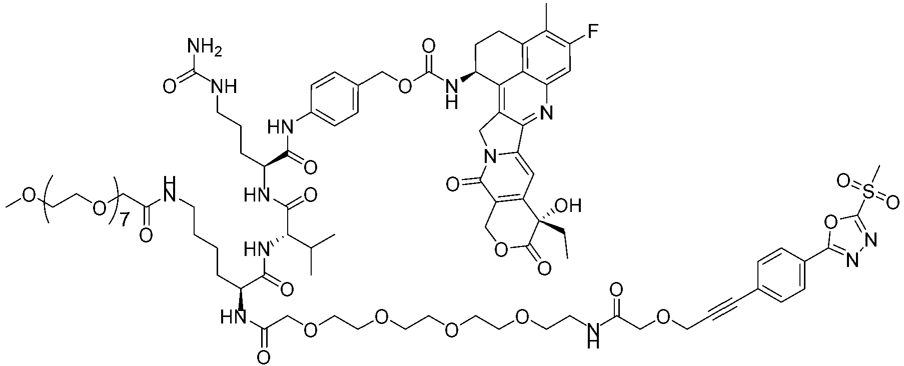

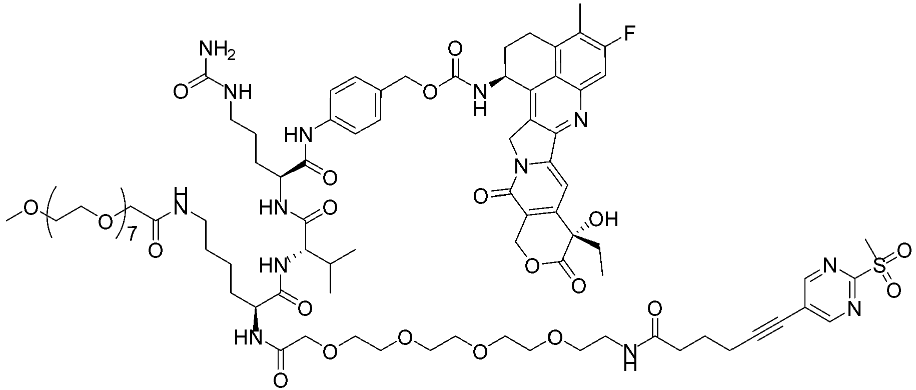

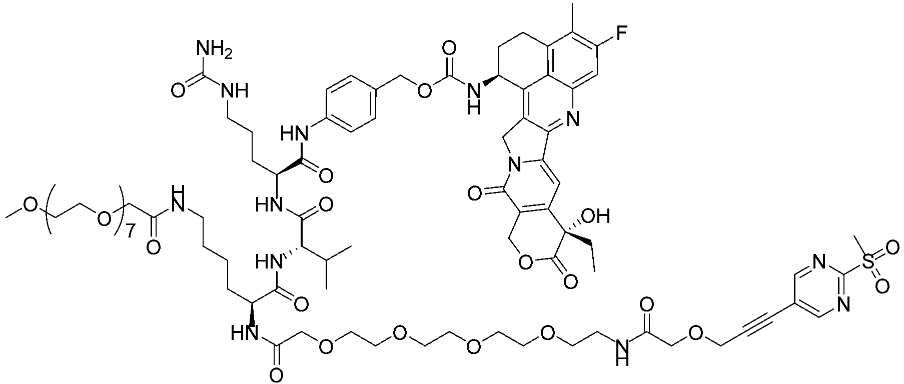

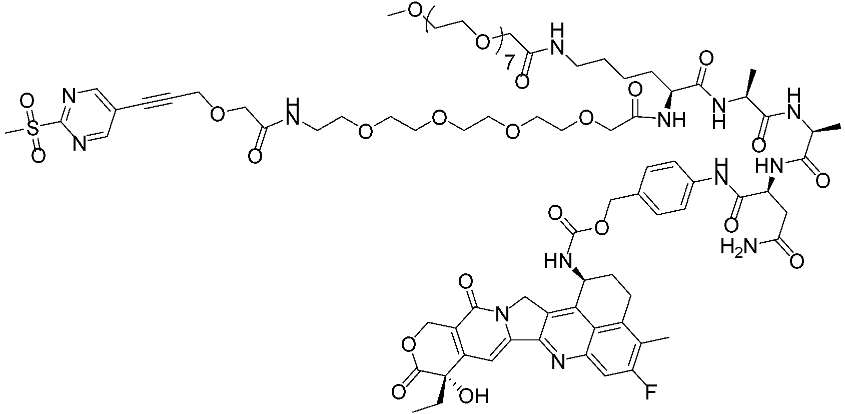

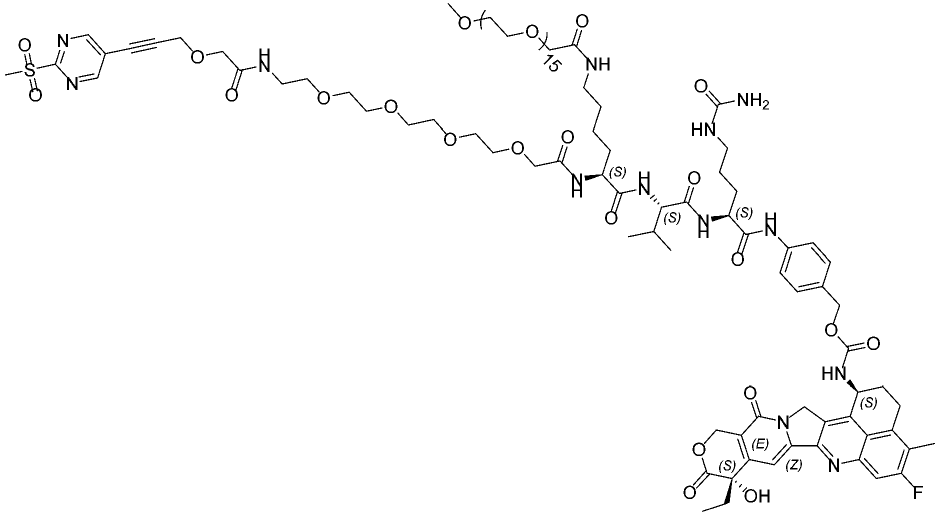

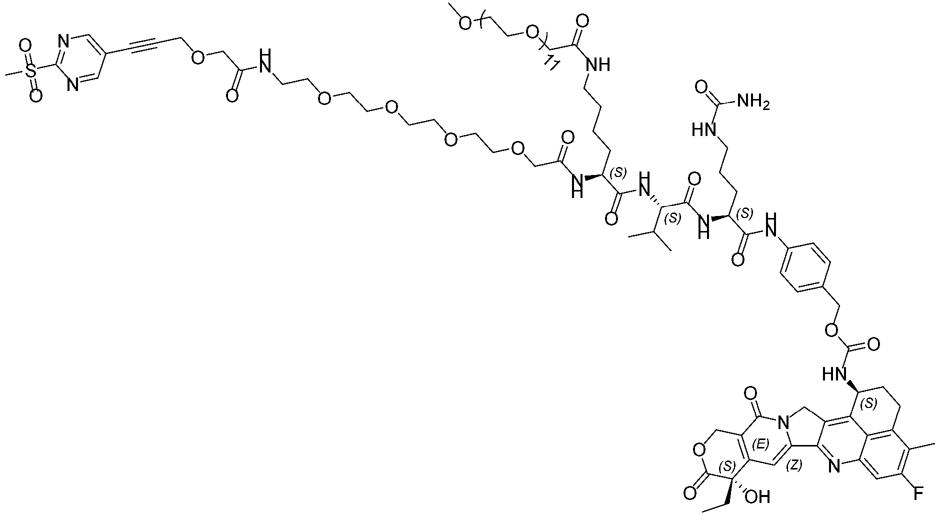

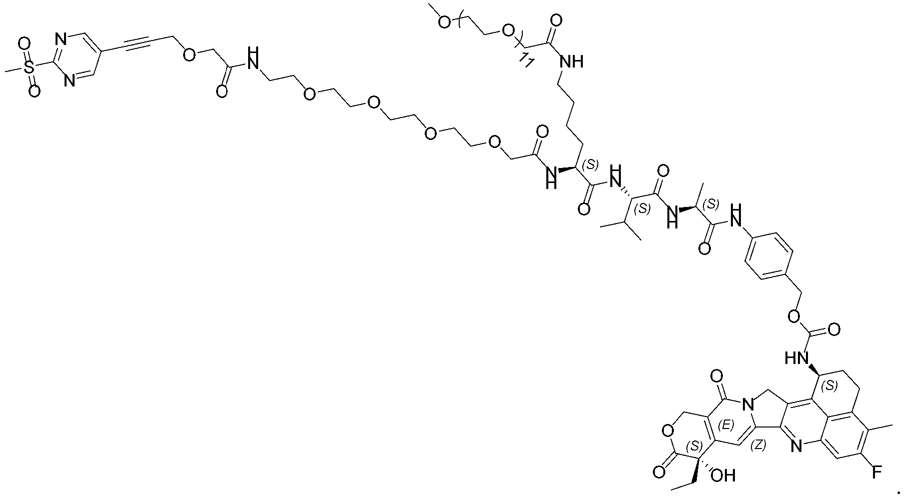

49. A compound as shown in formula IIIa or a pharmaceutically acceptable salt thereof, 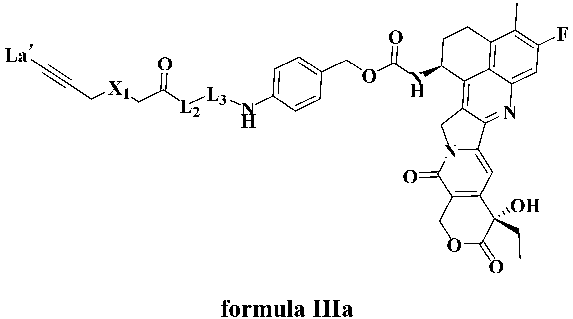 wherein, X₁, L₂ and L₃ have the same meanings as defined in claim 1 or 29, and La' is selected from the following groups: 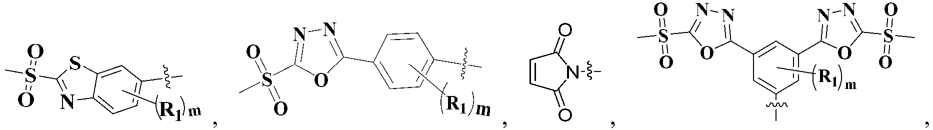 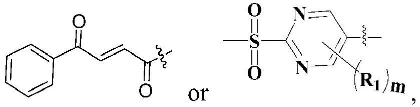 wherein R₁ is respectively independently selected from H, halogen atom, -CN, -OH, -NH₂, C₁₋₄ alkyl, C₁₋₄ haloalkyl, C₁₋₄ alkoxy, C₁₋₄ haloalkoxy, hydroxy-C₁₋₄ alkyl, and m is selected from 0, 1, 2 or 3; and preferably, La' is selected from the following groups

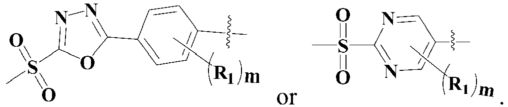

50. A compound as shown in formula IVa or a pharmaceutically acceptable salt thereof, 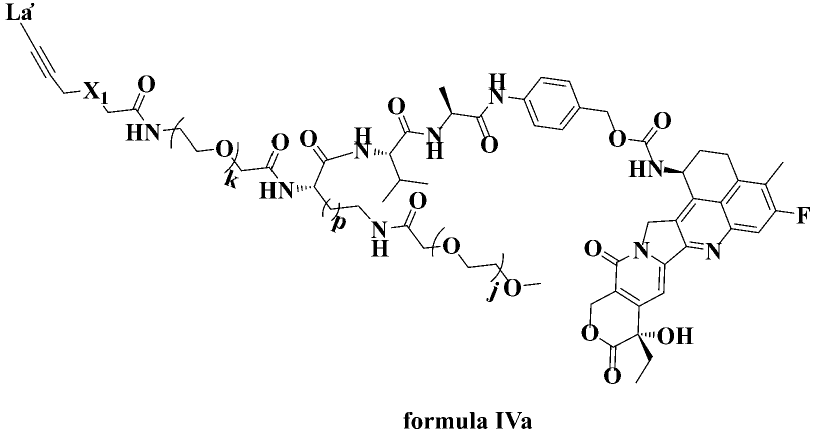 wherein, X₁, k, p and j have the same meanings as defined in any one of claim 1 and claims 38 to 40, and La' has the same meaning as defined in claim 49.

51. Structural fragments as follows:

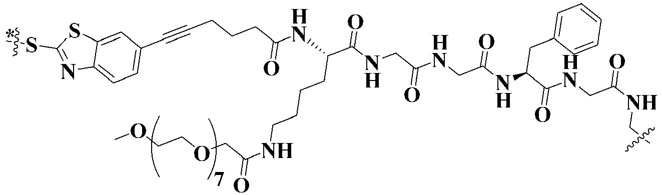

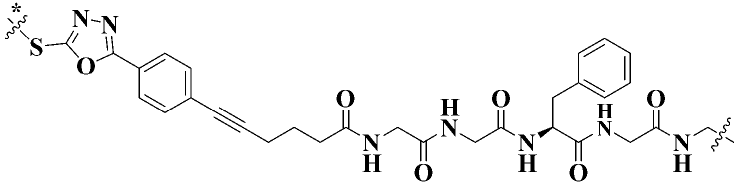

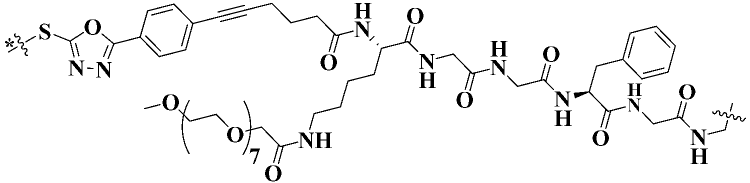

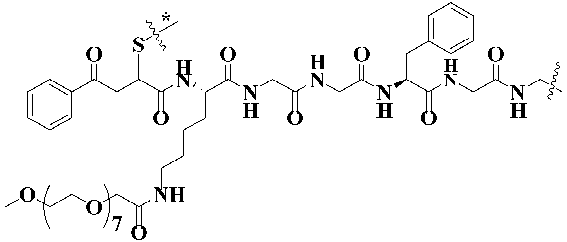

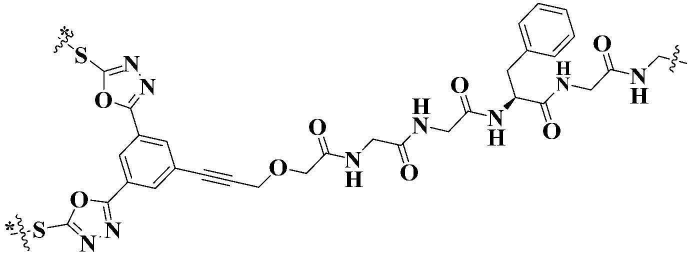

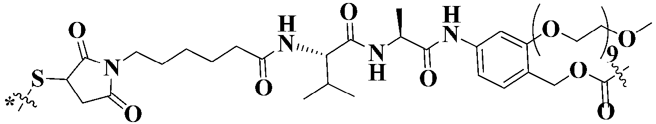

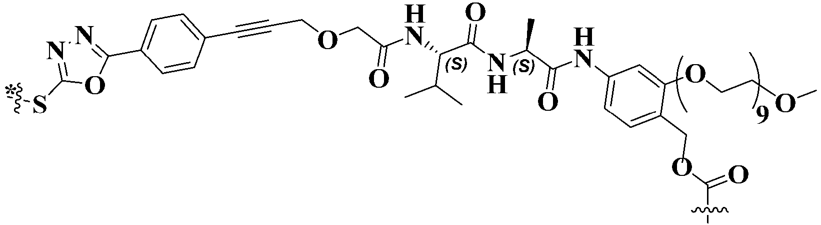

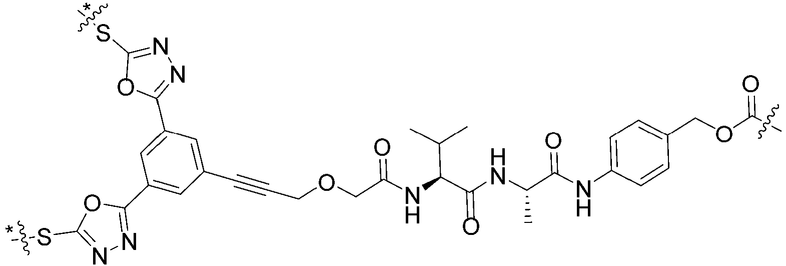

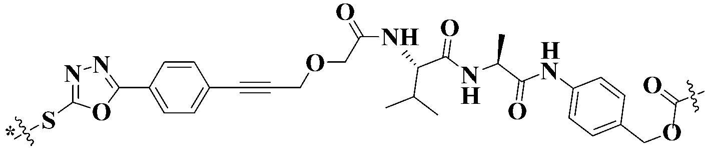

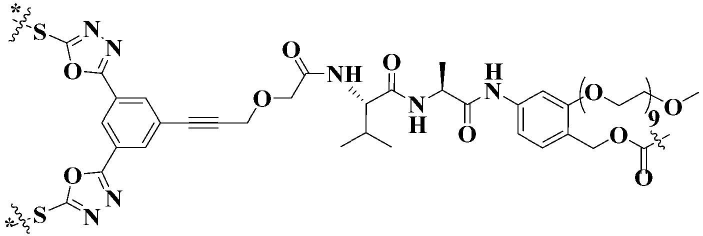

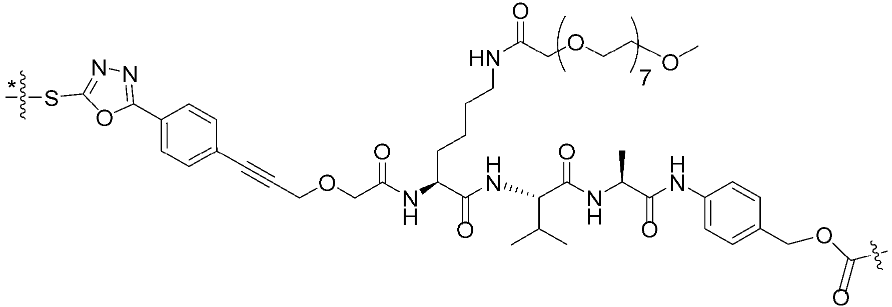

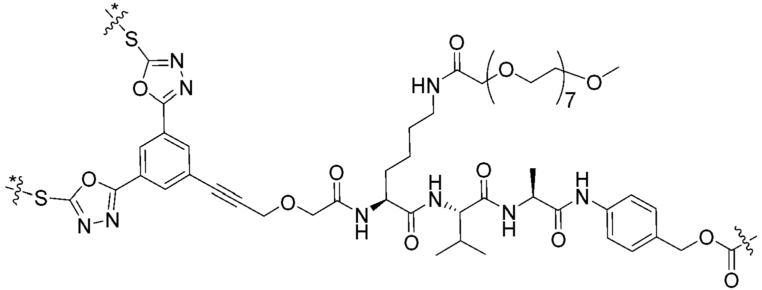

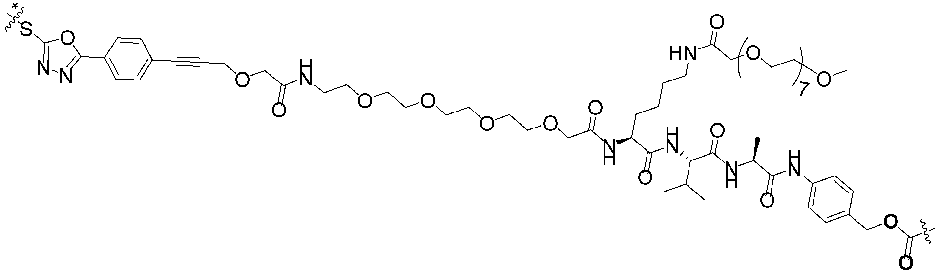

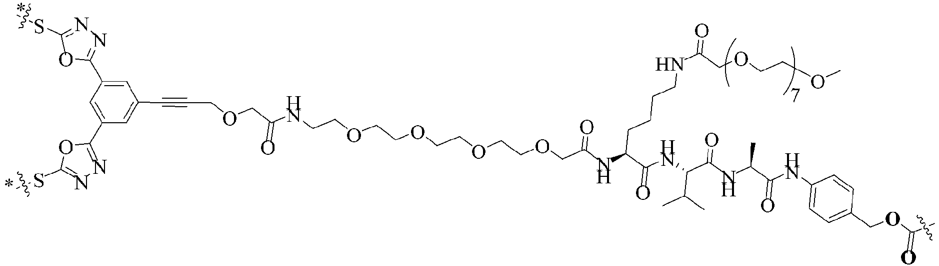

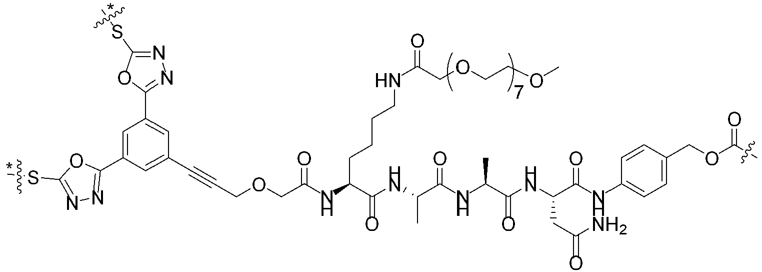

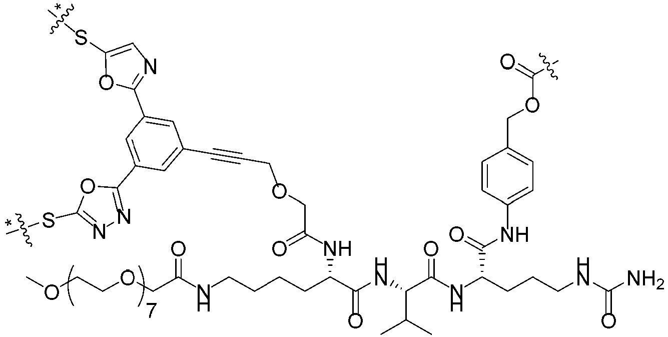

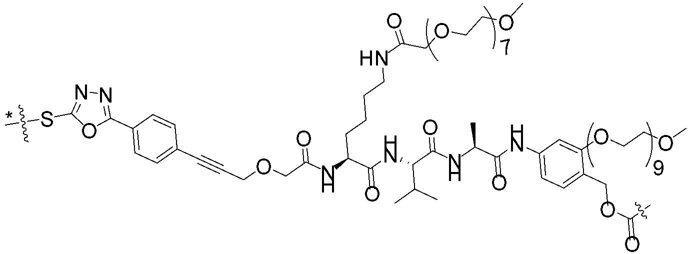

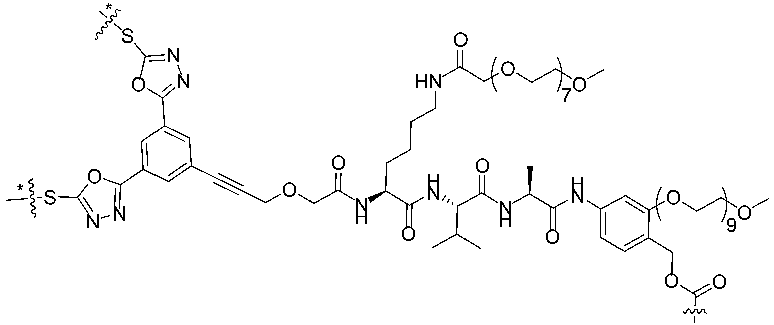

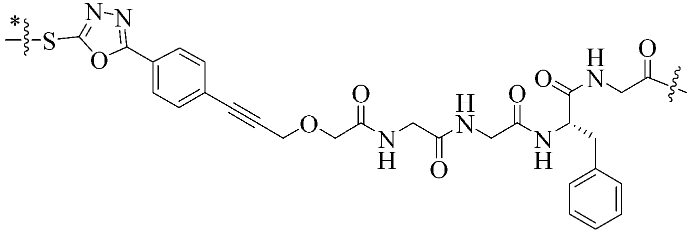

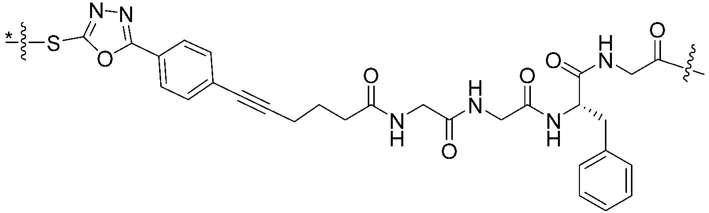

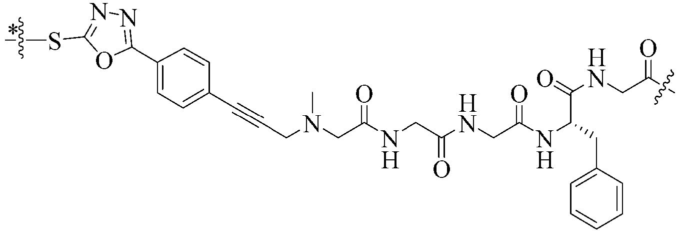

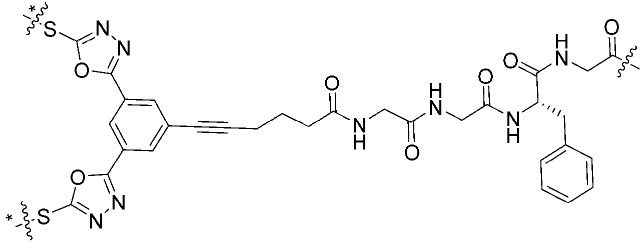

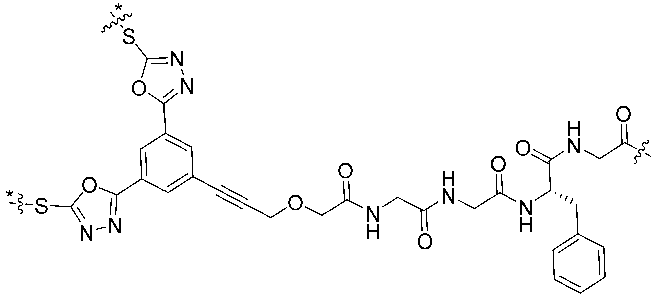

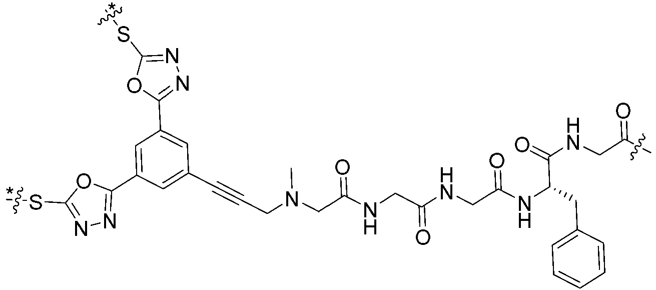

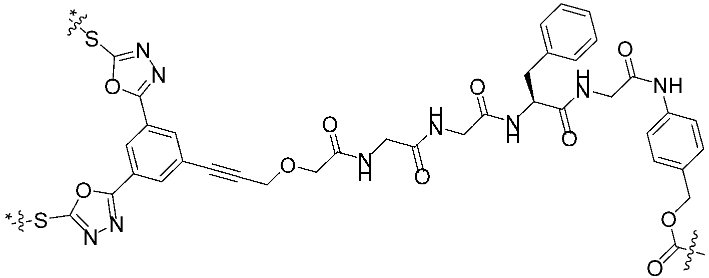

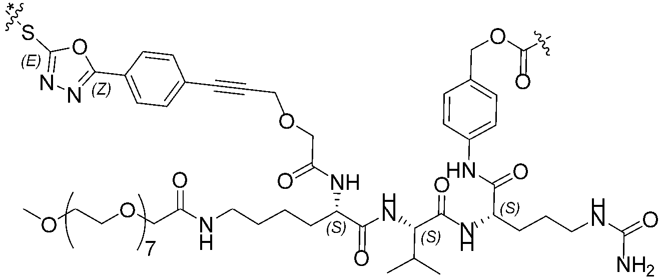

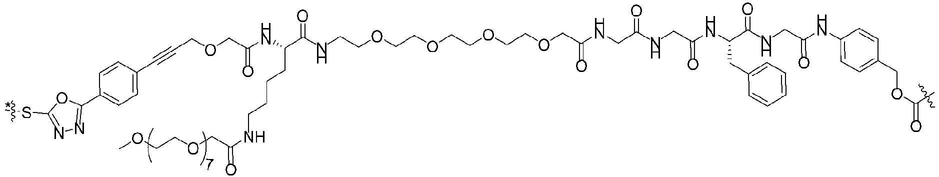

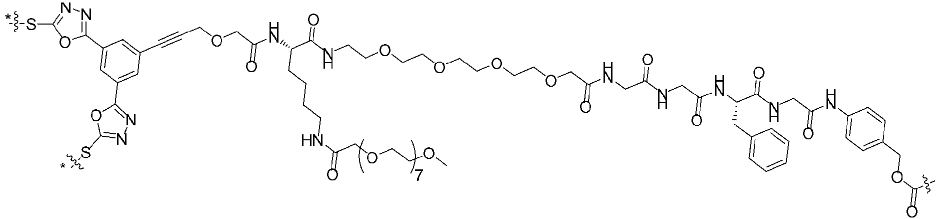

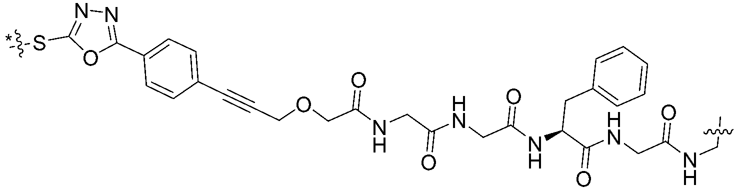

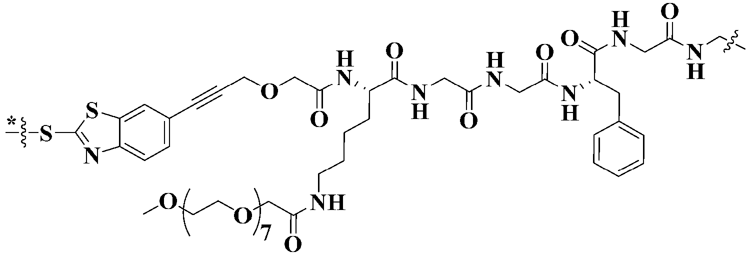

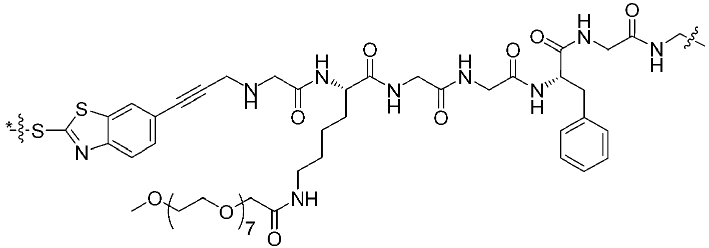

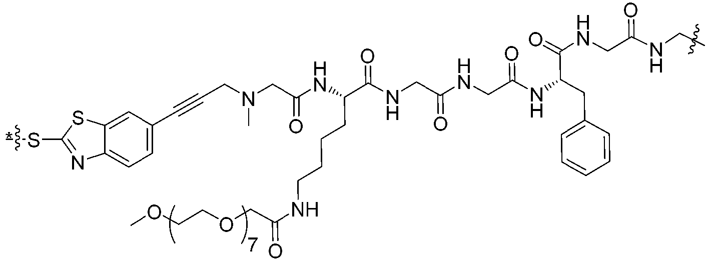

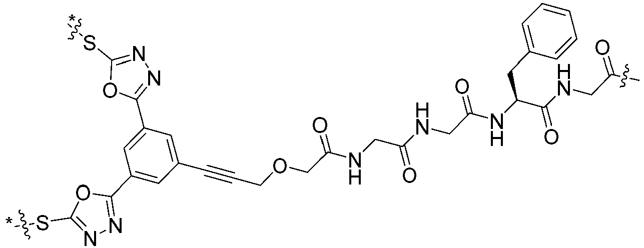

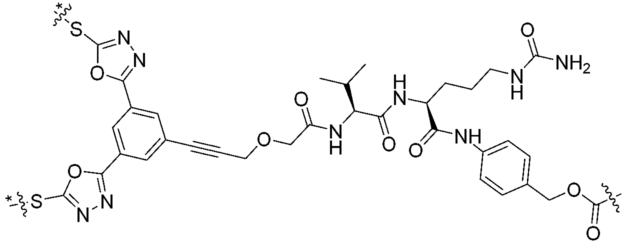

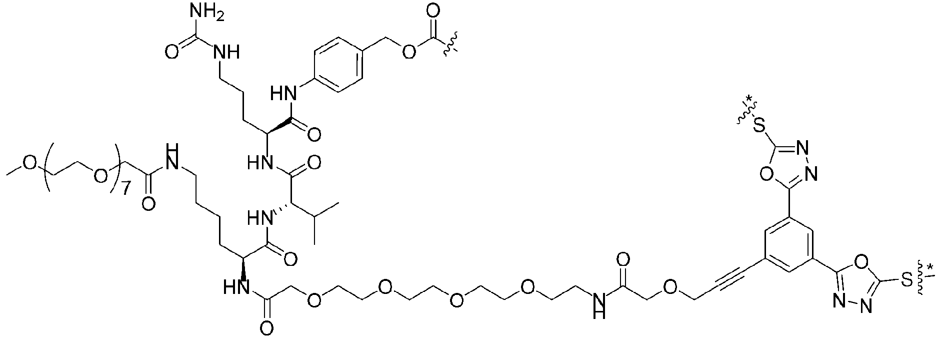

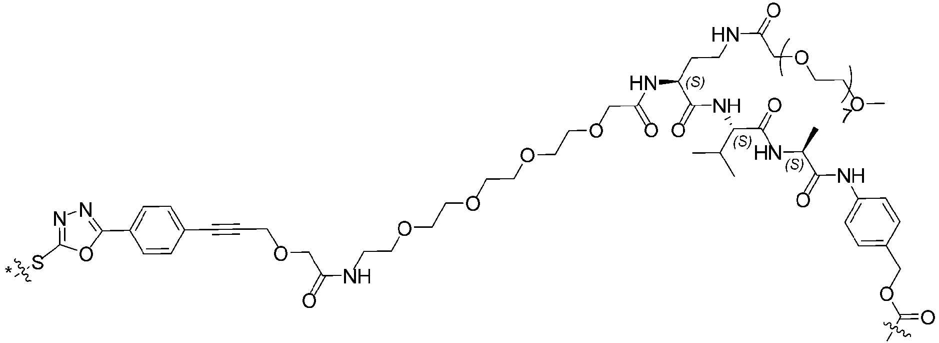

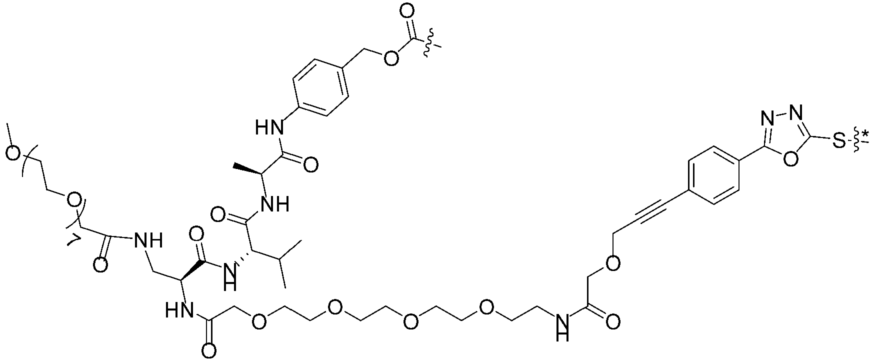

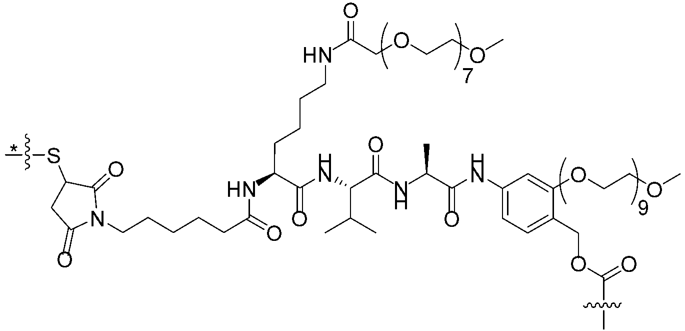

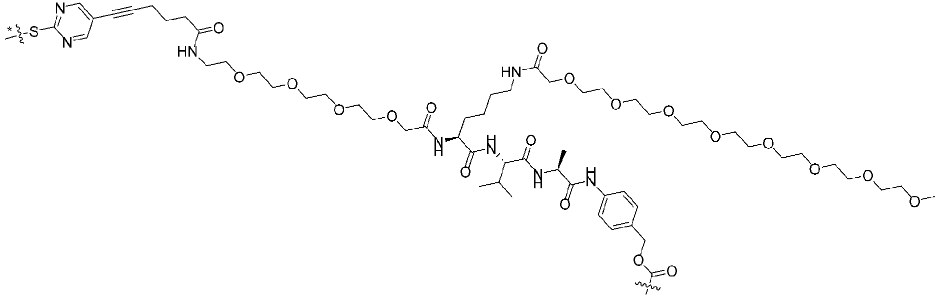

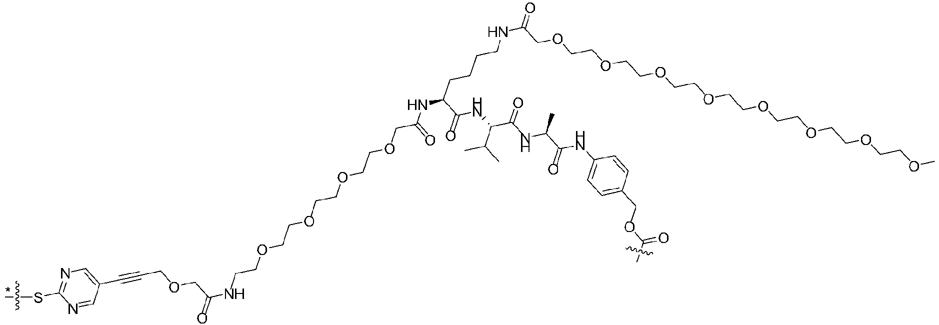

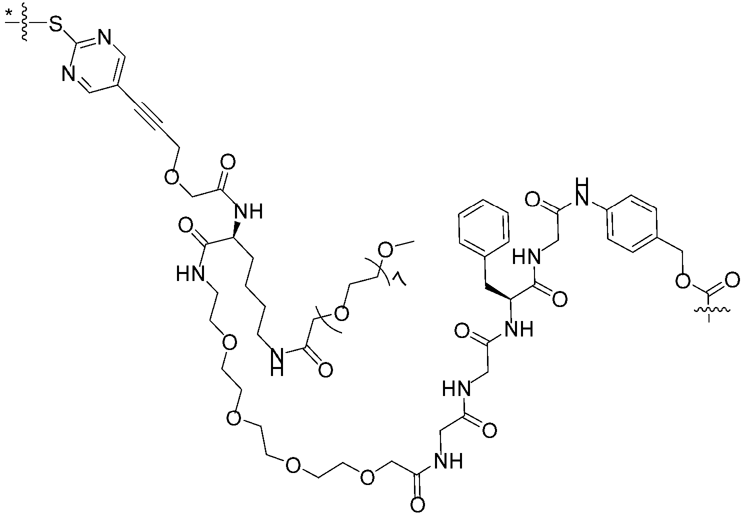

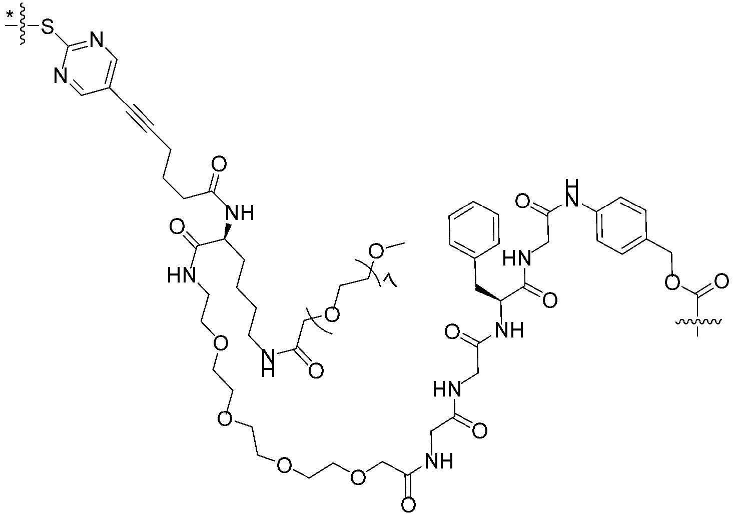

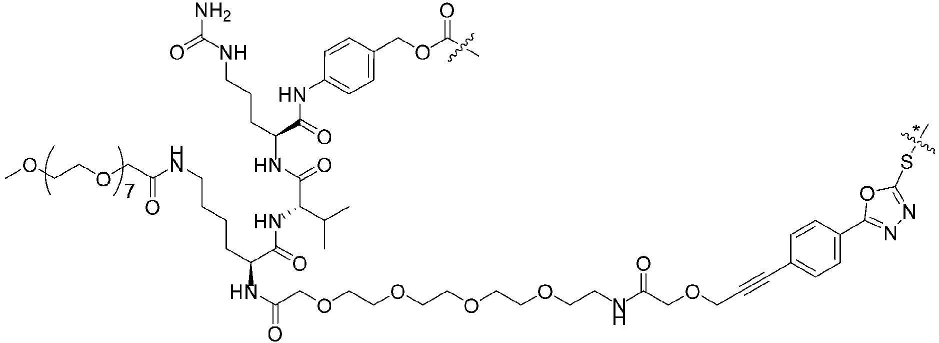

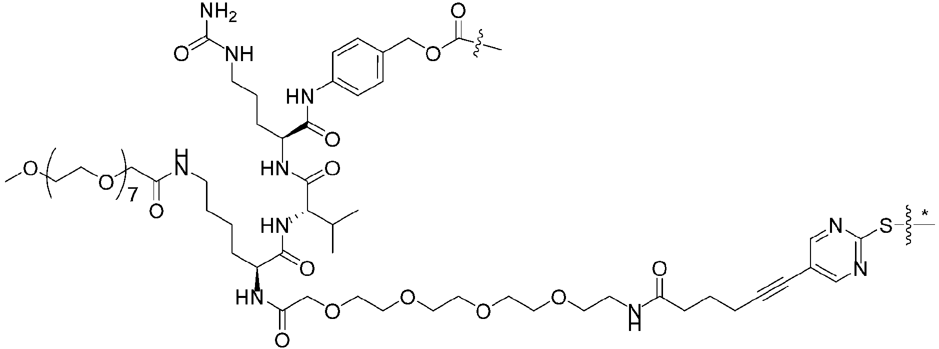

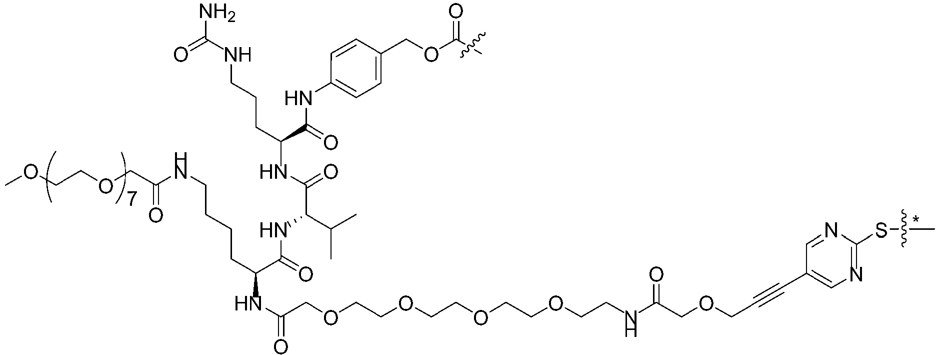

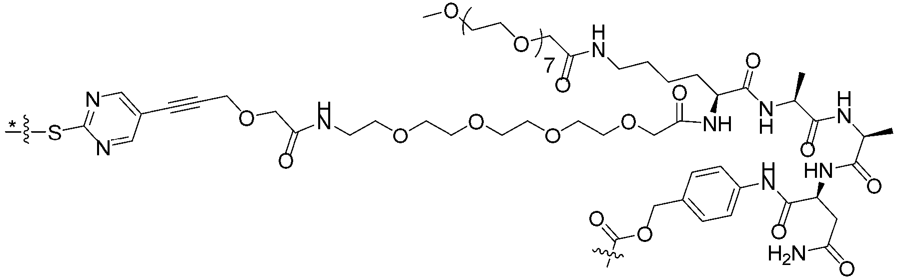

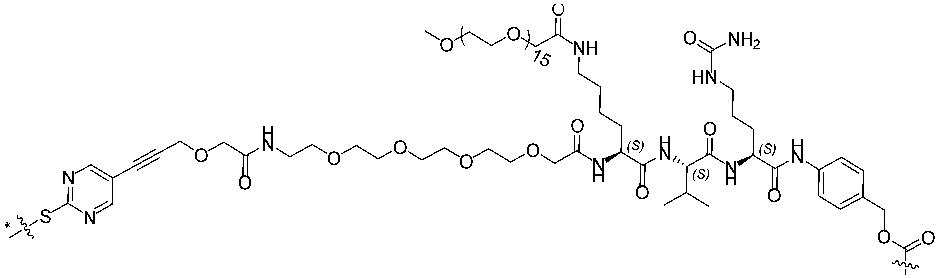

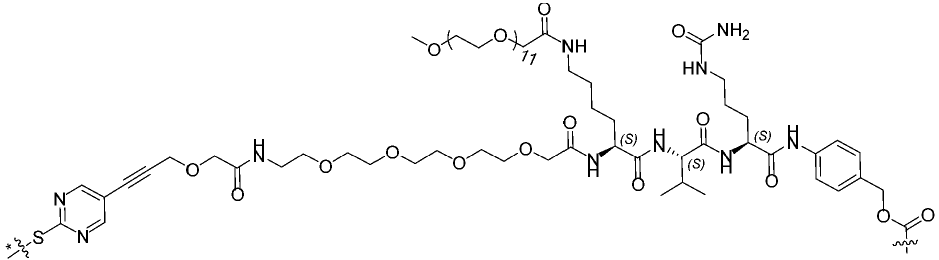

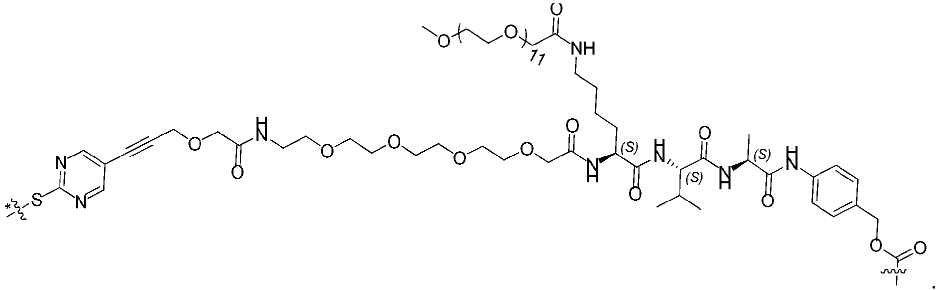

52. A use of the antibody drug conjugate or the pharmaceutically acceptable salt thereof according to any one of claims 1 to 41 in preparation of a drug for treating cancers.

53. A use of the compound or the pharmaceutically acceptable salt thereof according to any one of claims 42 to 47 in preparation of a drug for treating cancers.

54. A use of the compound or the pharmaceutically acceptable salt thereof according to any one of claims 42 to 47 in preparation of the antibody drug conjugate or the pharmaceutically acceptable salt thereof according to any one of claims 1 to 41.

55. A use of the compound or the pharmaceutically acceptable salt thereof according to any one of claims 42 to 47 in preparation of the compounds or the pharmaceutically acceptable salts thereof according to any one of claims 48 to 50.

56. A use of the compounds or the pharmaceutically acceptable salts thereof according to any one of claims 48 to 50 in preparation of the antibody drug conjugate or the pharmaceutically acceptable salt thereof according to any one of claims 1 to 41.

57. A use of the structural fragments according to claim 51 in preparation of the antibody drug conjugate or the pharmaceutically acceptable salt thereof according to any one of claims 1 to 41.

58. A pharmaceutical composition, comprising the antibody drug conjugate or the pharmaceutically acceptable salt thereof according to any one of claims 1 to 41.

59. A use of the pharmaceutical composition according to claim 58 in preparation of a drug for treating cancers.
